(19) Europäisches Patentamt
European Patent Office
Office européen des brevets

(11) Publication number: **0 204 513 B1**

(12) # EUROPEAN PATENT SPECIFICATION

(45) Date of publication of patent specification: **17.07.91**

(51) Int. Cl.$^5$: **A01N 47/36**, C07D 403/12, C07D 401/12, //C07D257/04

(21) Application number: **86304075.4**

(22) Date of filing: **29.05.86**

The file contains technical information submitted after the application was filed and not included in this specification

(54) **Herbicidal sulfonamides.**

(30) Priority: **30.05.85 US 739214**
**11.04.86 US 849618**

(43) Date of publication of application:
**10.12.86 Bulletin 86/50**

(45) Publication of the grant of the patent:
**17.07.91 Bulletin 91/29**

(84) Designated Contracting States:
**AT BE CH DE FR GB IT LI LU NL SE**

(56) References cited:
**EP-A- 0 083 975**
**EP-A- 0 096 593**
**EP-A- 0 111 442**

(73) Proprietor: **E.I. DU PONT DE NEMOURS AND COMPANY**
**1007 Market Street**
**Wilmington Delaware 19898(US)**

(72) Inventor: **Levitt, George**
**3218 Romilly Road**
**Wilmington Delaware 19810(US)**

(74) Representative: **Hildyard, Edward Martin et al**
**Frank B. Dehn & Co. Imperial House 15-19 Kingsway**
**London WC2B 6UZ(GB)**

**EP 0 204 513 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to tetrazole-substituted benzene sulfonamides which are useful as agricultural chemicals such as growth regulants and herbicides.

European Patent Application (EP-A) No. 83,975 (published July 20, 1983) discloses herbicidal benzenesulfonamides of formula

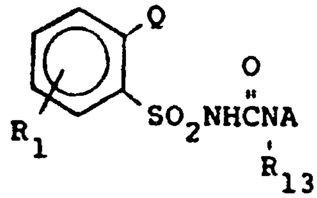

wherein

Q

is selected from various five or six-membered aromatic or partially unsaturated heterocyclic rings containing 2 or 3 heteroatoms selected from O, S or NR.

European Patent Application (EP-A) No. 85,476 (published August 10. 1983) discloses herbicidal benzenesulfonamides of formulae

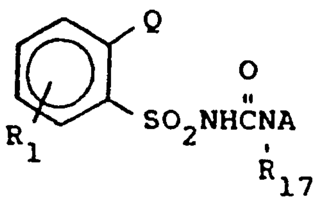

and

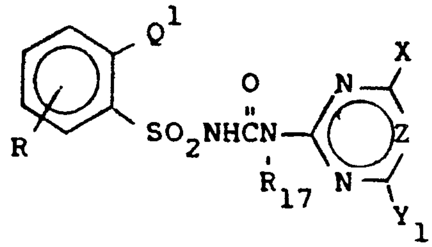

wherein

Q

is selected from varicus 5-membered aromatic heterocycles, and their dihydro and tetrahydro analogs, which contain one heteroatom selected from O, S or NR, or Q is a saturated or partially unsaturated 6-membered ring containing one heteroatom selected from O or S; and

$Q^1$

is a 6-membered aromatic heterocycle containing one to three N atoms.

South African Patent Application 83/8416 (published May 12, 1984) discloses herbicidal benzenesulfonamides of formula

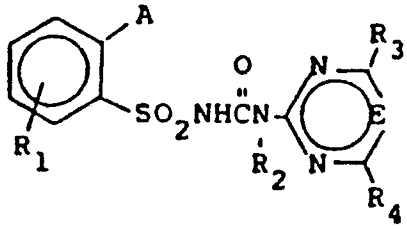

wherein

A

is an unsaturated or only partially saturated 5- or 6-membered heterocyclic ring system which is bonded through a carbon atom and contains 1, 2 or 3 heteroatoms.

European Patent Application 116,518 (Swiss priority 2/4/83, published 8/22/84) discloses herbicidal

sulfonamides of formula

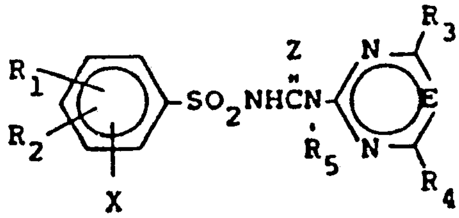

wherein
X is $NR_6R_7$, $N(SO_2R_9)_2$ or

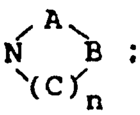

A is CO, $SO_2$, $CONR_{23}$ or $CO_2$;
B is $C_1$-$C_4$ alkyl or $C_2$-$C_4$ alkenyl; and
C is CO, $CR_{21}R_{22}$ or $SO_2$.

U.S. 4,475,944 discloses herbicidal sulfamates, possessing an ortho-heterocyclic ring, such as those of formula

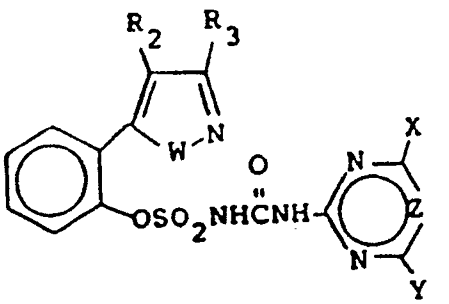

wherein
W is O, S or $NR_1$.

The presence of undesired vegetation causes substantial damage to useful crops, especially agricultural products that satisfy man's basic food needs, such as corn, soybeans, wheat and the like. The current population explosion and concomitant world food shortage demand improvements in the efficiency of producing these crops. Prevention or minimization of the loss of a portion of valuable crops by killing or inhibiting the growth of undesired vegetation is one way of improving this efficiency.

A wide variety of materials, useful for killing, or inhibiting (controlling) the growth of undesired vegetation is available. Such materials are commonly referred to as herbicides. The need exists, however, for still more effective herbicides that destroy or retard weeds without causing signficant damage to useful crops.

## Summary of the Invention

This invention relates to novel compounds of Formula I, agriculturally suitable compositions containing them, and their method-of-use as preemergent or postemergent herbicides or as plant growth regulants.

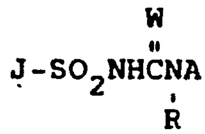

I

wherein

J is 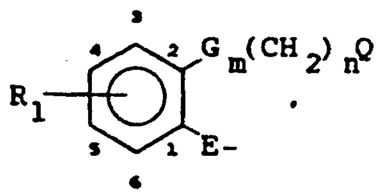 ,

J-1

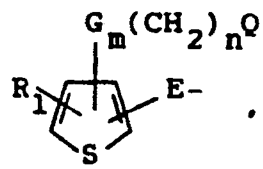 ,

J-2

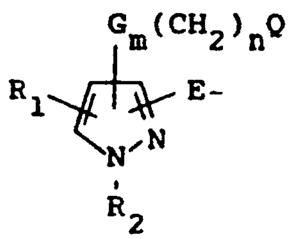 ,

J-3

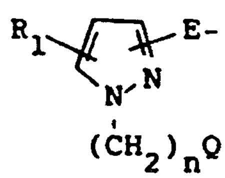 ,

J-4

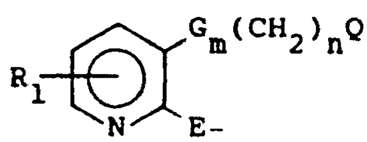 ,

J-5

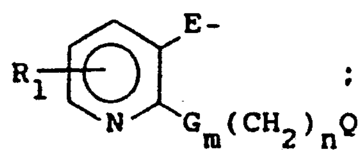 ;

J-6

W is O or S;
G is O, S, SO or $SO_2$;
m is 0 or 1;
n is 0, 1 or 2;
R is H or $CH_3$;
E is a single bond, $CH_2$ or O;

Q is 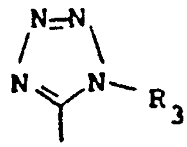 Q-1 , 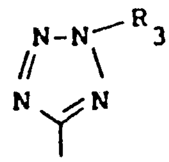 Q-2 , 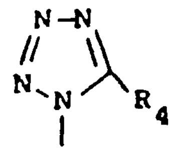 Q-3 , 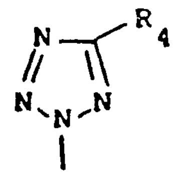 Q-4 ,

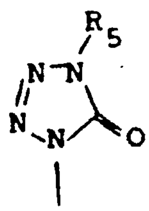 Q-5 , 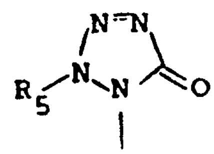 Q-6 , 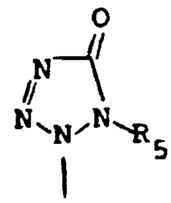 Q-7 ;

$R_1$
is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, CN, $CO_2R_c$, $C_1$-$C_3$ haloalkoxy, $C_1$-$C_3$ haloalkylthio, amino, $C_1$-$C_3$ alkylamino, di($C_1$-$C_3$ alkyl)amino or $C_1$-$C_2$ alkyl substituted with $C_1$-$C_2$ alkoxy, $C_1$-$C_2$ haloalkoxy, $C_1$-$C_2$ alkylthio, $C_1$-$C_2$ haloalkylthio or CN;

$R_2$
is H, $C_1$-$C_3$ alkyl, allyl or phenyl;

$R_3$
is H, $C_1$-$C_6$ alkyl, $C_3$-$C_6$ cycloalkyl, $C_1$-$C_6$ haloalkyl, $CH_2$-($C_2$-$C_5$ alkenyl), $CH_2$($C_2$-$C_5$ haloalkenyl), $CH_2$( $C_2$-$C_5$ alkynyl), $CH_2$($C_2$-$C_5$ haloalkynyl), $C_6H_5$ or $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl;

$R_4$
is H, halogen, $C_1$-$C_6$ alkyl, $C_3$-$C_6$ cycloalkyl, $C_1$-$C_6$ haloalkyl, $C_2$-$C_6$ alkenyl, $C_2$-$C_6$ haloalkenyl, $C_2$-$C_6$ alkynyl, $C_3$-$C_6$ haloalkynyl, $C_6H_5$, $C_1$-$C_4$ alkoxy, $C_1$-$C_4$ alkylthio, $C_1$-$C_4$ alkylsulfinyl, $C_1$-$C_4$ alkylsulfonyl, $OCH_2CH_2O$-($C_1$-$C_2$ alkyl) or di($C_1$-$C_3$ alkyl)amino;

$R_5$
is H, $C_1$-$C_3$ alkyl or allyl;

$R_a$
is H, $C_1$-$C_4$ alkyl, $C_2$-$C_3$ cyanoalkyl, methoxy or ethoxy;

$R_b$
is H, $C_1$-$C_4$ alkyl or $C_3$-$C_4$ alkenyl; or

$R_a$ and $R_b$
may be taken together as $-(CH_2)_3-$, $-(CH_2)_4-$, $-(CH_2)_5-$ or $-CH_2CH_2OCH_2CH_2-$;

$R_c$
is $C_1$-$C_4$ alkyl, $C_3$-$C_4$ alkenyl, $C_3$-$C_4$ alkynyl, $C_2$-$C_4$ haloalkyl, $C_1$-$C_2$ cyanoalkyl, $C_5$-$C_6$ cycloalkyl, $C_4$-$C_7$ cycloalkylalkyl or $C_2$-$C_4$ alkoxyalkyl;

A is 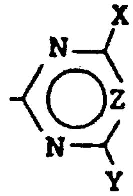 , 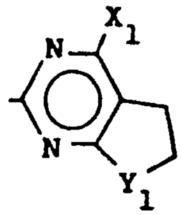 , 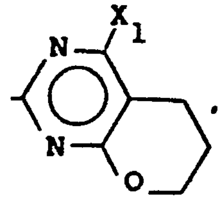 ,

A-1 A-2 A-3

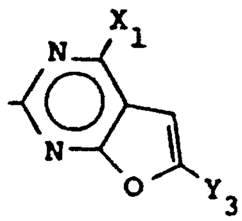 , 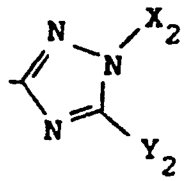 , 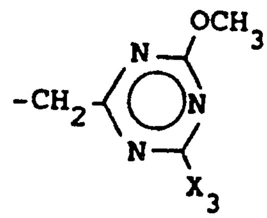

A-4 A-5 A-6

or 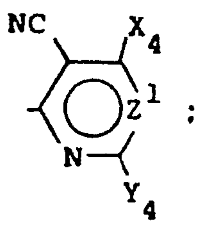 ;

A-7

X

is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y

is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_2$-$C_4$ alkynyl, azido, cyano,

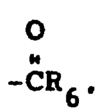 , 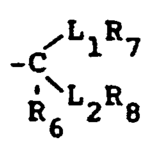 , 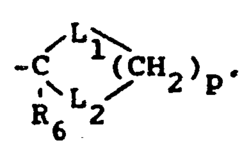 ,

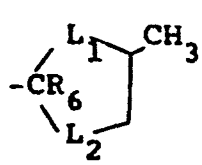

or -N($OCH_3$)$CH_3$;

p is 2 or 3;
$L_1$ and $L_2$ are independently O or S;
$R_6$ is H or $CH_3$;
$R_7$ and $R_8$ are independently $C_1$-$C_3$ alkyl;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl;
$Y_4$ is $CH_3$, $OCH_3$, $OC_2H_5$ or Cl; and
$Z^1$ is CH or N;
and their agriculturally suitable salts;
provided that
a) when X is Cl, F, Br or I. then Z is CH and Y is $OCH_3$, $OC_2H_5$, $N(OCH_3)CH_3$, $NHCH_3$, $N(CH_3)_2$ or $OCF_2H$;
b) when X or Y is $C_1$ haloalkoxy, then Z is CH;
c) when J is J-2, J-3 or J-4, the substituent $G_m(CH_2)_nQ$ or $(CH_2)_nQ$ and the sulfonylurea bridge are on adjacent ring positions;
d) when J is J-4 and n is O, then Q is Q-1 or Q-2;
e) when E is O, then J is J-1;
f) when W is S, then A is A-1, R is H, and Y is $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$
or

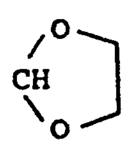 ;

g) when the total number of carbon atoms in X and Y is greater than 4, then the carbon content of $R_1$, $R_3$, $R_4$ and $R_5$ must each be less than or equal to 2;
h) $X_4$ and $Y_4$ cannot simultaneously be Cl;
i) when J is J-1 and m is 0, then n is 0; and
j) when n is 0 and m is 1, then Q is Q-1 or Q-2.

In the above definitions, the term "alkyl" used either alone or in compound words such as "alkylthio" or "haloalkyl", denotes straight chain or branched alkyl, e.g., methyl, ethyl, n-propyl, isopropyl or the different butyl, pentyl or hexyl isomers.

Alkoxy denotes methoxy, ethoxy, n-propoxy, isopropoxy and the different butyl isomers.

Alkenyl denotes straight chain or branched alkenes, e.g., vinyl, 1-propenyl, 2-propenyl, 3-propenyl and the different butenyl, pentenyl or hexenyl isomers.

Alkynyl denotes straight chain or branched alkynes, e.g., ethynyl, 1-propynyl, 2-propynyl and the different butynyl, pentynyl and hexynyl isomers.

Alkylsulfonyl denotes methylsulfonyl, ethylsulfonyl or the different propylsulfonyl isomers.

Alkylthio, alkylsulfinyl, alkylamino, etc. are defined in an analogous manner.

Cycloalkyl denotes cyclopropyl, cyclobutyl, cyclopentyl and cyclohexyl.

The term "halogen", either alone or in compound words such as "haloalkyl", denotes fluorine, chlorine, bromine or iodine.

In terms such as $C_2$-$C_3$ alkylthioalkyl, the specified number of carbon atoms is meant to define the total number of carbon atoms in that substituent group. For example, $C_2$-$C_3$ alkylthioalkyl would designate $CH_2SCH_3$, $CH_2SC_2H_5$, $CH_2CH_2SCH_3$ or $CH(CH_3)SCH_3$, and $C_2$-$C_5$ alkoxyalkoxy would represent $OCH_2OCH_3$ through $O(CH_2)_4OCH_3$ or $OCH_2O(CH_2)_3CH_3$ and the various structural isomers embraced therein.

Preferred for reasons of increased ease of synthesis and/or greater herbicidal efficacy are:

1) Compounds of Formula I where E is a single bond.

2) Compounds of Formula I where E is $CH_2$.

3) Compounds of Formula I where E is O.

4) Compounds of Preferred 1 where
m is 0;
W is O; and
R is H.

5) Compounds of Preferred 4 where
X
is $C_1$-$C_2$ alkyl, $C_1$-$C_2$ alkoxy, Cl, F, Br, I, $OCF_2H$, $CH_2F$, $CF_3$, $OCH_2CH_2F$, $OCH_2CHF_2$, $OCH_2CF_3$, $CH_2Cl$ or $CH_2Br$; and
Y
is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C{\equiv}CH$, $OCH_2CH_2OCH_3$, $CH_2SCH_3$,

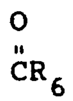, 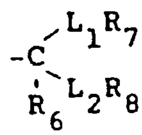, 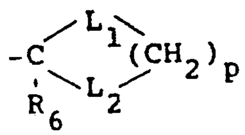,

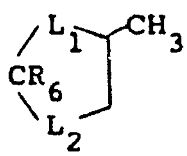,

$OCF_2H$, $SCF_2H$, cyclopropyl, $C{\equiv}CH$ or $C{\equiv}CCH_3$.

6) Compounds of Preferred 5 where $R_1$ is H, $CH_3$, F, Cl, Br, $OCH_3$, $SCH_3$, $CH_2CN$, $CH_2OCH_3$, $CF_3$ or $OCF_2H$; and n is O.

7) Compounds of Preferred 6 where
- $R_3$ is H, $C_1$-$C_3$ alkyl, $CH_2CH_2Cl$, $CH_2CH_2F$, $CH_2CHF_2$, $CH_2CF_3$, allyl or propargyl; and
- $R_4$ is H, $C_1$-$C_3$ alkyl, $C_1$-$C_2$ alkoxy, $C_1$-$C_2$ alkylthio, $N(CH_3)_2$, allyl, propargyl or $C_1$-$C_2$ alkyl substituted with 1-3 atoms of F, Cl or Br.

8) Compounds of Preferred 7 where
- A is A-1;
- Z is CH or N;
- X is $CH_3$, $OCH_3$, $OCH_2CH_3$, cyclopropyl, Cl or $OCF_2H$;
- Y is $CH_3$, $OCH_3$, $C_2H_5$, $CH_2OCH_3$, $NHCH_3$ or $CH(OCH_3)_2$.

9) Compounds of Preferred 8 where
- J is J-1.

10) Compounds of Preferred 9 where
- $R_3$ and $R_4$ are independently H, $CH_3$ or $C_2H_5$.

11) Compounds of Preferred 8 where
- J is J-2; and
- $R_1$ is H.

12) Compounds of Preferred 8 where
- J is J-3; and
- $R_1$ is H.

13) Compounds of Preferred 8 where
- J is J-4; and
- $R_1$ is H.

14) Compounds of Preferred 8 where

J is J-5; and
$R_1$ is H.

15) Compounds of Preferred 8 where
J is J-6; and
$R_1$ is H.

16) Compounds of Preferred 9 where
Q is Q-1 and $R_1$ is in the 5- or 6-position.

17) Compounds of Preferred 9 where
Q is Q-2 and $R_1$ is in the 5- or 6-position.

18) Compounds of Preferred 9 where
Q is Q-3 and $R_1$ is in the 5- or 6-position.

19) Compounds of Preferred 9 where
Q is Q-4 and $R_1$ is in the 5- or 6-position.

20) Compounds of Preferred 9 where
Q is Q-5 and $R_1$ is in the 5- or 6-position.

21) Compounds of Preferred 9 where
Q is Q-6 and $R_1$ is in the 5- or 6-position.

22) Compounds of Preferred 9 where
Q is Q-7 and $R_1$ is in the 5- or 6-position.

23) Compounds of Preferred 2 where
m is 0;
n is 0;
J is J-1;
W is O;
R is H;
$R_1$ is H;
$R_3$ is H or $C_1$-$C_3$ alkyl;
$R_4$ is H or $C_1$-$C_3$ alkyl;
A is A-1;
Z is CH or N;
X is $CH_3$, $OCH_3$, $OCH_2CH_3$, cyclopropyl, Cl or $OCF_2H$; and
Y is $CH_3$, $OCH_3$, $C_2H_5$, $CH_2OCH_3$, $NHCH_3$ or $CH(OCH_3)_2$.

24) Compounds of Preferred 3 where
m is O;
R is H;
$R_1$ is H;
$R_3$ is H or $C_1$-$C_3$ alkyl;
$R_4$ is H or $C_1$-$C_3$ alkyl;
A is A-1;
Z is CH or N;
X is $CH_3$, $OCH_3$, $OCH_2CH_3$, cyclopropyl, Cl or $OCF_2H$; and
Y is $CH_3$, $OCH_3$, $C_2H_5$, $CH_2OCH_3$, $NHCH_3$ or $CH(OCH_3)_2$.

Specifically preferred for reasons of greatest ease of synthesis and/or greatest herbicidal efficacy are:

N-[(4-chloro-6-methoxypyrimidin-2-yl)aminocarbonyl]-2-(1-ethyl-1H-tetrazol-5-yl)benzenesulfonamide, m.p. 223-225 °C;

N-[(4,6-dimethoxypyrimidin-2-yl)aminocarbonyl]-2-(1-ethyl-1H-tetrazol-5-yl)benzenesulfonamide, m.p. 214-217 °C;

N-[(4,6-dimethoxypyrimidin-2-yl)aminocarbonyl]-2-methyl-6-(1-methyl-1H-tetrazol-5-yl)benzenesulfonamide, m.p. 178-180 °C;

N-[(4-methoxy-6-methylpyrimidin-2-yl)aminocarbonyl]-2-methyl-6-(1-methyl-1H-tetrazol-5-yl)-benzenesulfonamide, m.p. 180-183 °C;

N-[(4,6-dimethylpyrimidin-2-yl)aminocarbonyl]-2-methyl-6-(1-methyl-1H-tetrazol-5-yl)benzenesulfonamide, m.p. 213-215 °C;

N-[4,6-dimethoxypyrimidin-2-yl)aminocarbonyl]-2-(5-methyl-1H-tetrazol-1-yl)benzenesulfonamide, m.p. 213-216 °C;

N-[(4,6-dimethoxypyrimidin-2-yl)aminocarbonyl]-2-(1-methyl-1H-tetrazol-5-yl)benzenesulfonamide, m.p. 214-222 °C;

N-[(4,6-dimethoxypyrimidin-2-yl)aminocarbonyl]-2-(1-methyl-1H-tetrazol-5-yl)benzenesulfonamide, m.p. 207-210° C;
N-[(4,6-dimethylpyrimidin-2-yl)aminocarbonyl]-2-(1-methyl-1H-tetrazol-5-yl)benzenesulfonamide, m.p. 224-226° C;
N-[(4-methoxy-6-methylpyrimidin-2-yl)aminocarbonyl]-2-(1-methyl-1H-tetrazol-5-yl)benzenesulfonamide, m.p. 222-224° C; and
N-[(4-chloro-6-methoxypyrimidin-2-yl)aminocarbonyl]-2-(1-methyl-1H-tetrazol-5-yl)benzenesulfonamide, m.p. 225-227° C.

## Detailed Description of the Invention

### Synthesis

Compounds of Formula I can be synthesized by one or both of the methods shown below in Equation 1 and 2.

Equation 1 illustrates the reaction of sulfonyl isocyanates and isothiocyanates of Formula II with the appropriate heterocyclic amines of Formula III to give the desired sulfonylureas and sulfonylthioureas of Formula I.

Equation 1

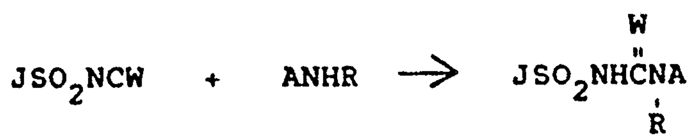

II III I

wherein
J, W, R, and A are as previously defined.

The reaction of Equation 1 is best carried out in an inert aprotic solvent such as methylene chloride, tetrahydrofuran or acetonitrile at a temperature between 0° and 82° C. A catalytic amount of 1,4-diazabicyclo[2,2,2]octane (DABCO®) may be used to accelerate the reaction. In cases in which the products are insoluble in the reaction solvent, they may be isolated by simple filtration. When the products are soluble, they can be isolated by evaporation of the solvent and trituration of the residue with solvents such as 1-chlorobutane, diethyl ether or ethyl acetate, and filtration.

Compounds of Formula I can be prepared as shown below in Equation 2 by the reaction of sulfonamides IV with the phenyl ester of the appropriate carbamic acid or thiocarbamic acid of Formula V, in the presence of an equimolar quantity of a tertiary amine base such as 1,8-diazabicyclo[5,4,0]undec-7-ene (DBU).

Equation 2

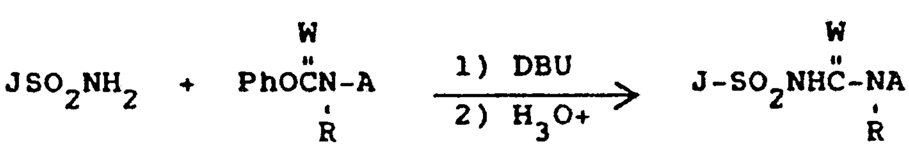


IV V Ia

wherein
J, R, W and A are as previously defined.

The reaction shown in Equation 2 is best carried out at 25° C in a solvent such as dioxane or acetonitrile for 1-2 hours under an inert atmosphere as described in European Patent Application No. 70,804

(published January 26, 1983). The desired products of Formula I can be conveniently isolated by acidifying the reaction solution with aqueous hydrochloric acid. Alternatively, the aqueous layer can be extracted with a solvent such as methylene chloride or ethyl acetate. Drying and evaporation of the solvent affords the desired products.

The phenylcarbamates and phenylthiocarbamates of Formula V can be prepared by methods, or modifications thereof known to those skilled in the art, described in South African Patent Application 82/5671 and South African Patent Application 82/5045.

A judicious choice of the appropriate methods for preparing compounds of Formula I must take into account the nature of the substituents contained within the J values ($J_1$-$J_6$), namely Q and $R_1$, and their chemical compatibility with the reaction conditions of Equations 1 and 2.

Sulfonyl isocyanates of Formula II can be prepared as shown in Equation 3 by the reaction of sulfonamides of the general structure IV with phosgene in the presence of n-butyl isocyanate and a catalytic amount of 1,4-diazabicyclo[2.2.2]octane (DABCO).

Equation 3

$$JSO_2NH_2 \xrightarrow[COCl_2]{\underline{n}\text{-}BuNCO} \underline{II}, \text{ where W is O}$$

IV — DABCO, xylenes

wherein
J is as previously defined, provided E is not oxygen.

The reaction depicted in Equation 3 is best carried out according to the procedure described in United States Patent 4,238,621.

Alternatively, sulfonyl isocyanates II can be prepared via phosgenation of the preformed n-butylureas of Formula VI as represented in Equation 4.

Equation 4

$$\underline{IV} \xrightarrow[K_2CO_3]{\underline{n}\text{-}BuNCO} JSO_2NHC(=O)NH\text{-}\underline{n}\text{-}Bu$$

VI

$$\underline{VI} \xrightarrow[DABCO]{COCl_2} \underline{II}, \text{ where W is O}$$

xylenes

wherein
J is as previously defined, provided E is not oxygen.

The compounds of Formula VI are conveniently prepared by stirring a mixture of the appropriate sulfonamide IV, anhydrous potassium carbonate, and n-butyl isocyanate in a suitable solvent such as acetone or methyl ethyl ketone at 25° to 80° C until all of the isocyanate has reacted. The products are isolated by quenching in dilute aqueous acid and recrystallizing the insoluble solid. The n-butylureas VI are then treated with phosgene and a catalytic amount of DABCO in refluxing xylenes or chlorobenzene in a manner analogous to that described in the reference cited for Equation 3.

Alternatively, treatment of the sulfonamides of Formula IV with thionyl chloride gives intermediate N-sulfinylsulfonamides VII, which afford sulfonylisocyanates II (where W is O) upon exposure to phosgene in

the presence of a catalytic amount of pyridine.

Equation 5

$$\underline{IV} \xrightarrow[\Delta]{SOCl_2} \underset{\underline{VII}}{JSO_2NSO}$$

$$\underline{VII} \xrightarrow[\substack{\text{Cat. Pyridine} \\ \text{toluene} \\ \Delta}]{COCl_2} \underline{II}\text{, where W is O}$$

The reaction of Equation 5 can best be performed according to the procedure of H. Ulrich, B. Tucker and A. Sayigh, J. Org. Chem., 34, 3200 (1969).

Sulfonyl isothiocyanates II (where W is S) are known in the art and are prepared from the corresponding sulfonamides (IV) by reaction with carbon disulfide and potassium hydroxide followed by treatment of the resulting dipotassium salt with phosgene. Such a procedure is described in Arch. Pharm. 299, 174 (1966).

A judicious choice of the appropriate method for preparing compounds of Formula II must take into account the nature of the substituents contained within the J values ($J_1$-$J_6$), namely Q and $R_1$, and their chemical compatability with reaction conditions of Equations 3-5.

The required sulfonamides of Formula IV can be synthesized by one or more of the methods shown below.

Equation 6 depicts the reaction of sulfonyl chlorides of Formula VIII with ammonia to give sulfonamides of Formula IVa.

Equation 6

$$\underset{\underline{VIII}}{JSO_2Cl} \xrightarrow{NH_3} \underset{\underline{IVa}}{JSO_2NH_2}$$

wherein

J is as previously defined, provided E is not oxygen.

The amination of Equation 6 can be effected by adding at least two molar equivalents of either anhydrous ammonia or concentrated ammonium hydroxide to a solution of the sulfonyl chloride VIII in a solvent such as diethyl ether, methylene chloride, or tetrahydrofuran at temperatures between -30° and 25° C. The sulfonamides of Formula IVa are isolated either by filtration, in which case the ammonium chloride by-product is removed by washing with water, or extraction into an organic solvent such as methylene chloride. Drying and evaporation of the solvent affords the sulfonamides IVa, which are usually sufficiently pure to be carried directly on to the next step.

Sulfonyl chlorides of Formula VIII can be prepared by one or more of the methods shown below in Equations 7, 8 and 9.

The reaction of an appropriately substituted N-arylhydroxylamine of Formula IX, as shown in Equation 7, and subsequent displacement with sulfur dioxide in the presence of cupric or cuprous chloride yields the desired sulfonyl chloride of Formula VIII.

Equation 7

$$\underset{\underline{IX}}{JNHOH} \xrightarrow[\text{2) } SO_2\text{, CuCl, AcOH}]{\text{1) } NaNO_2\text{, HCl}} \underset{\underline{VIII}}{JSO_2Cl}$$

The reaction of Equation 7 can be carried out by combining the appropriate arylhydroxylamine with aqueous concentrated hydrochloric acid and acetic acid and adding an aqueous solution of sodium nitrite at -10 to 10 °C. After being stirred for 10 minutes to two hours the reaction mixture is added to a mixture of acetic acid, a catalytic amount of cuprous or cupric chloride and excess sulfur dioxide at -5 to 20 °C. After being stirred for 0.5 to 24 hours the mixture is diluted with cold water and the product is extracted into a solvent such as methylene chloride or ethyl ether.

The solution of sulfonyl chloride thus obtained can be treated as in Equation 6 to prepare the desired sulfonamide IVa.

Diazotization of appropriately substituted amine derivatives of Formula X, as shown in Equation 8, and subsequent reaction with sulfur dioxide in the presence of either cupric or cuprous chloride gives the desired products of Formula VIII.

Equation 8

$$\underset{\underline{X}}{J\text{-}NH_2} \xrightarrow[\text{2) } SO_2\text{, } CuCl_2 \text{ or CuCl, HOAc}]{\text{1) } NaNO_2\text{, HCl}} \underset{\underline{VIII}}{JSO_2Cl}$$

The reaction of Equation 8 can be effected by methods analogous to those described in EP-A Nos. 83,975 and 85,476 (published August 10, 1983). In Equation 8, a substituted amine X, in concentrated hydrochloric acid is treated with a solution of sodium nitrite in water at -5° tc 5 °C. After being stirred for 10 minutes to one hour at about 0 °C, the solution is added to a mixture of excess sulfur dioxide and a catalytic amount of cupric or cuprous chloride in acetic acid at about 10 °C. After being stirred for 0.25 to 24 hours at temperatures between 10° to 25 °C, the solution is poured into a large excess of ice water. The sulfonyl chloride VIII can be isolated by filtration, or by extraction into a solvent such as methylene chloride or ether, followed by drying and evaporation of the solvent.

Sulfonyl chlorides can also be prepared as shown below in Equation 9 by metal halogen exchange or directed lithiation of appropriately substituted aryl or heterocyclic substrates XI, and trapping with sulfuryl chloride.

Equation 9

$$\underset{\underline{XI}}{J\text{-}Br\ (H)} \xrightarrow[\text{-78°C; 2) } SO_2Cl_2\text{/Hexanes}]{\text{1) } \underline{n\text{-}BuLi;}\ Et_2O \text{ or THF}} \underset{\underline{VIIIa}}{JSO_2Cl}$$

wherein

J is as previously described provided that $R_1$ is not $CO_2R_c$, $C_1$-$C_3$ haloalkyl, Br, nitro, $C_1$-$C_3$ alkylsulfonyl or CN.

The lithiation shown in Equation 10 can be performed according to the procedure of S. H. Bhattacharya, et al., J. Chem. Soc. (C), 1265 (1968) or by procedures reviewed by H. Gschwind and H. Rodriquez in Organic Reactions, Vol. 26, Wiley, New York, 1979, and references cited within.

Compounds of Formula VIII can be prepared via oxidative chlorination of the appropriate thioethers or mercaptans of Formula XII as represented in Equation 10.

Equation 10

$$J\text{-}SR_{15} \xrightarrow[H_2O,\ HOAc]{Cl_2} JSO_2Cl$$

XII VIII

wherein

J is as previously defined, $R_{15}$ is H, $C_2$-$C_4$ alkyl or benzyl, and $R_1$ is not $C_1$-$C_3$ alkylthio or $C_1$-$C_3$ alkylsulfinyl.

The reaction of Equation 10 can be carried out by treating a solution of the thioether XII in a solvent such as acetic acid in the presence of at least 2.5 equivalents of water and at least 3.0 equivalents of chlorine at 0-30°C for .25 to 5 hours. The reaction is poured into ice-water and the product is isolated by extraction with a suitable solvent such as methylene chloride, dried, and the solvent evaporated to yield a product sufficiently pure to be carried directly on to the next step.

Arylmethanesulfonyl chlorides of Formula VIIIb can be prepared from appropriately substituted benzyl chlorides or bromides of Formula XIII by a simple two-step procedure outlined in Equation 11.

Equation 11

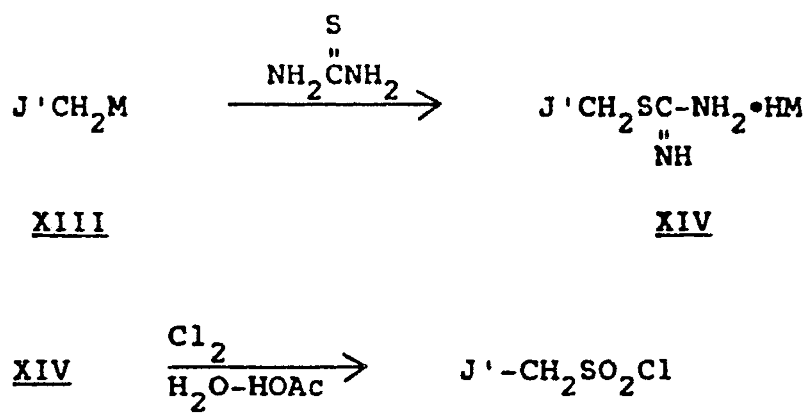


XIII XIV

XIV VIIIb

wherein

| | |
|---|---|
| J'$CH_2$ | is defined as for J-1 through J-6 where E is $CH_2$; |
| $R_1$ and Q | are as previously defined except $R_1$ is not $C_1$-$C_3$ alkylthio or $C_1$-$C_3$ alkylsulfinyl; and |
| M | is Cl or Br. |

The conversion of alkyl halides of Formula XIII to isothiouronium salts of Formula XIV can be carried out by the procedure or T. B. Johnson and J. M. Sprague, J. Am. Chem. Soc., 58, 1348 (1936); 59 1837 and 2439 (1937); 61 176 (1939). Oxidative chlorination of isothiouronium salts such as XIV to afford sulfonyl chlorides of Formula VIIIb is best carried out according to the procedure of Johnson as described in J. Am. Chem. Soc., 61 2548 (1939).

The requisite tetrazole substituted intermediates can be prepared by various methods depending on the

nature of the aromatic ring in J (J-1 to J-6) systems on which the tetrazole is substituted and the nature of the other substituents present on J (J-1 to J-6). These intermediates can be prepared by one skilled in the art by the methods referred to in the following reviews on tetrazole chemistry: F. R. Benson, "Heterocyclic Compounds" (R. C. Elderfield, Ed.) 8, 1-104, John Wiley and Sons, New York, (1967); R. N. Butler, "Advances in Heterocyclic Chem.", 21, 323-436, Academic Press (1977); R N. Butler. "Comprehensive Heterocyclic Chem.", (K. T. Potts. Ed.) 5, 791-838, Pergamon Press (1984) and F.R. Benson, "High Nitrogen Compounds", John Wiley and Sons, New York (1983).

The heterocyclic amines of Formula III in Equation 1 above can be prepared by methods known in the literature, or simple modifications thereof, by those skilled in the art. For instance, EP-A No. 84,224 (published July 27, 1983) and W. Braker et al., J. Am. Chem. Soc., 69, 3072 (1947) describes methods for preparing aminopyridines and triazines substituted by acetal groups such as dialkoxymethyl or 1,3-dioxolan-2-yl, among other groups. Also, for example, South African patent application Nos. 82/5045 and 82/5671 describe methods for preparing aminopyrimidines and triazines substituted by haloalkyl or haloalkylthio groups such as $OCH_2CH_2F$, $OCH_2CF_3$, $SCF_2H$, or $OCF_2H$, among other groups. South African patent application No. 83/7434 (published October 5, 1983) describes methods for the synthesis of cycopropylpyrimidines and triazines substituted by such groups as alkyl, haloalkyl, alkoxy, haloalkoxy, alkylamino, dialkylamino or alkoxyalkyl.

The 5,6-dihydrofuro[2,3-d]pyrimidine-2-amines, the cyclopenta[d]pyrimidin-2-amines (III, A is A-2) and the 6,7-dihydro-5H-pyrano[2,3-d]pyrimidin-2-amines (III, A is A-3) can be prepared as described in EP-A No. 15,683. The furo[2,3-d]pyrimidin-2-amines (III, A is A-4) are described in EP-A No. 46,677.

Compounds of Formula III, where A is A-5, are described in EP-A-73,562. Compounds of Formula III, where A is A-6 are described in EP-A-94,260.

In addition, general methods for preparing aminopyrimidines and triazines have been reviewed in the following publications:

- "The Chemistry of Heterocyclic Compounds", a series published by Interscience Publishers, Inc., New York and London;
- "Pyrimidines", Vol. 16 of the same series by D. J. Brown;
- "s-Triazines and Derivatives", Vol. 13 of the same series by E. M. Smolin and L. Rappoport; and
- F. C. Schaefer, U. S. Patent 3,154,547 and K. R. Huffman and F. C. Schaefer, J. Org. Chem., 28, 1812 (1963), which describes the synthesis of triazines.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide or carbonate). Quaternary amine salts can be made by similar techniques.

Salts of compounds of Formula I can also be prepared by exchange of one cation for another. Cationic exchange can be effected by direct contact of an aqueous solution of a salt of a compound of Formula I (e.g. alkali or quaternary amine salt) with a solution containing the cation to be exchanged. This method is most effective when the desired salt containing the exchange cation is insoluble in water and can be separated by filtration.

Exchange may also be effected by passing an aqueous solution of a salt of a compound of Formula I (e.g., an alkali metal or quaternary amine salt) through a column packed with a cation exchange resin containing the cation to be exchanged for that of the original salt and the desired product is eluted from the column. This method is particularly useful when the desired salt is water-soluble, e.g., a potassium sodium or calcium salt.

Acid addition salts, useful in this invention, can be obtained by reacting a compound of Formula I with a suitable acid, e.g., p-toluenesulfonic acid, trichloroacetic acid or the like.

The preparation of the compounds of this invention is further illustrated by the following specific examples.

## Example 1

### 1-(2-Chlorosulfonylphenyl)-5-methyl-1H-tetrazole

To 5 g of 1-(2-hydroxylaminophenyl)-5-methyl-1H-tetrazole in 12 mL of 37% hydrochloric acid and 5 mL of acetic acid at 0-5 °C was added 2.3 g of sodium nitrite in 10 mL of water. The resultant solution was stirred at -5° to +5 °C for one hour and then added portionwise, with stirring, to a mixture of 25 mL of acetic acid, 0.5 g of cupric chloride and 5 mL of liquified sulfur dioxide at below 5 °C. After stirring for two hours, during which time the mixture was allowed to warm to room temperature, it was poured into 150 mL

of ice and water and extracted twice with 150 mL of methylene chloride. The methylene chloride solution was washed three times with 100 mL portions of water, two times with 100 mL of saturated aqueous sodium bicarbonate followed by single washes with 100 mL of saturated aqueous sodium chloride and 100 mL of water. The methylene chloride solution was then dried over magnesium sulfate and filtered to yield a solution containing the desired sulfonylchloride. This solution was used without further work-up to prepare the sulfonamide of Example 2.

Example 2

1-(2-Aminosulfonylphenyl)-5-methyl-1H-tetrazole

Twenty-five mL of 28% ammonium hydroxide was added to the methylene chloride solution obtained from Example 2 and the mixture was stirred for two hours and concentrated in vacuo to yield a mixture of an oil and a solid. The solid product was separated by filtration and washed with water to yield 2.2 g of the desired product, which melted at 141-148 °C. Infrared absorption peaks at 3260 and 3360 $cm^{-1}$ were consistent for $NH_2$ and 1160 and 1350 $cm^{-1}$ consistent for the $SO_2$ of the desired product. Elem. anal. calc'd for $C_8H_9N_5O_2S$:C, 40.15; H, 3.79; N, 29.26, S, 13.39. Found: C, 40.43; H, 3.84; N, 29.61; S, 13.04.

Example 3

5-(2-Chlorosulfonylphenyl)-2-methyl-2H-tetrazole and 5-(2-chlorosulfonylphenyl)-1-methyl-1H-tetrazole

To 5g of a mixture of 5-(2-hydroxylaminophenyl)-2-methyl-2H-tetrazole and 5-(2-hydroxyaminophenyl)-1-methyl-1H-tetrazole dissolved in 12 mL of 37% hydrochloric acid and 5 mL of acetic acid was added with stirring 2.3 g of sodium nitrite dissolved in 10 mL of water at -5 ° to 5 °C. After one hour this solution was added, at 0-5 °C, portionwise to a mixture of 25 mL of acetic acid, 0.5 g of cupric chloride and 5 mL of liquified sulfur dioxide. After the addition was completed the cooling bath was removed and the reaction mixture was allowed to warm to 31 °C whereupon the cooling bath was reapplied and the stirring was continued until two and one half hours after completion of the addition. The mixture was then poured into ice and worked up as described in Example 1 except that the desired methylene chloride solution was concentrated in vacuo to yield an oil which solidified. This product, (3.28 g) which was a mixture of the desired sulfonylchlorides, was of sufficient purity for the preparation of the sulfonamides of Example 4.

Example 4

5-(2-Aminosulfonylphenyl)-2-methyl-2H-tetrazole and 5-(2-aminosulfonylphenyl)-1-methyl-1H-tetrazole

The solid product obtained in Example 3 was dissolved in 50 mL of tetrahydrofuran and added portionwise to 20 mL of 28% ammonium hydroxide. The mixture was stirred for one hour and concentrated in vacuo. Water was added to the residue, the mixture filtered and the solid washed with a small amount of cold water to yield 1.3 g of the isomer mixture. Infrared absorption spectra showed peaks at 3260 and 3360 $cm^{-1}$ consistent for $NH_2$ and 1160 and 1330 consistent for the $SO_2$ group of the desired product. Elem. anal. calc'd. for $C_8H_9N_5O_2S$:C, 40.15; H, 3.79; N, 29.26; S, 13.39. Found: C, 40.49; H, 3.86; N, 29.99; S, 13.75.

Example 5

5-(2-Aminosulfonylphenyl)-1-methyl-1H-tetrazole

To 4.4 g of 5-(2-ethylthiophenyl)-1-methyl-1H-tetrazole in 40 mL of propionic acid and 1.08 g of water at -10 °C was added slowly, dropwise, 5.2 mL, of liquid chlorine (1.367 g/mL). After the addition the mixture was allowed to warm to room temperature, and was then poured into approximately 100 g of ice. The resultant sulfonyl chloride was extracted with methylene chloride, washed with water and the methylene chloride solution added to 45 mL of concentrated aqueous ammonium hydroxide while maintaining the reaction temperature below 25 °C with ice bath cooling. Evaporation of the methylene chloride yielded a solid product which was removed by filtration from the aqueous residue and washed with water. The product, 3.72 g, melted at 176-179 °C and showed absorption peaks by infrared spectroscopy at 3210 and 3300 $cm^{-1}$, consistent for the desired sulfonamide.

Example 6

5-(2-Aminosulfonyl-4-methylphenyl)-1-methyl-1H-tetrazole

To a mixture of 4.68 g of 5-(4-methyl-2-propylthiophenyl)-1-methyl-1H-tetrazole, 40 mL of propionic acid and 1.08 g of water cooled to -10 °C was added dropwise 5.2 mL (liquified) of chlorine while maintaining the reaction temperature at -10 ° to 0 °C. After one-half hour the reaction mixture was allowed to warm to room temperature and stirred for one hour. It was then poured into 200 mL of ice and water and extracted with methylene chloride. The methylene chloride solution was added to 45 mL of concentrated ammonium hydroxide at 10 °C and stirred overnight. The mixture was concentrated in vacuo to an oil residue which was taken up in 100 mL of methylene chloride washed with three 100 mL portions of water and 100 mL of saturated aqueous sodium hydroxide. The methylene chloride solution was then dried over magnesium sulfate, filtered, and the filtrate evaporated to yield 4.1 g of the desired product melting at 161-171 °C.

NMR ($CDCl_3$): δ 2.56 (s, $CH_3$ on phenyl);
3.99 (s, $CH_3$ on tetrazole);
5.6 (s, w, $NH_2$); and
7.3-8.1 (m, 3 CH on phenyl).

Example 7

5-(2-Aminosulfonyl-3-methylphenyl)-1-methyl-1H-tetrazole

To a mixture of 14.6 mL of concentrated (12 M) hydrochloric acid, 6.1 mL of acetic acid, 25 mL of ethyl ether and 6.91 g of 5-(2-amino-3-methylphenyl)-1-methyl-1H-tetrazole at 0 °C was added dropwise 2.8 g of sodium nitrite dissolved in 12.2 mL of water while maintaining the reaction temperature at below 10 °C. Stirring was continued for one hour after the addition was completed. This mixture was then added slowly to 30.5 mL of acetic acid containing 0.6 g of cupric chloride and 6.1 mL of liquified sulfur dioxide. After stirring for two hours at ambient temperature, the mixture was poured into 200 mL of ice-water and extracted twice with 125 mL of methylene chloride. The organic phase was then washed twice with 200 ml of water, once with 200 mL saturated aqueous sodium chloride, once with 200 mL aqueous sodium bicarbonate followed by 200 mL of water and 200 mL saturated aqueous sodium chloride. The organic phase was then added dropwise to 25 mL of concentrated aqueous ammonium hydroxide and the mixture stirred overnight and evaporated to a solid, which was washed with water and dried. The crude product thus obtained melted at 140-161 °C, yield 6.26 g. Mass spectral analysis showed a molecular weight of 253 (calc. 253) for the product.

Example 8

N-[(4,6-Dimethoxypyrimidin-2-yl)aminocarbonyl]-2-(2-methyl-2H-tetrazol-5-yl)benzenesulfonamide

To 25 mL of acetonitrile was added at ambient temperature and pressure 0.48 g of 5-(2-aminosulfonylphenyl)-2-methyl-2H-tetrazole and 0.54 g of phenyl N-(4,6-dimethoxypyrimidin-2-yl)carbamate followed by 0.3 mL of DBU. After being stirred for three hours the reaction mixture was added to 25 g of ice and acidified to pH 3 with hydrochloric acid. The precipitate thus obtained was filtered, washed with water and dried to yield 0.66 g of the desired product, m.p. 214-222 °C. Infrared absorptions at 1720, 1600 and 1580 $cm^{-1}$ were consistent with the desired structure.

NMR($CDCl_3$):δ 3.91 (s, 2 X $CH_3O$)
5.82 (s, CH, pyrimidin)
4.43 (s, $CH_3$ on N-2 of tetrazole)
4.38 (s, $CH_3$ on N-1 of tetrazole)

Integration of the peaks at 4.43 d and 4.38 d total one $CH_3$ indicating a ratio of 2-methyl-2H-tetrazole to 1-methyl-1H-tetrazole of 2:1. This mixture is suitable for purposes of this invention. If desired, the isomer mixture obtained in Example 6 can be separated by chromatography into its component parts.

Example 9

N-[(4,6-Dimethoxypyrimidin-2-yl)aminocarbonyl]-2-(5-methyl-1H-tetrazol-1-yl)benzenesulfonamide

To 0.48 g of 1-(2-aminosulfonylphenyl)-5-methyl 1H-tetrazole in 25 mL of acetonitrile was added at ambient temperature and pressure 0.54 g of phenyl N-(4,6-dimethoxypyrimidin-2-yl)carbamate and 0.3 mL of DBU. The reaction mixture was stirred for three hours, added to 25 g of ice and acidified to pH 3 with hydrochloric acid. The precipitate thus obtained was filtered, washed with water and dried to yield 0.74 g of the desired product, melting point at 213-216 °C. Infrared absorption spectra showed peaks at 1710, 1605 and 1570 $cm^{-1}$, consistent for the desired product.

NMR($CDCl_3$): δ2.48 (s, $CH_3$ on tetrazole)
3.82 (s, 2X$CH_3$O)
5.81 (s, CH-pyrimidin).

Example 10

N-[(4-Methoxy-6-methyl-1,3,5-triazin-2-yl)aminocarbonyl]-2-(5-methyl-1H-tetrazol-1-yl)benzenesulfonamide

To 0.48 g of 1-(2-aminosulfonylphenyl)-5-methyl-1H-tetrazole and 0.52 g of phenyl N-(4-methoxy-6-methyl-1,3,5-triazin-2-yl)carbamate in 25 mL of acetonitrile at ambient temperature and pressure was added 0.3 mL of DBU. The mixture was stirred for three hours and then added to 25 g of ice and acidified with hydrochloric acid. The precipitate thus obtained was filtered, washed with water and dried *in vacuo* to yield 0.65 g of the desired product melting at 207-209 °C. Infrared absorption spectra showed absorption at 1700, 1600 and 1550 $cm^{-1}$, consistent for the desired structure.

Example 11

N-[(4-Chloro-6-methoxypyrimidine-2-yl)aminocarbonyl]-2-(1-methyl-1H-tetrazol-5-yl)benzenesulfonamide

To 0.48 g of 5-(2-aminosulfonylphenyl)-1-methyl-1H-tetrazole and 0.56 g of phenyl N-(4-chloro-6-methoxypyrimidine-2-yl)carbamate in 20 mL of dry acetonitrile at ambient temperature was added, with stirring, 0.3 mL of DBU. After stirring 16 hours the mixture was then poured into 25 g of ice followed by the addition of 10 mL of 2N hydrochloric acid. The precipitate thus formed was removed by filtration, washed with water and dried to yield 0.74 g of the desired product, m.p. 225-227 °C.

NMR ($CDCl_3$): δ 3.88 (s, $CH_3$ on tetrazole);
3.95 (s, $CH_3$O);
6.52 (s, CH of pyrimidine);
7.2-7.5 (m, CH of phenyl and NH);
7.84 (m, 2 CH of phenyl);
8.6 (m, CH of phenyl); and
11.96 (s, NH).

Example 12

N-[(4,6-Dimethoxypyrimidin-2-yl)aminocarbonyl]-5-chloro-2-(4-methyl-5-oxo-1H,4H-tetrazol-5-yl)-benzenesulfonamide

To a stirred solution of 0.29 g of 1-(2-aminosulfonyl-5-chlorophenyl)-4-methyl-1H-tetrazol-5-[4H]-one and 0.27 g of phenyl N-(4,6-dimethoxypyrimidin-2-yl)-carbamate in 10 mL of acetonitrile at ambient temperature was added 0.1 mL of DBU. The mixture was stirred for two hours, poured into 20 g of ice and acidified with 2N hydrochloric acid. The precipitate was removed by filtration, washed with water and dried to yield 0.37 g of product, m.p. 178-180 °C.

NMR ($CDCl_3$): δ 3.63 (s, $CH_3$ on tetrazole);
3.92 (s, 2 X $CH_3$O);
5.81 (s, CH of pyrimidine);
7.4-8.6 (m, 3 CH of phenyl); and
12.6 (s, w, NH).

Example 13

3-(2-Methyl-2H-tetrazole-5-yl)-1-phenyl-1H-pyrazole-5-sulfonamide

To a mixture of 3.1 mL of 12N hydrochloric acid, 12.5 mL of acetic acid, 3.7 mL of formic acid and 4.5 g of 5-amino-1-phenyl-3-(2-methyl-2H-tetrazol-5-yl)-1H-pyrazolewas added dropwise 1.4 g of sodium nitrite in 2.5 mL of water at -8° to -6° C. The mixture was stirred for 15 minutes and then added to 20 mL of acetic acid containing 3.7 mL (liquid) of sulfur dioxide and 0.75 g of cupric chloride dehydrate. After stirring for thirty minutes and allowing to warm, the mixture was poured into an ice and water mixture and the precipitate removed by filtration. After washing with water the solid was dissolved in tetrahydrofuran, cooled to -78° C, and ammonia (liquified, 1.0 mL) was added dropwise. The mixture was allowed to stand overnight at ambient temperature and filtered to yield a brown solid. This solid showed peaks at 3300 and 3200 $cm^{-1}$, consistent for $NH_2$, and 1370, 1185 and 1170 $cm^{-1}$, consistent for $SO_2$.

By using the procedures described in the foregoing equations and examples or modifications thereof, one skilled in the art can prepare the compounds shown in the following tables.

## Compounds

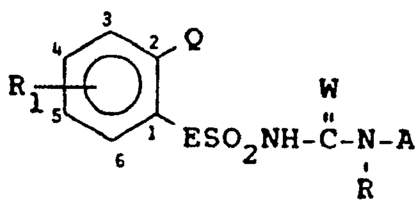

In General Structure 1, A is A-1
In General Structure 2, A is A-2
In General Structure 3, A is A-3
In General Structure 4, A is A-4
In General Structure 5, A is A-5
In General Structure 6, A is A-6
In General Structure 7, A is A-7

General Structure 8

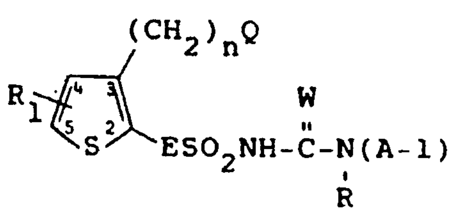

General Structure 9

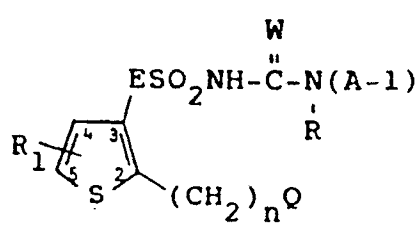

Compounds (continued)

General Structure 10

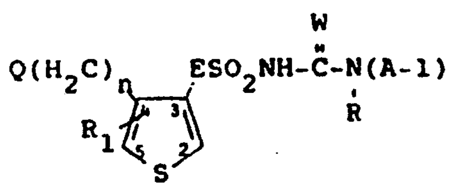

General Structure 11

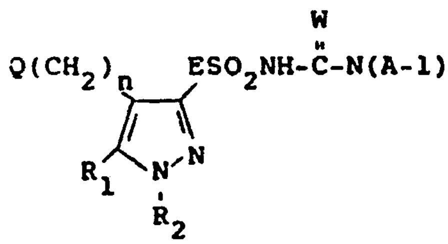

General Structure 12

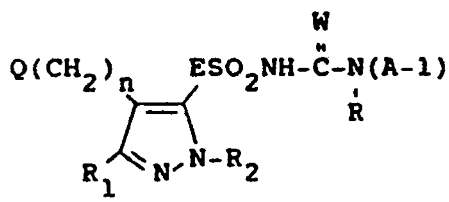

General Structure 13

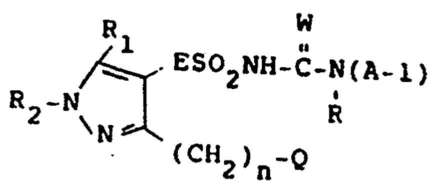

General Structure 14

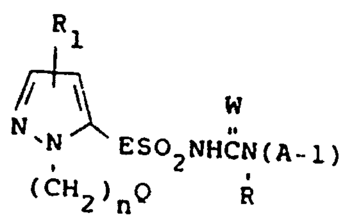

General Structure 15

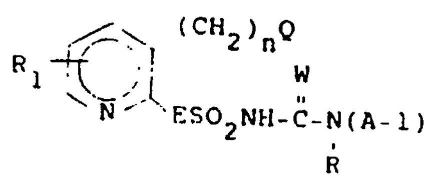

Compounds (continued)

General Structure 16

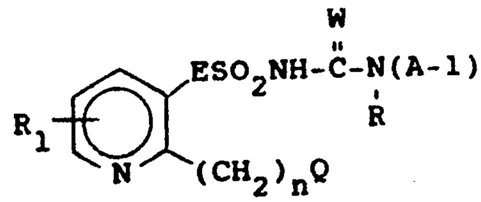

General Structure 17

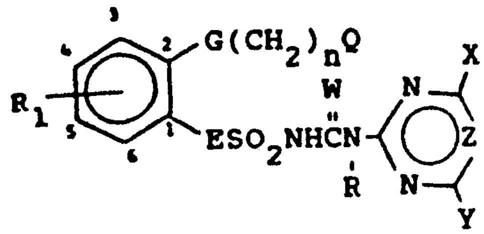

Table I

General Structure 1

| $R_1$ | E | Q | $R_3$ | W | R | X | Y | Z | m.p.(°C) |
|---|---|---|---|---|---|---|---|---|---|
| H | - | Q-1 | $CH_3$ | O | H | $CH_3$ | $CH_3$ | CH | 224-226 |
| H | - | Q-1 | $CH_3$ | O | H | $CH_3$ | $OCH_3$ | CH | 222-224 |
| H | - | Q-1 | $CH_3$ | O | H | $OCH_3$ | $OCH_3$ | CH | 207-210 |
| H | - | Q-1 | $CH_3$ | O | H | $OCH_3$ | Cl | CH | 225-227 |
| H | - | Q-1 | $CH_3$ | O | H | $CH_3$ | $OCH_3$ | N | 196-198 |
| H | - | Q-1 | $CH_3$ | O | H | $OCH_3$ | $OCH_3$ | N | 172-187 |
| H | - | Q-1 | $C_2H_5$ | O | H | $CH_3$ | $CH_3$ | CH | 200-207 |
| H | - | Q-1 | $C_2H_5$ | O | H | $CH_3$ | $OCH_3$ | CH | 203-205 |
| H | - | Q-1 | $C_2H_5$ | O | H | $OCH_3$ | $OCH_3$ | CH | 214-217 |
| H | - | Q-1 | $C_2H_5$ | O | H | $OCH_3$ | Cl | CH | 223-225 |
| H | - | Q-1 | $C_2H_5$ | O | H | $OC_2H_5$ | $NHCH_3$ | N | 193-197 |
| H | - | Q-1 | $C_2H_5$ | O | H | $CH_3$ | $OCH_3$ | N | 192-196 |
| H | - | Q-1 | $C_2H_5$ | O | H | $OCH_3$ | $OCH_3$ | N | 192-195 |
| H | - | Q-1 | $CH(CH_3)_2$ | O | H | $CH_3$ | $CH_3$ | CH | |
| H | - | Q-1 | $CH(CH_3)_2$ | O | H | $CH_3$ | $OCH_3$ | CH | |
| H | - | Q-1 | $CH(CH_3)_2$ | O | H | $OCH_3$ | $OCH_3$ | CH | |
| H | - | Q-1 | $CH(CH_3)_2$ | O | H | $OCH_3$ | Cl | CH | |
| H | - | Q-1 | $CH(CH_3)_2$ | O | H | $CH_3$ | $OCH_3$ | N | |
| H | - | Q-1 | $CH(CH_3)_2$ | O | H | $OCH_3$ | $OCH_3$ | N | |
| H | - | Q-1 | $(CH_2)_4CH_3$ | O | H | $CH_3$ | $CH_3$ | CH | |
| H | - | Q-1 | $(CH_2)_4CH_3$ | O | H | $CH_3$ | $OCH_3$ | CH | |
| H | - | Q-1 | $(CH_2)_4CH_3$ | O | H | $OCH_3$ | $OCH_3$ | CH | |
| H | - | Q-1 | $(CH_2)_4CH_3$ | O | H | $OCH_3$ | Cl | CH | |
| H | - | Q-1 | $(CH_2)_4CH_3$ | O | H | $CH_3$ | $OCH_3$ | N | |
| H | - | Q-1 | $(CH_2)_4CH_3$ | O | H | $OCH_3$ | $OCH_3$ | N | |
| H | - | Q-1 | cyclopentyl | O | H | $CH_3$ | $CH_3$ | CH | |
| H | - | Q-1 | cyclopentyl | O | H | $CH_3$ | $OCH_3$ | CH | |
| H | - | Q-1 | cyclopentyl | O | H | $OCH_3$ | $OCH_3$ | CH | |
| H | - | Q-1 | cyclopentyl | O | H | $OCH_3$ | Cl | CH | |
| H | - | Q-1 | cyclopentyl | O | H | $CH_3$ | $OCH_3$ | N | |
| H | - | Q-1 | cyclopentyl | O | H | $OCH_3$ | $OCH_3$ | N | |

Table 1 (Cont'd)

| $R_1$ | E | Q | $R_3$ | W | R | X | Y | Z | m.p.(°C) |
|---|---|---|---|---|---|---|---|---|---|
| H | - | Q-1 | $CH_2Cl$ | O | H | $CH_3$ | $CH_3$ | CH | |
| H | - | Q-1 | $CH_2Cl$ | O | H | $CH_3$ | $OCH_3$ | CH | |
| H | - | Q-1 | $CH_2Cl$ | O | H | $OCH_3$ | $OCH_3$ | CH | |
| H | - | Q-1 | $CH_2Cl$ | O | H | $OCH_3$ | Cl | CH | |
| H | - | Q-1 | $CH_2Cl$ | O | H | $CH_3$ | $OCH_3$ | N | |
| H | - | Q-1 | $CH_2Cl$ | O | H | $OCH_3$ | $OCH_3$ | N | |
| H | - | Q-1 | $(CH_2)_2F$ | O | H | $CH_3$ | $CH_3$ | CH | |
| H | - | Q-1 | $(CH_2)_2F$ | O | H | $CH_3$ | $OCH_3$ | CH | |
| H | - | Q-1 | $(CH_2)_2F$ | O | H | $OCH_3$ | $OCH_3$ | CH | |
| H | - | Q-1 | $(CH_2)_2F$ | O | H | $OCH_3$ | Cl | CH | |
| H | - | Q-1 | $(CH_2)_2F$ | O | H | $CH_3$ | $OCH_3$ | N | |
| H | - | Q-1 | $(CH_2)_2F$ | O | H | $OCH_3$ | $OCH_3$ | N | |
| H | - | Q-1 | $-CH_2CH=CH_2$ | O | H | $CH_3$ | $CH_3$ | CH | |
| H | - | Q-1 | $-CH_2CH=CH_2$ | O | H | $CH_3$ | $OCH_3$ | CH | |
| H | - | Q-1 | $-CH_2CH=CH_2$ | O | H | $OCH_3$ | $OCH_3$ | CH | |
| H | - | Q-1 | $-CH_2CH=CH_2$ | O | H | $OCH_3$ | Cl | CH | |
| H | - | Q-1 | $-CH_2CH=CH_2$ | O | H | $CH_3$ | $OCH_3$ | N | |
| H | - | Q-1 | $-CH_2CH=CH_2$ | O | H | $OCH_3$ | $OCH_3$ | N | |
| H | - | Q-1 | $CH_2CH=CHCH_2Cl$ | O | H | $CH_3$ | $CH_3$ | CH | |
| H | - | Q-1 | $CH_2CH=CHCH_2Cl$ | O | H | $CH_3$ | $OCH_3$ | CH | |
| H | - | Q-1 | $CH_2CH=CHCH_2Cl$ | O | H | $OCH_3$ | $OCH_3$ | CH | |
| H | - | Q-1 | $CH_2CH=CHCH_2Cl$ | O | H | $OCH_3$ | Cl | CH | |
| H | - | Q-1 | $CH_2CH=CHCH_2Cl$ | O | H | $CH_3$ | $OCH_3$ | N | |
| H | - | Q-1 | $CH_2CH=CHCH_2Cl$ | O | H | $OCH_3$ | $OCH_3$ | N | |
| H | - | Q-1 | $CH_2-C\equiv C-CH_3$ | O | H | $CH_3$ | $CH_3$ | CH | |
| H | - | Q-1 | $CH_2-C\equiv C-CH_3$ | O | H | $CH_3$ | $OCH_3$ | CH | |
| H | - | Q-1 | $CH_2-C\equiv C-CH_3$ | O | H | $OCH_3$ | $OCH_3$ | CH | |
| H | - | Q-1 | $CH_2-C\equiv C-CH_3$ | O | H | $OCH_3$ | Cl | CH | |
| H | - | Q-1 | $CH_2-C\equiv C-CH_3$ | O | H | $CH_3$ | $OCH_3$ | N | |
| H | - | Q-1 | $CH_2-C\equiv C-CH_3$ | O | H | $OCH_3$ | $OCH_3$ | N | |
| H | - | Q-1 | $C_6H_5$ | O | H | $CH_3$ | $CH_3$ | CH | |
| H | - | Q-1 | $C_6H_5$ | O | H | $CH_3$ | $OCH_3$ | CH | |

Table 1 (Cont'd)

| $R_1$ | E | Q | $R_3$ | W | R | X | Y | Z | m.p.(°C) |
|---|---|---|---|---|---|---|---|---|---|
| H | - | Q-1 | $C_6H_5$ | O | H | $OCH_3$ | $OCH_3$ | CH | |
| H | - | Q-1 | $C_6H_5$ | O | H | $OCH_3$ | Cl | CH | |
| H | - | Q-1 | $C_6H_5$ | O | H | $CH_3$ | $OCH_3$ | N | |
| H | - | Q-1 | $C_6H_5$ | O | H | $OCH_3$ | $OCH_3$ | N | |
| H | - | Q-1 | $(CH_2)_4OCH_3$ | O | H | $CH_3$ | $CH_3$ | CH | |
| H | - | Q-1 | $(CH_2)_4OCH_3$ | O | H | $CH_3$ | $OCH_3$ | CH | |
| H | - | Q-1 | $(CH_2)_4OCH_3$ | O | H | $OCH_3$ | $OCH_3$ | CH | |
| H | - | Q-1 | $(CH_2)_4OCH_3$ | O | H | $OCH_3$ | Cl | CH | |
| H | - | Q-1 | $(CH_2)_4OCH_3$ | O | H | $CH_3$ | $OCH_3$ | N | |
| H | - | Q-1 | $(CH_2)_4OCH_3$ | O | H | $OCH_3$ | $OCH_3$ | N | |
| H | - | Q-1 | $(CH_2)_2SCH_2CH_3$ | O | H | $CH_3$ | $CH_3$ | CH | |
| H | - | Q-1 | $(CH_2)_2SCH_2CH_3$ | O | H | $CH_3$ | $OCH_3$ | CH | |
| H | - | Q-1 | $(CH_2)_2SCH_2CH_3$ | O | H | $OCH_3$ | $OCH_3$ | CH | |
| H | - | Q-1 | $(CH_2)_2SCH_2CH_3$ | O | H | $OCH_3$ | Cl | CH | |
| H | - | Q-1 | $(CH_2)_2SCH_2CH_3$ | O | H | $CH_3$ | $OCH_3$ | N | |
| H | - | Q-1 | $(CH_2)_2SCH_2CH_3$ | O | H | $OCH_3$ | $OCH_3$ | N | |
| 5-$CH_3$ | - | Q-1 | $CH_3$ | O | H | $CH_3$ | $CH_3$ | CH | |
| 5-$CH_3$ | - | Q-1 | $CH_3$ | O | H | $CH_3$ | $OCH_3$ | CH | |
| 5-$CH_3$ | - | Q-1 | $CH_3$ | O | H | $OCH_3$ | $OCH_3$ | CH | |
| 5-$CH_3$ | - | Q-1 | $CH_3$ | O | H | $OCH_3$ | Cl | CH | |
| 5-$CH_3$ | - | Q-1 | $CH_3$ | O | H | $CH_3$ | $OCH_3$ | N | |
| 5-$CH_3$ | - | Q-1 | $CH_3$ | O | H | $OCH_3$ | $OCH_3$ | N | |
| 3-$CH_3$ | - | Q-1 | $CH_3$ | O | H | $OCH_3$ | $OCH_3$ | CH | 192-197 |
| 3-$CH_3$ | - | Q-1 | $CH_3$ | O | H | $OCH_3$ | $OCH_3$ | N | 201-206 |
| 3-$CH_3$ | - | Q-1 | $CH_3$ | O | H | $OCH_3$ | $CH_3$ | N | 159-173 |
| 3-$CH_3$ | - | Q-1 | $CH_3$ | O | H | $OCH_3$ | $CH_3$ | CH | 219-222 |
| 3-$CH_3$ | - | Q-1 | $CH_3$ | O | H | $CH_3$ | $CH_3$ | CH | 223-226 |
| 3-$CH_3$ | - | Q-1 | $CH_3$ | O | H | $OCH_3$ | Cl | CH | 181-186 |
| 3-$CH_3$ | - | Q-1 | $CH_3$ | O | H | $OC_2H_5$ | $NHCH_3$ | N | 187-190 |
| 4-$CH_3$ | - | Q-1 | $CH_3$ | O | H | $OCH_3$ | $CH_3$ | N | |
| 4-$CH_3$ | - | Q-1 | $CH_3$ | O | H | $OCH_3$ | $OCH_3$ | CH | 214-218 |
| 4-$CH_3$ | - | Q-1 | $CH_3$ | O | H | $OCH_3$ | $CH_3$ | CH | 225-229 |

Table 1 (Cont'd)

| $R_1$ | E | Q | $R_3$ | W | R | X | Y | Z | m.p.(°C) |
|---|---|---|---|---|---|---|---|---|---|
| 4-$CH_3$ | - | Q-1 | $CH_3$ | O | H | $OCH_3$ | $OCH_3$ | N | 191-196 |
| 4-$CH_3$ | - | Q-1 | $CH_3$ | O | H | $CH_3$ | $CH_3$ | CH | 182-196 |
| 4-$CH_3$ | - | Q-1 | $CH_3$ | O | H | $OCH_3$ | Cl | CH | 140-150 |
| 4-$CH_3$ | - | Q-1 | $CH_3$ | O | H | $OC_2H_5$ | $NHCH_3$ | N | 152-160 |
| 6-$CH_3$ | - | Q-1 | $CH_3$ | O | H | $OCH_3$ | $OCH_3$ | N | 197-200 |
| 6-$CH_3$ | - | Q-1 | $CH_3$ | O | H | $OCH_3$ | $CH_3$ | N | 198-202 |
| 6-$CH_3$ | - | Q-1 | $CH_3$ | O | H | $OCH_3$ | $OCH_3$ | CH | 178-180 |
| 6-$CH_3$ | - | Q-1 | $CH_3$ | O | H | $OCH_3$ | $CH_3$ | CH | 180-183 |
| 6-$CH_3$ | - | Q-1 | $CH_3$ | O | H | $CH_3$ | $CH_3$ | CH | 213-215 |
| 6-$CH_3$ | - | Q-1 | $CH_3$ | O | H | $OCH_3$ | Cl | CH | 196-199 |
| 5-Cl | - | Q-1 | $CH_3$ | O | H | $CH_3$ | $CH_3$ | CH | 221-223 |
| 5-Cl | - | Q-1 | $CH_3$ | O | H | $CH_3$ | $OCH_3$ | CH | 207-210 |
| 5-Cl | - | Q-1 | $CH_3$ | O | H | $OCH_3$ | $OCH_3$ | CH | 214-216 |
| 5-Cl | - | Q-1 | $CH_3$ | O | H | $OCH_3$ | Cl | CH | 203-205 |
| 5-Cl | - | Q-1 | $CH_3$ | O | H | $CH_3$ | $OCH_3$ | N | 191-193 |
| 5-Cl | - | Q-1 | $CH_3$ | O | H | $OCH_3$ | $OCH_3$ | N | 175-180 |
| 5-Cl | - | Q-1 | $CH_3$ | O | H | $OC_2H_5$ | $NHCH_3$ | N | 144-158 |
| 5-Cl | - | Q-1 | $CH_3$ | O | H | $OCH_3$ | $CH(OCH_3)_2$ | CH | 180-183 |
| 5-Br | - | Q-1 | $CH_3$ | O | H | $CH_3$ | $CH_3$ | CH | |
| 5-Br | - | Q-1 | $CH_3$ | O | H | $CH_3$ | $OCH_3$ | CH | |
| 5-Br | - | Q-1 | $CH_3$ | O | H | $OCH_3$ | $OCH_3$ | CH | |
| 5-Br | - | Q-1 | $CH_3$ | O | H | $OCH_3$ | Cl | CH | |
| 5-Br | - | Q-1 | $CH_3$ | O | H | $CH_3$ | $OCH_3$ | N | |
| 5-Br | - | Q-1 | $CH_3$ | O | H | $OCH_3$ | $OCH_3$ | N | |
| 5-$OCF_2H$ | - | Q-1 | $CH_3$ | O | H | $CH_3$ | $CH_3$ | CH | |
| 5-$OCF_2H$ | - | Q-1 | $CH_3$ | O | H | $CH_3$ | $OCH_3$ | CH | |
| 5-$OCF_2H$ | - | Q-1 | $CH_3$ | O | H | $OCH_3$ | $OCH_3$ | CH | |
| 5-$OCF_2H$ | - | Q-1 | $CH_3$ | O | H | $OCH_3$ | Cl | CH | |
| 5-$OCF_2H$ | - | Q-1 | $CH_3$ | O | H | $CH_3$ | $OCH_3$ | N | |
| 5-$OCF_2H$ | - | Q-1 | $CH_3$ | O | H | $OCH_3$ | $OCH_3$ | N | |
| 5-$OCH_3$ | - | Q-1 | $CH_3$ | O | H | $CH_3$ | $CH_3$ | CH | |
| 5-$OCH_3$ | - | Q-1 | $CH_3$ | O | H | $CH_3$ | $OCH_3$ | CH | |

Table 1 (Cont'd)

| $R_1$ | E | Q | $R_3$ | W | R | X | Y | Z | m.p.(°C) |
|---|---|---|---|---|---|---|---|---|---|
| 5-$OCH_3$ | - | Q-1 | $CH_3$ | O | H | $OCH_3$ | $OCH_3$ | CH | |
| 5-$OCH_3$ | - | Q-1 | $CH_3$ | O | H | $OCH_3$ | Cl | CH | |
| 5-$OCH_3$ | - | Q-1 | $CH_3$ | O | H | $CH_3$ | $OCH_3$ | N | |
| 5-$OCH_3$ | - | Q-1 | $CH_3$ | O | H | $OCH_3$ | $OCH_3$ | N | |
| 5-$CF_3$ | - | Q-1 | $CH_3$ | O | H | $CH_3$ | $CH_3$ | CH | |
| 5-$CF_3$ | - | Q-1 | $CH_3$ | O | H | $CH_3$ | $OCH_3$ | CH | |
| 5-$CF_3$ | - | Q-1 | $CH_3$ | O | H | $OCH_3$ | $OCH_3$ | CH | |
| 5-$CF_3$ | - | Q-1 | $CH_3$ | O | H | $OCH_3$ | Cl | CH | |
| 5-$CF_3$ | - | Q-1 | $CH_3$ | O | H | $CH_3$ | $OCH_3$ | N | |
| 5-$CF_3$ | - | Q-1 | $CH_3$ | O | H | $OCH_3$ | $OCH_3$ | N | |
| 5-$CH_2OCH_3$ | - | Q-1 | $CH_3$ | O | H | $CH_3$ | $CH_3$ | CH | |
| 5-$CH_2OCH_3$ | - | Q-1 | $CH_3$ | O | H | $CH_3$ | $OCH_3$ | CH | |
| 5-$CH_2OCH_3$ | - | Q-1 | $CH_3$ | O | H | $OCH_3$ | $OCH_3$ | CH | |
| 5-$CH_2OCH_3$ | - | Q-1 | $CH_3$ | O | H | $OCH_3$ | Cl | CH | |
| 5-$CH_2OCH_3$ | - | Q-1 | $CH_3$ | O | H | $CH_3$ | $OCH_3$ | N | |
| 5-$CH_2OCH_3$ | - | Q-1 | $CH_3$ | O | H | $OCH_3$ | $OCH_3$ | N | |
| 6-Cl | - | Q-1 | $CH_3$ | O | H | $CH_3$ | $CH_3$ | CH | |
| 6-Cl | - | Q-1 | $CH_3$ | O | H | $OCH_3$ | $CH_3$ | CH | |
| 6-Cl | - | Q-1 | $CH_3$ | O | H | $OCH_3$ | $OCH_3$ | CH | |
| 6-Cl | - | Q-1 | $CH_3$ | O | H | $OCH_3$ | Cl | CH | |
| 6-Cl | - | Q-1 | $CH_3$ | O | H | $CH_3$ | $OCH_3$ | N | |
| 6-Cl | - | Q-1 | $CH_3$ | O | H | $OCH_3$ | $OCH_3$ | N | |
| 6-$OCH_3$ | - | Q-1 | $CH_3$ | O | H | $CH_3$ | $CH_3$ | CH | |
| 6-$OCH_3$ | - | Q-1 | $CH_3$ | O | H | $OCH_3$ | $CH_3$ | CH | |
| 6-$OCH_3$ | - | Q-1 | $CH_3$ | O | H | $OCH_3$ | $OCH_3$ | CH | |
| 5-$SCH_3$ | - | Q-1 | $CH_3$ | O | H | $CH_3$ | $CH_3$ | CH | |
| 5-$SCH_3$ | - | Q-1 | $CH_3$ | O | H | $CH_3$ | $OCH_3$ | CH | |
| 5-$SCH_3$ | - | Q-1 | $CH_3$ | O | H | $OCH_3$ | $OCH_3$ | CH | |
| 5-$SCH_3$ | - | Q-1 | $CH_3$ | O | H | $OCH_3$ | Cl | CH | |
| 5-$SCH_3$ | - | Q-1 | $CH_3$ | O | H | $CH_3$ | $OCH_3$ | N | |
| 5-$SCH_3$ | - | Q-1 | $CH_3$ | O | H | $OCH_3$ | $OCH_3$ | N | |
| 5-$SCH_3$ | - | Q-1 | $C_2H_5$ | O | H | $CH_3$ | $CH_3$ | CH | |

Table 1 (Cont'd)

| $R_1$ | E | Q | $R_3$ | W | R | X | Y | Z | m.p.(°C) |
|---|---|---|---|---|---|---|---|---|---|
| 5-$SCH_3$ | - | Q-1 | $C_2H_5$ | O | H | $CH_3$ | $OCH_3$ | CH | |
| 5-$SCH_3$ | - | Q-1 | $C_2H_5$ | O | H | $OCH_3$ | $OCH_3$ | CH | |
| 5-$SCH_3$ | - | Q-1 | $C_2H_5$ | O | H | $OCH_3$ | Cl | CH | |
| 5-$SCH_3$ | - | Q-1 | $C_2H_5$ | O | H | $CH_3$ | $OCH_3$ | N | |
| 5-$SCH_3$ | - | Q-1 | $C_2H_5$ | O | H | $OCH_3$ | $OCH_3$ | N | |
| H | - | Q-1 | $CH_3$ | O | $CH_3$ | $CH_3$ | $CH_3$ | CH | |
| H | - | Q-1 | $CH_3$ | O | $CH_3$ | $CH_3$ | $OCH_3$ | CH | |
| H | - | Q-1 | $CH_3$ | O | $CH_3$ | $OCH_3$ | $OCH_3$ | CH | |
| H | - | Q-1 | $CH_3$ | O | $CH_3$ | $OCH_3$ | Cl | CH | |
| H | - | Q-1 | $CH_3$ | O | $CH_3$ | $CH_3$ | $OCH_3$ | N | |
| H | - | Q-1 | $CH_3$ | O | $CH_3$ | $OCH_3$ | $OCH_3$ | N | |
| H | - | Q-1 | $CH_3$ | S | H | $CH_3$ | $CH_3$ | CH | |
| H | - | Q-1 | $CH_3$ | S | H | $CH_3$ | $OCH_3$ | CH | |
| H | - | Q-1 | $CH_3$ | S | H | $OCH_3$ | $OCH_3$ | CH | |
| H | - | Q-1 | $CH_3$ | S | H | $OCH_3$ | Cl | CH | |
| H | - | Q-1 | $CH_3$ | S | H | $CH_3$ | $OCH_3$ | N | |
| H | - | Q-1 | $CH_3$ | S | H | $OCH_3$ | $OCH_3$ | N | |
| H | - | Q-1 | $CH_3$ | O | H | $OC_2H_5$ | $NHCH_3$ | CH | |
| H | - | Q-1 | $CH_3$ | O | H | H | $OCH_3$ | CH | |
| H | - | Q-1 | $CH_3$ | O | H | H | $OC_2H_5$ | CH | |
| H | - | Q-1 | $CH_3$ | O | H | H | $OCF_2H$ | CH | |
| H | - | Q-1 | $CH_3$ | O | H | $CH_3$ | $CF_3$ | CH | |
| H | - | Q-1 | $CH_3$ | O | H | $CH_3$ | $SCH_3$ | CH | |
| H | - | Q-1 | $CH_3$ | O | H | $CH_3$ | Cl | CH | |
| H | - | Q-1 | $CH_3$ | O | H | $CH_3$ | $CH_2OCH_3$ | CH | |
| H | - | Q-1 | $CH_3$ | O | H | $CH_3$ | $OCH_2OCH_3$ | CH | |
| H | - | Q-1 | $CH_3$ | O | H | $CH_3$ | $NHCH_3$ | CH | |
| H | - | Q-1 | $CH_3$ | O | H | $CH_3$ | $N(CH_3)_2$ | CH | |
| H | $CH_2$ | Q-1 | $CH_3$ | O | H | $CH_3$ | $CH_3$ | CH | |
| H | $CH_2$ | Q-1 | $CH_3$ | O | H | $CH_3$ | $OCH_3$ | CH | |
| H | $CH_2$ | Q-1 | $CH_3$ | O | H | $OCH_3$ | $OCH_3$ | CH | |
| H | $CH_2$ | Q-1 | $CH_3$ | O | H | $OCH_3$ | Cl | CH | |

Table 1 (Cont'd)

| $R_1$ | E | Q | $R_3$ | W | R | X | Y | Z | (m.p.(°C) |
|---|---|---|---|---|---|---|---|---|---|
| H | $CH_2$ | Q-1 | $CH_3$ | O | H | $CH_3$ | $OCH_3$ | N | |
| H | $CH_2$ | Q-1 | $CH_3$ | O | H | $OCH_3$ | $OCH_3$ | N | |
| H | O | Q-1 | $CH_3$ | O | H | $CH_3$ | $CH_3$ | CH | |
| H | O | Q-1 | $CH_3$ | O | H | $CH_3$ | $OCH_3$ | CH | |
| H | O | Q-1 | $CH_3$ | O | H | $OCH_3$ | $OCH_3$ | CH | |
| H | O | Q-1 | $CH_3$ | O | H | $CH_3$ | $OCH_3$ | N | |
| H | O | Q-1 | $CH_3$ | O | H | $CH_3$ | $CH_3$ | CH | |
| H | O | Q-1 | $CH_3$ | O | H | $CH_3$ | $OCH_3$ | CH | |
| H | O | Q-1 | $CH_3$ | O | H | $OCH_3$ | $OCH_3$ | CH | |
| H | O | Q-1 | $CH_3$ | O | H | $OCH_3$ | Cl | CH | |
| H | O | Q-1 | $CH_3$ | O | H | $CH_3$ | $OCH_3$ | N | |
| H | O | Q-1 | $CH_3$ | O | H | $OCH_3$ | $OCH_3$ | N | |
| H | - | Q-1 | $CH_3$ | O | H | $OC_2H_5$ | $NHCH_3$ | CH | |
| H | - | Q-1 | $CH_3$ | O | H | H | $OCH_3$ | CH | |
| H | - | Q-1 | $CH_3$ | O | H | H | $OC_2H_5$ | CH | |
| H | - | Q-1 | $CH_3$ | O | H | $CH_3$ | $CF_3$ | CH | |
| H | - | Q-1 | $CH_3$ | O | H | $CH_3$ | $SCH_3$ | CH | |
| H | - | Q-1 | $CH_3$ | O | H | $CH_3$ | $CH_2OCH_3$ | CH | |
| H | - | Q-1 | $CH_3$ | O | H | $CH_3$ | $NHCH_3$ | CH | |
| H | - | Q-1 | $CH_3$ | O | H | $CH_3$ | $N(CH_3)_2$ | CH | |
| H | - | Q-1 | $CH_3$ | O | H | $OC_2H_5$ | $NHCH_3$ | N | |
| H | - | Q-1 | $CH_3$ | O | H | H | $OCH_3$ | N | |
| H | - | Q-1 | $CH_3$ | O | H | H | $OC_2H_5$ | N | |
| H | - | Q-1 | $CH_3$ | O | H | H | $OCF_2H$ | N | |
| H | - | Q-1 | $CH_3$ | O | H | $CH_3$ | $CH_2OCH_3$ | N | |
| H | - | Q-1 | $CH_3$ | O | H | $CH_3$ | $NHCH_3$ | N | |
| H | - | Q-1 | $CH_3$ | O | H | $CH_3$ | $N(CH_3)_2$ | N | |
| 3-(n-$C_3H_7$) | - | Q-1 | $CH_3$ | O | H | $CH_3$ | $CH_3$ | CH | |
| 3-(n-$C_3H_7$) | - | Q-1 | $CH_3$ | O | H | $OCH_3$ | $OCH_3$ | CH | |
| 3-(n-$C_3H_7$) | - | Q-1 | $CH_3$ | O | H | $OCH_3$ | $CH_3$ | CH | |
| 5-$CH_2CH_2Cl$ | - | Q-1 | $CH_3$ | O | H | $OCH_3$ | $CH_3$ | CH | |
| 5-$CH_2CH_2Cl$ | - | Q-1 | $CH_3$ | O | H | $OCH_3$ | $OCH_3$ | CH | |

Table 1 (Cont'd)

| $R_1$ | E | Q | $R_3$ | W | R | X | Y | Z | m.p.(°C) |
|---|---|---|---|---|---|---|---|---|---|
| 5-$OCH_2CH_2CH_3$ | - | Q-1 | $CH_3$ | O | H | $OCH_3$ | $OCH_3$ | CH | |
| 5-$OCH_2CH_2CH_3$ | - | Q-1 | $CH_3$ | O | H | $OCH_3$ | $CH_3$ | CH | |
| 6-$SO_2N(CH_3)_2$ | - | Q-1 | $CH_3$ | O | H | $OCH_3$ | $OCH_3$ | CH | |
| 6-$SO_2N(CH_3)_2$ | - | Q-1 | $CH_3$ | O | H | $OCH_3$ | $CH_3$ | CH | |
| 6-$OCH_2CH_2CH_3$ | - | Q-1 | $CH_3$ | O | H | $OCH_3$ | $CH_3$ | CH | |
| 6-$OCH_2CH_2CH_3$ | - | Q-1 | $CH_3$ | O | H | $OCH_3$ | $OCH_3$ | CH | |
| 6-$SCH_2CH_2CH_3$ | - | Q-1 | $CH_3$ | O | H | $OCH_3$ | $OCH_3$ | CH | |
| 6-$SCH_2CH_2CH_3$ | - | Q-1 | $CH_3$ | O | H | $OCH_3$ | $CH_3$ | CH | |
| 6-$SOCH_3$ | - | Q-1 | $CH_3$ | O | H | $OCH_3$ | $OCH_3$ | CH | |
| 6-$SOCH_3$ | - | Q-1 | $CH_3$ | O | H | $OCH_3$ | $CH_3$ | CH | |
| 6-$SO_2CH_3$ | - | Q-1 | $CH_3$ | O | H | $OCH_3$ | $CH_3$ | CH | |
| 6-$SO_2CH_3$ | - | Q-1 | $CH_3$ | O | H | $OCH_3$ | $OCH_3$ | CH | |
| 5-CN | - | Q-1 | $CH_3$ | O | H | $OCH_3$ | $OCH_3$ | CH | |
| 5-CN | - | Q-1 | $CH_3$ | O | H | $OCH_3$ | $CH_3$ | CH | |
| 5-$CO_2CH_3$ | - | Q-1 | $CH_3$ | O | H | $OCH_3$ | $CH_3$ | CH | |
| 5-$CO_2CH_3$ | - | Q-1 | $CH_3$ | O | H | $OCH_3$ | $OCH_3$ | CH | |
| 5-$OCH_2CH_2Cl$ | - | Q-1 | $CH_3$ | O | H | $OCH_3$ | $OCH_3$ | CH | |
| 5-$OCH_2CH_2Cl$ | - | Q-1 | $CH_3$ | O | H | $OCH_3$ | $CH_3$ | CH | |
| 5-$OCH_2CF_3$ | - | Q-1 | $CH_3$ | O | H | $OCH_3$ | $CH_3$ | CH | |
| 5-$OCH_2CF_3$ | - | Q-1 | $CH_3$ | O | H | $OCH_3$ | $OCH_3$ | CH | |
| 5-$SCH_2CF_3$ | - | Q-1 | $CH_3$ | O | H | $OCH_3$ | $CH_3$ | CH | |
| 5-$SCH_2CF_3$ | - | Q-1 | $CH_3$ | O | H | $OCH_3$ | $OCH_3$ | CH | |
| 5-$NH_2$ | - | Q-1 | $CH_3$ | O | H | $OCH_3$ | $OCH_3$ | CH | |
| 5-$NH_2$ | - | Q-1 | $CH_3$ | O | H | $OCH_3$ | $CH_3$ | CH | |
| 5-$NHC_2H_5$ | - | Q-1 | $CH_3$ | O | H | $OCH_3$ | $CH_3$ | CH | |
| 5-$NHC_2H_5$ | - | Q-1 | $CH_3$ | O | H | $OCH_3$ | $OCH_3$ | CH | |
| 5-$N(CH_3)_2$ | - | Q-1 | $CH_3$ | O | H | $OCH_3$ | $OCH_3$ | CH | |
| 5-$N(CH_3)_2$ | - | Q-1 | $CH_3$ | O | H | $OCH_3$ | $CH_3$ | CH | |
| 5-$CH_2OCH_3$ | - | Q-1 | $CH_3$ | O | H | $OCH_3$ | $CH_3$ | CH | |
| 5-$CH_2OCH_3$ | - | Q-1 | $CH_3$ | O | H | $OCH_3$ | $OCH_3$ | CH | |
| 5-$C_2H_4OCH_3$ | - | Q-1 | $CH_3$ | O | H | $OCH_3$ | $OCH_3$ | CH | |
| 5-$C_2H_4OCH_3$ | - | Q-1 | $CH_3$ | O | H | $OCH_3$ | $CH_3$ | CH | |

Table 1 (Cont'd)

| $R_1$ | E | Q | $R_3$ | W | R | X | Y | Z | m.p.(°C) |
|---|---|---|---|---|---|---|---|---|---|
| $5-C_2H_4OC_2H_5$ | - | Q-1 | $CH_3$ | O | H | $OCH_3$ | $CH_3$ | CH | |
| $5-C_2H_4OC_2H_5$ | - | Q-1 | $CH_3$ | O | H | $OCH_3$ | $OCH_3$ | CH | |
| $5-CH_2OC_2H_4Cl$ | - | Q-1 | $CH_3$ | O | H | $OCH_3$ | $OCH_3$ | CH | |
| $5-CH_2OC_2H_4Cl$ | - | Q-1 | $CH_3$ | O | H | $OCH_3$ | $CH_3$ | CH | |
| $5-CH_2OCH_2CF_3$ | - | Q-1 | $CH_3$ | O | H | $OCH_3$ | $CH_3$ | CH | |
| $5-CH_2OCH_2CF_3$ | - | Q-1 | $CH_3$ | O | H | $OCH_3$ | $OCH_3$ | CH | |
| $5-CH_2SCH_3$ | - | Q-1 | $CH_3$ | O | H | $OCH_3$ | $OCH_3$ | CH | |
| $5-CH_2SCH_3$ | - | Q-1 | $CH_3$ | O | H | $OCH_3$ | $CH_3$ | CH | |
| $5-CH_2SC_2H_4Cl$ | - | Q-1 | $CH_3$ | O | H | $OCH_3$ | $CH_3$ | CH | |
| $5-CH_2SC_2H_4Cl$ | - | Q-1 | $CH_3$ | O | H | $OCH_3$ | $OCH_3$ | CH | |
| $5-CH_2CN$ | - | Q-1 | $CH_3$ | O | H | $OCH_3$ | $OCH_3$ | CH | |
| $5-CH_2CN$ | - | Q-1 | $CH_3$ | O | H | $OCH_3$ | $CH_3$ | CH | |
| H | - | Q-1 | $CH_3$ | O | H | $OCH_3$ | $OCH_2CH_2CH_2CH_3$ | CH | |
| H | - | Q-1 | $CH_3$ | O | H | $OCH_3$ | $OCH_2CH_2Cl$ | CH | |
| H | - | Q-1 | $CH_3$ | O | H | $OCH_3$ | $OCH_2CH_2CH_2Cl$ | CH | |
| H | - | Q-1 | $CH_3$ | O | H | $OCH_3$ | $SCH_2CH_2Cl$ | CH | |
| H | - | Q-1 | $CH_3$ | O | H | $OCH_3$ | $SCHF_2$ | CH | |
| H | - | Q-1 | $CH_3$ | O | H | $OCH_3$ | $SCH_2CH_3$ | CH | |
| H | - | Q-1 | $CH_3$ | O | H | $OCH_3$ | $CH_2OCH_3$ | CH | |
| H | - | Q-1 | $CH_3$ | O | H | $OCH_3$ | $C_2H_4OC_2H_5$ | CH | |
| H | - | Q-1 | $CH_3$ | O | H | $OCH_3$ | $OC_2H_4OC_2H_5$ | CH | |
| H | - | Q-1 | $CH_3$ | O | H | $OCH_3$ | $OCH_2CH{=}CH_2$ | CH | |
| H | - | Q-1 | $CH_3$ | O | H | $OCH_3$ | $OCH_2C{\equiv}CH$ | CH | |
| H | - | Q-1 | $CH_3$ | O | H | $OCH_3$ | $CH_2SCH_3$ | CH | |
| H | - | Q-1 | $CH_3$ | O | H | $OCH_3$ | $C_2H_4SCH_3$ | CH | |
| H | - | Q-1 | $CH_3$ | O | H | $OCH_3$ | $C_2H_4SC_2H_5$ | CH | |
| H | - | Q-1 | $CH_3$ | O | H | $OCH_3$ | $CH_2SO_2CH_3$ | CH | |
| H | - | Q-1 | $CH_3$ | O | H | $OCH_3$ | $C_2H_4SO_2CH_3$ | CH | |
| H | - | Q-1 | $CH_3$ | O | H | $OCH_3$ | $C_2H_4SOCH_3$ | CH | |
| H | - | Q-1 | $CH_3$ | O | H | $OCH_3$ | $CH_2CH_2CH_2Cl$ | CH | |
| H | - | Q-1 | $CH_3$ | O | H | $OCH_3$ | $CH_2C{\equiv}CH$ | CH | |
| H | - | Q-1 | $CH_3$ | O | H | $OCH_3$ | $N_3$ | CH | |

Table 1 (Cont'd)

| $R_1$ | E | Q | $R_3$ | W | R | X | Y | Z | m.p.(°C) |
|---|---|---|---|---|---|---|---|---|---|
| H | - | Q-1 | $CH_3$ | O | H | $OCH_3$ | CN | CH | |
| H | - | Q-1 | $CH_3$ | O | H | $OCH_3$ | $COCH_3$ | CH | |
| H | - | Q-1 | $CH_3$ | O | H | $OCH_3$ | $C(CH_3)(OCH_3)_2$ | CH | |
| H | - | Q-1 | $CH_3$ | O | H | $OCH_3$ | 2-methyldi-oxolan-2-yl | CH | |
| H | - | Q-1 | $CH_3$ | O | H | $OCH_3$ | $N(OCH_3)CH_3$ | CH | |
| H | - | Q-1 | $CH_3$ | O | H | $OC_2H_5$ | $NHCH_3$ | N | 204-208 |
| H | - | Q-2 | $CH_3$ | O | H | $CH_3$ | $CH_3$ | CH | |
| H | - | Q-2 | $CH_3$ | O | H | $CH_3$ | $OCH_3$ | CH | |
| H | - | Q-2 | $CH_3$ | O | H | $OCH_3$ | $OCH_3$ | CH | |
| H | - | Q-2 | $CH_3$ | O | H | $OCH_3$ | Cl | CH | |
| H | - | Q-2 | $CH_3$ | O | H | $CH_3$ | $OCH_3$ | N | |
| H | - | Q-2 | $CH_3$ | O | H | $OCH_3$ | $OCH_3$ | N | |
| H | - | Q-2 | $C_2H_5$ | O | H | $CH_3$ | $CH_3$ | CH | |
| H | - | Q-2 | $C_2H_5$ | O | H | $CH_3$ | $OCH_3$ | CH | |
| H | - | Q-2 | $C_2H_5$ | O | H | $OCH_3$ | $OCH_3$ | CH | |
| H | - | Q-2 | $C_2H_5$ | O | H | $OCH_3$ | Cl | CH | |
| H | - | Q-2 | $C_2H_5$ | O | H | $CH_3$ | $OCH_3$ | N | |
| H | - | Q-2 | $C_2H_5$ | O | H | $OCH_3$ | $OCH_3$ | N | |
| H | - | Q-2 | $CH(CH_3)_2$ | O | H | $CH_3$ | $CH_3$ | CH | |
| H | - | Q-2 | $CH(CH_3)_2$ | O | H | $CH_3$ | $OCH_3$ | CH | |
| H | - | Q-2 | $CH(CH_3)_2$ | O | H | $OCH_3$ | $OCH_3$ | CH | |
| H | - | Q-2 | $CH(CH_3)_2$ | O | H | $OCH_3$ | Cl | CH | |
| H | - | Q-2 | $CH(CH_3)_2$ | O | H | $CH_3$ | $OCH_3$ | N | |
| H | - | Q-2 | $CH(CH_3)_2$ | O | H | $OCH_3$ | $OCH_3$ | N | |
| H | - | Q-2 | $CH(CH_3)_2$ | O | H | $CH_3$ | $CH_3$ | CH | |
| H | - | Q-2 | $CH(CH_3)_2$ | O | H | $CH_3$ | $OCH_3$ | CH | |
| H | - | Q-2 | $CH(CH_3)_2$ | O | H | $OCH_3$ | $OCH_3$ | CH | |
| H | - | Q-2 | $CH(CH_3)_2$ | O | H | $OCH_3$ | Cl | CH | |
| H | - | Q-2 | $CH(CH_3)_2$ | O | H | $CH_3$ | $OCH_3$ | N | |
| H | - | Q-2 | $CH(CH_3)_2$ | O | H | $OCH_3$ | $OCH_3$ | N | |

Table 1 (Cont'd)

| $R_1$ | E | Q | $R_3$ | W | R | X | Y | Z | m.p.(°C) |
|---|---|---|---|---|---|---|---|---|---|
| H | - | Q-1 | $CH_2C{\equiv}CH$ | O | H | $CH_3$ | $CH_3$ | CH | |
| H | - | Q-1 | $CH_2C{\equiv}CH$ | O | H | $CH_3$ | $OCH_3$ | CH | |
| H | - | Q-1 | $CH_2C{\equiv}CH$ | O | H | $OCH_3$ | $OCH_3$ | CH | |
| H | - | Q-1 | $CH_2C{\equiv}CH$ | O | H | $OCH_3$ | Cl | CH | |
| H | - | Q-1 | $CH_2C{\equiv}CH$ | O | H | $CH_3$ | $OCH_3$ | N | |
| H | - | Q-1 | $CH_2C{\equiv}CH$ | O | H | $OCH_3$ | $OCH_3$ | N | |
| H | - | Q-1 | $CH_3$ | O | H | $OCH_3$ | $OCF_2H$ | CH | 160-165 |
| H | - | Q-1 | $OCH_3$ | O | H | $CH_3$ | $OCF_2H$ | CH | 195-198 |
| 5-$CH_3$ | - | Q-1 | $CH_3$ | O | H | $OCH_3$ | $OCF_2H$ | CH | 168-172 |
| 5-$CH_3$ | - | Q-1 | $CH_3$ | O | H | $CH_3$ | $OCF_2H$ | CH | 90-93 |
| H | - | Q-2 | $CH_2OCH_3$ | O | H | $CH_3$ | $CH_3$ | CH | |
| H | - | Q-2 | $CH_2OCH_3$ | O | H | $CH_3$ | $OCH_3$ | CH | |
| H | - | Q-2 | $CH_2OCH_3$ | O | H | $OCH_3$ | $OCH_3$ | CH | |
| H | - | Q-2 | $CH_2OCH_3$ | O | H | $OCH_3$ | Cl | CH | |
| H | - | Q-2 | $CH_2OCH_3$ | O | H | $CH_3$ | $OCH_3$ | N | |
| H | - | Q-2 | $CH_2OCH_3$ | O | H | $OCH_3$ | $OCH_3$ | N | |
| 5-$OCF_2H$ | - | Q-2 | $CH_3$ | O | H | $CH_3$ | $CH_3$ | CH | |
| 5-$OCF_2H$ | - | Q-2 | $CH_3$ | O | H | $CH_3$ | $OCH_3$ | CH | |
| 5-$OCF_2H$ | - | Q-2 | $CH_3$ | O | H | $OCH_3$ | $OCH_3$ | CH | |
| 5-$OCF_2H$ | - | Q-2 | $CH_3$ | O | H | $OCH_3$ | Cl | CH | |
| 5-$OCF_2H$ | - | Q-2 | $CH_3$ | O | H | $CH_3$ | $OCH_3$ | N | |
| 5-$OCF_2H$ | - | Q-2 | $CH_3$ | O | H | $OCH_3$ | $OCH_3$ | N | |
| 5-$OCH_3$ | - | Q-2 | $CH_3$ | O | H | $CH_3$ | $CH_3$ | CH | |
| 5-$OCH_3$ | - | Q-2 | $CH_3$ | O | H | $CH_3$ | $OCH_3$ | CH | |
| 5-$OCH_3$ | - | Q-2 | $CH_3$ | O | H | $OCH_3$ | $OCH_3$ | CH | |
| 5-$OCH_3$ | - | Q-2 | $CH_3$ | O | H | $OCH_3$ | Cl | CH | |
| 5-$OCH_3$ | - | Q-2 | $CH_3$ | O | H | $CH_3$ | $OCH_3$ | N | |
| 5-$OCH_3$ | - | Q-2 | $CH_3$ | O | H | $OCH_3$ | $OCH_3$ | N | |
| 6-Cl | - | Q-2 | $CH_3$ | O | H | $CH_3$ | $CH_3$ | CH | |
| 6-Cl | - | Q-2 | $CH_3$ | O | H | $OCH_3$ | $CH_3$ | CH | |

Table 1 (Cont'd)

| $R_1$ | E | Q | $R_3$ | W | R | X | Y | Z | m.p.(°C) |
|---|---|---|---|---|---|---|---|---|---|
| 6-Cl | - | Q-2 | $CH_3$ | O | H | $OCH_3$ | $OCH_3$ | CH | |
| 6-$OCH_3$ | - | Q-2 | $CH_3$ | O | H | $CH_3$ | $CH_3$ | CH | |
| 6-$OCH_3$ | - | Q-2 | $CH_3$ | O | H | $OCH_3$ | $CH_3$ | CH | |
| 6-$OCH_3$ | - | Q-2 | $CH_3$ | O | H | $OCH_3$ | $OCH_3$ | CH | |
| 5-$SCH_3$ | - | Q-2 | $CH_3$ | O | H | $CH_3$ | $CH_3$ | CH | |
| 5-$SCH_3$ | - | Q-2 | $CH_3$ | O | H | $CH_3$ | $OCH_3$ | CH | |
| 5-$SCH_3$ | - | Q-2 | $CH_3$ | O | H | $OCH_3$ | $OCH_3$ | CH | |
| 5-$SCH_3$ | - | Q-2 | $CH_3$ | O | H | $OCH_3$ | Cl | CH | |
| 5-$SCH_3$ | - | Q-2 | $CH_3$ | O | H | $CH_3$ | $OCH_3$ | N | |
| 5-$SCH_3$ | - | Q-2 | $CH_3$ | O | H | $OCH_3$ | $OCH_3$ | N | |
| 5-$SCH_3$ | - | Q-2 | $C_2H_5$ | O | H | $CH_3$ | $CH_3$ | CH | |
| 5-$SCH_3$ | - | Q-2 | $C_2H_5$ | O | H | $CH_3$ | $OCH_3$ | CH | |
| 5-$SCH_3$ | - | Q-2 | $C_2H_5$ | O | H | $OCH_3$ | $OCH_3$ | CH | |
| 5-$SCH_3$ | - | Q-2 | $C_2H_5$ | O | H | $OCH_3$ | Cl | CH | |
| 5-$SCH_3$ | - | Q-2 | $C_2H_5$ | O | H | $CH_3$ | $OCH_3$ | N | |
| 5-$SCH_3$ | - | Q-2 | $C_2H_5$ | O | H | $OCH_3$ | $OCH_3$ | N | |
| H | - | Q-2 | $CH_3$ | O | $CH_3$ | $CH_3$ | $CH_3$ | CH | |
| H | - | Q-2 | $CH_3$ | O | $CH_3$ | $CH_3$ | $OCH_3$ | CH | |
| H | - | Q-2 | $CH_3$ | O | $CH_3$ | $OCH_3$ | $OCH_3$ | CH | |
| H | - | Q-2 | $CH_3$ | O | $CH_3$ | $OCH_3$ | Cl | CH | |
| H | - | Q-2 | $CH_3$ | O | $CH_3$ | $CH_3$ | $OCH_3$ | N | |
| H | - | Q-2 | $CH_3$ | O | $CH_3$ | $OCH_3$ | $OCH_3$ | N | |
| H | - | Q-2 | $CH_3$ | S | H | $CH_3$ | $CH_3$ | CH | |
| H | - | Q-2 | $CH_3$ | S | H | $CH_3$ | $OCH_3$ | CH | |
| H | - | Q-2 | $CH_3$ | S | H | $OCH_3$ | $OCH_3$ | CH | |
| H | - | Q-2 | $CH_3$ | S | H | $OCH_3$ | Cl | CH | |
| H | - | Q-2 | $CH_3$ | S | H | $CH_3$ | $OCH_3$ | N | |
| H | - | Q-2 | $CH_3$ | S | H | $OCH_3$ | $OCH_3$ | N | |
| H | - | Q-2 | $CH_3$ | O | H | $OC_2H_5$ | $NHCH_3$ | N | |
| H | - | Q-2 | $CH_3$ | O | H | H | $OCH_3$ | CH | |
| H | - | Q-2 | $CH_3$ | O | H | H | $OC_2H_5$ | CH | |
| H | - | Q-2 | $CH_3$ | O | H | H | $OCF_2H$ | CH | |

Table 1 (Cont'd)

| $R_1$ | E | Q | $R_3$ | W | R | X | Y | Z | m.p.(°C) |
|---|---|---|---|---|---|---|---|---|---|
| H | - | Q-2 | $CH_3$ | O | H | $CH_3$ | $SCF_2H$ | CH | |
| H | - | Q-2 | $CH_3$ | O | H | $CH_3$ | Cl | CH | |
| H | - | Q-2 | $CH_3$ | O | H | $CH_3$ | $CH_2OCH_3$ | CH | |
| H | - | Q-2 | $CH_3$ | O | H | $CH_3$ | $OCH_2OCH_3$ | CH | |
| H | - | Q-2 | $CH_3$ | O | H | $OC_2H_5$ | $NHCH_3$ | N | |
| H | - | Q-2 | $CH_3$ | O | H | H | $OCH_3$ | CH | |
| H | - | Q-2 | $CH_3$ | O | H | H | $OC_2H_5$ | CH | |
| H | - | Q-2 | $CH_3$ | O | H | $CH_3$ | $CF_3$ | CH | |
| H | - | Q-2 | $CH_3$ | O | H | $CH_3$ | $SCH_3$ | CH | |
| H | - | Q-2 | $CH_3$ | O | H | $CH_3$ | Cl | CH | |
| H | - | Q-2 | $CH_3$ | O | H | $CH_3$ | $CH_2OCH_3$ | CH | |
| H | - | Q-2 | $CH_3$ | O | H | $CH_3$ | $OCH_2OCH_3$ | CH | |
| H | - | Q-2 | $CH_3$ | O | H | $OCH_3$ | $OC_2H_5$ | N | |
| H | - | Q-2 | $CH_3$ | O | H | $OCH_3$ | $OCH(CH_3)_2$ | N | |
| H | - | Q-2 | $CH_3$ | O | H | $OCH_3$ | $OCH_2CF_3$ | N | |
| H | - | Q-2 | $CH_3$ | O | H | $OCH_3$ | $CF_3$ | N | |
| H | - | Q-2 | $CH_3$ | O | H | $OCH_3$ | $O(CH_2)_2OCH_3$ | N | |
| H | - | Q-2 | $CH_3$ | O | H | $OCH_3$ | $C{\equiv}C-CH_3$ | N | |
| H | - | Q-2 | $CH_3$ | O | H | $OCH_3$ | $N(CH_3)OCH_3$ | N | |
| H | - | Q-2 | $C_2H_5$ | O | H | $CH_3$ | $CH_3$ | CH | |
| H | - | Q-2 | $C_2H_5$ | O | H | $CH_3$ | $OCH_3$ | CH | |
| H | - | Q-2 | $C_2H_5$ | O | H | $OCH_3$ | $OCH_3$ | CH | |
| H | - | Q-2 | $C_2H_5$ | O | H | $OCH_3$ | Cl | CH | |
| H | - | Q-2 | $C_2H_5$ | O | H | $CH_3$ | $OCH_3$ | N | |
| 6-$CH_3$ | - | Q-2 | $CH_3$ | O | H | $CH_3$ | $CH_3$ | CH | |
| 6-$CH_3$ | - | Q-2 | $CH_3$ | O | H | $CH_3$ | $OCH_3$ | CH | |
| 6-$CH_3$ | - | Q-2 | $CH_3$ | O | H | $OCH_3$ | $OCH_3$ | CH | |
| 6-$CH_3$ | - | Q-2 | $CH_3$ | O | H | $OCH_3$ | Cl | CH | |
| 6-$CH_3$ | - | Q-2 | $CH_3$ | O | H | $OCH_3$ | $CH_3$ | N | |

Table Ia

General Structure 1

| R1 | E | Q | R4 | W | R | X | Y | Z | m.p.(°C) |
|---|---|---|---|---|---|---|---|---|---|
| H | – | Q-3 | H | O | H | $CH_3$ | $CH_3$ | CH | 214 dec |
| H | – | Q-3 | H | O | H | $CH_3$ | $OCH_3$ | CH | 204-205 |
| H | – | Q-3 | H | O | H | $OCH_3$ | $OCH_3$ | CH | 187-188 |
| H | – | Q-3 | H | O | H | $OCH_3$ | Cl | CH | 195-196 |
| H | – | Q-3 | H | O | H | $CH_3$ | OCH | N | 199-202 |
| H | – | Q-3 | H | O | H | $OCH_3$ | $OCH_3$ | N | 191-192 |
| H | – | Q-3 | $CH_3$ | O | H | $CH_3$ | $CH_3$ | CH | |
| H | – | Q-3 | $CH_3$ | O | – | $OC_2H_5$ | $NHCH_3$ | N | >200 dec. |
| H | – | Q-3 | $CH_3$ | O | H | $CH_3$ | $OCH_3$ | CH | 221-223 |
| H | – | Q-3 | $CH_3$ | O | H | $OCH_3$ | $OCH_3$ | CH | 213-216 |
| H | – | Q-3 | $CH_3$ | O | H | $OCH_3$ | Cl | CH | 208-210 |
| H | – | Q-3 | $CH_3$ | O | H | $CH_3$ | $OCH_3$ | N | 207-209 |
| H | – | Q-3 | $CH_3$ | O | H | $OCH_3$ | $OCH_3$ | N | 193-196 |
| H | – | Q-3 | $C_2H_5$ | O | H | $CH_3$ | $CH_3$ | CH | 217-220 |
| H | – | Q-3 | $C_2H_5$ | O | H | $CH_2$ | $OCH_3$ | CH | 224-228 |
| H | – | Q-3 | $C_2H_5$ | O | H | $OCH_3$ | $OCH_3$ | CH | 193-196 |
| H | – | Q-3 | $C_2H_5$ | O | H | $OCH_3$ | Cl | CH | 200-204 |
| H | – | Q-3 | $C_2H_5$ | O | H | $CH_3$ | $OCH_3$ | N | 174-179 |
| H | – | Q-3 | $C_2H_5$ | O | H | $OCH_3$ | $OCH_3$ | N | 194-197 |
| H | – | Q-3 | $CH(CH_3)_2$ | O | H | $CH_3$ | $CH_3$ | CH | |
| H | – | Q-3 | $CH(CH_3)_2$ | O | H | $CH_3$ | $OCH_3$ | CH | |
| H | – | Q-3 | $CH(CH_3)_2$ | O | H | $OCH_3$ | $OCH_3$ | CH | |
| H | – | Q-3 | $CH(CH_3)_2$ | O | H | $OCH_3$ | Cl | CH | |
| H | – | Q-3 | $CH(CH_3)_2$ | O | H | $CH_3$ | $OCH_3$ | N | |
| H | – | Q-3 | $CH(CH_3)_2$ | O | H | $OCH_3$ | $OCH_3$ | N | |
| H | – | Q-3 | cyclopropyl | O | H | $CH_3$ | $CH_3$ | CH | |
| H | – | Q-3 | cyclopropyl | O | H | $CH_3$ | $OCH_3$ | CH | |
| H | – | Q-3 | cyclopropyl | O | H | $OCH_3$ | $OCH_3$ | CH | |
| H | – | Q-3 | cyclopropyl | O | H | $OCH_3$ | Cl | CH | |
| H | – | Q-3 | cyclopropyl | O | H | $CH_3$ | $OCH_3$ | N | |
| H | – | Q-3 | cyclopropyl | O | H | $OCH_2$ | $OCH_2$ | N | |
| H | – | Q-3 | $CH_2Cl$ | O | H | $CH_3$ | $CH_3$ | CH | |

Table 1a (Cont'd)

| $R_1$ | E | Q | $R_4$ | W | R | X | Y | Z | m.p.(°C) |
|---|---|---|---|---|---|---|---|---|---|
| H | - | Q-3 | $CH_2Cl$ | O | H | $CH_3$ | $OCH_3$ | CH | |
| H | - | Q-3 | $CH_2Cl$ | O | H | $OCH_3$ | $OCH_3$ | CH | |
| H | - | Q-3 | $CH_2Cl$ | O | H | $OCH_3$ | Cl | CH | |
| H | - | Q-3 | $CH_2Cl$ | O | H | $CH_3$ | $OCH_3$ | N | |
| H | - | Q-3 | $CH_2Cl$ | O | H | $OCH_3$ | $OCH_3$ | N | |
| H | - | Q-3 | $CF_3$ | O | H | $CH_3$ | $CH_3$ | CH | |
| H | - | Q-3 | $CF_3$ | O | H | $CH_3$ | $OCH_3$ | CH | |
| H | - | Q-3 | $CF_3$ | O | H | $OCH_3$ | $OCH_3$ | CH | |
| H | - | Q-3 | $CF_3$ | O | H | $OCH_3$ | Cl | CH | |
| H | - | Q-3 | $CF_3$ | O | H | $CH_3$ | $OCH_3$ | N | |
| H | - | Q-3 | $CF_3$ | O | H | $OCH_3$ | $OCH_3$ | N | |
| H | - | Q-3 | $OCH_3$ | O | H | $CH_3$ | $CH_3$ | CH | |
| H | - | Q-3 | $OCH_3$ | O | H | $CH_3$ | $OCH_3$ | CH | |
| H | - | Q-3 | $OCH_3$ | O | H | $OCH_3$ | $OCH_3$ | CH | |
| H | - | Q-3 | $OCH_3$ | O | H | $OCH_3$ | Cl | CH | |
| H | - | Q-3 | $OCH_3$ | O | H | $CH_3$ | $OCH_3$ | N | |
| H | - | Q-3 | $OCH_3$ | O | H | $OCH_3$ | $OCH_3$ | N | |
| H | - | Q-3 | $OC_2H_5$ | O | H | $CH_3$ | $CH_3$ | CH | |
| H | - | Q-3 | $OC_2H_5$ | O | H | $CH_3$ | $OCH_3$ | CH | |
| H | - | Q-3 | $OC_2H_5$ | O | H | $OCH_3$ | $OCH_3$ | CH | |
| H | - | Q-3 | $OC_2H_5$ | O | H | $OCH_3$ | Cl | CH | |
| H | - | Q-3 | $OC_2H_5$ | O | H | $CH_3$ | $OCH_3$ | N | |
| H | - | Q-3 | $OC_2H_5$ | O | H | $OCH_3$ | $OCH_3$ | N | |
| H | - | Q-3 | $SCH_3$ | O | H | $CH_3$ | $CH_3$ | CH | |
| H | - | Q-3 | $SCH_3$ | O | H | $CH_3$ | $OCH_3$ | CH | |
| H | - | Q-3 | $SCH_3$ | O | H | $OCH_3$ | $OCH_3$ | CH | |
| H | - | Q-3 | $SCH_3$ | O | H | $OCH_3$ | Cl | CH | |
| H | - | Q-3 | $SCH_3$ | O | H | $CH_3$ | $OCH_3$ | N | |
| H | - | Q-3 | $SCH_3$ | O | H | $OCH_3$ | $OCH_3$ | N | |
| H | - | Q-3 | $N(CH_3)_2$ | O | H | $CH_3$ | $CH_3$ | CH | |
| H | - | Q-3 | $N(CH_3)_2$ | O | H | $CH_3$ | $OCH_3$ | CH | |
| H | - | Q-3 | $N(CH_3)_2$ | O | H | $OCH_3$ | $OCH_3$ | CH | |

Table 1a (Cont'd)

| $R_1$ | E | Q | $R_4$ | W | R | X | Y | Z | m.p.(°C) |
|---|---|---|---|---|---|---|---|---|---|
| H | - | Q-3 | $N(CH_3)_2$ | O | H | $OCH_3$ | Cl | CH | |
| H | - | Q-3 | $N(CH_3)_2$ | O | H | $CH_3$ | $OCH_3$ | N | |
| H | - | Q-3 | $N(CH_3)_2$ | O | H | $OCH_3$ | $OCH_3$ | N | |
| 5-$CH_3$ | - | Q-3 | $CH_3$ | O | H | $CH_3$ | $CH_3$ | CH | |
| 5-$CH_3$ | - | Q-3 | $CH_3$ | O | H | $CH_3$ | $OCH_3$ | CH | |
| 5-$CH_3$ | - | Q-3 | $CH_3$ | O | H | $OCH_3$ | $OCH_3$ | CH | 177-186 |
| 5-$CH_3$ | - | Q-3 | $CH_3$ | O | H | $OCH_3$ | Cl | CH | 207-208 |
| 5-$CH_3$ | - | Q-3 | $CH_3$ | O | H | $CH_3$ | $OCH_3$ | N | 200-202 |
| 5-$CH_3$ | - | Q-3 | $CH_3$ | O | H | $OCH_3$ | $OCH_3$ | N | |
| 5-Cl | - | Q-3 | $CH_3$ | O | H | $CH_3$ | $CH_3$ | CH | 227-230 |
| 5-Cl | - | Q-3 | $CH_3$ | O | H | $CH_3$ | $OCH_3$ | CH | 173-180 |
| 5-Cl | - | Q-3 | $CH_3$ | O | H | $OCH_3$ | $OCH_3$ | CH | 216-220 |
| 5-Cl | - | Q-3 | $CH_3$ | O | H | $OCH_3$ | Cl | CH | 227-230 |
| 5-Cl | - | Q-3 | $CH_3$ | O | H | $CH_3$ | $OCH_3$ | N | 188-195 |
| 5-Cl | - | Q-3 | $CH_3$ | O | H | $OCH_3$ | $OCH_3$ | N | 192-194 |
| 5-Cl | - | Q-3 | $CH_3$ | O | H | $OC_2H_5$ | $NHCH_3$ | N | 181-185 |
| 5-Br | - | Q-3 | $CH_3$ | O | H | $CH_3$ | $CH_3$ | CH | |
| 5-Br | - | Q-3 | $CH_3$ | O | H | $CH_3$ | $OCH_3$ | CH | |
| 5-Br | - | Q-3 | $CH_3$ | O | H | $OCH_3$ | $OCH_3$ | CH | |
| 5-Br | - | Q-3 | $CH_3$ | O | H | $OCH_3$ | Cl | CH | |
| 5-Br | - | Q-3 | $CH_3$ | O | H | $CH_3$ | $OCH_3$ | N | |
| 5-Br | - | Q-3 | $CH_3$ | O | H | $OCH_3$ | $OCH_3$ | H | |
| 5-$OCF_2H$ | - | Q-3 | $CH_3$ | O | H | $CH_3$ | $CH_3$ | CH | |
| 5-$OCF_2H$ | - | Q-3 | $CH_3$ | O | H | $CH_3$ | $OCH_3$ | CH | |
| 5-$OCF_2H$ | - | Q-3 | $CH_3$ | O | H | $OCH_3$ | $OCH_3$ | CH | |
| 5-$OCF_2H$ | - | Q-3 | $CH_3$ | O | H | $OCH_3$ | Cl | CH | |
| 5-$OCF_2H$ | - | Q-3 | $CH_3$ | O | H | $CH_3$ | $OCH_3$ | N | |
| 5-$OCF_2H$ | - | Q-3 | $CH_3$ | O | H | $OCH_3$ | $OCH_3$ | N | |
| 5-$OCH_3$ | - | Q-3 | $CH_3$ | O | H | $CH_3$ | $CH_3$ | CH | |
| 5-$OCH_3$ | - | Q-3 | $CH_3$ | O | H | $CH_3$ | $OCH_3$ | CH | |
| 5-$OCH_3$ | - | Q-3 | $CH_3$ | O | H | $OCH_3$ | $OCH_3$ | CH | |
| 5-$OCH_3$ | - | Q-3 | $CH_3$ | O | H | $OCH_3$ | Cl | CH | |

Table 1a (Cont'd)

| $R_1$ | E | Q | $R_4$ | W | R | X | Y | Z | m.p.(°C) |
|---|---|---|---|---|---|---|---|---|---|
| 5-$OCH_3$ | - | Q-3 | $CH_3$ | O | H | $CH_3$ | $OCH_3$ | N | |
| 5-$OCH_3$ | - | Q-3 | $CH_3$ | O | H | $OCH_3$ | $OCH_3$ | N | |
| 5-$CF_3$ | - | Q-3 | $CH_3$ | O | H | $CH_3$ | $CH_3$ | CH | |
| 5-$CF_3$ | - | Q-3 | $CH_3$ | O | H | $CH_3$ | $OCH_3$ | CH | |
| 5-$CF_3$ | - | Q-3 | $CH_3$ | O | H | $OCH_3$ | $OCH_3$ | CH | |
| 5-$CF_3$ | - | Q-3 | $CH_3$ | O | H | $OCH_3$ | Cl | CH | |
| 5-$CF_3$ | - | Q-3 | $CH_3$ | O | H | $CH_3$ | $OCH_3$ | N | |
| 5-$CF_3$ | - | Q-3 | $CH_3$ | O | H | $OCH_3$ | $OCH_3$ | N | |
| 5-$SCH_3$ | - | Q-3 | $CH_3$ | O | H | $CH_3$ | $CH_3$ | CH | |
| 5-$SCH_3$ | - | Q-3 | $CH_3$ | O | H | $CH_3$ | $OCH_3$ | CH | |
| 5-$SCH_3$ | - | Q-3 | $CH_3$ | O | H | $OCH_3$ | $OCH_3$ | CH | |
| 5-$SCH_3$ | - | Q-3 | $CH_3$ | O | H | $OCH_3$ | Cl | CH | |
| 5-$SCH_3$ | - | Q-3 | $CH_3$ | O | H | $CH_3$ | $OCH_3$ | N | |
| 5-$SCH_3$ | - | Q-3 | $CH_3$ | O | H | $OCH_3$ | $OCH_3$ | N | |
| 5-$CH_2OCH_3$ | - | Q-3 | $CH_3$ | O | H | $CH_3$ | $CH_3$ | CH | |
| 5-$CH_2OCH_3$ | - | Q-3 | $CH_3$ | O | H | $CH_3$ | $OCH_3$ | CH | |
| 5-$CH_2OCH_3$ | - | Q-3 | $CH_3$ | O | H | $OCH_3$ | $OCH_3$ | CH | |
| 5-$CH_2OCH_3$ | - | Q-3 | $CH_3$ | O | H | $OCH_3$ | Cl | CH | |
| 5-$CH_2OCH_3$ | - | Q-3 | $CH_3$ | O | H | $CH_3$ | $OCH_3$ | N | |
| 5-$CH_2OCH_3$ | - | Q-3 | $CH_3$ | O | H | $OCH_3$ | $OCH_3$ | N | |
| 5-$OCH_2CH_3$ | - | Q-3 | $CH_3$ | O | H | $CH_3$ | $CH_3$ | CH | |
| 5-$OCH_2CH_3$ | - | Q-3 | $CH_3$ | O | H | $CH_3$ | $OCH_3$ | CH | |
| 5-$OCH_2CH_3$ | - | Q-3 | $CH_3$ | O | H | $OCH_3$ | $OCH_3$ | CH | |
| 5-$OCH_2CH_3$ | - | Q-3 | $CH_3$ | O | H | $OCH_3$ | Cl | CH | |
| 5-$OCH_2CH_3$ | - | Q-3 | $CH_3$ | O | H | $CH_3$ | $OCH_3$ | N | |
| 5-$OCH_2CH_3$ | - | Q-3 | $CH_3$ | O | H | $OCH_3$ | $OCH_3$ | N | |
| H | - | Q-3 | $CH_2OCH_3$ | O | H | $CH_3$ | $CH_3$ | CH | |
| H | - | Q-3 | $CH_2OCH_3$ | O | H | $CH_3$ | $OCH_3$ | CH | |
| H | - | Q-3 | $CH_2OCH_3$ | O | H | $OCH_3$ | $OCH_3$ | CH | |
| H | - | Q-3 | $CH_2OCH_3$ | O | H | $OCH_3$ | Cl | CH | |
| H | - | Q-3 | $CH_3OCH_3$ | O | H | $OCH_3$ | $CH_3$ | N | |
| H | - | Q-3 | $CH_3OCH_3$ | O | H | $OCH_3$ | $OCH_3$ | N | |

Table Ia (Cont'd)

| $R_1$ | E | Q | $R_4$ | W | R | X | Y | Z | m.p.(°C) |
|---|---|---|---|---|---|---|---|---|---|
| H | – | Q-3 | $CH_3$ | O | H | cyclopropyl | $OCH_3$ | CH | |
| H | – | Q-3 | $CH_3$ | O | H | $OCH_3$ | $N_3$ | CH | |
| H | – | Q-3 | $CH_3$ | O | H | $OCH_3$ | CN | CH | |
| H | – | Q-3 | $CH_3$ | O | H | $OCH_3$ | $C(O)CH_3$ | CH | |
| H | – | Q-3 | $CH_3$ | O | H | $OCH_3$ | $CH(OCH_3)_2$ | CH | |
| H | – | Q-3 | $CH_3$ | O | H | $OCH_3$ | 1,3-dioxo-lan-2-yl | CH | |
| H | – | Q-3 | $CH_3$ | O | H | $OCH_3$ | 1,3-diox-an-2-yl | CH | |
| H | $CH_2$ | Q-3 | $CH_3$ | O | H | $CH_3$ | $CH_3$ | CH | |
| H | $CH_2$ | Q-3 | $CH_3$ | O | H | $CH_3$ | $OCH_3$ | CH | |
| H | $CH_2$ | Q-3 | $CH_3$ | O | H | $OCH_3$ | $OCH_3$ | CH | |
| H | $CH_2$ | Q-3 | $CH_3$ | O | H | $OCH_3$ | Cl | CH | |
| H | $CH_2$ | Q-3 | $CH_3$ | O | H | $CH_3$ | $OCH_3$ | N | |
| H | $CH_2$ | Q-3 | $CH_3$ | O | H | $OCH_3$ | $OCH_3$ | N | |
| H | O | Q-3 | $CH_3$ | O | H | $CH_3$ | $CH_3$ | CH | |
| H | O | Q-3 | $CH_3$ | O | H | $CH_3$ | $OCH_3$ | CH | |
| H | O | Q-3 | $CH_3$ | O | H | $OCH_3$ | $OCH_3$ | CH | |
| H | O | Q-3 | $CH_3$ | O | H | $OCH_3$ | Cl | CH | |
| H | O | Q-3 | $CH_3$ | O | H | $CH_3$ | $OCH_3$ | N | |
| H | O | Q-3 | $CH_3$ | O | H | $OCH_3$ | $OCH_3$ | N | |
| H | – | Q-3 | $CH_3$ | S | H | $CH_3$ | $CH_3$ | CH | |
| H | – | Q-3 | $CH_3$ | S | H | $CH_3$ | $OCH_3$ | CH | |
| H | – | Q-3 | $CH_3$ | S | H | $OCH_3$ | $OCH_3$ | CH | |
| H | – | Q-3 | $CH_3$ | S | H | $OCH_3$ | Cl | CH | |
| H | – | Q-3 | $CH_3$ | S | H | $CH_3$ | $OCH_3$ | N | |
| H | – | Q-3 | $CH_3$ | S | H | $OCH_3$ | $OCH_3$ | N | |
| H | – | Q-3 | $CH_3$ | O | $CH_3$ | $CH_3$ | $CH_3$ | CH | |
| H | – | Q-3 | $CH_3$ | O | $CH_3$ | $CH_3$ | $OCH_3$ | CH | |
| H | – | Q-3 | $CH_3$ | O | $CH_3$ | $OCH_3$ | $OCH_3$ | CH | |
| H | – | Q-3 | $CH_3$ | O | $CH_3$ | $OCH_3$ | Cl | CH | |
| H | – | Q-3 | $CH_3$ | O | $CH_3$ | $CH_3$ | $OCH_3$ | N | |

Table 1a (Cont'd,

| $R_1$ | E | Q | $R_4$ | W | R | X | Y | Z | m.p.(°C) |
|---|---|---|---|---|---|---|---|---|---|
| H | - | Q-3 | $CH_3$ | O | $CH_3$ | $OCH_3$ | $OCH_3$ | N | |
| H | - | Q-3 | $CH_3$ | O | H | $OC_2H_5$ | $NHCH_3$ | CH | |
| H | - | Q-3 | $CH_3$ | O | H | H | $OCH_3$ | CH | |
| H | - | Q-3 | $CH_3$ | O | H | H | $OC_2H_5$ | CH | |
| H | - | Q-3 | $CH_3$ | O | H | H | $OCF_2H$ | CH | |
| H | - | Q-3 | $CH_3$ | O | H | $CH_3$ | $SCF_2H$ | CH | |
| H | - | Q-3 | $CH_3$ | O | H | $CH_3$ | $CH_2OCH_3$ | CH | |
| H | - | Q-3 | $CH_3$ | O | H | $OCH_3$ | $OC_2H_5$ | CH | |
| H | - | Q-3 | $CH_3$ | O | H | $OCH_3$ | $CF_3$ | CH | |
| H | - | Q-3 | $CH_3$ | O | H | $OCH_3$ | $O(CH_2)_2OCH_3$ | CH | |
| H | - | Q-3 | $CH_3$ | O | H | $OCH_3$ | $SCF_2H$ | CH | |
| H | - | Q-3 | $CH_3$ | O | H | $OCH_3$ | $C\equiv CH$ | CH | |
| H | - | Q-3 | $CH_3$ | O | H | $OCH_3$ | $C\equiv C-CH_3$ | CH | |
| H | - | Q-3 | $CH_3$ | O | H | $OCH_3$ | $N(OCH_3)CH_3$ | CH | |
| H | - | Q-4 | $CH_3$ | O | H | $CH_3$ | $CH_3$ | CH | |
| H | - | Q-4 | $CH_3$ | O | H | $CH_3$ | $OCH_3$ | CH | |
| H | - | Q-4 | $CH_3$ | O | H | $OCH_3$ | $OCH_3$ | CH | |
| H | - | Q-4 | $CH_3$ | O | H | $OCH_3$ | Cl | CH | |
| H | - | Q-4 | $CH_3$ | O | H | $CH_3$ | $OCH_3$ | N | |
| H | - | Q-4 | $CH_3$ | O | H | $OCH_3$ | $OCH_3$ | N | |
| H | - | Q-4 | $OCH_3$ | O | H | $CH_3$ | $CH_3$ | CH | |
| H | - | Q-4 | $OCH_3$ | O | H | $CH_3$ | $OCH_3$ | CH | |
| H | - | Q-4 | $OCH_3$ | O | H | $OCH_3$ | $OCH_3$ | CH | |
| H | - | Q-4 | $OCH_3$ | O | H | $OCH_3$ | Cl | CH | |
| H | - | Q-4 | $OCH_3$ | O | H | $CH_3$ | $OCH_3$ | N | |
| H | - | Q-4 | $OCH_3$ | O | H | $OCH_3$ | $OCH_3$ | N | |
| H | - | Q-4 | $SCH_3$ | O | H | $OCH_3$ | $OCH_3$ | N | |
| H | - | Q-4 | $SCH_3$ | O | H | $CH_3$ | $OCH_3$ | CH | |
| H | - | Q-4 | $SCH_3$ | O | H | $OCH_3$ | $OCH_3$ | CH | |
| H | - | Q-4 | $SCH_3$ | O | H | $OCH_3$ | Cl | CH | |
| H | - | Q-4 | $SCH_3$ | O | H | $CH_3$ | $OCH_3$ | N | |
| H | - | Q-4 | $SCH_3$ | O | H | $OCH_3$ | $OCH_3$ | N | |

Table 1a (Cont'd)

| $R_1$ | E | Q | $R_4$ | W | R | X | Y | Z | m.p.(°C) |
|---|---|---|---|---|---|---|---|---|---|
| H | – | Q-4 | $N(CH_2)_4$ | O | H | $CH_3$ | $CH_3$ | CH | |
| H | – | Q-4 | $N(CH_2)_4$ | O | H | $CH_3$ | $OCH_3$ | CH | |
| H | – | Q-4 | $N(CH_2)_4$ | O | H | $OCH_3$ | $OCH_3$ | CH | |
| H | – | Q-4 | $N(CH_2)_4$ | O | H | $OCH_3$ | Cl | CH | |
| H | – | Q-4 | $N(CH_2)_4$ | O | H | $CH_3$ | $OCH_3$ | N | |
| H | – | Q-4 | $N(CH_2)_4$ | O | H | $OCH_3$ | $OCH_3$ | N | |
| H | – | Q-4 | $C_6H_5$ | O | H | $OCH_3$ | $OCH_3$ | CH | |
| H | – | Q-4 | $C_6H_5$ | O | H | $OCH_3$ | $CH_3$ | CH | |
| H | – | Q-4 | $C_6H_5$ | O | H | $CH_3$ | $CH_3$ | CH | |
| H | – | Q-4 | $C_6H_5$ | O | H | $OCH_3$ | Cl | CH | |
| H | – | Q-4 | $C_6H_5$ | O | H | $CH_3$ | $OCH_3$ | N | |
| H | – | Q-4 | $CF_3$ | O | H | $CH_3$ | $OCH_3$ | CH | |
| H | – | Q-4 | $CF_3$ | O | H | $CH_3$ | $CH_3$ | CH | |
| H | – | Q-4 | $CF_3$ | O | H | $OCH_3$ | $OCH_3$ | CH | |
| H | – | Q-4 | $CF_3$ | O | H | $OCH_3$ | Cl | CH | |
| H | – | Q-4 | $CF_3$ | O | H | $OCH_3$ | $CH_3$ | N | |
| H | – | Q-4 | $CF_3$ | O | H | $OC_2H_5$ | $NHCH_3$ | N | |
| H | – | Q-4 | $CH_2CH=CH_2$ | O | H | $CH_3$ | $CH_3$ | CH | |
| H | – | Q-4 | $CH_2CH=CH_2$ | O | H | $CH_3$ | $OCH_3$ | CH | |
| H | – | Q-4 | $CH_2CH=CH_2$ | O | H | $OCH_3$ | $OCH_3$ | CH | |
| H | – | Q-4 | $CH_2CH=CH_2$ | O | H | $OCH_3$ | $CH_3$ | N | |
| 6-$CH_3$ | – | Q-3 | $CH_3$ | O | H | $CH_3$ | $CH_3$ | CH | |
| 6-$CH_3$ | – | Q-3 | $CH_3$ | O | H | $OCH_3$ | $CH_3$ | CH | |
| 6-$CH_3$ | – | Q-3 | $CH_3$ | O | H | $OCH_3$ | $OCH_3$ | CH | |
| 6-$CH_3$ | – | Q-3 | $CH_3$ | O | H | $OCH_3$ | Cl | CH | |
| 6-$CH_3$ | – | Q-3 | $CH_3$ | O | H | $OCH_3$ | $CH_3$ | N | |
| 6-Cl | – | Q-3 | $CH_3$ | O | H | $CH_3$ | $CH_3$ | CH | |
| 6-Cl | – | Q-3 | $CH_3$ | O | H | $OCH_3$ | $CH_3$ | CH | |
| 6-Cl | – | Q-3 | $CH_3$ | O | H | $OCH_3$ | $OCH_3$ | CH | |
| 6-Cl | – | Q-3 | $CH_3$ | O | H | $OCH_3$ | Cl | CH | |

Table 1b

General Structure 1

| $R_1$ | E | Q | $R_5$ | W | R | X | Y | Z | m.p.(°C) |
|---|---|---|---|---|---|---|---|---|---|
| H | - | Q-5 | $CH_3$ | O | H | $CH_3$ | $CH_3$ | CH | |
| H | - | Q-5 | $CH_3$ | O | H | $CH_3$ | $OCH_3$ | CH | |
| H | - | Q-5 | $CH_3$ | O | H | $OCH_3$ | $OCH_3$ | CH | 213-218 |
| H | - | Q-5 | $CH_3$ | O | H | $OCH_3$ | Cl | CH | |
| H | - | Q-5 | $CH_3$ | O | H | $CH_3$ | $OCH_3$ | N | |
| H | - | Q-5 | $CH_3$ | O | H | $OCH_3$ | $OCH_3$ | N | |
| H | - | Q-5 | $CH_2CH_3$ | O | H | $CH_3$ | $CH_3$ | CH | |
| H | - | Q-5 | $CH_2CH_3$ | O | H | $CH_3$ | $OCH_3$ | CH | |
| H | - | Q-5 | $CH_2CH_3$ | O | H | $OCH_3$ | $OCH_3$ | CH | |
| H | - | Q-5 | $CH_2CH_3$ | O | H | $OCH_3$ | Cl | CH | |
| H | - | Q-5 | $CH_2CH_3$ | O | H | $CH_3$ | $OCH_3$ | N | |
| H | - | Q-5 | $CH_2CH_3$ | O | H | $OCH_3$ | $OCH_3$ | N | |
| H | - | Q-6 | $CH_3$ | O | H | $CH_3$ | $CH_3$ | CH | |
| H | - | Q-6 | $CH_3$ | O | H | $CH_3$ | $OCH_3$ | CH | |
| H | - | Q-6 | $CH_3$ | O | H | $OCH_3$ | $OCH_3$ | CH | |
| H | - | Q-6 | $CH_3$ | O | H | $OCH_3$ | Cl | CH | |
| H | - | Q-6 | $CH_3$ | O | H | $CH_3$ | $OCH_3$ | N | |
| H | - | Q-6 | $CH_3$ | O | H | $OCH_3$ | $OCH_3$ | N | |
| H | - | Q-6 | $C_2H_5$ | O | H | $CH_3$ | $CH_3$ | CH | |
| H | - | Q-6 | $C_2H_5$ | O | H | $CH_3$ | $OCH_3$ | CH | |
| H | - | Q-6 | $C_2H_5$ | O | H | $OCH_3$ | $OCH_3$ | CH | |
| H | - | Q-6 | $C_2H_5$ | O | H | $OCH_3$ | Cl | CH | |
| H | - | Q-6 | $C_2H_5$ | O | H | $CH_3$ | $OCH_3$ | N | |
| H | - | Q-6 | $C_2H_5$ | O | H | $OCH_3$ | $OCH_3$ | N | |
| H | - | Q-6 | $CH_2CH=CH_2$ | O | H | $CH_3$ | $CH_3$ | CH | |
| H | - | Q-6 | $CH_2CH=CH_2$ | O | H | $CH_3$ | $OCH_3$ | CH | |
| H | - | Q-6 | $CH_2CH=CH_2$ | O | H | $OCH_3$ | $OCH_3$ | CH | |
| H | - | Q-6 | $CH_2CH=CH_2$ | O | H | $OCH_3$ | Cl | CH | |
| H | - | Q-6 | $CH_2CH=CH_2$ | O | H | $CH_3$ | $OCH_3$ | N | |
| H | - | Q-6 | $CH_2CH=CH_2$ | O | H | $OCH_3$ | $OCH_3$ | N | |
| H | - | Q-7 | $CH_3$ | O | H | $CH_3$ | $CH_3$ | CH | |
| H | - | Q-7 | $CH_3$ | O | H | $CH_3$ | $OCH_3$ | CH | |

Table 1b (Cont'd)

| $R_1$ | E | Q | $R_5$ | W | R | X | Y | Z | m.p. (°C) |
|---|---|---|---|---|---|---|---|---|---|
| H | - | Q-7 | $CH_3$ | O | H | $OCH_3$ | $OCH_3$ | CH | |
| H | - | Q-7 | $CH_3$ | O | H | $OCH_3$ | Cl | CH | |
| H | - | Q-7 | $CH_3$ | O | H | $CH_3$ | $OCH_3$ | N | |
| H | - | Q-7 | $CH_3$ | O | H | $OCH_3$ | $OCH_3$ | N | |
| H | - | Q-7 | $C_2H_5$ | O | H | $CH_3$ | $CH_3$ | CH | |
| H | - | Q-7 | $C_2H_5$ | O | H | $CH_3$ | $OCH_3$ | CH | |
| H | - | Q-7 | $C_2H_5$ | O | H | $OCH_3$ | $OCH_3$ | CH | |
| H | - | Q-7 | $C_2H_5$ | O | H | $OCH_3$ | Cl | CH | |
| H | - | Q-7 | $C_2H_5$ | O | H | $CH_3$ | $OCH_3$ | N | |
| H | - | Q-7 | $C_2H_5$ | O | H | $OCH_3$ | $OCH_3$ | N | |
| H | - | Q-7 | $CH_2CH=CH_2$ | O | H | $CH_3$ | $CH_3$ | CH | |
| H | - | Q-7 | $CH_2CH=CH_2$ | O | H | $CH_3$ | $OCH_3$ | CH | |
| H | - | Q-7 | $CH_2CH=CH_2$ | O | H | $OCH_3$ | $OCH_3$ | CH | |
| H | - | Q-7 | $CH_2CH=CH_2$ | O | H | $OCH_3$ | Cl | CH | |
| H | - | Q-7 | $CH_2CH=CH_2$ | O | H | $CH_3$ | $OCH_3$ | N | 202 |
| H | - | Q-7 | $CH_2CH=CH_2$ | O | H | $OCH_3$ | $OCH_3$ | N | |
| 5-Cl | - | Q-5 | $CH_3$ | O | H | $OCH_3$ | $OCH_3$ | CH | 178-180 |
| 5-Cl | - | Q-5 | $CH_3$ | O | H | $OCH_3$ | $CH_3$ | CH | 134-140 |
| 5-Cl | - | Q-5 | $CH_3$ | O | H | $OCH_3$ | Cl | CH | |
| 5-Cl | - | Q-5 | $CH_3$ | O | H | $CH_3$ | $CH_3$ | CH | |
| 5-Cl | - | Q-5 | $CH_3$ | O | H | $OCH_3$ | $CH_3$ | N | |
| 5-$CH_3$ | - | Q-5 | $CH_3$ | O | H | $OCH_3$ | $OCH_3$ | CH | |
| 5-$CH_3$ | - | Q-5 | $CH_3$ | O | H | $OCH_3$ | $CH_3$ | CH | |
| 5-$CH_3$ | - | Q-5 | $CH_3$ | O | H | $OCH_3$ | Cl | CH | |
| 5-$CH_3$ | - | Q-5 | $CH_3$ | O | H | $CH_3$ | $CH_3$ | CH | |
| 5-$CH_3$ | - | Q-5 | $CH_3$ | O | H | $OCH_3$ | $CH_3$ | N | |
| 6-$CH_3$ | - | Q-5 | $CH_3$ | O | H | $CH_3$ | $CH_3$ | CH | |
| 6-$CH_3$ | - | Q-5 | $CH_3$ | O | H | $OCH_3$ | $CH_3$ | CH | |
| 6-$CH_3$ | - | Q-5 | $CH_3$ | O | H | $OCH_3$ | $OCH_3$ | CH | |
| 6-$CH_3$ | - | Q-5 | $CH_3$ | O | H | $OCH_3$ | Cl | CH | |
| 6-$CH_3$ | - | Q-5 | $CH_3$ | O | H | $OCH_3$ | $CH_3$ | N | |

Table 1b (Cont'd)

| $R_1$ | E | Q | $R_5$ | W | R | X | Y | Z | m.p.(°C) |
|---|---|---|---|---|---|---|---|---|---|
| 5-$OCH_3$ | - | Q-5 | $CH_3$ | O | H | $CH_3$ | $CH_3$ | CH | |
| 5-$OCH_3$ | - | Q-5 | $CH_3$ | O | H | $OCH_3$ | $CH_3$ | CH | |
| 5-$OCH_3$ | - | Q-5 | $CH_3$ | O | H | $OCH_3$ | $OCH_3$ | CH | |
| 5-$OCH_3$ | - | Q-5 | $CH_3$ | O | H | $OCH_3$ | Cl | CH | |
| 5-$OCH_3$ | - | Q-5 | $CH_3$ | O | H | $OCH_3$ | $CH_3$ | N | |
| 6-Cl | - | Q-5 | $CH_3$ | O | H | $CH_3$ | $CH_3$ | CH | |
| 6-Cl | - | Q-5 | $CH_3$ | O | H | $OCH_3$ | $CH_3$ | CH | |
| 6-Cl | - | Q-5 | $CH_3$ | O | H | $OCH_3$ | $OCH_3$ | CH | |
| 6-Cl | - | Q-5 | $CH_3$ | O | H | $OCH_3$ | Cl | CH | |
| 6-Cl | - | Q-5 | $CH_3$ | O | H | $OCH_3$ | $CH_3$ | N | |
| 5-$(CH_3)_2N$ | - | Q-5 | $CH_3$ | O | H | $OCH_3$ | $CH_3$ | CH | |
| 5-$(CH_3)_2N$ | - | Q-5 | $CH_3$ | O | H | $OCH_3$ | $OCH_3$ | CH | |
| 5-$(CH_3)_2N$ | - | Q-5 | $CH_3$ | O | H | $CH_3$ | $CH_3$ | CH | |
| 5-$(CH_3)_2N$ | - | Q-5 | $CH_3$ | O | H | $OCH_3$ | Cl | CH | |
| 5-$(CH_3)_2N$ | - | Q-5 | $CH_3$ | O | H | $OCH_3$ | $CH_3$ | N | |
| 5-F | - | Q-5 | $CH_3$ | O | H | $OCH_3$ | $OCH_3$ | CH | |
| 5-F | - | Q-5 | $CH_3$ | O | H | $OCH_3$ | $CH_3$ | CH | |
| 5-F | - | Q-5 | $CH_3$ | O | H | $OCH_3$ | Cl | CH | |
| 5-F | - | Q-5 | $CH_3$ | O | H | $CH_3$ | $CH_3$ | CH | |
| H | - | Q-5 | $CH_3$ | O | H | $CH_3$ | $OCF_2H$ | CH | 196-198 |
| H | - | Q-5 | $CH_3$ | O | H | $OCH_3$ | $OCF_2H$ | CH | 162-166 |

Table II.

General Structure 2

| $R_1$ | E | Q | $R_3$ | W | R | $X_1$ | $Y_1$ | m.p.(°C) |
|---|---|---|---|---|---|---|---|---|
| H | - | Q-1 | $CH_3$ | O | H | $CH_3$ | O | |
| H | - | Q-1 | $CH_3$ | O | H | $OCH_3$ | O | |
| H | - | Q-1 | $CH_3$ | O | H | $OC_2H_5$ | O | |
| H | - | Q-1 | $CH_3$ | O | H | $OCF_2H$ | O | |
| H | - | Q-1 | $CH_3$ | O | H | $CH_3$ | $CH_2$ | |
| H | - | Q-1 | $CH_3$ | O | H | $OCH_3$ | $CH_2$ | |
| H | - | Q-1 | $CH_3$ | O | H | $OC_2H_5$ | $CH_2$ | |
| H | - | Q-1 | $CH_3$ | O | H | $OCF_2H$ | $CH_2$ | |
| H | - | Q-1 | $C_2H_5$ | O | H | $CH_3$ | O | |
| H | - | Q-1 | $C_2H_5$ | O | H | $OCH_3$ | O | |
| H | - | Q-1 | $C_2H_5$ | O | H | $OC_2H_5$ | O | |
| H | - | Q-1 | $C_2H_5$ | O | H | $OCF_2H$ | O | |
| H | - | Q-1 | $C_2H_5$ | O | H | $CH_3$ | $CH_2$ | |
| H | - | Q-1 | $C_2H_5$ | O | H | $OCH_3$ | $CH_2$ | |
| H | - | Q-1 | $C_2H_5$ | O | H | $OC_2H_5$ | $CH_2$ | |
| H | - | Q-1 | $C_2H_5$ | O | H | $OCF_2H$ | $CH_2$ | |
| H | - | Q-1 | $(CH_2)_5CH_3$ | O | H | $OCH_3$ | O | |
| H | - | Q-1 | $CH_2CH{=}CH{-}CH_2Cl$ | O | H | $OCH_3$ | O | |
| H | - | Q-1 | $C_2H_5$ | O | H | $OCH_3$ | O | |
| 5-$CH_3$ | - | Q-1 | $CH_3$ | O | H | $OCH_3$ | O | |
| 5-$CH_3$ | - | Q-1 | $CH_3$ | O | H | $OCH_3$ | $CH_2$ | |
| 5-$CH_3$ | - | Q-1 | $CH_3$ | O | H | $OCH_3$ | $CH_2$ | |
| 5-$CH_3$ | - | Q-1 | $CH_3$ | O | H | $OCH_3$ | $CH_2$ | |
| 5-Cl | - | Q-1 | $CH_3$ | O | H | $OCH_3$ | O | |
| 5-$OCH_3$ | - | Q-1 | $CH_3$ | O | H | $OCH_3$ | O | |
| 5-$OCH_3$ | - | Q-1 | $CH_3$ | O | H | $CH_3$ | O | |
| 5-$OC_2H_5$ | - | Q-1 | $CH_3$ | O | H | $OCH_3$ | O | |
| 5-$OC_2H_5$ | - | Q-1 | $CH_3$ | O | H | $CH_3$ | O | |
| 5-$SCH_3$ | - | Q-1 | $CH_3$ | O | H | $OCH_3$ | O | |
| 5-$SCH_3$ | - | Q-1 | $CH_3$ | O | H | $CH_3$ | O | |
| 5-$SC_2H_5$ | - | Q-1 | $CH_3$ | O | H | $OCH_3$ | O | |
| 5-$SC_2H_5$ | - | Q-1 | $CH_3$ | O | H | $CH_3$ | O | |

Table II (Cont'd)

| $R_1$ | E | Q | $R_3$ | W | R | $X_1$ | $Y_1$ | m.p.(°C) |
|---|---|---|---|---|---|---|---|---|
| 5-CN | - | Q-1 | $CH_3$ | O | H | $OCH_3$ | O | |
| 5-CN | - | Q-1 | $CH_3$ | O | H | $CH_3$ | O | |
| 5-$OCF_2H$ | - | Q-1 | $CH_3$ | O | H | $OCH_3$ | O | |
| 5-$OCF_2H$ | - | Q-1 | $CH_3$ | O | H | $CH_3$ | O | |
| H | - | Q-2 | $CH_3$ | O | H | $CH_3$ | O | |
| H | - | Q-2 | $CH_3$ | O | H | $OCH_3$ | O | |
| H | - | Q-2 | $CH_3$ | O | H | $OC_2H_5$ | O | |
| H | - | Q-2 | $CH_3$ | O | H | $OCF_2H$ | O | |
| H | - | Q-2 | $CH_3$ | O | H | $CH_3$ | $CH_2$ | |
| H | - | Q-2 | $CH_3$ | O | H | $OCH_3$ | $CH_2$ | |
| H | - | Q-2 | $CH_3$ | O | H | $OC_2H_5$ | $CH_2$ | |
| H | - | Q-2 | $C_2H_5$ | O | H | $CH_3$ | O | |
| H | - | Q-2 | $C_2H_5$ | O | H | $OCH_3$ | O | |
| H | - | Q-2 | $C_2H_5$ | O | H | $CH_3$ | $CH_2$ | |
| H | - | Q-2 | $C_2H_5$ | O | H | $OCH_3$ | $CH_2$ | |
| 5-$CH_3$ | - | Q-2 | $CH_3$ | O | H | $OCH_3$ | O | |
| 5-$CH_3$ | - | Q-2 | $CH_3$ | O | H | $OCH_3$ | $CH_2$ | |
| 5-$C_2H_5$ | - | Q-2 | $CH_3$ | O | H | $OCH_3$ | O | |
| 5-$OCH_3$ | - | Q-2 | $CH_3$ | O | H | $OCH_3$ | O | |
| 5-$OCH_3$ | - | Q-2 | $CH_3$ | O | H | $CH_3$ | O | |
| 5-$OC_2H_5$ | - | Q-2 | $CH_3$ | O | H | $OCH_3$ | O | |
| 5-$OC_2H_5$ | - | Q-2 | $CH_3$ | O | H | $CH_3$ | O | |
| 5-$SCH_3$ | - | Q-2 | $CH_3$ | O | H | $OCH_3$ | O | |
| 5-$SCH_3$ | - | Q-2 | $CH_3$ | O | H | $CH_3$ | O | |
| 5-$SC_2H_5$ | - | Q-2 | $CH_3$ | O | H | $OCH_3$ | O | |
| 5-$OCF_2H$ | - | Q-2 | $CH_3$ | O | H | $OCH_3$ | O | |
| 6-$CH_3$ | - | Q-1 | $CH_3$ | O | H | $OCH_3$ | O | |
| 6-$CH_3$ | - | Q-1 | $CH_3$ | O | H | $CH_3$ | O | |
| 6-$CH_3$ | - | Q-1 | $CH_3$ | O | H | $OCH_3$ | $CH_2$ | |
| 6-Cl | - | Q-1 | $CH_3$ | O | H | $OCH_3$ | O | |
| 6-Cl | - | Q-1 | $CH_3$ | O | H | $CH_3$ | O | |
| 6-Cl | - | Q-1 | $CH_3$ | O | H | $OCH_3$ | $CH_2$ | |

**Table IIa**

**General Structure 2**

| $R_1$ | E | Q | $R_4$ | W | R | $X_1$ | $Y_1$ | m.p.(°C) |
|---|---|---|---|---|---|---|---|---|
| H | - | Q-3 | $CH_3$ | O | H | $CH_3$ | O | |
| H | - | Q-3 | $CH_3$ | O | H | $OCH_3$ | O | |
| H | - | Q-3 | $CH_3$ | O | H | $OC_2H_5$ | O | |
| H | - | Q-3 | $CH_3$ | O | H | $OCF_2H$ | O | |
| H | - | Q-3 | $CH_3$ | O | H | $CH_3$ | $CH_2$ | |
| H | - | Q-3 | $CH_3$ | O | H | $OCH_3$ | $CH_2$ | |
| H | - | Q-3 | $CH_3$ | O | H | $OC_2H_5$ | $CH_2$ | |
| H | - | Q-3 | $C_2H_5$ | O | H | $CH_3$ | O | |
| H | - | Q-3 | $C_2H_5$ | O | H | $OCH_3$ | O | |
| H | - | Q-3 | $C_2H_5$ | O | H | $OC_2H_5$ | O | |
| H | - | Q-3 | $C_2H_5$ | O | H | $CH_3$ | $CH_2$ | |
| H | - | Q-3 | $C_2H_5$ | O | H | $OCH_3$ | $CH_2$ | |
| H | - | Q-3 | $C_2H_5$ | O | H | $OC_2H_5$ | $CH_2$ | |
| H | - | Q-3 | $C_2H_5$ | O | H | $OCF_2H$ | $CH_2$ | |
| H | - | Q-3 | $C_2H_5$ | O | H | $OCH_3$ | O | |
| H | - | Q-3 | $C_2H_5$ | O | H | $OCH_3$ | O | |
| 5-$CH_3$ | - | Q-3 | $CH_3$ | O | H | $OCH_3$ | O | |
| 5-$CH_3$ | - | Q-3 | $CH_3$ | O | H | $OCH_3$ | $CH_2$ | |
| 5-Cl | - | Q-3 | $CH_3$ | O | H | $OCH_3$ | O | |
| 5-Cl | - | Q-3 | $CH_3$ | O | H | $OCH_3$ | O | |
| 5-Cl | - | Q-3 | $CH_3$ | O | H | $CH_3$ | O | |
| 5-$OCH_3$ | - | Q-3 | $CH_3$ | O | H | $OCH_3$ | O | |
| 5-$OCH_3$ | - | Q-3 | $CH_3$ | O | H | $CH_3$ | O | |
| 5-$OC_2H_5$ | - | Q-3 | $CH_3$ | O | H | $OCH_3$ | O | |
| 5-$OC_2H_5$ | - | Q-3 | $CH_3$ | O | H | $CH_3$ | O | |
| 5-$SCH_3$ | - | Q-3 | $CH_3$ | O | H | $OCH_3$ | O | |
| 5-$SCH_3$ | - | Q-3 | $CH_3$ | O | H | $CH_3$ | O | |
| 5-$SC_2H_5$ | - | Q-3 | $CH_3$ | O | H | $OCH_3$ | O | |
| 5-$SC_2H_5$ | - | Q-3 | $CH_3$ | O | H | $CH_3$ | O | |
| 5-$OCF_2H$ | - | Q-3 | $CH_3$ | O | H | $OCH_3$ | O | |
| 5-$OCF_2H$ | - | Q-3 | $CH_3$ | O | H | $CH_3$ | O | |
| 5-$SCF_2H$ | - | Q-3 | $CH_3$ | O | H | $OCH_3$ | O | |

Table IIa (Cont'd)

| $R_1$ | E | Q | $R_4$ | W | R | $X_1$ | $Y_1$ | m.p.(°C) |
|---|---|---|---|---|---|---|---|---|
| 5-$SCF_2H$ | – | Q-3 | $CH_3$ | O | H | $CH_3$ | O | |
| H | – | Q-4 | $CH_3$ | O | H | $CH_3$ | O | |
| H | – | Q-4 | $CH_3$ | O | H | $OCH_3$ | O | |
| H | – | Q-4 | $CH_3$ | O | H | $OC_2H_5$ | O | |
| H | – | Q-4 | $CH_3$ | O | H | $OCF_2H$ | O | |
| H | – | Q-4 | $CH_3$ | O | H | $CH_3$ | $CH_2$ | |
| H | – | Q-4 | $CH_3$ | O | H | $OCH_3$ | $CH_2$ | |
| H | – | Q-4 | $CH_3$ | O | H | $OC_2H_5$ | $CH_2$ | |
| H | – | Q-4 | $CH_3$ | O | H | $OCF_2H$ | $CH_2$ | |
| H | – | Q-4 | $C_2H_5$ | O | H | $CH_3$ | O | |
| H | – | Q-4 | $C_2H_5$ | O | H | $OCH_3$ | O | |
| H | – | Q-4 | $C_2H_5$ | O | H | $OC_2H_5$ | O | |
| H | – | Q-4 | $C_2H_5$ | O | H | $OCF_2H$ | O | |
| H | – | Q-4 | $C_2H_5$ | O | H | $CH_3$ | $CH_2$ | |
| H | – | Q-4 | $C_2H_5$ | O | H | $OCH_3$ | $CH_2$ | |
| H | – | Q-4 | $C_2H_5$ | O | H | $OC_2H_5$ | $CH_2$ | |
| H | – | Q-4 | $C_2H_5$ | O | H | $OCF_2H$ | $CH_2$ | |
| 6-$CH_3$ | – | Q-3 | $CH_3$ | O | H | $OCH_3$ | $CH_2$ | |
| 6-$CH_3$ | – | Q-3 | $CH_3$ | O | H | $OCH_3$ | O | |
| 6-$CH_3$ | – | Q-3 | $CH_3$ | O | H | $CH_3$ | O | |
| 6-Cl | – | Q-3 | $CH_3$ | O | H | $OCH_3$ | $CH_2$ | |
| 6-Cl | – | Q-3 | $CH_3$ | O | H | $OCH_3$ | O | |
| 6-Cl | – | Q-3 | $CH_3$ | O | H | $CH_3$ | O | |
| 6-$CH_3$ | – | Q-4 | $CH_3$ | O | H | $OCH_3$ | $CH_2$ | |
| 6-$CH_3$ | – | Q-4 | $CH_3$ | O | H | $OCH_3$ | O | |
| 6-$CH_3$ | – | Q-4 | $CH_3$ | O | H | $CH_3$ | O | |
| 6-Cl | – | Q-4 | $CH_3$ | O | H | $OCH_3$ | $CH_2$ | |
| 6-Cl | – | Q-4 | $CH_3$ | O | H | $OCH_3$ | O | |
| 6-Cl | – | Q-4 | $CH_3$ | O | H | $CH_3$ | O | |

Table IIb

General Structure 2

| $R_1$ | E | Q | $R_5$ | W | R | $X_1$ | $Y_1$ | m.p.(°C) |
|---|---|---|---|---|---|---|---|---|
| H | - | Q-5 | $CH_3$ | O | H | $OCH_3$ | O | |
| H | - | Q-5 | $CH_3$ | O | H | $OCH_3$ | $CH_2$ | |
| H | - | Q-5 | $CH_3$ | O | H | $CH_3$ | O | |
| H | - | Q-5 | $C_2H_5$ | O | H | $OCH_3$ | O | |
| H | - | Q-5 | $C_2H_5$ | O | H | $OCH_3$ | $CH_2$ | |
| H | - | Q-5 | $C_2H_5$ | O | H | $CH_3$ | O | |
| 5-$CH_3$ | - | Q-5 | $CH_3$ | O | H | $OCH_3$ | O | |
| 5-$CH_3$ | - | Q-5 | $CH_3$ | O | H | $OCH_3$ | $CH_2$ | |
| 5-$CH_3$ | - | Q-5 | $CH_3$ | O | H | $CH_3$ | O | |
| 5-Cl | - | Q-5 | $CH_3$ | O | H | $OCH_3$ | O | |
| 5-Cl | - | Q-5 | $CH_3$ | O | H | $OCH_3$ | $CH_2$ | |
| 5-Cl | - | Q-5 | $CH_3$ | O | H | $CH_3$ | O | |
| 6-$CH_3$ | - | Q-5 | $CH_3$ | O | H | $OCH_3$ | O | |
| 6-$CH_3$ | - | Q-5 | $CH_3$ | O | H | $OCH_3$ | $CH_2$ | |
| 6-$CH_3$ | - | Q-5 | $CH_3$ | O | H | $CH_3$ | O | |
| 6-Cl | - | Q-5 | $CH_3$ | O | H | $OCH_3$ | O | |
| 6-Cl | - | Q-5 | $CH_3$ | O | H | $OCH_3$ | $CH_2$ | |
| 6-Cl | - | Q-5 | $CH_3$ | O | H | $CH_3$ | O | |
| 5-F | - | Q-5 | $CH_3$ | O | H | $OCH_3$ | O | |
| 5-F | - | Q-5 | $CH_3$ | O | H | $OCH_3$ | $CH_2$ | |
| 5-F | - | Q-5 | $CH_3$ | O | H | $CH_3$ | O | |
| 5-$OCH_3$ | - | Q-5 | $CH_3$ | O | H | $OCH_3$ | O | |
| 5-$OCH_3$ | - | Q-5 | $CH_3$ | O | H | $OCH_3$ | $CH_2$ | |
| 5-$OCH_3$ | - | Q-5 | $CH_3$ | O | H | $CH_3$ | O | |

Table III

General Structure 3

| $R_1$ | E | Q | $R_3$ | $R_4$ | $R_5$ | W | R | $X_1$ | m.p.(°C) |
|---|---|---|---|---|---|---|---|---|---|
| H | - | Q-1 | $CH_3$ | - | - | O | H | $CH_3$ | |
| H | - | Q-1 | $CH_3$ | - | - | O | H | $OCH_3$ | |
| H | - | Q-1 | $CH_3$ | - | - | O | H | $OC_2H_5$ | |
| H | - | Q-1 | $CH_3$ | - | - | O | H | $OCF_2H$ | |
| H | - | Q-1 | $C_2H_5$ | - | - | O | H | $CH_3$ | |
| H | - | Q-1 | $C_2H_5$ | - | - | O | H | $OCH_3$ | |
| H | - | Q-1 | $C_2H_5$ | - | - | O | H | $OC_2H_5$ | |
| H | - | Q-2 | $CH_3$ | - | - | O | H | $CH_3$ | |
| H | - | Q-2 | $CH_3$ | - | - | O | H | $OCH_3$ | |
| H | - | Q-2 | $CH_3$ | - | - | O | H | $OC_2H_5$ | |
| H | - | Q-2 | $C_2H_5$ | - | - | O | H | $OCH_3$ | |
| H | - | Q-2 | $C_2H_5$ | - | - | O | H | $CH_3$ | |
| H | - | Q-3 | - | $CH_3$ | - | O | H | $OC_2H_5$ | |
| H | - | Q-3 | - | $CH_3$ | - | O | H | $OCH_3$ | |
| H | - | Q-3 | - | $CH_3$ | - | O | H | $CH_3$ | |
| H | - | Q-3 | - | $C_2H_5$ | - | O | H | $CH_3$ | |
| H | - | Q-3 | - | $C_2H_5$ | - | O | H | $OCH_3$ | |
| 6-$CH_3$ | - | Q-1 | $CH_3$ | - | - | O | H | $OCH_3$ | |
| 6-$CH_3$ | - | Q-1 | $CH_3$ | - | - | O | H | $CH_3$ | |
| 6-Cl | - | Q-1 | $CH_3$ | - | - | O | H | $CH_3$ | |
| 6-Cl | - | Q-1 | $CH_3$ | - | - | O | H | $OCH_3$ | |
| H | - | Q-4 | - | $CH_3$ | - | O | H | $OCH_3$ | |
| H | - | Q-5 | - | - | $CH_3$ | O | H | $OCH_3$ | |
| H | - | Q-5 | - | - | $CH_3$ | O | H | $CH_3$ | |
| H | - | Q-6 | - | - | $CH_3$ | O | H | $OCH_3$ | |
| H | - | Q-7 | - | - | $CH_3$ | O | H | $OCH_3$ | |

Table IV

General Structure 4

| $R_1$ | E | Q | $R_3$ | $R_4$ | $R_5$ | W | R | $X_1$ | $Y_3$ | m.p.(°C) |
|---|---|---|---|---|---|---|---|---|---|---|
| H | - | Q-1 | $CH_3$ | - | - | O | H | $CH_3$ | H | |
| H | - | Q-1 | $CH_3$ | - | - | O | H | $OCH_3$ | H | |
| H | - | Q-1 | $CH_3$ | - | - | O | H | $OC_2H_5$ | H | |
| H | - | Q-1 | $CH_3$ | - | - | O | H | $CH_3$ | $CH_3$ | |
| H | - | Q-1 | $CH_3$ | - | - | O | H | $OCH_3$ | $CH_3$ | |
| H | - | Q-1 | $CH_3$ | - | - | O | H | $OCF_2H$ | $CH_3$ | |
| H | - | Q-1 | $C_2H_5$ | - | - | O | H | $CH_3$ | H | |
| H | - | Q-1 | $C_2H_5$ | - | - | O | H | $OCH_3$ | H | |
| H | - | Q-1 | $C_2H_5$ | - | - | O | H | $CH_3$ | $CH_3$ | |
| H | - | Q-2 | $CH_3$ | - | - | O | H | $OCH_3$ | $CH_3$ | |
| H | - | Q-2 | $CH_3$ | - | - | O | H | $OCH_3$ | H | |
| H | - | Q-2 | $CH_3$ | - | - | O | H | $CH_3$ | H | |
| H | - | Q-2 | $CH_3$ | - | - | O | H | $OCH_3$ | $CH_3$ | |
| H | - | Q-2 | $CH_3$ | - | - | O | H | $CH_3$ | $CH_3$ | |
| H | - | Q-3 | - | $CH_3$ | - | O | H | $OCH_3$ | $CH_3$ | |
| H | - | Q-3 | - | $CH_3$ | - | O | H | $OCH_3$ | $CH_3$ | |
| H | - | Q-3 | - | $C_2H_5$ | - | O | H | $CH_3$ | $CH_3$ | |
| H | - | Q-3 | - | $C_2H_5$ | - | O | H | $OCH_3$ | H | |
| H | - | Q-4 | - | $CH_3$ | - | O | H | $OCH_3$ | H | |
| H | - | Q-5 | - | - | $CH_3$ | O | H | $OCH_3$ | H | |
| H | - | Q-5 | - | - | $CH_3$ | O | H | $OCH_3$ | $CH_3$ | |
| H | - | Q-5 | - | - | $CH_3$ | O | H | $CH_3$ | H | |
| 5-Cl | - | Q-5 | - | - | $CH_3$ | O | H | $CH_3$ | H | |
| 6-$CH_3$ | - | Q-5 | - | - | $CH_3$ | O | H | $OCH_3$ | H | |

## Table V

## General Structure 5

| $R_1$ | E | Q | $R_3$ | $R_4$ | $R_5$ | W | R | $X_2$ | $Y_2$ | m.p.(°C) |
|---|---|---|---|---|---|---|---|---|---|---|
| H | - | Q-1 | $CH_3$ | - | - | O | H | $CH_3$ | $OCH_3$ | |
| H | - | Q-1 | $CH_3$ | - | - | O | H | $CH_2CF_3$ | $OCH_3$ | |
| H | - | Q-1 | $CH_3$ | - | - | O | H | $CH_3$ | $SC_2H_5$ | |
| H | - | Q-1 | $CH_3$ | - | - | O | H | $CH_3$ | $SCH_3$ | |
| H | - | Q-1 | $CH_3$ | - | - | O | H | $CH_3$ | $OC_2H_5$ | |
| H | - | Q-1 | $C_2H_5$ | - | - | O | H | $CH_3$ | $SCH_3$ | |
| H | - | Q-1 | $C_2H_5$ | - | - | O | H | $CH_3$ | $OCH_3$ | |
| H | - | Q-2 | $CH_3$ | - | - | O | H | $CH_3$ | $OCH_3$ | |
| H | - | Q-2 | $CH(CH_3)_2$ | - | - | O | H | $CH_3$ | $OCH_3$ | |
| H | - | Q-3 | - | $CH_3$ | - | O | H | $CH_3$ | $SCH_3$ | |
| H | - | Q-3 | - | $CH_3$ | - | O | H | $CH_3$ | $SC_2H_5$ | |
| H | - | Q-3 | - | $C_2H_5$ | - | O | H | $CH_3$ | $SCH_3$ | |
| H | - | Q-3 | - | $CF_3$ | - | O | H | $CH_3$ | $SCH_3$ | |
| H | - | Q-4 | - | $CH_3$ | - | O | H | $CH_3$ | $SCH_3$ | |
| 6-$CH_3$ | - | Q-4 | - | $CH_3$ | - | O | H | $CH_3$ | $SCH_3$ | |
| 6-Cl | - | Q-4 | - | $CH_3$ | - | O | H | $CH_3$ | $SCH_3$ | |
| H | - | Q-5 | - | - | $CH_3$ | O | H | $CH_3$ | $SCH_3$ | |
| H | - | Q-6 | - | - | $CH_3$ | O | H | $CH_3$ | $SCH_3$ | |
| H | - | Q-7 | - | - | $CH_3$ | O | H | $CH_3$ | $SCH_3$ | |
| 6-$CH_3$ | - | Q-1 | $CH_3$ | - | - | O | H | $CH_3$ | $SCH_3$ | |
| 5-Cl | - | Q-1 | $CH_3$ | - | - | O | H | $CH_3$ | $SCH_3$ | |

## Table VI

## General Structure 6

| $R_1$ | E | Q | $R_3$ | $R_4$ | $R_5$ | W | R | $X_3$ | m.p.(°C) |
|---|---|---|---|---|---|---|---|---|---|
| H | - | Q-1 | $CH_3$ | - | - | O | H | $CH_3$ | |
| H | - | Q-1 | $CH_3$ | - | - | O | H | $OCH_3$ | |
| H | - | Q-1 | $C_2H_5$ | - | - | O | H | $CH_3$ | |
| H | - | Q-1 | $C_2H_5$ | - | - | O | H | $OCH_3$ | |
| H | - | Q-2 | $CH_3$ | - | - | O | H | $CH_3$ | |
| H | - | Q-2 | $C_2H_5$ | - | - | O | H | $CH_3$ | |
| H | - | Q-3 | - | $CH_3$ | - | O | H | $CH_3$ | |
| H | - | Q-3 | - | $CH_3$ | - | O | H | $OCH_3$ | |
| H | - | Q-3 | - | $C_2H_5$ | - | O | H | $CH_3$ | |
| H | - | Q-3 | - | $C_2H_5$ | - | O | H | $OCH_3$ | |
| H | - | Q-3 | - | $CH(CH_3)_2$ | - | O | H | $OCH_3$ | |
| H | - | Q-3 | - | $CH_2Cl$ | - | O | H | $OCH_3$ | |
| H | - | Q-3 | - | $CF_3$ | - | O | H | $OCH_3$ | |
| H | - | Q-4 | - | $CH_3$ | - | O | H | $OCH_3$ | |
| 6-$CH_3$ | - | Q-1 | $CH_3$ | - | - | O | H | $OCH_3$ | |
| 6-Cl | - | Q-1 | $CH_3$ | - | - | O | H | $OCH_3$ | |
| 5-Cl | - | Q-1 | $CH_3$ | - | - | O | H | $OCH_3$ | |
| H | - | Q-5 | - | - | $CH_3$ | O | H | $OCH_3$ | |
| H | - | Q-5 | - | - | $CH_3$ | O | H | $CH_3$ | |
| H | - | Q-6 | - | - | $CH_3$ | O | H | $OCH_3$ | |
| H | - | Q-6 | - | - | $CH_3$ | O | H | $CH_3$ | |
| H | - | Q-7 | - | - | $CH_3$ | O | H | $OCH_3$ | |

**Table VII**

**General Structure 7**

| $R_1$ | E | Q | $R_3$ | $R_4$ | $R_5$ | W | R | $X_4$ | $Y_4$ | $Z^1$ | m.p.(°C) |
|---|---|---|---|---|---|---|---|---|---|---|---|
| H | - | Q-1 | $CH_3$ | - | - | O | H | $CH_3$ | $CH_3$ | N | |
| H | - | Q-1 | $CH_3$ | - | - | O | H | $OCH_3$ | $CH_3$ | N | |
| H | - | Q-1 | $CH_3$ | - | - | O | H | $OC_2H_5$ | $CH_3$ | N | |
| H | - | Q-1 | $CH_3$ | - | - | O | H | $CH_2OCH_3$ | $CH_3$ | N | |
| H | - | Q-1 | $CH_3$ | - | - | O | H | Cl | $CH_3$ | N | |
| H | - | Q-1 | $CH_3$ | - | - | O | H | $CH_3$ | $OCH_3$ | N | |
| H | - | Q-1 | $CH_3$ | - | - | O | H | $OCH_3$ | $OCH_3$ | N | |
| H | - | Q-1 | $CH_3$ | - | - | O | H | $OC_2H_5$ | $OCH_3$ | N | |
| H | - | Q-1 | $CH_3$ | - | - | O | H | $CH_2OCH_3$ | $OCH_3$ | N | |
| H | - | Q-1 | $CH_3$ | - | - | O | H | Cl | $OCH_3$ | N | |
| H | - | Q-1 | $CH_3$ | - | - | O | H | $CH_3$ | $CH_3$ | CH | |
| H | - | Q-1 | $CH_3$ | - | - | O | H | $OCH_3$ | $CH_3$ | CH | |
| H | - | Q-1 | $CH_3$ | - | - | O | H | $OC_2H_5$ | $CH_3$ | CH | |
| H | - | Q-1 | $CH_3$ | - | - | O | H | $CH_2OCH_3$ | $CH_3$ | CH | |
| H | - | Q-1 | $CH_3$ | - | - | O | H | Cl | $CH_3$ | CH | |
| H | - | Q-1 | $CH_3$ | - | - | O | H | $CH_3$ | $OCH_3$ | CH | |
| H | - | Q-1 | $CH_3$ | - | - | O | H | $OCH_3$ | $OCH_3$ | CH | |
| H | - | Q-1 | $CH_3$ | - | - | O | H | $OC_2H_5$ | $OCH_3$ | CH | |
| H | - | Q-1 | $CH_3$ | - | - | O | H | $CH_2OCH_3$ | $OCH_3$ | CH | |
| H | - | Q-1 | $CH_3$ | - | - | O | H | Cl | $OCH_3$ | CH | |
| H | - | Q-1 | $CH_3$ | - | - | O | H | $OCH_3$ | $OC_2H_5$ | CH | |
| H | - | Q-1 | $CH_3$ | - | - | O | H | $OCH_3$ | Cl | CH | |
| H | - | Q-1 | $CH_3$ | - | - | O | H | $CH_3$ | $OC_2H_5$ | CH | |
| H | - | Q-1 | $CH_3$ | - | - | O | H | $CH_3$ | Cl | CH | |
| 6-$CH_3$ | - | Q-1 | $CH_3$ | - | - | O | H | $CH_3$ | $CH_3$ | CH | |
| 6-$CH_3$ | - | Q-1 | $CH_3$ | - | - | O | H | $OCH_3$ | $OCH_3$ | CH | |
| 6-$CH_3$ | - | Q-1 | $CH_3$ | - | - | O | H | $CH_3$ | $OCH_3$ | CH | |
| 5-$CH_3$ | - | Q-1 | $CH_3$ | - | - | O | H | $CH_3$ | $OCH_3$ | CH | |
| 5-$CH_3$ | - | Q-1 | $CH_3$ | - | - | O | H | $OCH_3$ | $OCH_3$ | CH | |
| 5-$CH_3$ | - | Q-1 | $CH_3$ | - | - | O | H | $CH_3$ | $CH_3$ | CH | |

Table VII (Cont'd)

| $R_1$ | E | Q | $R_3$ | $R_4$ | $R_5$ | W | R | $X_4$ | $Y_4$ | $Z^1$ | m.p.(°C) |
|---|---|---|---|---|---|---|---|---|---|---|---|
| 5-$OCH_3$ | - | Q-1 | $CH_3$ | - | - | O | H | $CH_3$ | $CH_3$ | CH | |
| 5-$OCH_3$ | - | Q-1 | $CH_3$ | - | - | O | H | $OCH_3$ | $CH_3$ | CH | |
| 5-$OCH_3$ | - | Q-1 | $CH_3$ | - | - | O | H | $OCH_3$ | $OCH_3$ | CH | |
| H | - | Q-3 | - | $CH_3$ | - | O | H | $CH_3$ | $CH_3$ | CH | |
| H | - | Q-3 | - | $CH_3$ | - | O | H | $OCH_3$ | $OCH_3$ | CH | |
| H | - | Q-3 | - | $CH_3$ | - | O | H | $CH_3$ | $OCH_3$ | CH | |
| H | - | Q-3 | - | $CH_3$ | - | O | H | $OCH_3$ | $CH_3$ | CH | |
| 6-$CH_3$ | - | Q-3 | - | $CH_3$ | - | O | H | $OCH_3$ | $OCH_3$ | CH | |
| 6-$CH_3$ | - | Q-3 | - | $CH_3$ | - | O | H | $OCH_3$ | $OCH_3$ | N | |
| 5-Cl | - | Q-3 | - | $CH_3$ | - | O | H | $OCH_3$ | $OCH_3$ | CH | |
| 5-Cl | - | Q-3 | - | $CH_3$ | - | O | H | $OCH_3$ | $CH_3$ | CH | |
| 5-$OCH_3$ | - | Q-3 | - | $CH_3$ | - | O | H | $OCH_3$ | $OCH_3$ | CH | |
| 5-$OCH_3$ | - | Q-3 | - | $CH_3$ | - | O | H | $CH_3$ | $OCH_3$ | CH | |
| 5-$OCH_3$ | - | Q-3 | - | $CH_3$ | - | O | H | $OCH_3$ | $CH_3$ | CH | |
| H | - | Q-4 | - | $CH_3$ | - | O | H | $OCH_3$ | $OCH_3$ | CH | |
| H | - | Q-4 | - | $CH_3$ | - | O | H | $CH_3$ | $CH_3$ | CH | |
| H | - | Q-5 | - | - | $CH_3$ | O | H | $CH_3$ | $CH_3$ | CH | |
| H | - | Q-5 | - | - | $CH_3$ | O | H | $CH_3$ | $OCH_3$ | CH | |
| H | - | Q-6 | - | - | $CH_3$ | O | H | $CH_3$ | $OCH_3$ | CH | |
| H | - | Q-7 | - | - | $CH_3$ | O | H | $CH_3$ | $OCH_3$ | CH | |

## Table VIII

### General Structure 8

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-1 | $CH_3$ | - | - | O | H | $CH_3$ | $CH_3$ | CH | |
| H | O | - | Q-1 | $CH_3$ | - | - | O | H | $OCH_3$ | $CH_3$ | CH | |
| H | O | - | Q-1 | $CH_3$ | - | - | O | H | Cl | $OCH_3$ | CH | |
| H | O | - | Q-1 | $CH_3$ | - | - | O | H | $CH_3$ | $OCH_3$ | N | |
| H | O | - | Q-1 | $CH_3$ | - | - | O | H | $OCH_3$ | $OCH_3$ | N | |
| H | O | - | Q-1 | $C_2H_5$ | - | - | O | H | $OCH_3$ | $CH_3$ | CH | |
| H | O | - | Q-1 | $C_2H_5$ | - | - | O | H | $OCH_3$ | $OCH_3$ | CH | |
| H | O | - | Q-1 | $C_2H_5$ | - | - | O | H | Cl | $OCH_3$ | CH | |
| H | O | - | Q-1 | $C_2H_5$ | - | - | O | H | $CH_3$ | $CH_3$ | CH | |
| H | O | - | Q-1 | $C_2H_5$ | - | - | O | H | $CH_3$ | $OCH_3$ | N | |
| H | O | - | Q-2 | $CH_3$ | - | - | O | H | $OCH_3$ | $OCH_3$ | N | |
| H | O | - | Q-2 | $CH_3$ | - | - | O | H | $OCH_3$ | $OCH_3$ | CH | |
| H | O | - | Q-2 | $CH_3$ | - | - | O | H | $OCH_3$ | $CH_3$ | CH | |
| H | O | - | Q-2 | $CH_3$ | - | - | O | H | Cl | $OCH_3$ | CH | |
| H | O | - | Q-2 | $CH_3$ | - | - | O | H | $CH_3$ | $CH_3$ | CH | |
| H | O | - | Q-2 | $CH_3$ | - | - | O | H | $OCH_3$ | $CH_3$ | N | |
| H | O | - | Q-2 | $C_2H_5$ | - | - | O | H | $OCH_3$ | $OCH_3$ | N | |
| H | O | - | Q-2 | $C_2H_5$ | - | - | O | H | $CH_3$ | $CH_3$ | CH | |
| H | O | - | Q-2 | $C_2H_5$ | - | - | O | H | $CH_3$ | $OCH_3$ | CH | |
| H | O | - | Q-2 | $C_2H_5$ | - | - | O | H | Cl | $OCH_3$ | CH | |
| H | O | - | Q-2 | $C_2H_5$ | - | - | O | H | $CH_3$ | $OCH_3$ | N | |
| H | O | - | Q-2 | $C_2H_5$ | - | - | O | H | $OCH_3$ | $OCH_3$ | N | |
| H | O | - | Q-3 | - | $CH_3$ | - | O | H | $CH_3$ | $OCH_3$ | CH | |
| H | O | - | Q-3 | - | $CH_3$ | - | O | H | Cl | $OCH_3$ | CH | |
| H | O | - | Q-3 | - | $CH_3$ | - | O | H | $OCH_3$ | $OCH_3$ | CH | |
| H | O | - | Q-3 | - | $CH_3$ | - | O | H | $OCH_3$ | $OCH_3$ | N | |
| H | O | - | Q-3 | - | $CH_3$ | - | O | H | $CH_3$ | $OCH_3$ | N | |
| H | O | - | Q-3 | - | $C_2H_5$ | - | O | H | $CH_3$ | $CH_3$ | CH | |
| H | O | - | Q-3 | - | $C_2H_5$ | - | O | H | $CH_3$ | $OCH_3$ | CH | |
| H | O | - | Q-3 | - | $C_2H_5$ | - | O | H | Cl | $OCH_3$ | CH | |

Table VIII (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-3 | - | $C_2H_5$ | - | O | H | $OCH_3$ | $OCH_3$ | CH | |
| H | O | - | Q-3 | - | $C_2H_5$ | - | O | H | $CH_3$ | $OCH_3$ | N | |
| H | O | - | Q-3 | - | $CH_2Cl$ | - | O | H | $CH_3$ | $OCH_3$ | CH | |
| H | O | - | Q-3 | - | $CH_2Cl$ | - | O | H | $OCH_3$ | $OCH_3$ | CH | |
| H | O | - | Q-3 | - | $CH_2Cl$ | - | O | H | Cl | $OCH_3$ | CH | |
| H | O | - | Q-3 | - | $CH_2Cl$ | - | O | H | $CH_3$ | $OCH_3$ | N | |
| H | O | - | Q-3 | - | $CH_2Cl$ | - | O | H | $OCH_3$ | $OCH_3$ | N | |
| H | O | - | Q-3 | - | $CF_3$ | - | O | H | $CH_3$ | $CH_3$ | CH | |
| H | O | - | Q-3 | - | $CF_3$ | - | O | H | $OCH_3$ | $OCH_3$ | CH | |
| H | O | - | Q-3 | - | $CF_3$ | - | O | H | $OCH_3$ | $CH_3$ | CH | |
| H | O | - | Q-3 | - | $CF_3$ | - | O | H | $CH_3$ | $OCH_3$ | N | |
| H | O | - | Q-3 | - | $CH(CH_3)_2$ | - | O | H | $CH_3$ | $OCH_3$ | CH | |
| H | O | - | Q-3 | - | $CH(CH_3)_2$ | - | O | H | $OCH_3$ | $OCH_3$ | CH | |
| H | O | - | Q-3 | - | $CH(CH_3)_2$ | - | O | H | Cl | $OCH_3$ | CH | |
| H | O | - | Q-3 | - | $CH(CH_3)_2$ | - | O | H | $CH_3$ | $OCH_3$ | N | |
| H | O | - | Q-3 | - | $OCH_3$ | - | O | H | $OCH_3$ | $OCH_3$ | CH | |
| H | O | - | Q-3 | - | $OCH_3$ | - | O | H | $CH_3$ | $OCH_3$ | CH | |
| H | O | - | Q-3 | - | $OCH_3$ | - | O | H | Cl | $CH_3$ | CH | |
| H | O | - | Q-3 | - | $OCH_3$ | - | O | H | $OCH_3$ | $OCH_3$ | N | |
| H | O | - | Q-3 | - | $SCH_3$ | - | O | H | $CH_3$ | $OCH_3$ | CH | |
| H | O | - | Q-3 | - | $SCH_3$ | - | O | H | $OCH_3$ | $OCH_3$ | CH | |
| H | O | - | Q-3 | - | $SCH_3$ | - | O | H | $OCH_3$ | $OCH_3$ | N | |
| H | O | - | Q-3 | - | $SCH_3$ | - | O | H | $CH_3$ | $OCH_3$ | N | |
| H | O | - | Q-3 | - | $N(CH_3)_2$ | - | O | H | $CH_3$ | $CH_3$ | CH | |
| H | O | - | Q-3 | - | $N(CH_3)_2$ | - | O | H | $OCH_3$ | $OCH_3$ | CH | |
| H | O | - | Q-3 | - | $N(CH_3)_2$ | - | O | H | $OCH_3$ | $CH_3$ | N | |
| H | O | - | Q-4 | - | $CH_3$ | - | O | H | $OCH_3$ | $CH_3$ | CH | |
| H | O | - | Q-4 | - | $CH_3$ | - | O | H | $OCH_3$ | $OCH_3$ | CH | |
| H | O | - | Q-4 | - | $CH_3$ | - | O | H | $CH_3$ | $CH_3$ | CH | |
| H | O | - | Q-5 | - | - | $CH_3$ | O | H | $CH_3$ | $CH_3$ | CH | |
| H | O | - | Q-5 | - | - | $CH_3$ | O | H | $OCH_3$ | $CH_3$ | CH | |
| H | O | - | Q-5 | - | - | $CH_3$ | O | H | $OCH_3$ | $OCH_3$ | CH | |

Table VIII (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | 0 | - | Q-5 | - | - | $CH_3$ | O | H | Cl | $OCH_3$ | CH | |
| H | 0 | - | Q-5 | - | - | $CH_3$ | O | H | $CH_3$ | $OCH_3$ | CH | |
| H | 0 | - | Q-5 | - | - | $C_2H_5$ | O | H | $CH_3$ | $OCH_3$ | CH | |
| H | 0 | - | Q-5 | - | - | $C_2H_5$ | O | H | Cl | $OCH_3$ | CH | |
| H | 0 | - | Q-5 | - | - | $C_2H_5$ | O | H | $CH_3$ | $CH_3$ | CH | |
| H | 0 | - | Q-5 | - | - | $C_2H_5$ | O | H | $OCH_3$ | $OCH_3$ | CH | |
| H | 0 | - | Q-5 | - | - | $C_2H_5$ | O | H | $OCH_3$ | $CH_3$ | N | |
| 3-$CH_3$ | 0 | - | Q-1 | $CH_3$ | - | - | O | H | $OCH_3$ | $CH_3$ | CH | |
| 3-$CH_3$ | 0 | - | Q-1 | $CH_3$ | - | - | O | H | $OCH_3$ | $OCH_3$ | CH | |
| 3-$CH_3$ | 0 | - | Q-1 | $CH_3$ | - | - | O | H | Cl | $OCH_3$ | CH | |
| 3-$CH_3$ | 0 | - | Q-1 | $CH_3$ | - | - | O | H | $CH_3$ | $CH_3$ | CH | |
| 3-$CH_3$ | 0 | - | Q-1 | $CH_3$ | - | - | O | H | $OCH_3$ | $CH_3$ | N | |
| 3-Cl | 0 | - | Q-1 | $CH_3$ | - | - | O | H | $OCH_3$ | $CH_3$ | N | |
| 3-Cl | 0 | - | Q-1 | $CH_3$ | - | - | O | H | $OCH_3$ | $CH_3$ | CH | |
| 3-Cl | 0 | - | Q-1 | $CH_3$ | - | - | O | H | $OCH_3$ | $OCH_3$ | CH | |
| 3-Cl | 0 | - | Q-1 | $CH_3$ | - | - | O | H | Cl | $OCH_3$ | CH | |
| 3-Cl | 0 | - | Q-1 | $CH_3$ | - | - | O | H | $CH_3$ | $CH_3$ | CH | |
| 3-Cl | 0 | - | Q-2 | $CH_3$ | - | - | O | H | $OCH_3$ | $OCH_3$ | CH | |
| 3-Cl | 0 | - | Q-2 | $CH_3$ | - | - | O | H | Cl | $OCH_3$ | CH | |
| H | 1 | - | Q-1 | $CH_3$ | - | - | O | H | Cl | $OCH_3$ | CH | |
| H | 1 | - | Q-1 | $CH_3$ | - | - | O | H | $OCH_3$ | $OCH_3$ | CH | |
| H | 1 | - | Q-1 | $CH_3$ | - | - | O | H | $CH_3$ | $CH_3$ | CH | |
| H | 1 | - | Q-5 | - | - | $CH_3$ | O | H | $CH_3$ | $CH_3$ | CH | |
| H | 1 | - | Q-5 | - | - | $CH_3$ | O | H | $OCH_3$ | $OCH_3$ | CH | |
| H | 1 | - | Q-5 | - | - | $CH_3$ | O | H | Cl | $OCH_3$ | CH | |

Table IX

General Structure 9

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-1 | $CH_3$ | - | - | O | H | $CH_3$ | $CH_3$ | CH | |
| H | O | - | Q-1 | $CH_3$ | - | - | O | H | $OCH_3$ | $CH_3$ | CH | |
| H | O | - | Q-1 | $CH_3$ | - | - | O | H | Cl | $OCH_3$ | CH | |
| H | O | - | Q-1 | $CH_3$ | - | - | O | H | $CH_3$ | $OCH_3$ | N | |
| H | O | - | Q-1 | $CH_3$ | - | - | O | H | $OCH_3$ | $OCH_3$ | N | |
| H | O | - | Q-1 | $C_2H_5$ | - | - | O | H | $OCH_3$ | $CH_3$ | CH | |
| H | O | - | Q-1 | $C_2H_5$ | - | - | O | H | $OCH_3$ | $OCH_3$ | CH | |
| H | O | - | Q-1 | $C_2H_5$ | - | - | O | H | Cl | $OCH_3$ | CH | |
| H | O | - | Q-1 | $C_2H_5$ | - | - | O | H | $CH_3$ | $CH_3$ | CH | |
| H | O | - | Q-1 | $C_2H_5$ | - | - | O | H | $CH_3$ | $OCH_3$ | N | |
| H | O | - | Q-2 | $CH_3$ | - | - | O | H | $OCH_3$ | $OCH_3$ | N | |
| H | O | - | Q-2 | $CH_3$ | - | - | O | H | $OCH_3$ | $OCH_3$ | CH | |
| H | O | - | Q-2 | $CH_3$ | - | - | O | H | $OCH_3$ | $CH_3$ | CH | |
| H | O | - | Q-2 | $CH_3$ | - | - | O | H | Cl | $OCH_3$ | CH | |
| H | O | - | Q-2 | $CH_3$ | - | - | O | H | $CH_3$ | $CH_3$ | CH | |
| H | O | - | Q-2 | $CH_3$ | - | - | O | H | $OCH_3$ | $CH_3$ | N | |
| H | O | - | Q-2 | $C_2H_5$ | - | - | O | H | $OCH_3$ | $OCH_3$ | N | |
| H | O | - | Q-2 | $C_2H_5$ | - | - | O | H | $CH_3$ | $CH_3$ | CH | |
| H | O | - | Q-2 | $C_2H_5$ | - | - | O | H | $CH_3$ | $OCH_3$ | CH | |
| H | O | - | Q-2 | $C_2H_5$ | - | - | O | H | Cl | $OCH_3$ | CH | |
| H | O | - | Q-2 | $C_2H_5$ | - | - | O | H | $CH_3$ | $OCH_3$ | N | |
| H | O | - | Q-2 | $C_2H_5$ | - | - | O | H | $OCH_3$ | $OCH_3$ | N | |
| H | O | - | Q-3 | - | $CH_3$ | - | O | H | $CH_3$ | $OCH_3$ | CH | |
| H | O | - | Q-3 | - | $CH_3$ | - | O | H | Cl | $OCH_3$ | CH | |
| H | O | - | Q-3 | - | $CH_3$ | - | O | H | $OCH_3$ | $OCH_3$ | CH | |
| H | O | - | Q-3 | - | $CH_3$ | - | O | H | $OCH_3$ | $OCH_3$ | N | |
| H | O | - | Q-3 | - | $CH_3$ | - | O | H | $CH_3$ | $OCH_3$ | N | |
| H | O | - | Q-3 | - | $C_2H_5$ | - | O | H | $CH_3$ | $CH_3$ | CH | |
| H | O | - | Q-3 | - | $C_2H_5$ | - | O | H | $CH_3$ | $OCH_3$ | CH | |
| H | O | - | Q-3 | - | $C_2H_5$ | - | O | H | Cl | $OCH_3$ | CH | |

Table IX (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-3 | - | $C_2H_5$ | - | O | H | $OCH_3$ | $OCH_3$ | CH | |
| H | O | - | Q-3 | - | $C_2H_5$ | - | O | H | $CH_3$ | $OCH_3$ | N | |
| H | O | - | Q-3 | - | $CH_2Cl$ | - | O | H | $CH_3$ | $OCH_3$ | CH | |
| H | O | - | Q-3 | - | $CH_2Cl$ | - | O | H | $OCH_3$ | $OCH_3$ | CH | |
| H | O | - | Q-3 | - | $CH_2Cl$ | - | O | H | Cl | $OCH_3$ | CH | |
| H | O | - | Q-3 | - | $CH_2Cl$ | - | O | H | $CH_3$ | $OCH_3$ | N | |
| H | O | - | Q-3 | - | $CH_2Cl$ | - | O | H | $OCH_3$ | $OCH_3$ | N | |
| H | O | - | Q-3 | - | $CF_3$ | - | O | H | $CH_3$ | $CH_3$ | CH | |
| H | O | - | Q-3 | - | $CF_3$ | - | O | H | $OCH_3$ | $OCH_3$ | CH | |
| H | O | - | Q-3 | - | $CF_3$ | - | O | H | $OCH_3$ | $CH_3$ | CH | |
| H | O | - | Q-3 | - | $CF_3$ | - | O | H | $CH_3$ | $OCH_3$ | N | |
| H | O | - | Q-3 | - | $CH(CH_3)_2$ | - | O | H | $CH_3$ | $OCH_3$ | CH | |
| H | O | - | Q-3 | - | $CH(CH_3)_2$ | - | O | H | $OCH_3$ | $OCH_3$ | CH | |
| H | O | - | Q-3 | - | $CH(CH_3)_2$ | - | O | H | Cl | $OCH_3$ | CH | |
| H | O | - | Q-3 | - | $CH(CH_3)_2$ | - | O | H | $CH_3$ | $OCH_3$ | N | |
| H | O | - | Q-3 | - | $OCH_3$ | - | O | H | $OCH_3$ | $OCH_3$ | CH | |
| H | O | - | Q-3 | - | $OCH_3$ | - | O | H | $CH_3$ | $OCH_3$ | CH | |
| H | O | - | Q-3 | - | $OCH_3$ | - | O | H | Cl | $CH_3$ | CH | |
| H | O | - | Q-3 | - | $OCH_3$ | - | O | H | $OCH_3$ | $OCH_3$ | N | |
| H | O | - | Q-3 | - | $SCH_3$ | - | O | H | $CH_3$ | $OCH_3$ | CH | |
| H | O | - | Q-3 | - | $SCH_3$ | - | O | H | $OCH_3$ | $OCH_3$ | CH | |
| H | O | - | Q-3 | - | $SCH_3$ | - | O | H | $OCH_3$ | $OCH_3$ | N | |
| H | O | - | Q-3 | - | $SCH_3$ | - | O | H | $CH_3$ | $OCH_3$ | N | |
| H | O | - | Q-3 | - | $N(CH_3)_2$ | - | O | H | $CH_3$ | $CH_3$ | CH | |
| H | O | - | Q-3 | - | $N(CH_3)_2$ | - | O | H | $OCH_3$ | $OCH_3$ | CH | |
| H | O | - | Q-3 | - | $N(CH_3)_2$ | - | O | H | $OCH_3$ | $CH_3$ | N | |
| H | O | - | Q-4 | - | $CH_3$ | - | O | H | $OCH_3$ | $CH_3$ | N | |
| H | O | - | Q-4 | - | $CH_3$ | - | O | H | $OCH_3$ | $CH_3$ | CH | |
| H | O | - | Q-4 | - | $CH_3$ | - | O | H | $OCH_3$ | $OCH_3$ | CH | |
| H | O | - | Q-4 | - | $CH_3$ | - | O | H | Cl | $OCH_3$ | CH | |
| H | O | - | Q-4 | - | $CH_3$ | - | O | H | $CH_3$ | $CH_3$ | CH | |

Table IX (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | 0 | - | Q-5 | - | - | $CH_3$ | O | H | $CH_3$ | $CH_3$ | CH | |
| H | 0 | - | Q-5 | - | - | $CH_3$ | O | H | $OCH_3$ | $CH_3$ | CH | |
| H | 0 | - | Q-5 | - | - | $CH_3$ | O | H | $OCH_3$ | $OCH_3$ | CH | |
| H | 0 | - | Q-5 | - | - | $CH_3$ | O | H | Cl | $OCH_3$ | CH | |
| H | 0 | - | Q-5 | - | - | $CH_3$ | O | H | $CH_3$ | $OCH_3$ | N | |
| 4-$CH_3$ | 0 | - | Q-1 | $CH_3$ | - | - | O | H | $CH_3$ | $CH_3$ | CH | |
| 4-$CH_3$ | 0 | - | Q-1 | $CH_3$ | - | - | O | H | $CH_3$ | $OCH_3$ | CH | |
| 4-$CH_3$ | 0 | - | Q-1 | $CH_3$ | - | - | O | H | $OCH_3$ | $OCH_3$ | CH | |
| 4-$CH_3$ | 0 | - | Q-1 | $CH_3$ | - | - | O | H | Cl | $OCH_3$ | CH | |
| 4-$CH_3$ | 0 | - | Q-1 | $CH_3$ | - | - | O | H | $CH_3$ | $OCH_3$ | N | |
| 4-Br | 0 | - | Q-1 | $CH_3$ | - | - | O | H | $CH_3$ | $CH_3$ | CH | |
| 4-Br | 0 | - | Q-1 | $CH_3$ | - | - | O | H | $CH_3$ | $OCH_3$ | CH | |
| 4-Br | 0 | - | Q-1 | $CH_3$ | - | - | O | H | $OCH_3$ | $OCH_3$ | CH | |
| 4-Br | 0 | - | Q-1 | $CH_3$ | - | - | O | H | Cl | $OCH_3$ | CH | |
| 4-Cl | 0 | - | Q-1 | $CH_3$ | - | - | O | H | $CH_3$ | $OCH_3$ | N | |
| 4-Cl | 0 | - | Q-1 | $CH_3$ | - | - | O | H | $CH_3$ | $OCH_3$ | CH | |
| 4-Cl | 0 | - | Q-1 | $CH_3$ | - | - | O | H | $OCH_3$ | $OCH_3$ | CH | |
| 4-Cl | 0 | - | Q-1 | $CH_3$ | - | - | O | H | Cl | $OCH_3$ | CH | |
| 4-Cl | 0 | - | Q-1 | $CH_3$ | - | - | O | H | $CH_3$ | $CH_3$ | CH | |
| 4-$CH_3$ | 0 | - | Q-2 | $CH_3$ | - | - | O | H | $CH_3$ | $CH_3$ | CH | |
| 4-$CH_3$ | 0 | - | Q-2 | $CH_3$ | - | - | O | H | $CH_3$ | $OCH_3$ | CH | |
| 4-$CH_3$ | 0 | - | Q-2 | $CH_3$ | - | - | O | H | $OCH_3$ | $OCH_3$ | CH | |
| 4-$CH_3$ | 0 | - | Q-2 | $CH_3$ | - | - | O | H | Cl | $OCH_3$ | CH | |
| 4-$CH_3$ | 0 | - | Q-5 | - | - | $CH_3$ | O | H | Cl | $OCH_3$ | CH | |
| 4-$CH_3$ | 0 | - | Q-5 | - | - | $CH_3$ | O | H | $OCH_3$ | $OCH_3$ | CH | |
| 4-$CH_3$ | 0 | - | Q-5 | - | - | $CH_3$ | O | H | $CH_3$ | $OCH_3$ | CH | |
| 4-$CH_3$ | 0 | - | Q-5 | - | - | $CH_3$ | O | H | $CH_3$ | $CH_3$ | CH | |

Table X

General Structure 10

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-1 | $CH_3$ | - | - | O | H | $CH_3$ | $CH_3$ | CH | |
| H | O | - | Q-1 | $CH_3$ | - | - | O | H | $OCH_3$ | $CH_3$ | CH | |
| H | O | - | Q-1 | $CH_3$ | - | - | O | H | Cl | $OCH_3$ | CH | |
| H | O | - | Q-1 | $CH_3$ | - | - | O | H | $CH_3$ | $OCH_3$ | N | |
| H | O | - | Q-1 | $CH_3$ | - | - | O | H | $OCH_3$ | $OCH_3$ | N | |
| H | O | - | Q-1 | $C_2H_5$ | - | - | O | H | $OCH_3$ | $CH_3$ | CH | |
| H | O | - | Q-1 | $C_2H_5$ | - | - | O | H | $OCH_3$ | $OCH_3$ | CH | |
| H | O | - | Q-1 | $C_2H_5$ | - | - | O | H | Cl | $OCH_3$ | CH | |
| H | O | - | Q-1 | $C_2H_5$ | - | - | O | H | $CH_3$ | $CH_3$ | CH | |
| H | O | - | Q-1 | $C_2H_5$ | - | - | O | H | $CH_3$ | $OCH_3$ | N | |
| H | O | - | Q-2 | $CH_3$ | - | - | O | H | $OCH_3$ | $OCH_3$ | N | |
| H | O | - | Q-2 | $CH_3$ | - | - | O | H | $OCH_3$ | $OCH_3$ | CH | |
| H | O | - | Q-2 | $CH_3$ | - | - | O | H | $OCH_3$ | $CH_3$ | CH | |
| H | O | - | Q-2 | $CH_3$ | - | - | O | H | Cl | $OCH_3$ | CH | |
| H | O | - | Q-2 | $CH_3$ | - | - | O | H | $CH_3$ | $CH_3$ | CH | |
| H | O | - | Q-2 | $CH_3$ | - | - | O | H | $OCH_3$ | $CH_3$ | N | |
| H | O | - | Q-2 | $C_2H_5$ | - | - | O | H | $OCH_3$ | $OCH_3$ | N | |
| H | O | - | Q-2 | $C_2H_5$ | - | - | O | H | $CH_3$ | $CH_3$ | CH | |
| H | O | - | Q-2 | $C_2H_5$ | - | - | O | H | $CH_3$ | $OCH_3$ | CH | |
| H | O | - | Q-2 | $C_2H_5$ | - | - | O | H | Cl | $OCH_3$ | CH | |
| H | O | - | Q-2 | $C_2H_5$ | - | - | O | H | $CH_3$ | $OCH_3$ | N | |
| H | O | - | Q-2 | $C_2H_5$ | - | - | O | H | $OCH_3$ | $OCH_3$ | N | |
| H | O | - | Q-3 | - | $CH_3$ | - | O | H | $CH_3$ | $OCH_3$ | CH | |
| H | O | - | Q-3 | - | $CH_3$ | - | O | H | Cl | $OCH_3$ | CH | |
| H | O | - | Q-3 | - | $CH_3$ | - | O | H | $OCH_3$ | $OCH_3$ | CH | |
| H | O | - | Q-3 | - | $CH_3$ | - | O | H | $OCH_3$ | $OCH_3$ | N | |
| H | O | - | Q-3 | - | $CH_3$ | - | O | H | $CH_3$ | $OCH_3$ | N | |
| H | O | - | Q-3 | - | $C_2H_5$ | - | O | H | $CH_3$ | $CH_3$ | CH | |
| H | O | - | Q-3 | - | $C_2H_5$ | - | O | H | $CH_3$ | $OCH_3$ | CH | |
| H | O | - | Q-3 | - | $C_2H_5$ | - | O | H | Cl | $OCH_3$ | CH | |

Table X (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-3 | - | $C_2H_5$ | - | O | H | $OCH_3$ | $OCH_3$ | CH | |
| H | O | - | Q-3 | - | $C_2H_5$ | - | O | H | $CH_3$ | $OCH_3$ | N | |
| H | O | - | Q-3 | - | $CH_2Cl$ | - | O | H | $CH_3$ | $OCH_3$ | CH | |
| H | O | - | Q-3 | - | $CH_2Cl$ | - | O | H | $OCH_3$ | $OCH_3$ | CH | |
| H | O | - | Q-3 | - | $CH_2Cl$ | - | O | H | Cl | $OCH_3$ | CH | |
| H | O | - | Q-3 | - | $CH_2Cl$ | - | O | H | $CH_3$ | $OCH_3$ | N | |
| H | O | - | Q-3 | - | $CH_2Cl$ | - | O | H | $OCH_3$ | $OCH_3$ | N | |
| H | O | - | Q-3 | - | $CF_3$ | - | O | H | $CH_3$ | $CH_3$ | CH | |
| H | O | - | Q-3 | - | $CF_3$ | - | O | H | $OCH_3$ | $OCH_3$ | CH | |
| H | O | - | Q-3 | - | $CF_3$ | - | O | H | $OCH_3$ | $CH_3$ | CH | |
| H | O | - | Q-3 | - | $CF_3$ | - | O | H | $CH_3$ | $OCH_3$ | N | |
| H | O | - | Q-3 | - | $CH(CH_3)_2$ | - | O | H | $CH_3$ | $OCH_3$ | CH | |
| H | O | - | Q-3 | - | $CH(CH_3)_2$ | - | O | H | $OCH_3$ | $OCH_3$ | CH | |
| H | O | - | Q-3 | - | $CH(CH_3)_2$ | - | O | H | Cl | $OCH_3$ | CH | |
| H | O | - | Q-3 | - | $CH(CH_3)_2$ | - | O | H | $CH_3$ | $OCH_3$ | N | |
| H | O | - | Q-3 | - | $OCH_3$ | - | O | H | $OCH_3$ | $OCH_3$ | CH | |
| H | O | - | Q-3 | - | $OCH_3$ | - | O | H | $CH_3$ | $OCH_3$ | CH | |
| H | O | - | Q-3 | - | $OCH_3$ | - | O | H | Cl | $CH_3$ | CH | |
| H | O | - | Q-3 | - | $OCH_3$ | - | O | H | $OCH_3$ | $OCH_3$ | N | |
| H | O | - | Q-3 | - | $SCH_3$ | - | O | H | $CH_3$ | $OCH_3$ | CH | |
| H | O | - | Q-3 | - | $SCH_3$ | - | O | H | $OCH_3$ | $OCH_3$ | CH | |
| H | O | - | Q-3 | - | $SCH_3$ | - | O | H | $OCH_3$ | $OCH_3$ | N | |
| H | O | - | Q-3 | - | $SCH_3$ | - | O | H | $CH_3$ | $OCH_3$ | N | |
| H | O | - | Q-3 | - | $N(CH_3)_2$ | - | O | H | $CH_3$ | $CH_3$ | CH | |
| H | O | - | Q-3 | - | $N(CH_3)_2$ | - | O | H | $OCH_3$ | $OCH_3$ | CH | |
| H | O | - | Q-3 | - | $N(CH_3)_2$ | - | O | H | $OCH_3$ | $CH_3$ | N | |
| H | O | - | Q-4 | - | $CH_3$ | - | O | H | $OCH_3$ | $CH_3$ | N | |
| H | O | - | Q-4 | - | $CH_3$ | - | O | H | $OCH_3$ | $CH_3$ | CH | |
| H | O | - | Q-4 | - | $CH_3$ | - | O | H | $OCH_3$ | $OCH_3$ | CH | |
| H | O | - | Q-4 | - | $CH_3$ | - | O | H | Cl | $OCH_3$ | CH | |
| H | O | - | Q-4 | - | $CH_3$ | - | O | H | $CH_3$ | $CH_3$ | CH | |

## Table X (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | 0 | - | Q-5 | - | $CH_3$ | $CH_3$ | O | H | $CH_3$ | $CH_3$ | CH | |
| H | 0 | - | Q-5 | - | $CH_3$ | $CH_3$ | O | H | $CH_3$ | $OCH_3$ | CH | |
| H | 0 | - | Q-5 | - | $CH_3$ | $CH_3$ | O | H | $OCH_3$ | $OCH_3$ | CH | |
| H | 0 | - | Q-5 | - | $CH_3$ | $CH_3$ | O | H | Cl | $OCH_3$ | CH | |

Table XI

General Structure 11

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | 0 | – | Q-1 | $C_6H_5$ | $CH_3$ | – | – | O | H | $CH_3$ | $CH_3$ | CH | |
| H | 0 | – | Q-1 | $C_6H_5$ | $CH_3$ | – | – | O | H | $OCH_3$ | $CH_3$ | CH | |
| H | 0 | – | Q-1 | $C_6H_5$ | $CH_3$ | – | – | O | H | Cl | $OCH_3$ | CH | |
| H | 0 | – | Q-1 | $C_6H_5$ | $CH_3$ | – | – | O | H | $CH_3$ | $OCH_3$ | N | |
| H | 0 | – | Q-1 | $C_6H_5$ | $CH_3$ | – | – | O | H | $OCH_3$ | $OCH_3$ | N | |
| H | 0 | – | Q-1 | $C_6H_5$ | $C_2H_5$ | – | – | O | H | $OCH_3$ | $CH_3$ | CH | |
| H | 0 | – | Q-1 | $C_6H_5$ | $C_2H_5$ | – | – | O | H | $OCH_3$ | $OCH_3$ | CH | |
| H | 0 | – | Q-1 | $C_6H_5$ | $C_2H_5$ | – | – | O | H | Cl | $OCH_3$ | CH | |
| H | 0 | – | Q-1 | $C_6H_5$ | $C_2H_5$ | – | – | O | H | $CH_3$ | $CH_3$ | CH | |
| H | 0 | – | Q-1 | $C_6H_5$ | $C_2H_5$ | – | – | O | H | $CH_3$ | $OCH_3$ | N | |
| H | 0 | – | Q-2 | $C_6H_5$ | $CH_3$ | – | – | O | H | $OCH_3$ | $OCH_3$ | N | |
| H | 0 | – | Q-2 | $C_6H_5$ | $CH_3$ | – | – | O | H | $OCH_3$ | $OCH_3$ | CH | |
| H | 0 | – | Q-2 | $C_6H_5$ | $CH_3$ | – | – | O | H | $OCH_3$ | $CH_3$ | CH | |
| H | 0 | – | Q-2 | $C_6H_5$ | $CH_3$ | – | – | O | H | Cl | $OCH_3$ | CH | |
| H | 0 | – | Q-2 | $C_6H_5$ | $CH_3$ | – | – | O | H | $CH_3$ | $CH_3$ | CH | |
| H | 0 | – | Q-2 | $C_6H_5$ | $CH_3$ | – | – | O | H | $OCH_3$ | $CH_3$ | N | |
| H | 0 | – | Q-2 | $C_6H_5$ | $C_2H_5$ | – | – | O | H | $OCH_3$ | $OCH_3$ | N | |
| H | 0 | – | Q-2 | C $H_5$ | $C_2H_5$ | – | – | O | H | $CH_3$ | $CH_3$ | CH | |
| H | 0 | – | Q-2 | $C_6H_5$ | $C_2H_5$ | – | – | O | H | $CH_3$ | $OCH_3$ | CH | |
| H | 0 | – | Q-2 | $C_6H_5$ | $C_2H_5$ | – | – | O | H | Cl | $OCH_3$ | CH | |
| H | 0 | – | Q-2 | $C_6H_5$ | $C_2H_5$ | – | – | O | H | $CH_3$ | $OCH_3$ | N | |
| H | 0 | – | Q-2 | $C_6H_5$ | $C_2H_5$ | – | – | O | H | $OCH_3$ | $OCH_3$ | N | |
| H | 0 | – | Q-3 | $C_6H_5$ | – | $CH_3$ | – | O | H | $CH_3$ | $OCH_3$ | CH | |
| H | 0 | – | Q-3 | $C_6H_5$ | – | $CH_3$ | – | O | H | Cl | $OCH_3$ | CH | |
| H | 0 | – | Q-3 | $C_6H_5$ | – | $CH_3$ | – | O | H | $OCH_3$ | $OCH_3$ | CH | |
| H | 0 | – | Q-3 | $C_6H_5$ | – | $CH_3$ | – | O | H | $OCH_3$ | $OCH_3$ | N | |
| H | 0 | – | Q-3 | $C_6H_5$ | – | $CH_3$ | – | O | H | $CH_3$ | $OCH_3$ | N | |
| H | 0 | – | Q-3 | $C_6H_5$ | – | $C_2H_5$ | – | O | H | $CH_3$ | $CH_3$ | CH | |
| H | 0 | – | Q-3 | $C_6H_5$ | – | $C_2H_5$ | – | O | H | $CH_3$ | $OCH_3$ | CH | |
| H | 0 | – | Q-3 | $C_6H_5$ | – | $C_2H_5$ | – | O | H | Cl | $OCH_3$ | CH | |

Table XI (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-3 | $C_6H_5$ | - | $C_2H_5$ | - | O | H | $OCH_3$ | $OCH_3$ | CH | |
| H | O | - | Q-3 | $C_6H_5$ | - | $C_2H_5$ | - | O | H | $CH_3$ | $OCH_3$ | N | |
| H | O | - | Q-3 | $C_6H_5$ | - | $CH_2Cl$ | - | O | H | $CH_3$ | $OCH_3$ | CH | |
| H | O | - | Q-3 | $C_6H_5$ | - | $CH_2Cl$ | - | O | H | $OCH_3$ | $OCH_3$ | CH | |
| H | O | - | Q-3 | $C_6H_5$ | - | $CH_2Cl$ | - | O | H | Cl | $OCH_3$ | CH | |
| H | O | - | Q-3 | $C_6H_5$ | - | $CH_2Cl$ | - | O | H | $CH_3$ | $OCH_3$ | N | |
| H | O | - | Q-3 | $C_6H_5$ | - | $CH_2Cl$ | - | O | H | $OCH_3$ | $OCH_3$ | N | |
| H | O | - | Q-3 | $C_6H_5$ | - | $CF_3$ | - | O | H | $CH_3$ | $CH_3$ | CH | |
| H | O | - | Q-3 | $C_6H_5$ | - | $CF_3$ | - | O | H | $OCH_3$ | $OCH_3$ | CH | |
| H | O | - | Q-3 | $C_6H_5$ | - | $CF_3$ | - | O | H | $OCH_3$ | $CH_3$ | CH | |
| H | O | - | Q-3 | $C_6H_5$ | - | $CF_3$ | - | O | H | $CH_3$ | $OCH_3$ | N | |
| H | O | - | Q-3 | $C_6H_5$ | - | $CH(CH_3)_2$ | - | O | H | $CH_3$ | $OCH_3$ | CH | |
| H | O | - | Q-3 | $C_6H_5$ | - | $CH(CH_3)_2$ | - | O | H | $OCH_3$ | $OCH_3$ | CH | |
| H | O | - | Q-3 | $C_6H_5$ | - | $CH(CH_3)_2$ | - | O | H | Cl | $OCH_3$ | CH | |
| H | O | - | Q-3 | $C_6H_5$ | - | $CH(CH_3)_2$ | - | O | H | $CH_3$ | $OCH_3$ | N | |
| H | O | - | Q-3 | $C_6H_5$ | - | $OCH_3$ | - | O | H | $OCH_3$ | $OCH_3$ | CH | |
| H | O | - | Q-3 | $C_6H_5$ | - | $OCH_3$ | - | O | H | $CH_3$ | $OCH_3$ | CH | |
| H | O | - | Q-3 | $C_6H_5$ | - | $OCH_3$ | - | O | H | Cl | $CH_3$ | CH | |
| H | O | - | Q-3 | $C_6H_5$ | - | $OCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | CH | |
| H | O | - | Q-3 | $C_6H_5$ | - | $SCH_3$ | - | O | H | $OCH_3$ | $OCH_3$ | CH | |
| H | O | - | Q-3 | $C_6H_5$ | - | $SCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | N | |
| H | O | - | Q-3 | $C_6H_5$ | - | $N(CH_3)_2$ | - | O | H | $CH_3$ | $CH_3$ | CH | |
| H | O | - | Q-3 | $C_6H_5$ | - | $N(CH_3)_2$ | - | O | H | $OCH_3$ | $OCH_3$ | CH | |
| H | O | - | Q-3 | $C_6H_5$ | - | $N(CH_3)_2$ | - | O | H | $OCH_3$ | $CH_3$ | N | |
| H | O | - | Q-5 | $C_6H_5$ | - | - | $CH_3$ | O | H | $OCH_3$ | $CH_3$ | N | |
| H | O | - | Q-5 | $C_6H_5$ | - | - | $CH_3$ | O | H | $OCH_3$ | $CH_3$ | CH | |
| H | O | - | Q-5 | $C_6H_5$ | - | - | $CH_3$ | O | H | $OCH_3$ | $OCH_3$ | CH | |
| H | O | - | Q-5 | $C_6H_5$ | - | - | $CH_3$ | O | H | Cl | $OCH_3$ | CH | |
| H | O | - | Q-5 | $C_6H_5$ | - | - | $CH_3$ | O | H | $CH_3$ | $CH_3$ | CH | |

Table XI (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | – | Q-1 | $CH_3$ | $CH_3$ | – | – | O | H | $CH_3$ | $CH_3$ | CH | |
| H | O | – | Q-1 | $CH_3$ | $CH_3$ | – | – | O | H | $OCH_3$ | $CH_3$ | CH | |
| H | O | – | Q-1 | $CH_3$ | $CH_3$ | – | – | O | H | Cl | $OCH_3$ | CH | |
| H | O | – | Q-1 | $CH_3$ | $CH_3$ | – | – | O | H | $CH_3$ | $OCH_3$ | N | |
| H | O | – | Q-1 | $CH_3$ | $CH_3$ | – | – | O | H | $OCH_3$ | $OCH_3$ | N | |
| H | O | – | Q-1 | $CH_3$ | $C_2H_5$ | – | – | O | H | $OCH_3$ | $CH_3$ | CH | |
| H | O | – | Q-1 | $CH_3$ | $C_2H_5$ | – | – | O | H | $OCH_3$ | $OCH_3$ | CH | |
| H | O | – | Q-1 | $CH_3$ | $C_2H_5$ | – | – | O | H | Cl | $OCH_3$ | CH | |
| H | O | – | Q-1 | $CH_3$ | $C_2H_5$ | – | – | O | H | $CH_3$ | $CH_3$ | CH | |
| H | O | – | Q-1 | $CH_3$ | $C_2H_5$ | – | – | O | H | $CH_3$ | $OCH_3$ | N | |
| H | O | – | Q-2 | $CH_3$ | $CH_3$ | – | – | O | H | $OCH_3$ | $OCH_3$ | N | |
| H | O | – | Q-2 | $CH_3$ | $CH_3$ | – | – | O | H | $OCH_3$ | $OCH_3$ | CH | |
| H | O | – | Q-2 | $CH_3$ | $CH_3$ | – | – | O | H | $OCH_3$ | $CH_3$ | CH | |
| H | O | – | Q-2 | $CH_3$ | $CH_3$ | – | – | O | H | Cl | $OCH_3$ | CH | |
| H | O | – | Q-2 | $CH_3$ | $CH_3$ | – | – | O | H | $CH_3$ | $CH_3$ | CH | |
| H | O | – | Q-2 | $CH_3$ | $CH_3$ | – | – | O | H | $OCH_3$ | $CH_3$ | N | |
| H | O | – | Q-2 | $CH_3$ | $C_2H_5$ | – | – | O | H | $OCH_3$ | $OCH_3$ | N | |
| H | O | – | Q-2 | $CH_3$ | $C_2H_5$ | – | – | O | H | $CH_3$ | $CH_3$ | CH | |
| H | O | – | Q-2 | $CH_3$ | $C_2H_5$ | – | – | O | H | $CH_3$ | $OCH_3$ | CH | |
| H | O | – | Q-2 | $CH_3$ | $C_2H_5$ | – | – | O | H | Cl | $OCH_3$ | CH | |
| H | O | – | Q-2 | $CH_3$ | $C_2H_5$ | – | – | O | H | $CH_3$ | $OCH_3$ | N | |
| H | O | – | Q-2 | $CH_3$ | $C_2H_5$ | – | – | O | H | $OCH_3$ | $OCH_3$ | N | |
| H | O | – | Q-3 | $CH_3$ | – | $CH_3$ | – | O | H | $CH_3$ | $OCH_3$ | CH | |
| H | O | – | Q-3 | $CH_3$ | – | $CH_3$ | – | O | H | Cl | $OCH_3$ | CH | |
| H | O | – | Q-3 | $CH_3$ | – | $CH_3$ | – | O | H | $OCH_3$ | $OCH_3$ | CH | |
| H | O | – | Q-3 | $CH_3$ | – | $CH_3$ | – | O | H | $OCH_3$ | $OCH_3$ | N | |
| H | O | – | Q-3 | $CH_3$ | – | $CH_3$ | – | O | H | $CH_3$ | $OCH_3$ | N | |
| H | O | – | Q-3 | $CH_3$ | – | $C_2H_5$ | – | O | H | $CH_3$ | $CH_3$ | CH | |
| H | O | – | Q-3 | $CH_3$ | – | $C_2H_5$ | – | O | H | $CH_3$ | $OCH_3$ | CH | |
| H | O | – | Q-3 | $CH_3$ | – | $C_2H_5$ | – | O | H | Cl | $OCH_3$ | CH | |

Table XI (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-3 | $CH_3$ | - | $C_2H_5$ | - | O | H | $OCH_3$ | $OCH_3$ | CH | |
| H | O | - | Q-3 | $CH_3$ | - | $C_2H_5$ | - | O | H | $CH_3$ | $OCH_3$ | N | |
| H | O | - | Q-3 | $CH_3$ | - | $CH_2Cl$ | - | O | H | $CH_3$ | $OCH_3$ | CH | |
| H | O | - | Q-3 | $CH_3$ | - | $CH_2Cl$ | - | O | H | $OCH_3$ | $OCH_3$ | CH | |
| H | O | - | Q-3 | $CH_3$ | - | $CH_2Cl$ | - | O | H | Cl | $OCH_3$ | CH | |
| H | O | - | Q-3 | $CH_3$ | - | $CH_2Cl$ | - | O | H | $CH_3$ | $OCH_3$ | N | |
| H | O | - | Q-3 | $CH_3$ | - | $CH_2Cl$ | - | O | H | $OCH_3$ | $OCH_3$ | N | |
| H | O | - | Q-3 | $CH_3$ | - | $CF_3$ | - | O | H | $CH_3$ | $CH_3$ | CH | |
| H | O | - | Q-3 | $CH_3$ | - | $CF_3$ | - | O | H | $OCH_3$ | $OCH_3$ | CH | |
| H | O | - | Q-3 | $CH_3$ | - | $CF_3$ | - | O | H | $OCH_3$ | $CH_3$ | CH | |
| H | O | - | Q-3 | $CH_3$ | - | $CF_3$ | - | O | H | $CH_3$ | $OCH_3$ | N | |
| H | O | - | Q-3 | $CH_3$ | - | $CH(CH_3)_2$ | - | O | H | $CH_3$ | $OCH_3$ | CH | |
| H | O | - | Q-3 | $CH_3$ | - | $CH(CH_3)_2$ | - | O | H | $OCH_3$ | $OCH_3$ | CH | |
| H | O | - | Q-3 | $CH_3$ | - | $CH(CH_3)_2$ | - | O | H | Cl | $OCH_3$ | CH | |
| H | O | - | Q-3 | $CH_3$ | - | $CH(CH_3)_2$ | - | O | H | $CH_3$ | $OCH_3$ | N | |
| H | O | - | Q-3 | $CH_3$ | - | $OCH_3$ | - | O | H | $OCH_3$ | $OCH_3$ | CH | |
| H | O | - | Q-3 | $CH_3$ | - | $OCH_3$ | - | O | H | $CH_3$ | $OCH_3$ | CH | |
| H | O | - | Q-3 | $CH_3$ | - | $OCH_3$ | - | O | H | Cl | $CH_3$ | CH | |
| H | O | - | Q-3 | $CH_3$ | - | $OCH_3$ | - | O | H | $OCH_3$ | $OCH_3$ | N | |
| H | O | - | Q-3 | $CH_3$ | - | $SCH_3$ | - | O | H | $CH_3$ | $OCH_3$ | CH | |
| H | O | - | Q-3 | $CH_3$ | - | $SCH_3$ | - | O | H | $OCH_3$ | $OCH_3$ | CH | |
| H | O | - | Q-3 | $CH_3$ | - | $SCH_3$ | - | O | H | $OCH_3$ | $OCH_3$ | N | |
| H | O | - | Q-3 | $CH_3$ | - | $SCH_3$ | - | O | H | $CH_3$ | $OCH_3$ | N | |
| H | O | - | Q-3 | $CH_3$ | - | $N(CH_3)_2$ | - | O | H | $CH_3$ | $CH_3$ | CH | |
| H | O | - | Q-3 | $CH_3$ | - | $N(CH_3)_2$ | - | O | H | $OCH_3$ | $OCH_3$ | CH | |
| H | O | - | Q-3 | $CH_3$ | - | $N(CH_3)_2$ | - | O | H | $OCH_3$ | $CH_3$ | N | |
| H | O | - | Q-5 | $CH_3$ | - | - | $CH_3$ | O | H | $OCH_3$ | $CH_3$ | N | |

## Table XII

General Structure 12

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-1 | $C_6H_5$ | $CH_3$ | - | - | O | H | $CH_3$ | $CH_3$ | CH | |
| H | O | - | Q-1 | $C_6H_5$ | $CH_3$ | - | - | O | H | $OCH_3$ | $CH_3$ | CH | |
| H | O | - | Q-1 | $C_6H_5$ | $CH_3$ | - | - | O | H | Cl | $OCH_3$ | CH | |
| H | O | - | Q-1 | $C_6H_5$ | $CH_3$ | - | - | O | H | $CH_3$ | $OCH_3$ | N | |
| H | O | - | Q-1 | $C_6H_5$ | $CH_3$ | - | - | O | H | $OCH_3$ | $OCH_3$ | N | |
| H | O | - | Q-1 | $C_6H_5$ | $C_2H_5$ | - | - | O | H | $OCH_3$ | $CH_3$ | CH | |
| H | O | - | Q-1 | $C_6H_5$ | $C_2H_5$ | - | - | O | H | $OCH_3$ | $OCH_3$ | CH | |
| H | O | - | Q-1 | $C_6H_5$ | $C_2H_5$ | - | - | O | H | Cl | $OCH_3$ | CH | |
| H | O | - | Q-1 | $C_6H_5$ | $C_2H_5$ | - | - | O | H | $CH_3$ | $CH_3$ | CH | |
| H | O | - | Q-1 | $C_6H_5$ | $C_2H_5$ | - | - | O | H | $CH_3$ | $OCH_3$ | N | |
| H | O | - | Q-2 | $C_6H_5$ | $CH_3$ | - | - | O | H | $OCH_3$ | $OCH_3$ | N | |
| H | O | - | Q-2 | $C_6H_5$ | $CH_3$ | - | - | O | H | $OCH_3$ | $OCH_3$ | CH | 232-233 |
| H | O | - | Q-2 | $C_6H_5$ | $CH_3$ | - | - | O | H | $OCH_3$ | $CH_3$ | CH | 193-195 |
| H | O | - | Q-2 | $C_6H_5$ | $CH_3$ | - | - | O | H | Cl | $OCH_3$ | CH | 195-198 |
| H | O | - | Q-2 | $C_6H_5$ | $CH_3$ | - | - | O | H | $CH_3$ | $CH_3$ | CH | 165-170 |
| H | O | - | Q-2 | $C_6H_5$ | $CH_3$ | - | - | O | H | $OCH_3$ | $CH_3$ | N | 140-145 |
| H | O | - | Q-2 | $C_6H_5$ | $C_2H_5$ | - | - | O | H | $OCH_3$ | $OCH_3$ | N | 195-196 |
| H | O | - | Q-2 | $C\ H_5$ | $C_2H_5$ | - | - | O | H | $CH_3$ | $CH_3$ | CH | |
| H | O | - | Q-2 | $C_6H_5$ | $C_2H_5$ | - | - | O | H | $CH_3$ | $OCH_3$ | CH | |
| H | O | - | Q-2 | $C_6H_5$ | $C_2H_5$ | - | - | O | H | Cl | $OCH_3$ | CH | |
| H | O | - | Q-2 | $C_6H_5$ | $C_2H_5$ | - | - | O | H | $CH_3$ | $OCH_3$ | N | |
| H | O | - | Q-2 | $C_6H_5$ | $C_2H_5$ | - | - | O | H | $OCH_3$ | $OCH_3$ | N | |
| H | O | - | Q-3 | $C_6H_5$ | - | $CH_3$ | - | O | H | $CH_3$ | $OCH_3$ | CH | |
| H | O | - | Q-3 | $C_6H_5$ | - | $CH_3$ | - | O | H | Cl | $OCH_3$ | CH | |
| H | O | - | Q-3 | $C_6H_5$ | - | $CH_3$ | - | O | H | $OCH_3$ | $OCH_3$ | CH | |
| H | O | - | Q-3 | $C_6H_5$ | - | $CH_3$ | - | O | H | $OCH_3$ | $OCH_3$ | N | |
| H | O | - | Q-3 | $C_6H_5$ | - | $CH_3$ | - | O | H | $CH_3$ | $OCH_3$ | N | |
| H | O | - | Q-3 | $C_6H_5$ | - | $C_2H_5$ | - | O | H | $CH_3$ | $CH_3$ | CH | |
| H | O | - | Q-3 | $C_6H_5$ | - | $C_2H_5$ | - | O | H | $CH_3$ | $OCH_3$ | CH | |
| H | O | - | Q-3 | $C_6H_5$ | - | $C_2H_5$ | - | O | H | Cl | $OCH_3$ | CH | |

Table XII (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-3 | $C_6H_5$ | - | $C_2H_5$ | - | O | H | $OCH_3$ | $OCH_3$ | CH | |
| H | O | - | Q-3 | $C_6H_5$ | - | $C_2H_5$ | - | O | H | $CH_3$ | $OCH_3$ | N | |
| H | O | - | Q-3 | $C_6H_5$ | - | $CH_2Cl$ | - | O | H | $CH_3$ | $OCH_3$ | CH | |
| H | O | - | Q-3 | $C_6H_5$ | - | $CH_2Cl$ | - | O | H | $OCH_3$ | $OCH_3$ | CH | |
| H | O | - | Q-3 | $C_6H_5$ | - | $CH_2Cl$ | - | O | H | Cl | $OCH_3$ | CH | |
| H | O | - | Q-3 | $C_6H_5$ | - | $CH_2Cl$ | - | O | H | $CH_3$ | $OCH_3$ | N | |
| H | O | - | Q-3 | $C_6H_5$ | - | $CH_2Cl$ | - | O | H | $OCH_3$ | $OCH_3$ | N | |
| H | O | - | Q-3 | $C_6H_5$ | - | $CF_3$ | - | O | H | $CH_3$ | $CH_3$ | CH | |
| H | O | - | Q-3 | $C_6H_5$ | - | $CF_3$ | - | O | H | $OCH_3$ | $OCH_3$ | CH | |
| H | O | - | Q-3 | $C_6H_5$ | - | $CF_3$ | - | O | H | $OCH_3$ | $CH_3$ | CH | |
| H | O | - | Q-3 | $C_6H_5$ | - | $CF_3$ | - | O | H | $CH_3$ | $OCH_3$ | N | |
| H | O | - | Q-3 | $C_6H_5$ | - | $CH(CH_3)_2$ | - | O | H | $CH_3$ | $OCH_3$ | CH | |
| H | O | - | Q-3 | $C_6H_5$ | - | $CH(CH_3)_2$ | - | O | H | $OCH_3$ | $OCH_3$ | CH | |
| H | O | - | Q-3 | $C_6H_5$ | - | $CH(CH_3)_2$ | - | O | H | Cl | $OCH_3$ | CH | |
| H | O | - | Q-3 | $C_6H_5$ | - | $CH(CH_3)_2$ | - | O | H | $CH_3$ | $OCH_3$ | N | |
| H | O | - | Q-3 | $C_6H_5$ | - | $OCH_3$ | - | O | H | $OCH_3$ | $OCH_3$ | CH | |
| H | O | - | Q-3 | $C_6H_5$ | - | $OCH_3$ | - | O | H | $CH_3$ | $OCH_3$ | CH | |
| H | O | - | Q-3 | $C_6H_5$ | - | $OCH_3$ | - | O | H | Cl | $CH_3$ | CH | |
| H | O | - | Q-3 | $C_6H_5$ | - | $OCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | CH | |
| H | O | - | Q-3 | $C_6H_5$ | - | $SCH_3$ | - | O | H | $OCH_3$ | $OCH_3$ | CH | |
| H | O | - | Q-3 | $C_6H_5$ | - | $SCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | N | |
| H | O | - | Q-3 | $C_6H_5$ | - | $N(CH_3)_2$ | - | O | H | $CH_3$ | $CH_3$ | CH | |
| H | O | - | Q-3 | $C_6H_5$ | - | $N(CH_3)_2$ | - | O | H | $OCH_3$ | $OCH_3$ | CH | |
| H | O | - | Q-3 | $C_6H_5$ | - | $N(CH_3)_2$ | - | O | H | $OCH_3$ | $CH_3$ | N | |

Table XII (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-1 | $CH_3$ | $CH_3$ | - | - | O | H | $CH_3$ | $CH_3$ | CH | |
| H | O | - | Q-1 | $CH_3$ | $CH_3$ | - | - | O | H | $OCH_3$ | $CH_3$ | CH | |
| H | O | - | Q-1 | $CH_3$ | $CH_3$ | - | - | O | H | Cl | $OCH_3$ | CH | |
| H | O | - | Q-1 | $CH_3$ | $CH_3$ | - | - | O | H | $CH_3$ | $OCH_3$ | N | |
| H | O | - | Q-1 | $CH_3$ | $CH_3$ | - | - | O | H | $OCH_3$ | $OCH_3$ | N | |
| H | O | - | Q-1 | $CH_3$ | $C_2H_5$ | - | - | O | H | $OCH_3$ | $CH_3$ | CH | |
| H | O | - | Q-1 | $CH_3$ | $C_2H_5$ | - | - | O | H | $OCH_3$ | $OCH_3$ | CH | |
| H | O | - | Q-1 | $CH_3$ | $C_2H_5$ | - | - | O | H | Cl | $OCH_3$ | CH | |
| H | O | - | Q-1 | $CH_3$ | $C_2H_5$ | - | - | O | H | $CH_3$ | $CH_3$ | CH | |
| H | O | - | Q-1 | $CH_3$ | $C_2H_5$ | - | - | O | H | $CH_3$ | $OCH_3$ | N | |
| H | O | - | Q-2 | $CH_3$ | $CH_3$ | - | - | O | H | $OCH_3$ | $OCH_3$ | N | |
| H | O | - | Q-2 | $CH_3$ | $CH_3$ | - | - | O | H | $OCH_3$ | $OCH_3$ | CH | |
| H | O | - | Q-2 | $CH_3$ | $CH_3$ | - | - | O | H | $OCH_3$ | $CH_3$ | CH | |
| H | O | - | Q-2 | $CH_3$ | $CH_3$ | - | - | O | H | Cl | $OCH_3$ | CH | |
| H | O | - | Q-2 | $CH_3$ | $CH_3$ | - | - | O | H | $CH_3$ | $CH_3$ | CH | |
| H | O | - | Q-2 | $CH_3$ | $CH_3$ | - | - | O | H | $OCH_3$ | $CH_3$ | N | |
| H | O | - | Q-2 | $CH_3$ | $C_2H_5$ | - | - | O | H | $OCH_3$ | $OCH_3$ | N | |
| H | O | - | Q-2 | $CH_3$ | $C_2H_5$ | - | - | O | H | $CH_3$ | $CH_3$ | CH | |
| H | O | - | Q-2 | $CH_3$ | $C_2H_5$ | - | - | O | H | $CH_3$ | $OCH_3$ | CH | |
| H | O | - | Q-2 | $CH_3$ | $C_2H_5$ | - | - | O | H | Cl | $OCH_3$ | CH | |
| H | O | - | Q-2 | $CH_3$ | $C_2H_5$ | - | - | O | H | $CH_3$ | $OCH_3$ | N | |
| H | O | - | Q-2 | $CH_3$ | $C_2H_5$ | - | - | O | H | $OCH_3$ | $OCH_3$ | N | |
| H | O | - | Q-3 | $CH_3$ | - | $CH_3$ | - | O | H | $CH_3$ | $OCH_3$ | CH | |
| H | O | - | Q-3 | $CH_3$ | - | $CH_3$ | - | O | H | Cl | $OCH_3$ | CH | |
| H | O | - | Q-3 | $CH_3$ | - | $CH_3$ | - | O | H | $OCH_3$ | $OCH_3$ | CH | |
| H | O | - | Q-3 | $CH_3$ | - | $CH_3$ | - | O | H | $OCH_3$ | $OCH_3$ | N | |
| H | O | - | Q-3 | $CH_3$ | - | $CH_3$ | - | O | H | $CH_3$ | $OCH_3$ | N | |
| H | O | - | Q-3 | $CH_3$ | - | $C_2H_5$ | - | O | H | $CH_3$ | $CH_3$ | CH | |
| H | O | - | Q-3 | $CH_3$ | - | $C_2H_5$ | - | O | H | $CH_3$ | $OCH_3$ | CH | |
| H | O | - | Q-3 | $CH_3$ | - | $C_2H_5$ | - | O | H | Cl | $OCH_3$ | CH | |

Table XII (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-3 | $CH_3$ | - | $C_2H_5$ | - | O | H | $OCH_3$ | $OCH_3$ | CH | |
| H | O | - | Q-3 | $CH_3$ | - | $C_2H_5$ | - | O | H | $CH_3$ | $OCH_3$ | N | |
| H | O | - | Q-3 | $CH_3$ | - | $CH_2Cl$ | - | O | H | $CH_3$ | $OCH_3$ | CH | |
| H | O | - | Q-3 | $CH_3$ | - | $CH_2Cl$ | - | O | H | $OCH_3$ | $OCH_3$ | CH | |
| H | O | - | Q-3 | $CH_3$ | - | $CH_2Cl$ | - | O | H | Cl | $OCH_3$ | CH | |
| H | O | - | Q-3 | $CH_3$ | - | $CH_2Cl$ | - | O | H | $CH_3$ | $OCH_3$ | N | |
| H | O | - | Q-3 | $CH_3$ | - | $CH_2Cl$ | - | O | H | $OCH_3$ | $OCH_3$ | N | |
| H | O | - | Q-3 | $CH_3$ | - | $CF_3$ | - | O | H | $CH_3$ | $CH_3$ | CH | |
| H | O | - | Q-3 | $CH_3$ | - | $CF_3$ | - | O | H | $OCH_3$ | $OCH_3$ | CH | |
| H | O | - | Q-3 | $CH_3$ | - | $CF_3$ | - | O | H | $OCH_3$ | $CH_3$ | CH | |
| H | O | - | Q-3 | $CH_3$ | - | $CF_3$ | - | O | H | $CH_3$ | $OCH_3$ | N | |
| H | O | - | Q-3 | $CH_3$ | - | $CH(CH_3)_2$ | - | O | H | $CH_3$ | $OCH_3$ | CH | |
| H | O | - | Q-3 | $CH_3$ | - | $CH(CH_3)_2$ | - | O | H | $OCH_3$ | $OCH_3$ | CH | |
| H | O | - | Q-3 | $CH_3$ | - | $CH(CH_3)_2$ | - | O | H | Cl | $OCH_3$ | CH | |
| H | O | - | Q-3 | $CH_3$ | - | $CH(CH_3)_2$ | - | O | H | $CH_3$ | $OCH_3$ | N | |
| H | O | - | Q-3 | $CH_3$ | - | $OCH_3$ | - | O | H | $OCH_3$ | $OCH_3$ | CH | |
| H | O | - | Q-3 | $CH_3$ | - | $OCH_3$ | - | O | H | $CH_3$ | $OCH_3$ | CH | |
| H | O | - | Q-3 | $CH_3$ | - | $OCH_3$ | - | O | H | Cl | $CH_3$ | CH | |
| H | O | - | Q-3 | $CH_3$ | - | $OCH_3$ | - | O | H | $OCH_3$ | $OCH_3$ | N | |
| H | O | - | Q-3 | $CH_3$ | - | $SCH_3$ | - | O | H | $CH_3$ | $OCH_3$ | CH | |
| H | O | - | Q-3 | $CH_3$ | - | $SCH_3$ | - | O | H | $OCH_3$ | $OCH_3$ | CH | |
| H | O | - | Q-3 | $CH_3$ | - | $SCH_3$ | - | O | H | $OCH_3$ | $OCH_3$ | N | |
| H | O | - | Q-3 | $CH_3$ | - | $SCH_3$ | - | O | H | $CH_3$ | $OCH_3$ | N | |
| H | O | - | Q-3 | $CH_3$ | - | $N(CH_3)_2$ | - | O | H | $CH_3$ | $CH_3$ | CH | |
| H | O | - | Q-3 | $CH_3$ | - | $N(CH_3)_2$ | - | O | H | $OCH_3$ | $OCH_3$ | CH | |
| H | O | - | Q-3 | $CH_3$ | - | $N(CH_3)_2$ | - | O | H | $OCH_3$ | $CH_3$ | N | |
| H | O | - | Q-5 | $CH_3$ | - | - | $CH_3$ | O | H | $OCH_3$ | $CH_3$ | N | |
| H | O | - | Q-5 | $CH_3$ | - | - | $CH_3$ | O | H | $OCH_3$ | $CH_3$ | CH | |
| H | O | - | Q-5 | $CH_3$ | - | - | $CH_3$ | O | H | $OCH_3$ | $OCH_3$ | CH | |
| H | O | - | Q-5 | $CH_3$ | - | - | $CH_3$ | O | H | Cl | $OCH_3$ | CH | |
| H | O | - | Q-5 | $CH_3$ | - | - | $CH_3$ | O | H | $CH_3$ | $CH_3$ | CH | |

**Table XIII**

**General Structure 13**

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | 0 | - | Q-1 | $C_6H_5$ | $CH_3$ | - | - | 0 | H | $CH_3$ | $CH_3$ | CH | |
| H | 0 | - | Q-1 | $C_6H_5$ | $CH_3$ | - | - | 0 | H | $OCH_3$ | $CH_3$ | CH | |
| H | 0 | - | Q-1 | $C_6H_5$ | $CH_3$ | - | - | 0 | H | Cl | $OCH_3$ | CH | |
| H | 0 | - | Q-1 | $C_6H_5$ | $CH_3$ | - | - | 0 | H | $CH_3$ | $OCH_3$ | N | |
| H | 0 | - | Q-1 | $C_6H_5$ | $CH_3$ | - | - | 0 | H | $OCH_3$ | $OCH_3$ | N | |
| H | 0 | - | Q-1 | $C_6H_5$ | $C_2H_5$ | - | - | 0 | H | $OCH_3$ | $CH_3$ | CH | |
| H | 0 | - | Q-1 | $C_6H_5$ | $C_2H_5$ | - | - | 0 | H | $OCH_3$ | $OCH_3$ | CH | |
| H | 0 | - | Q-1 | $C_6H_5$ | $C_2H_5$ | - | - | 0 | H | Cl | $OCH_3$ | CH | |
| H | 0 | - | Q-1 | $C_6H_5$ | $C_2H_5$ | - | - | 0 | H | $CH_3$ | $CH_3$ | CH | |
| H | 0 | - | Q-1 | $C_6H_5$ | $C_2H_5$ | - | - | 0 | H | $CH_3$ | $OCH_3$ | N | |
| H | 0 | - | Q-2 | $C_6H_5$ | $CH_3$ | - | - | 0 | H | $OCH_3$ | $OCH_3$ | N | |
| H | 0 | - | Q-2 | $C_6H_5$ | $CH_3$ | - | - | 0 | H | $OCH_3$ | $OCH_3$ | CH | |
| H | 0 | - | Q-2 | $C_6H_5$ | $CH_3$ | - | - | 0 | H | $OCH_3$ | $CH_3$ | CH | |
| H | 0 | - | Q-2 | $C_6H_5$ | $CH_3$ | - | - | 0 | H | Cl | $OCH_3$ | CH | |
| H | 0 | - | Q-2 | $C_6H_5$ | $CH_3$ | - | - | 0 | H | $CH_3$ | $CH_3$ | CH | |
| H | 0 | - | Q-2 | $C_6H_5$ | $CH_3$ | - | - | 0 | H | $OCH_3$ | $CH_3$ | N | |
| H | 0 | - | Q-2 | $C_6H_5$ | $C_2H_5$ | - | - | 0 | H | $OCH_3$ | $OCH_3$ | N | |
| H | 0 | - | Q-2 | $C\ H_5$ | $C_2H_5$ | - | - | 0 | H | $CH_3$ | $CH_3$ | CH | |
| H | 0 | - | Q-2 | $C_6H_5$ | $C_2H_5$ | - | - | 0 | H | $CH_3$ | $OCH_3$ | CH | |
| H | 0 | - | Q-2 | $C_6H_5$ | $C_2H_5$ | - | - | 0 | H | Cl | $OCH_3$ | CH | |
| H | 0 | - | Q-2 | $C_6H_5$ | $C_2H_5$ | - | - | 0 | H | $CH_3$ | $OCH_3$ | N | |
| H | 0 | - | Q-2 | $C_6H_5$ | $C_2H_5$ | - | - | 0 | H | $OCH_3$ | $OCH_3$ | N | |
| H | 0 | - | Q-3 | $C_6H_5$ | - | $CH_3$ | - | 0 | H | $CH_3$ | $OCH_3$ | CH | |
| H | 0 | - | Q-3 | $C_6H_5$ | - | $CH_3$ | - | 0 | H | Cl | $OCH_3$ | CH | |
| H | 0 | - | Q-3 | $C_6H_5$ | - | $CH_3$ | - | 0 | H | $OCH_3$ | $OCH_3$ | CH | |
| H | 0 | - | Q-3 | $C_6H_5$ | - | $CH_3$ | - | 0 | H | $OCH_3$ | $OCH_3$ | N | |
| H | 0 | - | Q-3 | $C_6H_5$ | - | $CH_3$ | - | 0 | H | $CH_3$ | $OCH_3$ | N | |
| H | 0 | - | Q-3 | $C_6H_5$ | - | $C_2H_5$ | - | 0 | H | $CH_3$ | $CH_3$ | CH | |
| H | 0 | - | Q-3 | $C_6H_5$ | - | $C_2H_5$ | - | 0 | H | $CH_3$ | $OCH_3$ | CH | |
| H | 0 | - | Q-3 | $C_6H_5$ | - | $C_2H_5$ | - | 0 | H | Cl | $OCH_3$ | CH | |

Table XIII (Cont'd)

| $R_1$ | n | E | Q | $R_2$ | $R_3$ | $R_4$ | $R_5$ | W | R | X | Y | Z | m.p.(°C) |
|---|---|---|---|---|---|---|---|---|---|---|---|---|---|
| H | O | - | Q-3 | $C_6H_5$ | - | $C_2H_5$ | - | O | H | $OCH_3$ | $OCH_3$ | CH | |
| H | O | - | Q-3 | $C_6H_5$ | - | $C_2H_5$ | - | O | H | $CH_3$ | $OCH_3$ | N | |
| H | O | - | Q-3 | $C_6H_5$ | - | $CH_2Cl$ | - | O | H | $CH_3$ | $OCH_3$ | CH | |
| H | O | - | Q-3 | $C_6H_5$ | - | $CH_2Cl$ | - | O | H | $OCH_3$ | $OCH_3$ | CH | |
| H | O | - | Q-3 | $C_6H_5$ | - | $CH_2Cl$ | - | O | H | Cl | $OCH_3$ | CH | |
| H | O | - | Q-3 | $C_6H_5$ | - | $CH_2Cl$ | - | O | H | $CH_3$ | $OCH_3$ | N | |
| H | O | - | Q-3 | $C_6H_5$ | - | $CH_2Cl$ | - | O | H | $OCH_3$ | $OCH_3$ | N | |
| H | O | - | Q-3 | $C_6H_5$ | - | $CF_3$ | - | O | H | $CH_3$ | $CH_3$ | CH | |
| H | O | - | Q-3 | $C_6H_5$ | - | $CF_3$ | - | O | H | $OCH_3$ | $OCH_3$ | CH | |
| H | O | - | Q-3 | $C_6H_5$ | - | $CF_3$ | - | O | H | $OCH_3$ | $CH_3$ | CH | |
| H | O | - | Q-3 | $C_6H_5$ | - | $CF_3$ | - | O | H | $CH_3$ | $OCH_3$ | N | |
| H | O | - | Q-3 | $C_6H_5$ | - | $CH(CH_3)_2$ | - | O | H | $CH_3$ | $OCH_3$ | CH | |
| H | O | - | Q-3 | $C_6H_5$ | - | $CH(CH_3)_2$ | - | O | H | $OCH_3$ | $OCH_3$ | CH | |
| H | O | - | Q-3 | $C_6H_5$ | - | $CH(CH_3)_2$ | - | O | H | Cl | $OCH_3$ | CH | |
| H | O | - | Q-3 | $C_6H_5$ | - | $CH(CH_3)_2$ | - | O | H | $CH_3$ | $OCH_3$ | N | |
| H | O | - | Q-3 | $C_6H_5$ | - | $OCH_3$ | - | O | H | $OCH_3$ | $OCH_3$ | CH | |
| H | O | - | Q-3 | $C_6H_5$ | - | $OCH_3$ | - | O | H | $CH_3$ | $OCH_3$ | CH | |
| H | O | - | Q-3 | $C_6H_5$ | - | $OCH_3$ | - | O | H | Cl | $CH_3$ | CH | |
| H | O | - | Q-3 | $C_6H_5$ | - | $OCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | CH | |
| H | O | - | Q-3 | $C_6H_5$ | - | $SCH_3$ | - | O | H | $OCH_3$ | $OCH_3$ | CH | |
| H | O | - | Q-3 | $C_6H_5$ | - | $SCH_3$ | - | O | H | $OCH_3$ | $OCH_3$ | N | |
| H | O | - | Q-3 | $C_6H_5$ | - | $SCH_3$ | - | O | H | $CH_3$ | $OCH_3$ | N | |
| H | O | - | Q-3 | $C_6H_5$ | - | $N(CH_3)_2$ | - | O | H | $CH_3$ | $CH_3$ | CH | |
| H | O | - | Q-3 | $C_6H_5$ | - | $N(CH_3)_2$ | - | O | H | $OCH_3$ | $OCH_3$ | CH | |
| H | O | - | Q-3 | $C_6H_5$ | - | $N(CH_3)_2$ | - | O | H | $OCH_3$ | $CH_3$ | N | |
| H | O | - | Q-5 | $C_6H_5$ | - | - | $CH_3$ | O | H | $OCH_3$ | $CH_3$ | N | |

**Table XIV**

**General Structure 14**

| $R_1$ | n | E | Q | $R_3$ | $R_4$ | W | R | X | Y | Z | m.p.(°C) |
|---|---|---|---|---|---|---|---|---|---|---|---|
| H | O | - | Q-1 | $CH_3$ | - | O | H | $CH_3$ | $CH_3$ | CH | |
| H | O | - | Q-1 | $CH_3$ | - | O | H | $OCH_3$ | $CH_3$ | CH | |
| H | O | - | Q-1 | $CH_3$ | - | O | H | Cl | $OCH_3$ | CH | |
| H | O | - | Q-1 | $CH_3$ | - | O | H | $CH_3$ | $OCH_3$ | N | |
| H | O | - | Q-1 | $CH_3$ | - | O | H | $OCH_3$ | $OCH_3$ | N | |
| H | O | - | Q-1 | $C_2H_5$ | - | O | H | $OCH_3$ | $CH_3$ | CH | |
| H | O | - | Q-1 | $C_2H_5$ | - | O | H | $OCH_3$ | $OCH_3$ | CH | |
| H | O | - | Q-1 | $C_2H_5$ | - | O | H | Cl | $OCH_3$ | CH | |
| H | O | - | Q-1 | $C_2H_5$ | - | O | H | $CH_3$ | $CH_3$ | CH | |
| H | O | - | Q-1 | $C_2H_5$ | - | O | H | $CH_3$ | $OCH_3$ | N | |
| H | O | - | Q-2 | $CH_3$ | - | O | H | $OCH_3$ | $OCH_3$ | N | |
| H | O | - | Q-2 | $CH_3$ | - | O | H | $OCH_3$ | $OCH_3$ | CH | |
| H | O | - | Q-2 | $CH_3$ | - | O | H | $OCH_3$ | $CH_3$ | CH | |
| H | O | - | Q-2 | $CH_3$ | - | O | H | Cl | $OCH_3$ | CH | |
| H | O | - | Q-2 | $CH_3$ | - | O | H | $CH_3$ | $CH_3$ | CH | |
| H | O | - | Q-2 | $CH_3$ | - | O | H | $OCH_3$ | $CH_3$ | N | |
| H | O | - | Q-2 | $C_2H_5$ | - | O | H | $OCH_3$ | $OCH_3$ | N | |
| H | O | - | Q-2 | $C_2H_5$ | - | O | H | $CH_3$ | $CH_3$ | CH | |
| H | O | - | Q-2 | $C_2H_5$ | - | O | H | $CH_3$ | $OCH_3$ | CH | |
| H | O | - | Q-2 | $C_2H_5$ | - | O | H | Cl | $OCH_3$ | CH | |
| H | O | - | Q-2 | $C_2H_5$ | - | O | H | $CH_3$ | $OCH_3$ | N | |
| H | O | - | Q-2 | $C_2H_5$ | - | O | H | $OCH_3$ | $OCH_3$ | N | |

**Table XV**

**General Structure 15**

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-1 | $CH_3$ | - | - | O | H | $CH_3$ | $CH_3$ | CH | |
| H | O | - | Q-1 | $CH_3$ | - | - | O | H | $OCH_3$ | $CH_3$ | CH | |
| H | O | - | Q-1 | $CH_3$ | - | - | O | H | Cl | $OCH_3$ | CH | |
| H | O | - | Q-1 | $CH_3$ | - | - | O | H | $CH_3$ | $OCH_3$ | N | |
| H | O | - | Q-1 | $CH_3$ | - | - | O | H | $OCH_3$ | $OCH_3$ | N | |
| H | O | - | Q-1 | $C_2H_5$ | - | - | O | H | $OCH_3$ | $CH_3$ | CH | |
| H | O | - | Q-1 | $C_2H_5$ | - | - | O | H | $OCH_3$ | $OCH_3$ | CH | |
| H | O | - | Q-1 | $C_2H_5$ | - | - | O | H | Cl | $OCH_3$ | CH | |
| H | O | - | Q-1 | $C_2H_5$ | - | - | O | H | $CH_3$ | $CH_3$ | CH | |
| H | O | - | Q-1 | $C_2H_5$ | - | - | O | H | $CH_3$ | $OCH_3$ | N | |
| H | O | - | Q-2 | $CH_3$ | - | - | O | H | $OCH_3$ | $OCH_3$ | N | |
| H | O | - | Q-2 | $CH_3$ | - | - | O | H | $OCH_3$ | $OCH_3$ | CH | 138-143 |
| H | O | - | Q-2 | $CH_3$ | - | - | O | H | $OCH_3$ | $CH_3$ | CH | 155-159 |
| H | O | - | Q-2 | $CH_3$ | - | - | O | H | Cl | $OCH_3$ | CH | 157-158 |
| H | O | - | Q-2 | $CH_3$ | - | - | O | H | $CH_3$ | $CH_3$ | CH | 135-140 |
| H | O | - | Q-2 | $CH_3$ | - | - | O | H | $OCH_3$ | $CH_3$ | N | |
| H | O | - | Q-2 | $CH_3$ | - | - | O | H | $OCH_3$ | $OCH_3$ | N | 75-78 |
| H | O | - | Q-2 | $C_2H_5$ | - | - | O | H | $CH_3$ | $CH_3$ | CH | |
| H | O | - | Q-2 | $C_2H_5$ | - | - | O | H | $CH_3$ | $OCH_3$ | CH | |
| H | O | - | Q-2 | $C_2H_5$ | - | - | O | H | Cl | $OCH_3$ | CH | |
| H | O | - | Q-2 | $C_2H_5$ | - | - | O | H | $CH_3$ | $OCH_3$ | N | |
| H | O | - | Q-2 | $C_2H_5$ | - | - | O | H | $OCH_3$ | $OCH_3$ | N | |
| H | O | - | Q-3 | - | $CH_3$ | - | O | H | $CH_3$ | $OCH_3$ | CH | |
| H | O | - | Q-3 | - | $CH_3$ | - | O | H | Cl | $OCH_3$ | CH | |
| H | O | - | Q-3 | - | $CH_3$ | - | O | H | $OCH_3$ | $OCH_3$ | CH | |
| H | O | - | Q-3 | - | $CH_3$ | - | O | H | $OCH_3$ | $OCH_3$ | N | |
| H | O | - | Q-3 | - | $CH_3$ | - | O | H | $CH_3$ | $OCH_3$ | N | |
| H | O | - | Q-3 | - | $C_2H_5$ | - | O | H | $CH_3$ | $CH_3$ | CH | |
| H | O | - | Q-3 | - | $C_2H_5$ | - | O | H | $CH_3$ | $OCH_3$ | CH | |
| H | O | - | Q-3 | - | $C_2H_5$ | - | O | H | Cl | $OCH_3$ | CH | |

Table XV (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-3 | - | $C_2H_5$ | - | O | H | $OCH_3$ | $OCH_3$ | CH | |
| H | O | - | Q-3 | - | $C_2H_5$ | - | O | H | $CH_3$ | $OCH_3$ | N | |
| H | O | - | Q-3 | - | $CH_2Cl$ | - | O | H | $CH_3$ | $OCH_3$ | CH | |
| H | O | - | Q-3 | - | $CH_2Cl$ | - | O | H | $OCH_3$ | $OCH_3$ | CH | |
| H | O | - | Q-3 | - | $CH_2Cl$ | - | O | H | Cl | $OCH_3$ | CH | |
| H | O | - | Q-3 | - | $CH_2Cl$ | - | O | H | $CH_3$ | $OCH_3$ | N | |
| H | O | - | Q-3 | - | $CH_2Cl$ | - | O | H | $OCH_3$ | $OCH_3$ | N | |
| H | O | - | Q-3 | - | $CF_3$ | - | O | H | $CH_3$ | $CH_3$ | CH | |
| H | O | - | Q-3 | - | $CF_3$ | - | O | H | $OCH_3$ | $OCH_3$ | CH | |
| H | O | - | Q-3 | - | $CF_3$ | - | O | H | $OCH_3$ | $CH_3$ | CH | |
| H | O | - | Q-3 | - | $CF_3$ | - | O | H | $CH_3$ | $OCH_3$ | N | |
| H | O | - | Q-3 | - | $CH(CH_3)_2$ | - | O | H | $CH_3$ | $OCH_3$ | CH | |
| H | O | - | Q-3 | - | $CH(CH_3)_2$ | - | O | H | $OCH_3$ | $OCH_3$ | CH | |
| H | O | - | Q-3 | - | $CH(CH_3)_2$ | - | O | H | Cl | $OCH_3$ | CH | |
| H | O | - | Q-3 | - | $CH(CH_3)_2$ | - | O | H | $CH_3$ | $OCH_3$ | N | |
| H | O | - | Q-3 | - | $OCH_3$ | - | O | H | $OCH_3$ | $OCH_3$ | CH | |
| H | O | - | Q-3 | - | $OCH_3$ | - | O | H | $CH_3$ | $OCH_3$ | CH | |
| H | O | - | Q-3 | - | $OCH_3$ | - | O | H | Cl | $CH_3$ | CH | |
| H | O | - | Q-3 | - | $OCH_3$ | - | O | H | $OCH_3$ | $OCH_3$ | N | |
| H | O | - | Q-3 | - | $SCH_3$ | - | O | H | $CH_3$ | $OCH_3$ | CH | |
| H | O | - | Q-3 | - | $SCH_3$ | - | O | H | $OCH_3$ | $OCH_3$ | CH | |
| H | O | - | Q-3 | - | $SCH_3$ | - | O | H | $OCH_3$ | $OCH_3$ | N | |
| H | O | - | Q-3 | - | $SCH_3$ | - | O | H | $CH_3$ | $OCH_3$ | N | |
| H | O | - | Q-3 | - | $N(CH_3)_2$ | - | O | H | $CH_3$ | $CH_3$ | CH | |
| H | O | - | Q-3 | - | $N(CH_3)_2$ | - | O | H | $OCH_3$ | $OCH_3$ | CH | |
| H | O | - | Q-3 | - | $N(CH_3)_2$ | - | O | H | $OCH_3$ | $CH_3$ | N | |
| H | O | - | Q-4 | - | $CH_3$ | - | O | H | $OCH_3$ | $CH_3$ | N | |
| H | O | - | Q-4 | - | $CH_3$ | - | O | H | $OCH_3$ | $CH_3$ | CH | |
| H | O | - | Q-4 | - | $CH_3$ | - | O | H | $OCH_3$ | $OCH_3$ | CH | |
| H | O | - | Q-4 | - | $CH_3$ | - | O | H | Cl | $OCH_3$ | CH | |
| H | O | - | Q-4 | - | $CH_3$ | - | O | H | $CH_3$ | $CH_3$ | CH | |

Table XV (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | 0 | - | Q-5 | - | - | $CH_3$ | O | H | $CH_3$ | $CH_3$ | CH | |
| H | 0 | - | Q-5 | - | - | $CH_3$ | O | H | $OCH_3$ | $CH_3$ | CH | |
| H | 0 | - | Q-5 | - | - | $CH_3$ | O | H | $OCH_3$ | $OCH_3$ | CH | |
| H | 0 | - | Q-5 | - | - | $CH_3$ | O | H | Cl | $OCH_3$ | CH | |

Table XVI

General Structure 16

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | 0 | - | Q-1 | $CH_3$ | - | - | O | H | $CH_3$ | $CH_3$ | CH | |
| H | 0 | - | Q-1 | $CH_3$ | - | - | O | H | $OCH_3$ | $CH_3$ | CH | |
| H | 0 | - | Q-1 | $CH_3$ | - | - | O | H | Cl | $OCH_3$ | CH | |
| H | 0 | - | Q-1 | $CH_3$ | - | - | O | H | $CH_3$ | $OCH_3$ | N | |
| H | 0 | - | Q-1 | $CH_3$ | - | - | O | H | $OCH_3$ | $OCH_3$ | N | |
| H | 0 | - | Q-1 | $C_2H_5$ | - | - | O | H | $OCH_3$ | $CH_3$ | CH | |
| H | 0 | - | Q-1 | $C_2H_5$ | - | - | O | H | $OCH_3$ | $OCH_3$ | CH | |
| H | 0 | - | Q-1 | $C_2H_5$ | - | - | O | H | Cl | $OCH_3$ | CH | |
| H | 0 | - | Q-1 | $C_2H_5$ | - | - | O | H | $CH_3$ | $CH_3$ | CH | |
| H | 0 | - | Q-1 | $C_2H_5$ | - | - | O | H | $CH_3$ | $OCH_3$ | N | |
| H | 0 | - | Q-2 | $CH_3$ | - | - | O | H | $OCH_3$ | $OCH_3$ | N | |
| H | 0 | - | Q-2 | $CH_3$ | - | - | O | H | $OCH_3$ | $OCH_3$ | CH | |
| H | 0 | - | Q-2 | $CH_3$ | - | - | O | H | $OCH_3$ | $CH_3$ | CH | |
| H | 0 | - | Q-2 | $CH_3$ | - | - | O | H | Cl | $OCH_3$ | CH | |
| H | 0 | - | Q-2 | $CH_3$ | - | - | O | H | $CH_3$ | $CH_3$ | CH | |
| H | 0 | - | Q-2 | $CH_3$ | - | - | O | H | $OCH_3$ | $CH_3$ | N | |
| H | 0 | - | Q-2 | $C_2H_5$ | - | - | O | H | $OCH_3$ | $OCH_3$ | N | |
| H | 0 | - | Q-2 | $C_2H_5$ | - | - | O | H | $CH_3$ | $CH_3$ | CH | |
| H | 0 | - | Q-2 | $C_2H_5$ | - | - | O | H | $CH_3$ | $OCH_3$ | CH | |
| H | 0 | - | Q-2 | $C_2H_5$ | - | - | O | H | Cl | $OCH_3$ | CH | |
| H | 0 | - | Q-2 | $C_2H_5$ | - | - | O | H | $CH_3$ | $OCH_3$ | N | |
| H | 0 | - | Q-2 | $C_2H_5$ | - | - | O | H | $OCH_3$ | $OCH_3$ | N | |
| H | 0 | - | Q-3 | - | $CH_3$ | - | O | H | $CH_3$ | $OCH_3$ | CH | |
| H | 0 | - | Q-3 | - | $CH_3$ | - | O | H | Cl | $OCH_3$ | CH | |
| H | 0 | - | Q-3 | - | $CH_3$ | - | O | H | $OCH_3$ | $OCH_3$ | CH | |
| H | 0 | - | Q-3 | - | $CH_3$ | - | O | H | $OCH_3$ | $OCH_3$ | N | |
| H | 0 | - | Q-3 | - | $CH_3$ | - | O | H | $CH_3$ | $OCH_3$ | N | |
| H | 0 | - | Q-3 | - | $C_2H_5$ | - | O | H | $CH_3$ | $CH_3$ | CH | |
| H | 0 | - | Q-3 | - | $C_2H_5$ | - | O | H | $CH_3$ | $OCH_3$ | CH | |
| H | 0 | - | Q-3 | - | $C_2H_5$ | - | O | H | Cl | $OCH_3$ | CH | |

Table XVI (Cont'd)

| $R_1$ | n | E | Q | $R_3$ | $R_4$ | $R_5$ | W | R | X | Y | Z | m.p.(°C) |
|---|---|---|---|---|---|---|---|---|---|---|---|---|
| H | O | - | Q-5 | - | - | $CH_3$ | O | H | Cl | $OCH_3$ | CH | |
| H | O | - | Q-5 | - | - | $CH_3$ | O | H | $CH_3$ | $OCH_3$ | CH | |
| H | O | - | Q-5 | - | - | $CH_3$ | O | H | $OCH_3$ | $OCH_3$ | CH | |
| H | O | - | Q-5 | - | - | $CH_3$ | O | H | $CH_3$ | $CH_3$ | CH | |
| H | O | - | Q-5 | - | - | $CH_3$ | O | H | $OCH_3$ | $CH_3$ | N | |

## Table XVII

### General Structure 17

| $R_1$ | E | Q | G | n | $R_3$ | W | R | X | Y | Z | m.p. (°C) |
|---|---|---|---|---|---|---|---|---|---|---|---|
| H | - | Q-1 | O | 0 | $CH_3$ | O | H | $OCH_3$ | $OCH_3$ | CH | |
| H | - | Q-1 | S | 0 | $CH_3$ | O | H | $CH_3$ | $OCH_3$ | CH | |
| H | - | Q-1 | SO | 0 | $CH_3$ | O | H | $CH_3$ | $CH_3$ | CH | |
| H | - | Q-1 | $SO_2$ | 0 | $CH_3$ | O | H | $OCH_3$ | $OCH_3$ | N | |
| H | - | Q-1 | O | 1 | $CH_3$ | O | H | $CH_3$ | $OCH_3$ | N | |
| H | - | Q-1 | S | 1 | $CH_3$ | O | H | $OCH_3$ | $OCH_3$ | CH | |
| H | - | Q-1 | SO | 1 | $CH_3$ | O | H | $OCH_3$ | $OCH_3$ | CH | |
| H | - | Q-1 | $SO_2$ | 1 | $CH_3$ | O | H | $CH_3$ | $OCH_3$ | CH | |
| H | - | Q-1 | O | 2 | $CH_3$ | O | H | $CH_3$ | $OCH_3$ | N | |
| H | - | Q-1 | S | 2 | $CH_3$ | O | H | $OCH_3$ | $OCH_3$ | N | |
| H | - | Q-1 | SO | 2 | $CH_3$ | O | H | $OCH_3$ | $OCH_3$ | CH | |
| H | - | Q-1 | $SO_2$ | 2 | $CH_3$ | O | H | $OCH_3$ | $OCH_3$ | CH | |
| 6-$CH_3$ | - | Q-1 | O | 0 | $CH_3$ | O | H | $OCH_3$ | $OCH_3$ | CH | |
| 6-$CH_3$ | - | Q-1 | S | 1 | $CH_3$ | O | H | $CH_3$ | $OCH_3$ | CH | |
| 6-$CH_3$ | - | Q-1 | SO | 2 | $CH_3$ | O | H | $OCH_3$ | $OCH_3$ | N | |
| 6-$CH_3$ | - | Q-1 | $SO_2$ | 1 | $CH_3$ | O | H | $CH_3$ | $OCH_3$ | N | |
| 5-Cl | - | Q-1 | O | 1 | $CH_3$ | O | H | $OCH_3$ | $OCH_3$ | CH | |
| 5-Cl | - | Q-1 | S | 2 | $CH_3$ | O | H | $CH_3$ | $OCH_3$ | CH | |
| 5-Cl | - | Q-1 | SO | 1 | $CH_3$ | O | H | $OCH_3$ | $OCH_3$ | N | |
| 5-Cl | - | Q-1 | $SO_2$ | 2 | $CH_3$ | O | H | $CH_3$ | $OCH_3$ | N | |
| H | - | Q-2 | O | 0 | $CH_3$ | O | H | $CH_3$ | $OCH_3$ | CH | |
| H | - | Q-2 | S | 0 | $CH_3$ | O | H | $OCH_3$ | $OCH_3$ | CH | |
| H | - | Q-2 | SO | 0 | $CH_3$ | O | H | $CH_3$ | $OCH_3$ | N | |
| H | - | Q-2 | $SO_2$ | 0 | $CH_3$ | O | H | $OCH_3$ | $OCH_3$ | N | |
| H | - | Q-2 | O | 1 | $CH_3$ | O | H | $CH_3$ | $OCH_3$ | CH | |
| H | - | Q-2 | S | 1 | $CH_3$ | O | H | $OCH_3$ | $OCH_3$ | CH | |
| H | - | Q-2 | SO | 1 | $CH_3$ | O | H | $CH_3$ | $OCH_3$ | N | |
| H | - | Q-2 | $SO_2$ | 1 | $CH_3$ | O | H | $OCH_3$ | $OCH_3$ | N | |
| H | - | Q-2 | O | 2 | $CH_3$ | O | H | $CH_3$ | $CH_3$ | CH | |
| H | - | Q-2 | S | 2 | $CH_3$ | O | H | $OCH_3$ | $OCH_3$ | CH | |
| H | - | Q-2 | SO | 2 | $CH_3$ | O | H | $CH_3$ | $CH_3$ | N | |
| H | - | Q-2 | $SO_2$ | 2 | $CH_3$ | O | H | $OCH_3$ | $OCH_3$ | N | |

Table XVIIa

General Structure 17

| $R_1$ | E | Q | G | n | $R_4$ | W | R | X | Y | Z | m.p. (°C) |
|---|---|---|---|---|---|---|---|---|---|---|---|
| H | - | Q-3 | O | 1 | $CH_3$ | O | H | $CH_3$ | $OCH_3$ | N | |
| H | - | Q-3 | S | 1 | $CH_3$ | O | H | $OCH_3$ | $OCH_3$ | CH | |
| H | - | Q-3 | SO | 1 | $CH_3$ | O | H | $CH_3$ | $OCH_3$ | CH | |
| H | - | Q-3 | $SO_2$ | 1 | $CH_3$ | O | H | $OCH_3$ | $OCH_3$ | N | |
| H | - | Q-3 | O | 2 | $CH_3$ | O | H | $CH_3$ | $OCH_3$ | CH | |
| H | - | Q-3 | S | 2 | $CH_3$ | O | H | $OCH_3$ | $OCH_3$ | N | |
| H | - | Q-3 | SO | 2 | $CH_3$ | O | H | $CH_3$ | $OCH_3$ | N | |
| H | - | Q-3 | $SO_2$ | 2 | $CH_3$ | O | H | $OCH_3$ | $OCH_3$ | CH | |
| H | - | Q-4 | O | 1 | $CH_3$ | O | H | $CH_3$ | $OCH_3$ | N | |
| H | - | Q-4 | S | 1 | $CH_3$ | O | H | $OCH_3$ | $OCH_3$ | CH | |
| H | - | Q-4 | SO | 1 | $CH_3$ | O | H | $CH_3$ | $OCH_3$ | CH | |
| H | - | Q-4 | $SO_2$ | 1 | $CH_3$ | O | H | $OCH_3$ | $OCH_3$ | N | |
| H | - | Q-4 | O | 2 | $CH_3$ | O | H | $CH_3$ | $OCH_3$ | N | |
| H | - | Q-4 | S | 2 | $CH_3$ | O | H | $OCH_3$ | $OCH_3$ | CH | |
| H | - | Q-4 | SO | 2 | $CH_3$ | O | H | $CH_3$ | $OCH_3$ | CH | |
| H | - | Q-4 | $SO_2$ | 2 | $CH_3$ | O | H | $OCH_3$ | $OCH_3$ | N | |

Table XVIIb

General Structure 17

| $R_1$ | E | Q | G | n | $R_5$ | W | R | X | Y | Z | m.p. (°C) |
|---|---|---|---|---|---|---|---|---|---|---|---|
| H | - | Q-5 | O | 1 | $CH_3$ | O | H | $CH_3$ | $OCH_3$ | CH | |
| H | - | Q-5 | S | 1 | $CH_3$ | O | H | $OCH_3$ | $OCH_3$ | N | |
| H | - | Q-5 | SO | 1 | $CH_3$ | O | H | $CH_3$ | $OCH_3$ | N | |
| H | - | Q-5 | $SO_2$ | 1 | $CH_3$ | O | H | $OCH_3$ | $OCH_3$ | CH | |
| H | - | Q-5 | O | 2 | $CH_3$ | O | H | $CH_3$ | $OCH_3$ | N | |
| H | - | Q-5 | S | 2 | $CH_3$ | O | H | $OCH_3$ | $OCH_3$ | CH | |
| H | - | Q-5 | SO | 2 | $CH_3$ | O | H | $CH_3$ | $OCH_3$ | CH | |
| H | - | Q-5 | $SO_2$ | 2 | $CH_3$ | O | H | $OCH_3$ | $OCH_3$ | N | |
| H | - | Q-6 | O | 1 | $CH_3$ | O | H | $CH_3$ | $OCH_3$ | N | |
| H | - | Q-6 | S | 1 | $CH_3$ | O | H | $OCH_3$ | $OCH_3$ | CH | |
| H | - | Q-6 | SO | 1 | $CH_3$ | O | H | $CH_3$ | $OCH_3$ | CH | |
| H | - | Q-6 | $SO_2$ | 1 | $CH_3$ | O | H | $OCH_3$ | $OCH_3$ | N | |
| H | - | Q-6 | O | 2 | $CH_3$ | O | H | $CH_3$ | $OCH_3$ | CH | |
| H | - | Q-6 | S | 2 | $CH_3$ | O | H | $OCH_3$ | $OCH_3$ | N | |
| H | - | Q-6 | SO | 2 | $CH_3$ | O | H | $CH_3$ | $OCH_3$ | N | |
| H | - | Q-6 | $SO_2$ | 2 | $CH_3$ | O | H | $OCH_3$ | $OCH_3$ | CH | |
| H | - | Q-7 | O | 1 | $CH_3$ | O | H | $CH_3$ | $OCH_3$ | CH | |
| H | - | Q-7 | S | 1 | $CH_3$ | O | H | $OCH_3$ | $OCH_3$ | N | |
| H | - | Q-7 | SO | 1 | $CH_3$ | O | H | $CH_3$ | $OCH_3$ | N | |
| H | - | Q-7 | $SO_2$ | 1 | $CH_3$ | O | H | $OCH_3$ | $OCH_3$ | CH | |
| H | - | Q-7 | O | 2 | $CH_3$ | O | H | $CH_3$ | $OCH_3$ | CH | |
| H | - | Q-7 | S | 2 | $CH_3$ | O | H | $OCH_3$ | $OCH_3$ | N | |
| H | - | Q-7 | SO | 2 | $CH_3$ | O | H | $CH_3$ | $OCH_3$ | N | |
| H | - | Q-7 | $SO_2$ | 2 | $CH_3$ | O | H | $OCH_3$ | $OCH_3$ | CH | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

| | Active Ingredient | Diluent(s) | Surfactant(s) |
|---|---|---|---|
| | Weight Percent* | | |
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are somtimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0° C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning. "Agglomeration", Chemical Engineering, December 4, 1967, pp. 147ff. and "Perry's Chemical Engineer's Handbook", 5th Ed., McGraw-Hill, New York, 1973, pp. 8-57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138-140, 162-164, 166, 167 and 169-182;

H. Gysin and E. Knusli, U.S. Patent 2,891,855, June 23, 1959, Col. 3, line 66 through Col. 5, line 17 and Examples 1-4;

G. C. Klingman, "Weed Control as a Science", John Wiley & Sons, Inc., New York, 1961, pp. 81-96; and

J. D. Fryer and S. A. Evans, "Weed Control Handbook", 5th Ed., Blackwell Scientific Publications, Oxford, 1968, pp. 101-103.

In the following examples, all parts are by weight unless otherwise indicated.

Example 14

Wettable Powder

| | |
|---|---|
| N-[(4,6-dimethoxypyrimidin-2-yl)aminocarbonyl]-2-(1-methyl-1H-tetrazol-5-yl)benzenesulfonamide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 15

Wettable Powder

| | |
|---|---|
| N-[(4,6-dimethoxypyrimidin-2-yl)aminocarbonyl]-2-(5-methyl-1H-tetrazol-1-yl)benzenesulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 16

Granule

| | |
|---|---|
| Wettable Powder of Example 15 (above) | 5% |
| attapulgite granules (U.S.S. 20-40 mesh; 0.84-0.42 mm) | 95% |

A slurry of wettable powder containing 25% solids is sprayed on the surface of attapulgite granules while tumbling in a double-cone blender. The granules are dried and packaged.

Example 17

Extruded Pellet

| | |
|---|---|
| N-[(4,6-dimethoxypyrimidin-2-yl)aminocarbonyl]-2-(5-methyl-1H-tetrazol-1-yl)benzenesulfonamide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 18

Oil Suspension

| | |
|---|---|
| N-[(4,6-dimethoxypyrimidin-2-yl)aminocarbonyl]-2-(1-methyl-1H-tetrazol-5-yl)benzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 19

Wettable Powder

| | |
|---|---|
| N-[(4,6-dimethoxypyrimidin-2-yl)aminocarbonyl]-2-(5-methyl-1H-tetrazol-1-yl)benzenesulfonamide | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is reblended and sifted through a U.S.S. No. 50 sieve (0.3 mm opening) and packaged.

Example 20

Low Strength Granule

| | |
|---|---|
| N-[(4,6-dimethoxypyrimidin-2-yl)aminocarbonyl]-2-(1-methyl-1H-tetrazol-5-yl)benzenesulfonamide | 1% |
| N,N-dimethylformamide | 9% |
| attapulgite granules (U.S.S. 20-40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 21

Aqueous Suspension

| | |
|---|---|
| N-[(4,6-dimethoxypyrimidin-2-yl)aminocarbonyl]-2-(1-methyl-1H-tetrazol-5-yl)benzenesulfonamide | 40% |
| polyacrylic acid thickener | 0.3% |
| dodecylphenol polyethylene glycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 22

Solution

| | |
|---|---|
| N-[(4,6-dimethoxypyrimidin-2-yl)aminocarbonyl]-2-(1-methyl-1H-tetrazol-5-yl)benzenesulfonamide, Sodium Salt | 5% |
| water | 95% |

The salt is added directly to the water with stirring to produce the solution, which may then be packaged for use.

Example 23

Low Strength Granule

| | |
|---|---|
| N-[(4,6-dimethoxypyrimidin-2-yl)aminocarbonyl]-2-(1-methyl-1H-tetrazol-5-yl)benzenesulfonamide | 0.1% |
| attapulgite granules (U.S.S. 20-40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 24

Granule

| | |
|---|---|
| N-[(4,6-dimethoxypyrimidin-2-yl)aminocarbonyl]-2-(1-methyl-1H-tetrazol-5-yl)benzenesulfonamide | 80% |
| wetting agent | 1% |
| crude ligninsulfonate salt (containing 5-20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14-100 mesh (1410-149 microns), and packaged for use.

Example 25

High Strength Concentrate

| | |
|---|---|
| N-[(4,6-dimethoxypyrimidin-2-yl)aminocarbonyl]-2-(1-methyl-1H-tetrazol-5-yl)benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 26

Wettable Powder

| | |
|---|---|
| N-[(4,6-dimethoxypyrimidin-2-yl)aminocarbonyl]-2-(1-methyl-1H-tetrazol-5-yl)benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 27

Wettable Powder

| | |
|---|---|
| N-[(4,6-dimethoxypyrimidin-2-yl)aminocarbonyl]-2-(1-methyl-1H-tetrazol-5-yl)benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 28

Oil Suspension

| | |
|---|---|
| N-[(4,6-dimethoxypyrimidin-2-yl)aminocarbonyl]-2-(1-methyl-1H-tetrazol-5-yl)benzenesulfonamide | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

Example 29

Dust

| | |
|---|---|
| N-[(4,6-dimethoxypyrimidin-2-yl)aminocarbonyl]-2-(1-methyl-1H-tetrazol-5-yl)benzenesulfonamide | 10% |
| attapulgite | 10% |
| Pyrophyllite | 80% |

The active ingredient is blended with attapulgite and then passed through a hammer-mill to produce particles substantially all below 200 microns. The ground concentrate is then blended with powdered pyrophyllite until homogeneous.

Utility

Test results indicate that the compounds of the present invention are highly active preemergent or postemergent herbicides or plant growth regulants. They have utility for broad-spectrum pre- and/or postemergence weed control in areas where complete control of all vegetation is desired, such as around fuel storage tanks, ammunition depots, industrial storage areas, parking lots, drive-in theaters, around billboards, highway and railroad structures. Some of the compounds have utility for selective weed control in crops such as corn, rice, wheat, barley and rape. Alternatively, many of the subject compounds are useful to modify plant growth.

The rates of application for the compounds of the invention are determined by a number of factors, including their use as plant growth modifiers or as herbicides, the crop species involved, the types of weeds to be controlled, weather and climate, formulations selected, mode of application, amount of foliage present, etc. In general terms, the subject compounds should be applied at levels of about 0.001 to 10 kg/ha, the lower rates being suggested for use on lighter soils and/or those having a low organic matter content, for plant growth modification or for situations where only short-term persistence is required.

The compounds of the invention may be used in combination with any other commercial herbicide, examples of which are those of the triazine, triazole, uracil, urea, amide, diphenylether, carbamate, phenoxyacetic acid and bipyridylium types, as well as other sulfonylureas. They are particularly useful in combination with the following herbicides.

| Common Name | Chemical Name |
|---|---|
| alachlor | 2-chloro-2',6'-diethyl-N-(methoxymethyl)-acetanilide |
| atrazine | 2-chloro-4-(ethylamino)-6-(isopropyl-amino)-s-triazine |
| butylate | S-ethyl-diisobutylthiocarbamate |
| cyanazine | 2-[[4-chloro-6-(ethylamino)-s-triazin-2-yl]amino]-2-methylpropionitrile |
| dicamba | 3,6-dichloro-o-anisic acid |
| EPTC | S-ethyl dipropylthiocarbamate |
| linuron | 3-(3,4-dichlorophenyl)-1-methoxy-1-methylurea |
| metolachlor | 2-chloro-N-(2-ethyl-6-methylphenyl)-N-(2-methoxy-1-methylethyl)acetamide |
| metribuzin | 4-amino-6-tert-butyl-3-(methylthio)-as-triazine-5(4H)-one |
| tridiphane | 2-(3,5-dichlorophenyl)-2-(2,2,2-trichloroethyl)oxirane |
| 2,4-D | (2,4-dichlorophenoxy)acetic acid |

| Trade Name | Chemical Name |
|---|---|
| Harmony™ | 3-[[(4-methoxy-6-methyl-1,3,5-triazin-2-yl)aminocarbonyl]aminosulfonyl]-2-thiophenecarboxylic acid, methyl ester |

The herbicidal properties of the subject compounds were discovered in a number of greenhouse tests. The test procedures and results follow.

COMPOUNDS

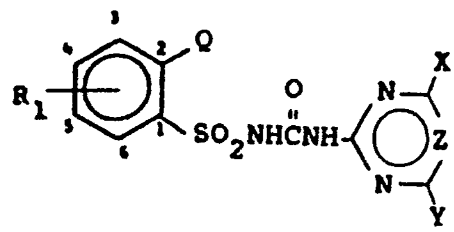

Q is 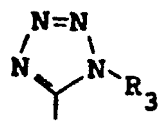 , 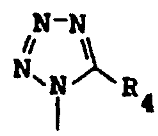 or 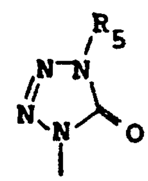

Q-1 Q-3 Q-5

| Compound | Q | $R_1$ | $R_3$ | $R_4$ | $R_5$ | X | Y | Z |
|---|---|---|---|---|---|---|---|---|
| 1 | Q-3 | H | - | $CH_3$ | - | $OCH_3$ | $CH_3$ | N |
| 2 | Q-3 | H | - | $CH_3$ | - | $OCH_3$ | $OCH_3$ | CH |
| 3 | Q-1 | H | $CH_3$ | - | - | $OCH_3$ | $OCH_3$ | CH |
| 4 | Q-3 | H | - | $CH_3$ | - | $CH_3$ | $OCH_3$ | CH |
| 5 | Q-3 | H | - | $CH_3$ | - | Cl | $OCH_3$ | CH |
| 6 | Q-3 | H | - | $CH_3$ | - | $OCH_3$ | $OCH_3$ | N |
| 7 | Q-3 | H | - | $CH_3$ | - | $NHCH_3$ | $OCH_2CH_3$ | N |
| 8 | Q-3 | H | - | H | - | $OCH_3$ | $OCH_3$ | N |
| 9 | Q-3 | H | - | H | - | Cl | $OCH_3$ | CH |
| 10 | Q-3 | H | - | H | - | $CH_3$ | $OCH_3$ | CH |
| 11 | Q-3 | H | - | H | - | $CH_3$ | $CH_3$ | CH |
| 12 | Q-3 | H | - | H | - | $CH_3$ | $OCH_3$ | N |
| 13 | Q-3 | H | - | H | - | $OCH_3$ | $OCH_3$ | CH |
| 14 | Q-3 | 5-$CH_3$ | - | $CH_3$ | - | $OCH_3$ | $OCH_3$ | CH |
| 15 | Q-3 | 5-$CH_3$ | - | $CH_3$ | - | $CH_3$ | $OCH_3$ | N |
| 16 | Q-3 | 5-$CH_3$ | - | $CH_3$ | - | Cl | $OCH_3$ | CH |
| 17 | Q-3 | H | - | $CH_2CH_3$ | - | $CH_3$ | $OCH_3$ | CH |
| 18 | Q-3 | H | - | $CH_2CH_3$ | - | $OCH_3$ | $OCH_3$ | CH |
| 19 | Q-3 | H | - | $CH_2CH_3$ | - | $CH_3$ | $CH_3$ | CH |

Compounds (Cont'd)

| Cmpd. | Q | $R_1$ | $R_3$ | $R_4$ | $R_5$ | X | Y | Z |
|---|---|---|---|---|---|---|---|---|
| 20 | Q-3 | H | - | $CH_2CH_3$ | - | Cl | $OCH_3$ | CH |
| 21 | Q-3 | H | - | $CH_2CH_3$ | - | $OCH_3$ | $OCH_3$ | N |
| 22 | Q-3 | H | - | $CH_2CH_3$ | - | $CH_3$ | $OCH_3$ | N |
| 23 | Q-3 | 5-Cl | - | $CH_3$ | - | $OCH_3$ | $OCH_3$ | CH |
| 24 | Q-3 | 5-Cl | - | $CH_3$ | - | $CH_3$ | $OCH_3$ | CH |
| 25 | Q-3 | 5-Cl | - | $CH_3$ | - | $OCH_3$ | $OCH_3$ | N |
| 26 | Q-3 | 5-Cl | - | $CH_3$ | - | $CH_3$ | $OCH_3$ | N |
| 27 | Q-3 | 5-Cl | - | $CH_3$ | - | $CH_3$ | $CH_3$ | CH |
| 28 | Q-3 | 5-Cl | - | $CH_3$ | - | Cl | $OCH_3$ | CH |
| 29 | Q-3 | 5-Cl | - | $CH_3$ | - | $NHCH_3$ | $OCH_2CH_3$ | N |
| 30 | Q-1 | H | $CH_2CH_3$ | - | - | $OCH_3$ | $OCH_3$ | N |
| 31 | Q-1 | H | $CH_2CH_3$ | - | - | $CH_3$ | $OCH_3$ | N |
| 32 | Q-1 | H | $CH_2CH_3$ | - | - | $OCH_3$ | $OCH_3$ | CH |
| 33 | Q-1 | H | $CH_2CH_3$ | - | - | $CH_3$ | $OCH_3$ | CH |
| 34 | Q-1 | H | $CH_2CH_3$ | - | - | $CH_3$ | $CH_3$ | CH |
| 35 | Q-1 | H | $CH_2CH_3$ | - | - | Cl | $OCH_3$ | CH |
| 36 | Q-1 | H | $CH_2CH_3$ | - | - | $NHCH_3$ | $OCH_2CH_3$ | N |
| 37 | Q-1 | 3-$CH_3$ | $CH_3$ | - | - | $OCH_3$ | $OCH_3$ | N |
| 38 | Q-1 | 3-$CH_3$ | $CH_3$ | - | - | $CH_3$ | $OCH_3$ | N |
| 39 | Q-1 | 3-$CH_3$ | $CH_3$ | - | - | $OCH_3$ | $OCH_3$ | CH |
| 40 | Q-1 | 3-$CH_3$ | $CH_3$ | - | - | $CH_3$ | $OCH_3$ | CH |
| 41 | Q-1 | 3-$CH_3$ | $CH_3$ | - | - | $CH_3$ | $CH_3$ | CH |
| 42 | Q-1 | 3-$CH_3$ | $CH_3$ | - | - | Cl | $OCH_3$ | CH |
| 43 | Q-1 | 3-$CH_3$ | $CH_3$ | - | - | $NHCH_3$ | $OCH_2CH_3$ | N |
| 44 | Q-1 | 4-$CH_3$ | $CH_3$ | - | - | $OCH_3$ | $OCH_3$ | N |
| 45 | Q-1 | 4-$CH_3$ | $CH_3$ | - | - | $CH_3$ | $OCH_3$ | N |
| 46 | Q-1 | 4-$CH_3$ | $CH_3$ | - | - | $OCH_3$ | $OCH_3$ | CH |
| 47 | Q-1 | 4-$CH_3$ | $CH_3$ | - | - | $CH_3$ | $OCH_3$ | CH |
| 48 | Q-1 | 4-$CH_3$ | $CH_3$ | - | - | $CH_3$ | $CH_3$ | CH |
| 49 | Q-1 | 4-$CH_3$ | $CH_3$ | - | - | Cl | $OCH_3$ | CH |
| 50 | Q-1 | 4-$CH_3$ | $CH_3$ | - | - | $NHCH_3$ | $OCH_2CH_3$ | N |
| 51 | Q-1 | 6-$CH_3$ | $CH_3$ | - | - | $OCH_3$ | $OCH_3$ | N |

Compounds (Cont'd)

| Cmpd. | Q | $R_1$ | $R_3$ | $R_4$ | $R_5$ | X | Y | Z |
|---|---|---|---|---|---|---|---|---|
| 52 | Q-1 | 6-$CH_3$ | $CH_3$ | - | - | $CH_3$ | $OCH_3$ | N |
| 53 | Q-1 | 6-$CH_3$ | $CH_3$ | - | - | $OCH_3$ | $OCH_3$ | CH |
| 54 | Q-1 | 6-$CH_3$ | $CH_3$ | - | - | $CH_3$ | $OCH_3$ | CH |
| 55 | Q-1 | 6-$CH_3$ | $CH_3$ | - | - | $CH_3$ | $CH_3$ | CH |
| 56 | Q-1 | 6-$CH_3$ | $CH_3$ | - | - | Cl | $OCH_3$ | CH |
| 57 | Q-5 | H | - | - | $CH_3$ | $OCH_3$ | $OCH_3$ | CH |
| 58 | Q-5 | H | - | - | $CH_3$ | $CH_3$ | $OCH_3$ | N |

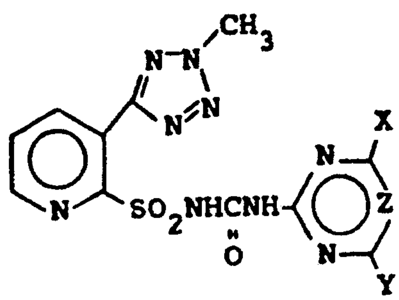

| Compound | X | Y | Z |
|---|---|---|---|
| 59 | $OCH_3$ | $OCH_3$ | CH |
| 60 | $CH_3$ | $OCH_3$ | CH |
| 61 | $CH_3$ | $CH_3$ | CH |
| 62 | $OCH_3$ | $OCH_3$ | N |
| 63 | Cl | $OCH_3$ | CH |

TEST A

Seeds of crabgrass (Digitaria spp.), giant foxtail (Setaria faberii) (in some cases), barnyardgrass (Echinochloa crusgalli), cheatgrass (Bromus secalinus), wild oats (Avena fatua), velvetleaf (Abutilon theophrasti), morningglory (Ipomoea spp.), cocklebur (Xanthium pensylvanicum), sorghum, corn, soybean, sugarbeet, cotton, rice, barley (in some cases), wheat and purple nutsedge (Cyperus rotundus) tubers were planted and treated preemergence with the test chemicals dissolved in a non-phytotoxic solvent. At the same time, these crop and weed species were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings, summarized in Table A, are based on a numerical scale extending from 0 = no injury, to 10 = complete kill. The accompanying descriptive symbols have the following meanings:

C = chlorosis/necrosis;

| | |
|---|---|
| B = | burn; |
| D = | defoliation; |
| E = | emergence inhibition; |
| G = | growth retardation; |
| H = | formative effect; |
| U = | unusual pigmentation; |
| X = | axillary stimulation; |
| S = | albinism; and |
| 6Y = | abscised buds or flowers. |

Table A

| | Cmpd. 1 | Cmpd. 2 | Cmpd. 3 |
|---|---|---|---|
| Rate kg/ha | 0.05 | 0.05 | 0.05 |
| POSTEMERGENCE | | | |
| Morningglory | 3C,8G | 10C | 10C |
| Cocklebur | 3C,7H | 9C | 9C |
| Velvetleaf | 2C,4G | 10C | 9C |
| Nutsedge | 8G | 9C | 9C |
| Crabgrass | 5G | 5C,9G | 9C |
| Barnyardgrass | 5C,9G | 9C | 5C,9G |
| Cheatgrass | 0 | 7G | 9C |
| Wild Oats | 3C,8G | 9C | 9C |
| Wheat | 4C,9G | 9C | 9C |
| Corn | 9C | 9C | 9C |
| Soybean | 5C,9G | 9C | 9C |
| Rice | 6C,9G | 9C | 9C |
| Sorghum | 6C,9H | 9C | 9C |
| Sugar Beets | 6C,9H | 9C | 9C |
| Cotton | 6C,9H | 9C | 9C |
| PREEMERGENCE | | | |
| Morningglory | 3G | 9H | 9G |
| Cocklebur | 0 | 9H | 9H |
| Velvetleaf | 0 | 10C | 10C |
| Nutsedge | 0 | 10E | 10E |
| Crabgrass | 0 | 3C,8G | 8G |
| Barnyardgrass | 0 | 4C,9H | 9H |
| Cheatgrass | 0 | 7G | 9H |
| Wild Oats | 0 | 5C,9G | 5C,9G |
| Wheat | 5G | 10E | 10E |
| Corn | 3C,8G | 4C,9G | 3C,9H |
| Soybean | 5G | 9H | 9H |
| Rice | 1C | 10E | 10E |
| Sorghum | 3C,6G | 5C,9H | 10H |
| Sugar Beets | 0 | 5C,9G | 9C |
| Cotton | 0 | 3C,9G | 9C |

Table A (continued)

| | Compound 4 | | Compound 5 | |
|---|---|---|---|---|
| Rate kg/ha | 0.05 | 0.01 | 0.05 | 0.01 |
| POSTEMERGENCE | | | | |
| Morningglory | 10C | 10C | 10C | 9C |
| Cocklebur | 9C | 9C | 10C | 9C |
| Velvetleaf | 9C | 9C | 9C | 4C,9H |
| Nutsedge | 4C,9G | 3C,9G | 9C | 4C,9G |
| Crabgrass | 5C,9G | 3C,8G | 4C,8G | 3C,5G |
| Giant Foxtail | - | - | - | - |
| Barnyardgrass | 9C | 9C | 9C | 9C |
| Cheatgrass | 3C,9G | 6G | 4G | 0 |
| Wild Oats | 5C,9G | 5C,9G | 4C,9G | 3C,9G |
| Wheat | 5C,9G | 2C,9G | 4C,9H | 8G |
| Corn | 5C,9G | 3C,9H | 5C,9G | 9H |
| Barley | - | - | - | - |
| Soybean | 5C,9G | 5C,9G | 4C,9G | 4C,9H |
| Rice | 9C | 9C | 6C,9G | 9C |
| Sorghum | 5C,9G | 6C,9G | 9C | 9C |
| Sugar beet | 8G | 10C | 9C | 9C |
| Cotton | 9C | 9C | 9C | 4C,9G |
| PREEMERGENCE | | | | |
| Morningglory | 9G | 9G | 9G | 9G |
| Cocklebur | 9H | 9H | 9H | 9H |
| Velvetleaf | 9C | 9C | 9C | 4C,8G |
| Nutsedge | 10E | 4C,8G | 10E | 10E |
| Crabgrass | 5C,9G | 5C,9G | 4C,9G | 7G |
| Giant Foxtail | - | - | - | - |
| Barnyardgrass | 5C,9H | 5C,9H | 4C,9H | 4C,9H |
| Cheatgrass | 9G | 7G | 5G | 3G |
| Wild Oats | 6C,9H | 4C,8H | 5C,9G | 3C,7G |
| Wheat | 10E | 5C,9H | 4C,9H | 9G |
| Corn | 9H | 5C,9H | 3C,9G | 8G |
| Barley | - | - | - | - |
| Soybean | 9H | 4C,7G | 3C,8H | 4C,6G |
| Rice | 10E | 10E | 10E | 10E |
| Sorghum | 10E | 5C,9H | 10E | 9H |
| Sugar beet | 5C,9G | 5C,9G | 5C,9G | 5C,9G |
| Cotton | 3C,9G | 3C,9G | 3C,9G | 3C,9G |

Table A (continued)

| | Compound 6 | | Compound 7 | |
|---|---|---|---|---|
| Rate kg/ha | 0.05 | 0.01 | 0.05 | 0.01 |
| POSTEMERGENCE | | | | |
| Morningglory | 1C,1H | 1C | 6C,9G | 4C,9H |
| Cocklebur | 1H | 0 | 8G | 2G |
| Velvetleaf | 0 | 0 | 4C,8H | 3C,5G |
| Nutsedge | 0 | 0 | 6G | 0 |
| Crabgrass | 0 | 0 | 4C,8G | 2C,5G |
| Giant Foxtail | - | - | - | - |
| Barnyardgrass | 4C,9H | 0 | 5C,9H | 3C,9H |
| Cheatgrass | 0 | 0 | 6G | 2G |
| Wild Oats | 2G | 0 | 5C,9G | 4C,9G |
| Wheat | 3G | 0 | 4C,9G | 2C,9G |
| Corn | 4C,9H | 3C,7H | 6C,9G | 4C,9H |
| Barley | - | - | - | - |
| Soybean | 4C,8H | 1C,1H | 3C,7G | 3G |
| Rice | 5C,9G | 5G | 9C | 9C |
| Sorghum | 4C,8H | 3G | 5C,9G | 4C,9H |
| Sugar beet | 4C,6G | 0 | 5C,9H | 4C,6H |
| Cotton | 4C,6G | 3C,4G | 3C,5G | 3C,5G |
| PREEMERGENCE | | | | |
| Morningglory | 0 | 0 | 9G | 7G |
| Cocklebur | 0 | 0 | 8G | 7G |
| Velvetleaf | 0 | 0 | 4C,9G | 0 |
| Nutsedge | 0 | 0 | 0 | 0 |
| Crabgrass | 0 | 0 | 4C,8G | 0 |
| Giant Foxtail | - | - | - | - |
| Barnyardgrass | 0 | 0 | 9H | 3G |
| Cheatgrass | 0 | 0 | 5G | 0 |
| Wild Oats | 0 | 0 | 5C,9H | 3C,6G |
| Wheat | 4G | 0 | 4C,9H | 2C,6G |
| Corn | 2G | 0 | 9G | 3C,7G |
| Barley | - | - | - | - |
| Soybean | 0 | 0 | 2G | 0 |
| Rice | 3G | 0 | 10E | 4C,8H |
| Sorghum | 0 | 0 | 10E | 4C,9H |
| Sugar beet | 0 | 0 | 5C,9G | 4C,8G |
| Cotton | 0 | 0 | 2C,5G | 0 |

Table A (continued)

| | Compound 9 | |
|---|---|---|
| Rate kg/ha | 0.05 | 0.01 |
| POSTEMERGENCE | | |
| Morningglory | 5C,9G | 2C,5G |
| Cocklebur | 9C | 3C,9H |
| Velvetleaf | 4C,8G | 1C |
| Nutsedge | 4C,9G | 3C,9G |
| Crabgrass | 2G | 0 |
| Giant Foxtail | - | - |
| Barnyardgrass | 9H | 2C,5H |
| Cheatgrass | 7G | 0 |
| Wild Oats | 0 | 0 |
| Wheat | 0 | 0 |
| Corn | 7H | 1C,3G |
| Barley | - | - |
| Soybean | 5G | 0 |
| Rice | 4C,8G | 0 |
| Sorghum | 5C,9H | 3C,8H |
| Sugar beet | 10C | 4C,9H |
| Cotton | 9C | 3C,8H |
| PREEMERGENCE | | |
| Morningglory | 9G | 4C,8H |
| Cocklebur | 8H | 2C,5G |
| Velvetleaf | 7H | 4G |
| Nutsedge | 10E | 3G |
| Crabgrass | 2C | 0 |
| Giant Foxtail | - | - |
| Barnyardgrass | 3C,7G | 0 |
| Cheatgrass | 3C,7H | 4G |
| Wild Oats | 2C,7G | 0 |
| Wheat | 6G | 0 |
| Corn | 3C,8H | 0 |
| Barley | - | - |
| Soybean | 3G | 0 |
| Rice | 10E | 7G |
| Sorghum | 4C,9H | 4C,8G |
| Sugar beet | 9C | 4C,8G |
| Cotton | 9G | 8G |

Table A (continued)

| | Compound 10 | | Compound 11 | |
|---|---|---|---|---|
| Rate kg/ha | 0.05 | 0.01 | 0.05 | 0.01 |
| POSTEMERGENCE | | | | |
| Morningglory | 10C | 9C | 2C,6G | 2H |
| Cocklebur | 9C | 9C | 9C | 5C,9G |
| Velvetleaf | 9C | 4C,8G | 5C,9H | 4C,9H |
| Nutsedge | 9C | 9C | 4C,9G | 3C,9G |
| Crabgrass | 3C,7G | 2H | 3C,5G | 1H |
| Giant Foxtail | - | - | - | - |
| Barnyardgrass | 9H | 3C,7H | 5C,9H | 2C,3H |
| Cheatgrass | 9C | 3C,9G | 9C | 8G |
| Wild Oats | 5C,9G | 2C,7H | 9C | 9H |
| Wheat | 9G | 3G | 9G | 5G |
| Corn | 9C | 3C,9H | 5C,9G | 3C,9H |
| Barley | - | - | - | - |
| Soybean | 5C,9G | 3C,8H | 3C,7H | 2H |
| Rice | 9C | 2C,7G | 9C | 5C,9G |
| Sorghum | 9C | 3C,9H | 9C | 4C,9H |
| Sugar beet | 10C | 10C | 9C | 9C |
| Cotton | 9C | 9C | 9C | 9H |
| PREEMERGENCE | | | | |
| Morningglory | 9G | 8G | 8G | 3C,5H |
| Cocklebur | - | 9H | 9H | 8H |
| Velvetleaf | 9C | 6C,9G | 9C | 3C,7H |
| Nutsedge | 10E | 9G | 10E | 4G |
| Crabgrass | 4C,7G | 2C,4G | 2C,3G | 0 |
| Giant Foxtail | - | - | - | - |
| Barnyardgrass | 4C,9H | 2C,6G | 3C,5G | 0 |
| Cheatgrass | 10E | 4C,9H | 5C,9H | 5G |
| Wild Oats | 9C | 4C,9G | 9C | 3C,7G |
| Wheat | 4C,9G | 7G | 9G | 5G |
| Corn | 9H | 4C,9H | 2C,9H | 3C,7H |
| Barley | - | - | - | - |
| Soybean | 9H | 4C,8H | 2C,7G | 2C,3G |
| Rice | 10E | 4C,9H | 10E | 3C,8G |
| Sorghum | 10E | 5C,9H | 9H | 3C,8H |
| Sugar beet | 5C,9G | 5C,9G | 9C | 5C,9G |
| Cotton | 9C | 9G | 9G | 9G |

Table A (continued)

| | Cmpd. 12 | Cmpd. 13 | Compound 14 | |
|---|---|---|---|---|
| Rate kg/ha | 0.01 | 0.01 | 0.05 | 0.01 |
| POSTEMERGENCE | | | | |
| Morningglory | 2G | 10C | 5C,9G | 4G |
| Cocklebur | 0 | 10C | 10C | 10C |
| Velvetleaf | 0 | 9C | 10C | 10C |
| Nutsedge | 0 | 9C | 9C | 5C,9G |
| Crabgrass | 0 | 4G | 2C,7G | 1C |
| Giant Foxtail | - | - | - | - |
| Barnyardgrass | 0 | 5C,9H | 9C | 9C |
| Cheatgrass | 0 | 4C,9G | 3G | 0 |
| Wild Oats | 0 | 3C,5G | 5C,9G | 3C,9G |
| Wheat | 0 | 0 | 5C,9G | 2C,9G |
| Corn | 0 | 9H | 9C | 5C,9H |
| Barley | - | - | - | - |
| Soybean | 1H | 9C | 9C | 9C |
| Rice | 0 | 2C,8G | 9C | 9C |
| Sorghum | 0 | 9H | 9C | 5C,9H |
| Sugar beet | 1C,1H | 5C,9G | 5C,9G | 9C |
| Cotton | 1C | 9C | 9C | 5C,9G |
| PREEMERGENCE | | | | |
| Morningglory | 0 | 9G | 9G | 8G |
| Cocklebur | 0 | 9H | 9H | 9H |
| Velvetleaf | 0 | 5C,9G | 10C | 9C |
| Nutsedge | 0 | 10E | 10E | 4C,9G |
| Crabgrass | 0 | 3G | 2C,7G | 3G |
| Giant Foxtail | - | - | - | - |
| Barnyardgrass | 0 | 3C,8H | 5C,9H | 3C,8H |
| Cheatgrass | 0 | 8G | 6G | 2G |
| Wild Oats | 0 | 5G | 9C | 5C,9G |
| Wheat | 0 | 0 | 4C,9G | 3C,8G |
| Corn | 2G | 8G | 3C,9G | 7G |
| Barley | - | - | - | - |
| Soybean | 2C | 3C,8H | 3C,9H | 2C,7G |
| Rice | 0 | 2C,9H | 10E | 3C,8G |
| Sorghum | 1C | 3C,9H | 10E | 3C,9H |
| Sugar beet | 3H | 10C | 5C,9G | 5C,9G |
| Cotton | 0 | 9G | 9G | 9G |

Table A (continued)

| | Compound 15 | | Compound 16 | |
|---|---|---|---|---|
| Rate kg/ha | 0.05 | 0.01 | 0.05 | 0.01 |
| POSTEMERGENCE | | | | |
| Morningglory | 3C,8H | 0 | 10C | 5C,9G |
| Cocklebur | 2C,4G | 0 | 10C | 8G |
| Velvetleaf | 2C,4H | 0 | 9C | 3C,8G |
| Nutsedge | 0 | 0 | 5C,9G | 2C,8G |
| Crabgrass | 0 | 0 | 5G | 0 |
| Giant Foxtail | - | - | - | - |
| Barnyardgrass | 3C,8H | 3G | 9C | 3C,8H |
| Cheatgrass | 0 | 0 | 0 | 0 |
| Wild Oats | 2C,9G | 2C,2G | 3C,9G | 2C,5G |
| Wheat | 0 | 0 | 8G | 5G |
| Corn | 1C,5G | 2G | 4G | 2G |
| Barley | - | - | - | - |
| Soybean | 4C,9G | 3H | 3C,9G | 2H,7G |
| Rice | 5G | 0 | 5C,9G | 7G |
| Sorghum | 3C,9H | 0 | 9C | 3C,9H |
| Sugar beet | 3C,6G | 2C,3G | 9C | 4C,8G |
| Cotton | 3C,9H | 1H | 2C,9G | 8G |
| PREEMERGENCE | | | | |
| Morningglory | 5H | 3H | 9C | 9H |
| Cocklebur | 7H | 0 | 9H | 9H |
| Velvetleaf | 6G | 0 | 9C | 2C,4G |
| Nutsedge | 0 | 0 | 10E | 3C,9G |
| Crabgrass | 0 | 0 | 4G | 2G |
| Giant Foxtail | - | - | - | - |
| Barnyardgrass | 1C | 0 | 3C,9H | 3C,3G |
| Cheatgrass | 0 | 0 | 0 | 0 |
| Wild Oats | 2C,5G | 0 | 5C,9G | 3C,6G |
| Wheat | 0 | 0 | 8G | 3G |
| Corn | 6G | 0 | 6G | 2G |
| Barley | - | - | - | - |
| Soybean | 2C,2H | 1C | 3C,7G | 3C,3H |
| Rice | 5G | 1C | 10E | 2C,8G |
| Sorghum | 3C,6G | 0 | 10H | 3C,9H |
| Sugar beet | 3C,7H | 0 | 10E | 5C,9G |
| Cotton | 2C | 0 | 9G | 2C,5G |

Table A (continued)

| | Compound 17 | | Compound 18 | |
|---|---|---|---|---|
| Rate kg/ha | 0.05 | 0.01 | 0.05 | 0.01 |
| POSTEMERGENCE | | | | |
| Morningglory | 10C | 10C | 10C | 9C |
| Cocklebur | 10C | 10C | 10C | 10C |
| Velvetleaf | 9C | 9C | 10C | 9C |
| Nutsedge | 5C,9G | 3C,9G | 10C | 10C |
| Crabgrass | 3C,8G | 3C,5G | 9C | 3C,7G |
| Giant Foxtail | 9C | 9C | 9C | 5C,9H |
| Barnyardgrass | 9C | 9C | 9C | 9C |
| Cheatgrass | 5C,9G | 3C,8G | 9C | 2C,8G |
| Wild Oats | 9C | 5C,9G | 5C,9G | 4C,9G |
| Wheat | 4C,9G | 9G | 9C | 3C,9G |
| Corn | 9C | 5C,9G | 10C | 3C,9H |
| Barley | 5C,9G | 3C,8H | 6C,9G | 3C,8G |
| Soybean | 9C | 9C | 9C | 9C |
| Rice | 9C | 9C | 9C | 9C |
| Sorghum | 9C | 9C | 9C | 9C |
| Sugar beet | 10C | 5C,9G | 10C | 9C |
| Cotton | 9C | 5C,9G | 9C | 10C |
| PREEMERGENCE | | | | |
| Morningglory | 9C | 7H | 9G | 8G |
| Cocklebur | 9H | 9H | 9H | 9H |
| Velvetleaf | 9C | 3C,8G | 9C | 9C |
| Nutsedge | 10E | 10E | 10E | 10E |
| Crabgrass | 4C,8G | 3C,7G | 8G | 4G |
| Giant Foxtail | 4C,9H | 3C,8H | 9H | 3C,6G |
| Barnyardgrass | 5C,9H | 4C,9H | 5C,9H | 3C,9H |
| Cheatgrass | 4C,8H | 3G | 7H | 6G |
| Wild Oats | 5C,9G | 4C,9G | 5C,9G | 2C,9G |
| Wheat | 10H | 3C,8G | 10H | 9G |
| Corn | 10H | 4C,9H | 4C,9H | 8G |
| Barley | 9G | 3C,9G | 3C,9G | 9G |
| Soybean | 9H | 4C,8H | 9H | 4C,8H |
| Rice | 10E | 9H | 10E | 10E |
| Sorghum | 10H | 6C,9H | 10H | 10H |
| Sugar beet | 10E | 9C | 5C,9G | 5C,9G |
| Cotton | 9C | 2C,9G | 4C,9G | 4C,9G |

Table A (continued)

| | Compound 19 | | Compound 20 | |
|---|---|---|---|---|
| Rate kg/ha | 0.05 | 0.01 | 0.05 | 0.01 |
| POSTEMERGENCE | | | | |
| Morningglory | 10C | 4C,9G | 10C | 9C |
| Cocklebur | 9C | 7G | 10C | 9C |
| Velvetleaf | 9C | 9C | 9C | 2C,7G |
| Nutsedge | 5C,9G | 3C,8G | 5C,9G | 4C,9G |
| Crabgrass | 4C,8G | 3C,4G | 3C,7G | 3C,3G |
| Giant Foxtail | 9C | 5C,9G | 5C,9H | 4C,8G |
| Barnyardgrass | 9C | 9C | 9C | 9C |
| Cheatgrass | 3C,7G | 5G | 5G | 2G |
| Wild Oats | 9C | 9C | 3C,9G | 3C,7G |
| Wheat | 9C | 5C,9G | 3C,9G | 9G |
| Corn | 9C | 4C,9H | 4C,9H | 3C,9H |
| Barley | 9C | 4C,9G | 2C,8G | 3C,7G |
| Soybean | 9C | 4C,9G | 9C | 4C,9G |
| Rice | 9C | 9C | 9C | 5C,9G |
| Sorghum | 10C | 9C | 9C | 9C |
| Sugar beet | 10C | 4C,8G | 9C | 4C,9G |
| Cotton | 9C | 4C,9G | 5C,9G | 9C |
| PREEMERGENCE | | | | |
| Morningglory | 8H | 3C,7G | 9G | 7H |
| Cocklebur | 8H | - | 9H | 9H |
| Velvetleaf | 9C | 3C,7H | 5C,9G | 7G |
| Nutsedge | 9G | 7G | 10E | 9G |
| Crabgrass | 5G | 0 | 9C | 2G |
| Giant Foxtail | 9H | 5G | 4C,8G | 4G |
| Barnyardgrass | 3C,8H | 3C,6G | 9H | 3C,8H |
| Cheatgrass | 2G | 0 | 3G | 0 |
| Wild Oats | 5C,9G | 3C,8H | 3C,9H | 2C,5G |
| Wheat | 5C,9H | 3C,8H | 3C,9H | 7G |
| Corn | 4C,9H | 3C,7H | 3C,9H | 9H |
| Barley | 4C,9G | 9G | 3C,9G | 8G |
| Soybean | 9H | 3C,6H | 3C,8H | 3C,5G |
| Rice | 10E | 4C,8H | 10E | 9H |
| Sorghum | 5C,9H | 6C,9H | 10H | 10H |
| Sugar beet | 9C | 5G | 9C | 5C,9G |
| Cotton | 9G | 2C,8G | 9G | 8G |

Table A (continued)

| | Compound 21 | | Compound 22 | |
|---|---|---|---|---|
| Rate kg/ha | 0.05 | 0.01 | 0.05 | 0.01 |
| POSTEMERGENCE | | | | |
| Morningglory | 4C,9G | 3C,7H | 4C,8H | 3C,7H |
| Cocklebur | 5C,9H | 5H | 5C,9G | 3C,7H |
| Velvetleaf | 3C,6G | 1H | 3C,7H | 3C,5H |
| Nutsedge | 4G | 3C,8G | 5G | 0 |
| Crabgrass | 3C,5G | 2G | 3C,7H | 3C,6G |
| Giant Foxtail | 9C | 4C,9G | 9C | 4C,9H |
| Barnyardgrass | 9C | 9C | 9C | 9C |
| Cheatgrass | 0 | 0 | 2C | 0 |
| Wild Oats | 2C | 0 | 2C,6G | 0 |
| Wheat | 5G | 0 | 8G | 0 |
| Corn | 10C | 9C | 10C | 9C |
| Barley | 0 | 0 | 2G | 0 |
| Soybean | 5C,9G | 4C,9G | 9C | 5C,9G |
| Rice | 5C,9G | 7G | 9C | 6G |
| Sorghum | 9C | 4C,9G | 9C | 9C |
| Sugar beet | 3C,6H | 3C,5G | 9C | 3C,8G |
| Cotton | 4C,9H | 4C,9H | 4C,9H | 3C,8H |
| PREEMERGENCE | | | | |
| Morningglory | 5H | 0 | 3C,8H | 2H |
| Cocklebur | 9H | - | 9H | 2C |
| Velvetleaf | 7G | 0 | 8H | 5G |
| Nutsedge | 0 | 0 | 0 | 0 |
| Crabgrass | 2C,5G | 0 | 2C,5G | 4G |
| Giant Foxtail | 2C,5G | 2G | 3C,7G | 3G |
| Barnyardgrass | 3C,6G | 0 | 3C,8G | 0 |
| Cheatgrass | 0 | 0 | 0 | 0 |
| Wild Oats | 2G | 0 | 2C | 0 |
| Wheat | 0 | 0 | 3G | 0 |
| Corn | 4C,9H | 2C,6G | 9H | 3C,8H |
| Barley | 0 | 0 | 7G | 0 |
| Soybean | 3C,7H | 0 | 3C,7H | 2C,3G |
| Rice | 8G | 2G | 9H | 0 |
| Sorghum | 5C,9H | 3C,6G | 4C,9H | 3C,8H |
| Sugar beet | 5G | 3G | 4C,7H | 5G |
| Cotton | 5G | 0 | 2C,5G | 1C |

Table A (continued)

| | Compound 23 | | Compound 24 |
|---|---|---|---|
| Rate kg/ha | 0.05 | 0.01 | 0.01 |
| POSTEMERGENCE | | | |
| Morningglory | 10C | 8G | 5G |
| Cocklebur | 10C | 10C | 10C |
| Velvetleaf | 10C | 5C,9G | 5C,9G |
| Nutsedge | 10C | 10C | 5C,9G |
| Crabgrass | 5G | 3G | 0 |
| Giant Foxtail | 5C,9G | 3C,8G | 3C,7G |
| Barnyardgrass | 9C | 3C,9H | 3C,7G |
| Cheatgrass | 5G | 2G | 2G |
| Wild Oats | 5G | 2G | 0 |
| Wheat | 8G | 6G | 5G |
| Corn | 9H | 6H | 0 |
| Barley | 3C,9H | 6G | 3G |
| Soybean | 9C | 9C | 9C |
| Rice | 9C | 4C,9G | 3C,7G |
| Sorghum | 9C | 9C | 4C,9H |
| Sugar beet | 10C | 10C | 10C |
| Cotton | 9C | 9C | 4C,9G |
| PREEMERGENCE | | | |
| Morningglory | 9H | 7H | 7H |
| Cocklebur | 9H | 9H | 9H |
| Velvetleaf | 10C | 9G | 9C |
| Nutsedge | 10E | 9G | 3C,7G |
| Crabgrass | 0 | 0 | 2G |
| Giant Foxtail | 4C,9H | 1C | 3C,5G |
| Barnyardgrass | 5C,9H | 3C,8H | 3C,6G |
| Cheatgrass | 4G | 0 | 0 |
| Wild Oats | 6G | 0 | 2C |
| Wheat | 4C,9G | 4G | 5G |
| Corn | 3C,8G | 2C,5G | 0 |
| Barley | 3C,9G | 2C,8G | 2C,8G |
| Soybean | 3C,8H | 3C,8H | 3C,7H |
| Rice | 9H | 4C,9G | 3C,6H |
| Sorghum | 10E | 3C,9H | 4C,8H |
| Sugar beet | 5C,9G | 5C,9G | 5C,9G |
| Cotton | 10C | 9C | 2C,7H |

Table A (continued)

| | Compound 25 | | Compound 26 | |
|---|---|---|---|---|
| Rate kg/ha | 0.05 | 0.01 | 0.05 | 0.01 |
| POSTEMERGENCE | | | | |
| Morningglory | 0 | 0 | 3C,5G | 0 |
| Cocklebur | 3H | 2H | 4C,8H | 2H |
| Velvetleaf | 0 | 0 | 2C,5G | 0 |
| Nutsedge | 0 | 0 | 3C,6G | 0 |
| Crabgrass | 0 | 0 | 3C,4H | 0 |
| Giant Foxtail | 3C,8H | 2H | 4C,9H | 3C,5G |
| Barnyardgrass | 5C,9H | 0 | 5C,9H | 2C,5G |
| Cheatgrass | 0 | 0 | 3G | 0 |
| Wild Oats | 4C,8G | 0 | 4C,9G | 3C,4G |
| Wheat | 5C,9G | 0 | 3C,9G | 7G |
| Corn | 3C,9H | 2H | 9H | 0 |
| Barley | 9G | 2G | 8G | 0 |
| Soybean | 2H,4G | 0 | 3C,8G | 2H |
| Rice | 9C | 2G | 9C | 8G |
| Sorghum | 4C,9H | 3G | 5C,9G | 3C,7H |
| Sugar beet | 5G | 0 | 9C | 2H |
| Cotton | 0 | 0 | 3C,7G | 0 |
| PREEMERGENCE | | | | |
| Morningglory | 0 | 0 | 2H | 1H |
| Cocklebur | 0 | 0 | 2C | 0 |
| Velvetleaf | 3G | 0 | 0 | 0 |
| Nutsedge | 0 | 0 | 0 | 0 |
| Crabgrass | 0 | 0 | 0 | 0 |
| Giant Foxtail | 3G | 0 | 0 | 0 |
| Barnyardgrass | 0 | 0 | 2C | 0 |
| Cheatgrass | 0 | 0 | 0 | 0 |
| Wild Oats | 0 | 0 | 0 | 0 |
| Wheat | 0 | 0 | 0 | 0 |
| Corn | 3G | 0 | 0 | 0 |
| Barley | 5G | 0 | 5G | 3G |
| Soybean | 0 | 0 | 0 | 0 |
| Rice | 5G | 0 | 3C,5G | 2C |
| Sorghum | 2C | 0 | 3C,5G | 2G |
| Sugar beet | 0 | 0 | 2H | 3G |
| Cotton | - | 0 | 1C | 0 |

Table A (continued)

| | Compound 27 | | Compound 28 | |
|---|---|---|---|---|
| Rate kg/ha | 0.05 | 0.01 | 0.05 | 0.01 |
| POSTEMERGENCE | | | | |
| Morningglory | 2C,6G | 2G | 10C | 3C,8G |
| Cocklebur | 10C | 5C,9G | 10C | 9C |
| Velvetleaf | 9C | 3C,7G | 4C,9G | 3C,9G |
| Nutsedge | 10C | 3C,8G | 10C | 4C,9G |
| Crabgrass | 2G | 2G | 3G | 0 |
| Giant Foxtail | 4C,9G | 4G | 7G | 2G |
| Barnyardgrass | 4C,9H | 4C,8H | 3C,7H | 1C,4G |
| Cheatgrass | 4G | 0 | 0 | 0 |
| Wild Oats | 9G | 3C,6G | 2G | 0 |
| Wheat | 5C,9G | 2C,6G | 4G | 0 |
| Corn | 2G | 0 | 0 | 0 |
| Barley | 9G | 3G | 2G | 0 |
| Soybean | 5C,9G | 4C,9H | 3H,8G | 3H,8G |
| Rice | 9C | 5C,9G | 2C,8G | 6G |
| Sorghum | 9C | 4C,9G | 4C,9H | 4C,9G |
| Sugar beet | 10C | 9C | 9C | 9C |
| Cotton | 9C | 9H | 9H | 9G |
| PREEMERGENCE | | | | |
| Morningglory | 6G | 0 | 3C,9G | 5H |
| Cocklebur | 8H | 6H | 9H | 3C,4H |
| Velvetleaf | 3C,8H | 7G | 5C,9H | 2G |
| Nutsedge | 5G | 0 | 10E | 0 |
| Crabgrass | 0 | 0 | 0 | 0 |
| Giant Foxtail | 3C,6G | 3C | 3C,3H | 3G |
| Barnyardgrass | 4C,9H | 3C,3G | 5C,9H | 3C,3G |
| Cheatgrass | 0 | 0 | 3G | 3G |
| Wild Oats | 3C,7G | 2C | 2C | 0 |
| Wheat | 7G | 2G | 6G | 0 |
| Corn | 2C | 0 | 2G | 0 |
| Barley | 9G | 6G | 8G | 5G |
| Soybean | 2C,7G | 2C,2H | 3C,5G | 3C,4G |
| Rice | 9H | 3C,7H | 5C,9H | 4C,8H |
| Sorghum | 9H | 2C,6G | 5C,9H | 5C,9H |
| Sugar beet | 4C,8G | 3C,7H | 10C | 3C,7G |
| Cotton | 4C,8G | 2C,3G | 3C,7G | 2C,4G |

Table A (continued)

| | Compound 29 | | Compound 30 | |
|---|---|---|---|---|
| Rate kg/ha | 0.05 | 0.01 | 0.05 | 0.01 |
| POSTEMERGENCE | | | | |
| Morningglory | 3C,9G | 3C,7H | 10C | 4C,8H |
| Cocklebur | 3H | 0 | 9C | 4C,9G |
| Velvetleaf | 3C,7H | 0 | 9C | 7G |
| Nutsedge | 0 | 0 | 4C,8G | 4G |
| Crabgrass | 3C,5G | 0 | 6C,9H | 4C,6G |
| Giant Foxtail | 5C,9G | 3C,7G | 9C | 10C |
| Barnyardgrass | 3C,8H | 2H | 10C | 9C |
| Cheatgrass | 0 | 0 | 3G | 0 |
| Wild Oats | 4C,9G | 7G | 4G | 3G |
| Wheat | 9G | 7G | 4G | 0 |
| Corn | 4C,8H | 5H | 10C | 10C |
| Barley | 9C | 9G | 0 | 0 |
| Soybean | 3C,7H | 4H | 9C | 5C,9G |
| Rice | 9C | 3C,8G | 5C,9G | 4C,9G |
| Sorghum | 5C,9G | 4C,9G | 9C | 9C |
| Sugar beet | 3C,7H | 4H | 10C | 5C,9G |
| Cotton | 5H | 0 | 9C | 9H |
| PREEMERGENCE | | | | |
| Morningglory | 5H | 1C | 9C | 4C,4H |
| Cocklebur | 0 | 0 | 9H | 3C |
| Velvetleaf | 0 | 0 | 9C | 9C |
| Nutsedge | 0 | 0 | 4G | 0 |
| Crabgrass | 0 | 0 | 3C,7G | 3C,4G |
| Giant Foxtail | 5G | 0 | 9H | 3G |
| Barnyardgrass | 3C,5G | 0 | 5C,9H | 3C,7G |
| Cheatgrass | 0 | 0 | 3G | 0 |
| Wild Oats | 3C,6G | 0 | 2C | 0 |
| Wheat | 9H | 2G | 2G | 0 |
| Corn | 2C,5G | 0 | 5C,9H | 9H |
| Barley | 2C,8G | 2G | 5G | 0 |
| Soybean | 2C,2H | 0 | 3C,8H | 3C,7H |
| Rice | 3C,8H | 2C | 9H | 3C,6H |
| Sorghum | 3C,8H | 1C | 6C,9H | 4C,8H |
| Sugar beet | 3G | 4H | 10E | 5H |
| Cotton | 0 | 2H | 4C,9G | 2C,2H |

Table A (continued)

| | Compound 31 | | Compound 32 | |
|---|---|---|---|---|
| Rate kg/ha | 0.05 | 0.01 | 0.05 | 0.01 |
| POSTEMERGENCE | | | | |
| Morningglory | 10C | 3C,8H | 10C | 10C |
| Cocklebur | 10C | 9C | 10C | 10C |
| Velvetleaf | 9C | 4C,8H | 10C | 10C |
| Nutsedge | 7G | 0 | 10C | 9C |
| Crabgrass | 9C | 3C,8G | 5C,8G | 7G |
| Giant Foxtail | 10C | 9C | 9C | 9C |
| Barnyardgrass | 10C | 9C | 9C | 9C |
| Cheatgrass | 3G | 0 | 7G | 5G |
| Wild Oats | 7G | 4G | 4C,9G | 9G |
| Wheat | 2C,6G | 3G | 10C | 6C,9G |
| Corn | 10C | 9C | 10C | 2C,7H |
| Barley | 1C,4G | 0 | 9C | 8G |
| Soybean | 9C | 4C,9G | 9C | 9C |
| Rice | 9C | 4C,9G | 9C | 9C |
| Sorghum | 10C | 9C | 10C | 9C |
| Sugar beet | 10C | 9C | 10C | 10C |
| Cotton | 9C | 9C | 9C | 10C |
| PREEMERGENCE | | | | |
| Morningglory | 4C,8H | 3C,5H | 9C | 8H |
| Cocklebur | 9H | 2C,5G | 9H | 8H |
| Velvetleaf | 9C | 0 | 10C | 10C |
| Nutsedge | 4G | 0 | 10E | 10E |
| Crabgrass | 3C,5G | 0 | 7G | 4G |
| Giant Foxtail | 3C,6G | 1C | 5C,9G | 4C,7G |
| Barnyardgrass | 9C | 3C,4H | 9C | 9H |
| Cheatgrass | 2G | 0 | 7G | 0 |
| Wild Oats | 2C,4G | 0 | 5C,9G | 3C,8G |
| Wheat | 2G | 0 | 10E | 4C,9G |
| Corn | 9H | 0 | 3C,9H | 3C,7G |
| Barley | 5G | 0 | 5C,9G | 4C,9G |
| Soybean | 3C,8H | 2H | 9H | 3C,7H |
| Rice | 4C,9H | 2C,4G | 10E | 10E |
| Sorghum | 10H | 4C,7G | 6C,9H | 9C |
| Sugar beet | 5C,9G | 5H | 4C,8G | 9C |
| Cotton | 4C,8H | 2C | 9C | 9G |

Table A (continued)

| | Compound 33 | | Compound 34 | |
|---|---|---|---|---|
| Rate kg/ha | 0.05 | 0.01 | 0.05 | 0.01 |
| POSTEMERGENCE | | | | |
| Morningglory | 10C | 10C | 10C | 5C,9G |
| Cocklebur | 10C | 9C | 10C | 5C,9G |
| Velvetleaf | 10C | 9C | 10C | 5C,9G |
| Nutsedge | 9C | 5C,9G | 5C,9G | 5C,9G |
| Crabgrass | 3C,7G | 2G | 2C,9G | 2C,4G |
| Giant Foxtail | 9C | 4C,9H | 10C | 9C |
| Barnyardgrass | 10C | 9C | 10C | 4C,9H |
| Cheatgrass | 7G | 5G | 6G | 2G |
| Wild Oats | 5C,9G | 3C,7G | 9C | 4C,9G |
| Wheat | 9C | 4C,9G | 9C | 3C,9G |
| Corn | 3C,9H | 3C,7H | 4C,9H | 1H |
| Barley | 3C,9G | 4C,9G | 9C | 2C,8G |
| Soybean | 10C | 5C,9G | 9C | 4C,9G |
| Rice | 9C | 9C | 9C | 9C |
| Sorghum | 10C | 10C | 9C | 4C,9H |
| Sugar beet | 9C | 5C,9G | 10C | 9C |
| Cotton | 9C | 9C | 10C | 9C |
| PREEMERGENCE | | | | |
| Morningglory | 9C | 9G | 9H | 8H |
| Cocklebur | 9H | - | 8H | 9H |
| Velvetleaf | 4C,9G | 9G | 9H | 3C,9G |
| Nutsedge | 10E | 3C,5G | 4C,8G | 2C,3G |
| Crabgrass | 3C,7G | 2C,4G | 3C,5G | 1C |
| Giant Foxtail | 4C,8H | 3C,4G | 4C,8G | 2G |
| Barnyardgrass | 4C,9H | 2C,2H | 3C,7H | 0 |
| Cheatgrass | 8G | 2G | 6G | 0 |
| Wild Oats | 4C,9H | 6C,9G | 4C,9H | 4C,8G |
| Wheat | 9C | 4C,9G | 4C,8G | 8G |
| Corn | 3C,8H | 1C,5G | 4C,8H | 2G |
| Barley | 5C,9G | 9G | 5C,9G | 9G |
| Soybean | 9H | 9H | 9H | 3C,7H |
| Rice | 10E | 10E | 10E | 3C,8H |
| Sorghum | 10H | 7C,9H | 6C,9H | 4C,8H |
| Sugar beet | 9C | 9C | 9C | 4C,8H |
| Cotton | 9G | 8G | 4C,8G | 2G |

Table A (continued)

| | Compound 35 | | Compound 36 | |
|---|---|---|---|---|
| Rate kg/ha | 0.05 | 0.01 | 0.05 | 0.01 |
| POSTEMERGENCE | | | | |
| Morningglory | 10C | 10C | 9C | 10C |
| Cocklebur | 10C | 10C | 9C | 3C,8H |
| Velvetleaf | 10C | 4C,9G | 9C | 9C |
| Nutsedge | 10C | 4C,9G | 3C,7G | 3G |
| Crabgrass | 3C,7H | 3G | 9C | 3C,7G |
| Giant Foxtail | 9C | 4C,9G | 9C | 9C |
| Barnyardgrass | 10C | 10C | 9C | 10C |
| Cheatgrass | 6G | 2G | 7G | 3G |
| Wild Oats | 3C,9G | 3C,6G | 9C | 9C |
| Wheat | 5C,9G | 2C,8G | 9C | 5C,9G |
| Corn | 4G | 0 | 9C | 9C |
| Barley | 9C | 6G | 9C | 4C,9G |
| Soybean | 4C,9G | 4C,9G | 5C,9G | 4C,9G |
| Rice | 9C | 5C,9G | 9C | 9C |
| Sorghum | 9C | 9C | 9C | 9C |
| Sugar beet | 10C | 10C | 9C | 10C |
| Cotton | 10C | 10C | 9C | 9C |
| PREEMERGENCE | | | | |
| Morningglory | 9C | 8H | 2C,9H | 3C,7H |
| Cocklebur | 9H | 7H | 9H | 3H |
| Velvetleaf | 9G | 8G | 9G | 3C,5H |
| Nutsedge | 10E | 9G | 5G | 0 |
| Crabgrass | 2C,5G | 3C,4G | 4C,9G | 3C,6G |
| Giant Foxtail | 3C,6G | 4G | 4C,9H | 3C,5G |
| Barnyardgrass | 4C,9H | 3C,7G | 3C,9H | 3C,7H |
| Cheatgrass | 0 | 0 | 8G | 0 |
| Wild Oats | 3C,8G | 3C,5G | 6C,9H | 3C,8G |
| Wheat | 5C,9H | 7G | 6C,9H | 5G |
| Corn | 2C,8G | 4G | 10H | 4C,9H |
| Barley | 9G | 9G | 9C | 5C,9G |
| Soybean | 3C,8H | 3C,7H | 9H | 3C,7H |
| Rice | 10E | 10E | 10E | 3C,9H |
| Sorghum | 10H | 10H | 10H | 6C,9H |
| Sugar beet | 4C,9G | 3C,8G | 10C | 4C,9G |
| Cotton | 9G | 4C,9G | 8G | 1C |

Table A (continued)

| | Compound 37 | | Compound 38 | |
|---|---|---|---|---|
| Rate kg/ha | 0.05 | 0.01 | 0.05 | 0.01 |
| POSTEMERGENCE | | | | |
| Morningglory | 3C,8H | 3C,5H | 3C,8H | 1C |
| Cocklebur | 2C,2G | 0 | 3C,7H | 0 |
| Velvetleaf | 2C,4G | 0 | 2C,5G | 1C |
| Nutsedge | 0 | 0 | 0 | 0 |
| Crabgrass | 2C,3G | 0 | 2G | 0 |
| Giant Foxtail | 4C,8G | 4G | 3C,8H | 2G |
| Barnyardgrass | 9C | 3C,7H | 5C,9G | 4H |
| Cheatgrass | 2C,6G | 2G | 2G | 0 |
| Wild Oats | 2C,6G | 0 | 2C,5G | 0 |
| Wheat | 0 | 0 | 4G | 0 |
| Corn | 4C,9G | 3C,8H | 3C,9H | 3C,7H |
| Barley | 0 | 0 | 0 | 0 |
| Soybean | 4C,9G | 3C,3G | 3C,8G | 2C,4H |
| Rice | 5C,9G | 4C,9G | 5C,9G | 5G |
| Sorghum | 4C,9G | 4C,9G | 4C,9G | 2C,6G |
| Sugar beet | 3C,7H | 2H | 3C,7H | 3C,4H |
| Cotton | 3C,8H | 2C,3G | 3C,7H | 2C,2G |
| PREEMERGENCE | | | | |
| Morningglory | 0 | 0 | 1C | 0 |
| Cocklebur | - | 0 | 0 | 0 |
| Velvetleaf | 5G | 0 | 0 | 0 |
| Nutsedge | 2G | 0 | 3G | 0 |
| Crabgrass | 0 | 0 | - | 0 |
| Giant Foxtail | 0 | 0 | 0 | 0 |
| Barnyardgrass | 1C | 0 | 3G | 0 |
| Cheatgrass | 0 | 0 | 2G | 0 |
| Wild Oats | 0 | 0 | 0 | 0 |
| Wheat | 0 | 0 | 0 | 0 |
| Corn | 3C,7H | 2C | 2C,5G | 0 |
| Barley | 0 | 0 | 0 | 0 |
| Soybean | 3C,3H | 1C | 3C,3H | 0 |
| Rice | 3C,6H | 3C,3G | 2C,7G | 2G |
| Sorghum | 3C,8G | 3C,4G | 3C,8G | 2C |
| Sugar beet | 3C,5G | 7G | 5H | 3G |
| Cotton | 2C,4G | 1C | 2G | 0 |

Table A (continued)

| | Compound 39 | | Compound 40 | |
|---|---|---|---|---|
| Rate kg/ha | 0.05 | 0.01 | 0.05 | 0.01 |
| POSTEMERGENCE | | | | |
| Morningglory | 10C | 10C | 10C | 9C |
| Cocklebur | 9C | 9C | 9C | 9C |
| Velvetleaf | 9C | 10C | 10C | 5C,9H |
| Nutsedge | 9C | 5C,9G | 2C,8G | 5G |
| Crabgrass | 5C,9G | 3C,7G | 3C,7G | 4G |
| Giant Foxtail | 5C,9G | 3C,7G | 3C,7G | 2G |
| Barnyardgrass | 9C | 9C | 9C | 3C,7H |
| Cheatgrass | 10C | 9C | 9C | 4C,9G |
| Wild Oats | 5C,9G | 9G | 4C,9G | 3C,6G |
| Wheat | 3C,9G | 9G | 9G | 9G |
| Corn | 3C,9G | 2U,9G | 3C,8H | 1C,3G |
| Barley | 4C,9G | 9G | 5G | 2G |
| Soybean | 6C,9G | 5C,9G | 9C | 4C,9G |
| Rice | 9C | 5C,9G | 9C | 4C,9G |
| Sorghum | 3U,9G | 9G | 4C,9H | 3C,8G |
| Sugar beet | 10C | 9C | 10C | 3C,8H |
| Cotton | 9C | 4C,9G | 4C,9G | 4C,9H |
| PREEMERGENCE | | | | |
| Morningglory | 9C | 8G | 3C,8H | 2C,3H |
| Cocklebur | 9H | 8H | 3C,7H | 2C,3H |
| Velvetleaf | 3C,9G | 4C,9G | 3C,8H | 3C,5H |
| Nutsedge | 10E | 8G | 4G | 0 |
| Crabgrass | 3C,3G | 2G | 2C,5G | 0 |
| Giant Foxtail | 4C,9H | 3C,6G | 3C,7G | 2G |
| Barnyardgrass | 5C,9H | 4C,9H | 3C,8H | 2C,3G |
| Cheatgrass | 5C,9H | 9H | 9H | 3C,6G |
| Wild Oats | 4C,9H | 3C,8H | 3C,8G | 3C,5G |
| Wheat | 5C,9H | 3C,9G | 3C,8G | 5G |
| Corn | 3C,9H | 2C,8G | 3C,5G | 2C,2G |
| Barley | 9G | 9G | 3C,9G | 8G |
| Soybean | 9H | 3C,6H | 4C,6H | 3C,4H |
| Rice | 10E | 5C,9H | 4C,9H | 2C,6G |
| Sorghum | 4C,9G | 4C,8H | 4C,9G | 3C,6G |
| Sugar beet | 9G | 8G | 5C,9G | 4C,8G |
| Cotton | 9G | 9G | 8G | 2C,6G |

Table A (continued)

| | Compound 41 | | Compound 42 | |
|---|---|---|---|---|
| Rate kg/ha | 0.05 | 0.01 | 0.05 | 0.01 |
| POSTEMERGENCE | | | | |
| Morningglory | 4C,8H | 2C,4G | 2C,8G | 2C,7G |
| Cocklebur | 5C,9H | 2C,4G | 5C,9H | 3C,8G |
| Velvetleaf | 4C,8H | 3C,5H | 4C,8H | 5G |
| Nutsedge | 0 | 0 | 3G | 4G |
| Crabgrass | 2G | 0 | 0 | 0 |
| Giant Foxtail | 5G | 0 | 4G | 0 |
| Barnyardgrass | 2C,7H | 3H | 4C,9H | 4G |
| Cheatgrass | 3C,8G | 4G | 7G | 0 |
| Wild Oats | 7G | 3G | 2C,7G | 0 |
| Wheat | 4G | 2G | 2G | 0 |
| Corn | 0 | 0 | 2G | 0 |
| Barley | 0 | 0 | 0 | 0 |
| Soybean | 4C,8H | 3C,5H | 2C,7G | 1H |
| Rice | 5C,9G | 3C,5G | 2C,9G | 5G |
| Sorghum | 2C,6G | 2C,4G | 9G | 7G |
| Sugar beet | 3C,6H | 2C,3G | 3C,8G | 2C,6G |
| Cotton | 4C,9H | 2C,3G | 2C,8G | 5G |
| PREEMERGENCE | | | | |
| Morningglory | 1C | 2G | 2C,4H | 1H |
| Cocklebur | 3C,3G | - | 3C,7H | 7H |
| Velvetleaf | 1H | 0 | 0 | 2G |
| Nutsedge | 0 | 0 | 0 | 0 |
| Crabgrass | 0 | 0 | 6G | 0 |
| Giant Foxtail | 0 | 0 | 2C,7G | 0 |
| Barnyardgrass | 0 | 0 | 9H | 6G |
| Cheatgrass | 0 | 0 | 8G | 4G |
| Wild Oats | 0 | 0 | 2G | 0 |
| Wheat | 0 | 0 | 2G | 0 |
| Corn | 2G | 0 | 2G | 0 |
| Barley | 2G | 0 | 5G | 0 |
| Soybean | 3C,3H | 1C | 7G | 0 |
| Rice | 0 | 0 | 8H | 7G |
| Sorghum | 3C,3G | 0 | 8G | 2C,7G |
| Sugar beet | 6G | 3H | 8G | 0 |
| Cotton | 2C,3G | 0 | 5G | 2G |

Table A (continued)

| | Compound 43 | | Compound 44 | |
|---|---|---|---|---|
| Rate kg/ha | 0.05 | 0.01 | 0.05 | 0.01 |
| POSTEMERGENCE | | | | |
| Morningglory | 3C,8H | 3C,7H | 3C,7H | 3C,3H |
| Cocklebur | 3G | 0 | 2C,5G | 2C |
| Velvetleaf | 7G | 2C,5G | 3C,6H | 0 |
| Nutsedge | 0 | 0 | 0 | 0 |
| Crabgrass | 3G | 0 | 0 | 0 |
| Giant Foxtail | 2G | 0 | 1C | 0 |
| Barnyardgrass | 4G | 3G | 3C,7H | 2C,4G |
| Cheatgrass | 8G | 7G | 0 | 0 |
| Wild Oats | 4C,9G | 5C,9G | 0 | 0 |
| Wheat | 9G | 9G | 0 | 0 |
| Corn | 0 | 0 | 3C,7G | 0 |
| Barley | 9G | 9G | 2C | 0 |
| Soybean | 3H,8G | 2H,5G | 2C,7H | 3H |
| Rice | 4C,9G | 4C,9G | 4C,9G | 4G |
| Sorghum | 4C,8G | 2C,5G | 5C,9H | 5G |
| Sugar beet | 3C,7G | 2C,5G | 3C,7H | 3C,6G |
| Cotton | 5G | 0 | 3C,6G | 1C |
| PREEMERGENCE | | | | |
| Morningglory | 3H | 0 | 2C | 5G |
| Cocklebur | 8G | 0 | 1H | 0 |
| Velvetleaf | 0 | 0 | 7G | 0 |
| Nutsedge | 0 | 0 | 0 | 0 |
| Crabgrass | 0 | 0 | 0 | 0 |
| Giant Foxtail | 5G | 0 | 3G | 0 |
| Barnyardgrass | 0 | 0 | 3G | 0 |
| Cheatgrass | 7G | 3G | 0 | 0 |
| Wild Oats | 2C,6G | 0 | 0 | 0 |
| Wheat | 6G | 0 | 0 | 0 |
| Corn | 2C,6G | 0 | 3C,7H | 0 |
| Barley | 9G | 7G | 0 | 0 |
| Soybean | 1C | 0 | 0 | 0 |
| Rice | 9H | 4G | 3C,8H | 2C,4G |
| Sorghum | 3C,6H | 0 | 3C,8H | 2C |
| Sugar beet | 9G | 2G | 7G | 5G |
| Cotton | 0 | 0 | 2C | 0 |

Table A (continued)

| | Compound 45 | | Compound 46 | |
|---|---|---|---|---|
| Rate kg/ha | 0.05 | 0.01 | 0.05 | 0.01 |
| POSTEMERGENCE | | | | |
| Morningglory | 4C,9H | 2C,5G | 10C | 10C |
| Cocklebur | 4C,9H | 1C | 10C | 8H |
| Velvetleaf | 3C,7H | 3C,3G | 10C | 9C |
| Nutsedge | 3G | 0 | 2C,9G | 4C,8G |
| Crabgrass | 2G | 0 | 7G | 5G |
| Giant Foxtail | 2G | 0 | 5C,9G | 3C,8G |
| Barnyardgrass | 3C,9H | 2C,5H | 10C | 5C,9G |
| Cheatgrass | 3G | 0 | 9G | 8G |
| Wild Oats | 2G | 0 | 2C,9G | 9G |
| Wheat | 2G | 0 | 5C,9G | 9G |
| Corn | 2C,6G | 0 | 2C,9G | 9H |
| Barley | 0 | 0 | 3C,9G | 9G |
| Soybean | 3C,8G | 2C,6H | 9C | 5C,9G |
| Rice | 5C,9G | 5G | 9C | 9C |
| Sorghum | 4C,9H | 2C,8G | 9C | 5C,9G |
| Sugar beet | 5C,9G | 3C,7H | 10C | 9C |
| Cotton | 3C,8H | 2C,4G | 10C | 2C,8G |
| PREEMERGENCE | | | | |
| Morningglory | 4H | 4H | 9G | 9G |
| Cocklebur | 6H | 4H | 9H | 8G |
| Velvetleaf | 2G | 3G | 9C | 9G |
| Nutsedge | 0 | 0 | 4C,9G | 0 |
| Crabgrass | 0 | 0 | 3G | 0 |
| Giant Foxtail | 0 | 0 | 9H | 7G |
| Barnyardgrass | 4G | 0 | 9H | 3C,9H |
| Cheatgrass | 0 | 0 | 7G | 3G |
| Wild Oats | 0 | 0 | 4C,9H | 8H |
| Wheat | 0 | 0 | 10H | 3C,9H |
| Corn | 3C,8H | 0 | 10H | 3C,9H |
| Barley | 0 | 0 | 9G | 9H |
| Soybean | 2C,2H | 0 | 9H | 8H |
| Rice | 3C,7H | 0 | 10E | 10E |
| Sorghum | 3C,8G | 2C,4G | 10H | 5C,9H |
| Sugar beet | 4G | 4G | 5C,9G | 5C,9G |
| Cotton | 5G | 2C | 9G | 9G |

Table A (continued)

| | Compound 47 | | Compound 48 | |
|---|---|---|---|---|
| Rate kg/ha | 0.05 | 0.01 | 0.05 | 0.01 |
| POSTEMERGENCE | | | | |
| Morningglory | 9C | 4C,9H | 4C,9H | 2C,2H |
| Cocklebur | 7H | 2G | 1H | 1H |
| Velvetleaf | 5C,9G | 3C,8G | 3C,7H | 3G |
| Nutsedge | 7G | 3G | 4G | 0 |
| Crabgrass | 4G | 0 | 0 | 0 |
| Giant Foxtail | 5G | 2G | 0 | 0 |
| Barnyardgrass | 3C,9H | 3C,8H | 3C,7H | 1H |
| Cheatgrass | 2C,6G | 4G | 2G | 0 |
| Wild Oats | 3C,8G | 3G | 2G | 0 |
| Wheat | 8G | 2G | 2G | 0 |
| Corn | 3C,8H | 2G | 1H | 0 |
| Barley | 3C,8G | 4G | 2G | 0 |
| Soybean | 4C,9G | 3C,8G | 2C,7G | 2H |
| Rice | 4C,9G | 4C,9G | 4C,9G | 2G |
| Sorghum | 4C,9G | 4C,9G | 4C,8G | 6G |
| Sugar beet | 9C | 9C | 4C,8H | 2C,6G |
| Cotton | 8H | 3C,8G | 6H | 0 |
| PREEMERGENCE | | | | |
| Morningglory | 9G | 7G | 2G | 3G |
| Cocklebur | 8H | - | 0 | - |
| Velvetleaf | 4C,9G | 7H | 0 | - |
| Nutsedge | 4G | 0 | 0 | 0 |
| Crabgrass | 2G | 0 | 0 | 0 |
| Giant Foxtail | 8G | 2C | 0 | 0 |
| Barnyardgrass | 8H | 2C | 0 | 0 |
| Cheatgrass | 6G | 0 | 0 | 0 |
| Wild Oats | 2C,8G | 2G | 0 | 0 |
| Wheat | 8G | 3G | 0 | 0 |
| Corn | 2C,9G | 2C,6G | 0 | 0 |
| Barley | 9G | 2C,7G | 2G | 0 |
| Soybean | 3C,7H | 7H | 2C | 0 |
| Rice | 5C,9H | 6G | 0 | 3G |
| Sorghum | 5C,9H | 2C,8G | 2C | - |
| Sugar beet | 4C,9G | 7G | - | 2G |
| Cotton | 9G | 2C,5G | 2G | - |

Table A (contin d)

| | Compound 49 | | Compound 50 | |
|---|---|---|---|---|
| Rate kg/ha | 0.05 | 0.01 | 0.05 | 0.01 |
| POSTEMERGENCE | | | | |
| Morningglory | 4C,8G | 2C,5G | 2C,5H | 0 |
| Cocklebur | 4C,9H | 5G | 0 | 0 |
| Velvetleaf | 3C,8H | 3H | 2C,5G | 0 |
| Nutsedge | 5G | 0 | 0 | 0 |
| Crabgrass | 0 | 0 | 0 | 0 |
| Giant Foxtail | 3C,6G | 0 | 2C,4G | 0 |
| Barnyardgrass | 3C,8H | 3H | 3C,7H | 0 |
| Cheatgrass | 0 | 0 | 0 | 0 |
| Wild Oats | 0 | 0 | 0 | 0 |
| Wheat | 2G | 0 | 0 | 0 |
| Corn | 1H | 0 | 2G | 0 |
| Barley | 1C | 0 | 0 | 0 |
| Soybean | 2H | 1H | 0 | 0 |
| Rice | 4C,9G | 4C,9G | 6G | 0 |
| Sorghum | 4C,9G | 4C,9H | 8G | 4G |
| Sugar beet | 3C,8H | 2C,5G | 3C,7G | 0 |
| Cotton | 7G | 3G | 2G | 0 |
| PREEMERGENCE | | | | |
| Morningglory | 8G | 7G | 0 | 0 |
| Cocklebur | 9H | 7H | 8G | - |
| Velvetleaf | 3H | 0 | 7G | - |
| Nutsedge | 4G | 0 | 0 | 0 |
| Crabgrass | 0 | 0 | 0 | 0 |
| Giant Foxtail | 3C,4G | 2G | 0 | 0 |
| Barnyardgrass | 3C,8H | 2G | 0 | 0 |
| Cheatgrass | 0 | 0 | 0 | 0 |
| Wild Oats | 2G | 0 | 0 | 0 |
| Wheat | 3G | 0 | 0 | 0 |
| Corn | 4G | 0 | 0 | 0 |
| Barley | 8G | 0 | 0 | 0 |
| Soybean | 4G | 0 | 0 | 0 |
| Rice | 3C,8H | 4G | 2C | 0 |
| Sorghum | 10H | 3C,8H | 2C,5G | 0 |
| Sugar beet | 3C,8G | 8G | 4G | 0 |
| Cotton | 2C,7G | 1C | 0 | 0 |

Table A (continued)

| | Compound 51 | | Compound 52 | |
|---|---|---|---|---|
| Rate kg/ha | 0.05 | 0.01 | 0.05 | 0.01 |
| POSTEMERGENCE | | | | |
| Morningglory | 4C,8H | 1C | 5C,9G | 4C,9H |
| Cocklebur | 3C,6G | 0 | 4C,9H | 3C,4G |
| Velvetleaf | 2G | 0 | 3C,8H | 3C,4G |
| Nutsedge | 8G | 0 | 9G | 2C,7G |
| Crabgrass | 2C,2G | 0 | 3C,7G | 2C |
| Giant Foxtail | 6C,9H | 3C,7H | 9C | 4C,8G |
| Barnyardgrass | 9C | 3C,8H | 9C | 9H |
| Cheatgrass | 8G | 0 | 3C,8G | 5G |
| Wild Oats | 4C,9G | 0 | 4C,9G | 3C,7G |
| Wheat | 8G | 2G | 8G | 4G |
| Corn | 4C,9H | 2C,5G | 3C,8H | 0 |
| Barley | 3G | 0 | 3C,7G | 2G |
| Soybean | 3C,7H | 2C,5G | 4C,9G | 2C,8H |
| Rice | 9C | 5C,9G | 9C | 4C,9G |
| Sorghum | 9C | 4C,9H | 9C | 5C,9G |
| Sugar beet | 9C | 3C,7H | 9C | 4C,8H |
| Cotton | 8H | 7H | 4C,9G | 3C,8H |
| PREEMERGENCE | | | | |
| Morningglory | 5G | 7G | 8G | 0 |
| Cocklebur | 4H | - | 7H | 0 |
| Velvetleaf | 0 | 5G | 5G | 0 |
| Nutsedge | 0 | 0 | 5G | 0 |
| Crabgrass | 0 | 0 | 0 | 0 |
| Giant Foxtail | 4G | 2G | 6G | 2G |
| Barnyardgrass | 2G | 2G | 5G | 0 |
| Cheatgrass | 2G | 0 | 5G | 0 |
| Wild Oats | 0 | 0 | 2G | 0 |
| Wheat | 2G | 0 | 7G | 0 |
| Corn | 2C,8G | 0 | 3C,8H | 2G |
| Barley | 6G | 0 | 8G | 3G |
| Soybean | 2C,5G | 1H | 8H | 5H |
| Rice | 9H | 2G | 9H | 5H |
| Sorghum | 2C,9H | 2C,5G | 3C,9H | 3C,8G |
| Sugar beet | 5G | 5G | 9G | 8G |
| Cotton | 7G | 0 | 2C,7G | 2G |

Table A (continued)

| | Compound 53 | | Compound 54 | |
|---|---|---|---|---|
| Rate kg/ha | 0.05 | 0.01 | 0.05 | 0.01 |
| POSTEMERGENCE | | | | |
| Morningglory | 9C | 10C | 10C | 10C |
| Cocklebur | 9C | 9C | 10C | 10C |
| Velvetleaf | 9C | 9C | 10C | 9C |
| Nutsedge | 9C | 9C | 9C | 5C,9G |
| Crabgrass | 8G | 3G | 6G | 3G |
| Giant Foxtail | 9C | 3C,8G | 5C,9G | 3C,9G |
| Barnyardgrass | 9C | 9C | 10C | 9C |
| Cheatgrass | 9C | 9G | 9C | 4C,9G |
| Wild Oats | 2G | 0 | 3C,5G | 0 |
| Wheat | 9G | 7G | 3C,9G | 9G |
| Corn | 3H | 0 | 2H | 0 |
| Barley | 3C,9G | 2C,6G | 8G | 5G |
| Soybean | 9C | 5C,9G | 9C | 4C,9G |
| Rice | 9C | 5C,9G | 9C | 9C |
| Sorghum | 9C | 10C | 9C | 9C |
| Sugar beet | 9C | 10C | 10C | 9C |
| Cotton | 9C | 9C | 9C | 5C,9G |
| PREEMERGENCE | | | | |
| Morningglory | 9C | 9G | 9G | 9G |
| Cocklebur | 9H | - | 9H | 9H |
| Velvetleaf | 9C | 9C | 5C,9G | 5C,9G |
| Nutsedge | 10E | 10E | 10E | 10E |
| Crabgrass | 2G | 0 | 3G | 0 |
| Giant Foxtail | 9H | 8H | 9H | 3C,8H |
| Barnyardgrass | 9H | 9H | 9H | 9H |
| Cheatgrass | 10H | 9H | 9H | 10E |
| Wild Oats | 0 | 0 | 8G | 2C,5G |
| Wheat | 9H | 7H | 10H | 4C,9H |
| Corn | 2C,7G | 4G | 7G | 6G |
| Barley | 9G | 9G | 9G | 9G |
| Soybean | 9H | 9H | 9H | 3C,8H |
| Rice | 10E | 10E | 10E | 10E |
| Sorghum | 5C,9H | 9H | 10H | 5C,9H |
| Sugar beet | 9C | 9C | 9C | 5C,9G |
| Cotton | 9G | 9G | 4C,9G | 9G |

Table A (continued)

| | Compound 55 | | Compound 56 | |
|---|---|---|---|---|
| Rate kg/ha | 0.05 | 0.01 | 0.05 | 0.01 |
| POSTEMERGENCE | | | | |
| Morningglory | 10C | 8C | 10C | 10C |
| Cocklebur | 10C | 9C | 10C | 4C,9G |
| Velvetleaf | 10C | 9C | 10C | 3C,8G |
| Nutsedge | 9C | 9G | 5C,9G | 4C,9G |
| Crabgrass | 3C,6G | 2C,5G | 6G | 2G |
| Giant Foxtail | 9C | 3C,8G | 4C,9G | 7G |
| Barnyardgrass | 9C | 10C | 9C | 9H |
| Cheatgrass | 9C | 5C,9G | 4C,9G | 9G |
| Wild Oats | 5C,9G | 3C,3G | 3G | 0 |
| Wheat | 9G | 9G | 8G | 3G |
| Corn | 2H | 0 | 0 | 0 |
| Barley | 9G | 3C,7G | 2G | 0 |
| Soybean | 5C,9G | 4C,8G | 3C,8G | 3C,5H |
| Rice | 5C,9G | 5C,9G | 9C | 5C,9G |
| Sorghum | 5C,9G | 5C,9G | 9C | 5C,9G |
| Sugar beet | 9C | 9C | 10C | 9C |
| Cotton | 9G | 9G | 10C | 3C,8G |
| PREEMERGENCE | | | | |
| Morningglory | 9G | 8G | 9G | 9G |
| Cocklebur | 9H | 9H | 9H | 5H |
| Velvetleaf | 4C,9G | 8G | 8H | 6H |
| Nutsedge | 10E | 9G | 10E | 4G |
| Crabgrass | 2G | 0 | 2G | 0 |
| Giant Foxtail | 9H | 3C,8G | 3C,7G | 6G |
| Barnyardgrass | 9H | 3C,8H | 9H | 8H |
| Cheatgrass | 9H | 8G | 8H | 6G |
| Wild Oats | 8G | 0 | 0 | 0 |
| Wheat | 9G | 2G | 8G | 5G |
| Corn | 3G | 1C,2G | 1C | 0 |
| Barley | 9G | 8G | 4C,8G | 5G |
| Soybean | 3C,8H | 2C,6G | 7H | 2H |
| Rice | 10E | 9H | 10E | 8H |
| Sorghum | 10H | 9H | 9H | 5C,9H |
| Sugar beet | 9G | 9G | 4C,9G | 8G |
| Cotton | 9G | 9G | 9G | 7G |

Table A (contin' d)

| | Compound 57 | | Compound 58 | |
|---|---|---|---|---|
| Rate kg/ha | 0.05 | 0.01 | 0.05 | 0.01 |
| POSTEMERGENCE | | | | |
| Morningglory | 10C | 2C,8G | 1C,7G | 0 |
| Cocklebur | 10C | 10C | 0 | 0 |
| Velvetleaf | 10C | 2C,9H | 2G | 0 |
| Nutsedge | 5C,9G | 2C,9G | 0 | 0 |
| Crabgrass | 7G | 0 | 0 | 0 |
| Giant Foxtail | 3C,9G | 2C,7G | 0 | 0 |
| Barnyardgrass | 2C,9G | 2C,5H | 0 | 0 |
| Cheatgrass | 8C | 2C,9G | 2G | 0 |
| Wild Oats | 2C,9G | 2C,6G | 0 | 0 |
| Wheat | 1C,6G | 0 | 0 | 0 |
| Corn | 1C,8H | 3C,8H | 0 | 0 |
| Barley | 5C,9G | 3C,9G | 0 | 0 |
| Soybean | 5C,8H | 2C,6H | 0 | 0 |
| Rice | 5C,9G | 2C,8G | 3C | 0 |
| Sorghum | 3C,9G | 3C,9G | 1C,5G | 0 |
| Sugar beet | 9C | 10C | 2C,4H | 0 |
| Cotton | 10C | 2C,8H | 2G | 0 |
| PREEMERGENCE | | | | |
| Morningglory | 8H | 3C,7H | 0 | 0 |
| Cocklebur | 2C,8H | 2C,7H | 0 | 0 |
| Velvetleaf | 2C,7H | 2C,5H | 0 | 0 |
| Nutsedge | 10E | 9G | 0 | 0 |
| Crabgrass | 1C,5G | 2C,4G | 0 | 0 |
| Giant Foxtail | 2C,7H | 2C,6G | 0 | 0 |
| Barnyardgrass | 2C,6H | 2C,4H | 0 | 0 |
| Cheatgrass | 3C,9H | 2C,9H | 0 | 0 |
| Wild Oats | 2C,6G | 2C,6G | 0 | 0 |
| Wheat | 4G | 0 | 0 | 0 |
| Corn | 2C,7G | 3C,4G | 0 | 0 |
| Barley | 6G | 6G | 0 | 0 |
| Soybean | 2C,5G | 1C,2G | 0 | 0 |
| Rice | 3C,9H | 2C,8H | 0 | 0 |
| Sorghum | 2C,9H | 2C,8H | 0 | 0 |
| Sugar beet | 9C | 4C,8G | 1C | 0 |
| Cotton | 2C,8G | 2C,8G | 0 | 0 |

Table A (continued)

| | Compound 59 | | Compound 60 | |
|---|---|---|---|---|
| Rate kg/ha | 0.05 | 0.01 | 0.05 | 0.01 |
| POSTEMERGENCE | | | | |
| Morningglory | 10C | 10C | 3C,8G | 1C,1H |
| Cocklebur | 10C | 10C | 2C,8G | 2H |
| Velvetleaf | 10C | 9C | 3C,9G | 4G |
| Nutsedge | 10C | 10C | 2C,5G | 0 |
| Crabgrass | 3C,9G | 7G | 0 | 0 |
| Giant Foxtail | 9C | 9C | 3C,8G | 4G |
| Barnyardgrass | 9C | 9C | 3C,7H | 2H |
| Cheatgrass | 9C | 10C | 9G | 3G |
| Wild Oats | 9C | 5C,9G | 2C,7G | 0 |
| Wheat | 9C | 4C,9G | 9G | 0 |
| Corn | 9C | 10C | 3C,8H | 0 |
| Barley | 9C | 5C,9G | 2C,4G | 0 |
| Soybean | 9C | 9C | 3G | 2H |
| Rice | 9C | 9C | 5C,9G | 0 |
| Sorghum | 9C | 9C | 2C,9G | 5G |
| Sugar beet | 9C | 9C | 9C | 3G |
| Cotton | 10C | 9C | 3C,8G | 0 |
| PREEMERGENCE | | | | |
| Morningglory | 9G | 9G | 0 | 0 |
| Cocklebur | 9H | 9H | 2H | 0 |
| Velvetleaf | 9C | 3C,9G | 0 | 0 |
| Nutsedge | 10E | 10E | 0 | 0 |
| Crabgrass | 3C,7G | 3G | 0 | 0 |
| Giant Foxtail | 5C,9H | 3C,7G | 0 | 0 |
| Barnyardgrass | 5C,9H | 4C,9H | 0 | 0 |
| Cheatgrass | 10E | 9H | 0 | 0 |
| Wild Oats | 4C,9H | 3C,6G | 0 | 0 |
| Wheat | 10H | 4C,9H | 0 | 0 |
| Corn | 5C,9H | 4C,9H | 0 | 0 |
| Barley | 4C,9G | 3C,9G | 0 | 0 |
| Soybean | 9H | 9H | 0 | 0 |
| Rice | 10E | 10E | 0 | 0 |
| Sorghum | 10H | 4C,9H | 0 | 0 |
| Sugar beet | 5C,9G | 4C,9G | 0 | 0 |
| Cotton | 9G | 9G | 0 | 0 |

Table A (conti ed)

| | Compound 61 | | Compound 62 | |
|---|---|---|---|---|
| Rate kg/ha | 0.05 | 0.01 | 0.05 | 0.01 |
| POSTEMERGENCE | | | | |
| Morningglory | 1C,1H | 0 | 0 | 0 |
| Cocklebur | 3H | 0 | 0 | 0 |
| Velvetleaf | 4H | 0 | 0 | 0 |
| Nutsedge | 0 | 0 | 0 | 0 |
| Crabgrass | 0 | 0 | 0 | 0 |
| Giant Foxtail | 5G | 2G | 2G | 0 |
| Barnyardgrass | 2C,5G | 3G | 2C,5G | 0 |
| Cheatgrass | 6G | 0 | 6G | 0 |
| Wild Oats | 2C,5G | 2G | 2C,5G | 0 |
| Wheat | 9G | 8G | 6G | 0 |
| Corn | 2C,7G | 3G | 3G | 0 |
| Barley | 2C,5G | 3G | 4G | 0 |
| Soybean | 2C,6H | 3H | 0 | 0 |
| Rice | 3C,9G | 3G | 7G | 0 |
| Sorghum | 4G | 2G | 7G | 0 |
| Sugar beet | 2C,4G | 0 | 2C | 0 |
| Cotton | 0 | 0 | 0 | 0 |
| PREEMERGENCE | | | | |
| Morningglory | 0 | 0 | 0 | 0 |
| Cocklebur | 0 | 0 | 0 | 0 |
| Velvetleaf | 0 | 0 | 0 | 0 |
| Nutsedge | 0 | 0 | 0 | 0 |
| Crabgrass | 0 | 0 | 0 | 0 |
| Giant Foxtail | 0 | 0 | 0 | 0 |
| Barnyardgrass | 0 | 0 | 0 | 0 |
| Cheatgrass | 0 | 0 | 0 | 0 |
| Wild Oats | 0 | 0 | 0 | 0 |
| Wheat | 0 | 0 | 0 | 0 |
| Corn | 0 | 0 | 0 | 0 |
| Barley | 0 | 0 | 0 | 0 |
| Soybean | 0 | 0 | 0 | 0 |
| Rice | 0 | 0 | 0 | 0 |
| Sorghum | 0 | 0 | 0 | 0 |
| Sugar beet | 0 | 0 | 0 | 0 |
| Cotton | 0 | 0 | 0 | 0 |

Table A (continued)

| | Compound 63 | |
|---|---|---|
| Rate kg/ha | 0.05 | 0.01 |
| POSTEMERGENCE | | |
| Morningglory | 10C | 4C,8G |
| Cocklebur | 4C,9H | 3C,7H |
| Velvetleaf | 4C,8H | 3C,7H |
| Nutsedge | 9G | 2C,8G |
| Crabgrass | 3C,6G | 5G |
| Giant Foxtail | 9C | 2C,8G |
| Barnyardgrass | 9C | 3C,9G |
| Cheatgrass | 5C,9G | 9G |
| Wild Oats | 3C,8G | 2C,5G |
| Wheat | 9G | 7G |
| Corn | 5C,9G | 3C,9G |
| Barley | 9G | 8G |
| Soybean | 3C,8H | 3C,4H |
| Rice | 6C,9G | 4C,9G |
| Sorghum | 3C,9G | 4C,9G |
| Sugar beet | 9C | 5C,9G |
| Cotton | 4C,9G | 3C,8H |
| PREEMERGENCE | | |
| Morningglory | 3H | 0 |
| Cocklebur | 4G | 1H |
| Velvetleaf | 0 | 0 |
| Nutsedge | 7G | 0 |
| Crabgrass | 3G | 0 |
| Giant Foxtail | 3G | 2G |
| Barnyardgrass | 4G | 2G |
| Cheatgrass | 5G | 0 |
| Wild Oats | 2C | 0 |
| Wheat | 3G | 0 |
| Corn | 3C,3G | 0 |
| Barley | 3C,9G | 4G |
| Soybean | 1C | 0 |
| Rice | 4C,8H | 3G |
| Sorghum | 4C,9H | 3C,5G |
| Sugar beet | 7G | 3C,5G |
| Cotton | 3C,6G | 5G |

Test B

Postemergence

Two round pans (25 cm diameter by 12.5 cm deep) were filled with Sassafras sandy loam soil. One pan was planted with blackgrass. (*Alopecurus myosuroides*), sugar beets, nutsedge (*Cyperus rotundus*) tubers, rape (*Brassica napus*), crabgrass (*Digitaria sanguinalis*), sicklepod (*Cassia obtusifolia*), teaweed (*Sida spinosa*), jimsonweed (*Datura stramonium*), velvetleaf (*Abutilon theophrasti*), and giant foxtail (*Setaria*

faberii). The other pan was planted with wheat, barley, cotton, rice, corn, soybean, wild oats (Avena fatua), cocklebur (Xanthium pensylvanicum), morningglory (Ipomoea hederacea), johnsongrass (Sorghum halepense) and barnyardgrass (Echinochloa crusgalli). The plants were grown for approximately fourteen days, then sprayed postemergence with the chemicals dissolved in a non-phytotoxic solvent.

Preemergence

Two round pans (25 cm diameter by 12.5 cm deep) were filled with Sassafras sandy loam soil. One pan was planted with blackgrass, sugar beets, nutsedge, rape, crabgrass, sicklepod, teaweed, jimsonweed, velvetleaf, and giant foxtail. The other pan was planted with wheat, barley, cotton, rice, corn, soybeans, wild oats, cocklebur, morningglory, johnsongrass, and barnyardgrass. The two pans were sprayed preemergence with the chemicals dissolved in a non-phytotoxic solvent.

Treated plants and controls were maintained in the greenhouse for 28 days, then all treated plants were compared to controls and visually rated for plant response. Response ratings are based on a scale of 0 to 100 where 0 = no effect, and 100 = complete control. A dash (-) response means no test.

Response ratings are contained in Table B.

Table B

| | Compound 2 | | | | | | |
|---|---|---|---|---|---|---|---|
| POSTEMERGENCE | | | | | | | |
| Rate g/ha | 62 | 16 | 16 | 4 | 4 | 1 | .25 |
| Corn | 100 | 100 | 100 | 100 | 100 | 60 | 40 |
| Wheat | 100 | 100 | 100 | 60 | 100 | 40 | 0 |
| Barley | - | - | - | - | - | - | - |
| Rice | 100 | 100 | 100 | 90 | 70 | 50 | 30 |
| Soybean | 100 | 100 | 100 | 100 | 90 | 90 | 60 |
| Cotton | 100 | 100 | 60 | 70 | 20 | 40 | 0 |
| Sugar beet | 100 | 100 | 100 | 100 | 100 | 100 | 100 |
| Rape | 100 | 100 | 100 | 100 | 100 | 100 | 100 |
| Crabgrass | 100 | 80 | 100 | 50 | 60 | 40 | 0 |
| Johnsongrass | 100 | 100 | 100 | 100 | 90 | 60 | 40 |
| Blackgrass | 100 | 100 | 100 | 100 | 90 | 50 | 30 |
| Barnyardgrass | 100 | 100 | 100 | 100 | 100 | 60 | 40 |
| Nutsedge | 100 | 100 | 100 | 100 | 80 | 90 | 80 |
| Giant Foxtail | 100 | 100 | 100 | 90 | 90 | 50 | 30 |
| Wild Oats | 90 | 100 | 80 | 60 | 70 | 40 | 0 |
| Cocklebur | 100 | 100 | 90 | 70 | 60 | 30 | 0 |
| Morningglory | 100 | 100 | 90 | 100 | 70 | 90 | 60 |
| Teaweed | 100 | 100 | 90 | 70 | 30 | 40 | 0 |
| Sicklepod | 100 | 100 | 100 | 90 | 70 | 60 | 0 |
| Jimsonweed | 100 | 100 | 100 | 90 | 70 | 70 | 40 |
| Velvetleaf | 100 | 100 | 100 | 100 | 80 | 80 | 50 |
| PREEMERGENCE | | | | | | | |
| Rate g/ha | 250 | 62 | 62 | 16 | 16 | 4 | 1 |
| Corn | 100 | 100 | 100 | 80 | 90 | 20 | 0 |
| Wheat | 100 | 90 | 100 | 80 | 100 | 30 | 0 |
| Barley | - | - | - | - | - | - | - |
| Rice | 100 | 100 | 100 | 100 | 100 | 90 | 70 |
| Soybean | 100 | 100 | 100 | 80 | 100 | 40 | 20 |
| Cotton | 100 | 80 | 100 | 80 | 80 | 30 | 0 |
| Sugar beet | 100 | 100 | 100 | 100 | 100 | 100 | 30 |
| Rape | 100 | 100 | 100 | 90 | 100 | 20 | 0 |
| Crabgrass | 100 | 100 | 100 | 90 | 90 | 30 | 0 |
| Johnsongrass | 100 | 100 | 100 | 100 | 100 | 90 | 30 |
| Blackgrass | 100 | 90 | 100 | 100 | 100 | 70 | 20 |
| Barnyardgrass | 100 | 100 | 100 | 90 | 100 | 80 | 20 |
| Nutsedge | 100 | 100 | 100 | 100 | 100 | 70 | 20 |
| Giant Foxtail | 100 | 100 | 100 | 90 | 100 | 80 | 50 |
| Wild Oats | 100 | 90 | 100 | 90 | 90 | 20 | 0 |
| Cocklebur | 100 | 90 | 100 | 80 | 90 | 20 | 0 |
| Morningglory | 100 | 50 | 100 | 40 | 70 | 0 | 0 |
| Teaweed | 100 | 100 | 100 | 80 | 90 | 50 | 20 |
| Sicklepod | 100 | 90 | 100 | 80 | 80 | 60 | 20 |
| Jimsonweed | 100 | 100 | 100 | 90 | 90 | 60 | 20 |
| Velvetleaf | 100 | 100 | 100 | 100 | 100 | 60 | 0 |

Table B (continued)

Compound 3

| POSTEMERGENCE Rate g/ha | 62 | 16 | 16 | 16 | 4 | 4 | 4 | 1 | 1 | .25 | .25 |
|---|---|---|---|---|---|---|---|---|---|---|---|
| Corn | 100 | 100 | 100 | 100 | 100 | 90 | 100 | 80 | 70 | 60 | 0 |
| Wheat | 100 | 100 | 30 | 70 | 40 | 0 | 20 | 0 | 0 | 0 | 0 |
| Barley | - | - | - | - | - | - | - | - | - | - | - |
| Rice | 100 | 100 | 80 | 100 | 90 | 60 | 100 | 50 | 30 | 30 | 0 |
| Soybean | 100 | 100 | 100 | 100 | 100 | 90 | 90 | 80 | 70 | 80 | 0 |
| Cotton | 100 | 100 | 100 | 100 | 100 | 80 | 100 | 70 | 40 | 30 | 0 |
| Sugar beet | 100 | 100 | 100 | 100 | 100 | 100 | 100 | 100 | 100 | 100 | 0 |
| Rape | 100 | 100 | 100 | 100 | 100 | 100 | 100 | 100 | 100 | 100 | 0 |
| Crabgrass | 100 | 90 | 50 | 70 | 70 | 30 | 30 | 50 | 0 | 30 | 0 |
| Johnsongrass | 100 | 100 | 100 | 100 | 100 | 70 | 90 | 70 | 30 | 30 | 0 |
| Blackgrass | 100 | 100 | 100 | 100 | 100 | 60 | 100 | 80 | 30 | 70 | 0 |
| Barnyardgrass | 100 | 100 | 100 | 100 | 100 | 70 | 100 | 100 | 30 | 40 | 0 |
| Nutsedge | 100 | 100 | 100 | 100 | 100 | 100 | 100 | 100 | 100 | 100 | 0 |
| Giant Foxtail | 100 | 100 | 80 | 60 | 60 | 30 | 30 | 30 | 0 | 0 | 0 |
| Wild Oats | 100 | 100 | 50 | 100 | 40 | 0 | 80 | 0 | 0 | 0 | 0 |
| Cocklebur | 100 | 100 | 90 | 100 | 100 | 70 | 90 | 60 | 60 | 30 | 0 |
| Morningglory | 100 | 100 | 100 | 100 | 100 | 100 | 100 | 100 | 80 | 70 | 0 |
| Teaweed | 100 | 100 | 60 | 90 | 80 | 30 | 60 | 50 | 0 | 30 | 0 |
| Sicklepod | 100 | 100 | 100 | 100 | 100 | 100 | 100 | 90 | 100 | 80 | 0 |
| Jimsonweed | 100 | 100 | 90 | 100 | 90 | 60 | 100 | 70 | 30 | 40 | 0 |
| Velvetleaf | 100 | 100 | 90 | 100 | 100 | 80 | 100 | 90 | 50 | 50 | 0 |

| PREEMERGENCE Rate g/ha | 250 | 62 | 62 | 16 | 16 | 16 | 4 | 4 | 1 | 1 |
|---|---|---|---|---|---|---|---|---|---|---|
| Corn | 100 | 100 | 100 | 70 | 70 | 100 | 20 | 40 | 0 | 0 |
| Wheat | 100 | 100 | 100 | 20 | 0 | 40 | 0 | 0 | 0 | 0 |
| Barley | - | - | - | - | - | - | - | - | - | - |
| Rice | 100 | 100 | 100 | 100 | 100 | 100 | 100 | 100 | 90 | 0 |
| Soybean | 100 | 100 | 100 | 70 | 70 | 90 | 40 | 40 | 0 | 0 |
| Cotton | 100 | 100 | 100 | 70 | 70 | 100 | 30 | 0 | 0 | 0 |
| Sugar beet | 100 | 100 | 100 | 100 | 100 | 100 | 100 | 90 | 80 | 0 |
| Rape | 100 | 100 | 100 | 90 | 90 | 100 | 90 | 80 | 20 | 0 |
| Crabgrass | 100 | 90 | 100 | 60 | 30 | 80 | 30 | 0 | 0 | 0 |
| Johnsongrass | 100 | 100 | 100 | 90 | 70 | 100 | 80 | 30 | 50 | 0 |
| Blackgrass | 100 | 100 | 100 | 100 | 90 | 100 | 100 | 70 | 50 | 0 |
| Barnyardgrass | 100 | 100 | 100 | 80 | 50 | 100 | 30 | 0 | 0 | 0 |
| Nutsedge | 100 | 100 | 100 | 100 | 90 | 100 | 90 | 50 | 50 | 0 |
| Giant Foxtail | 100 | 100 | 100 | 80 | 30 | 100 | 50 | 0 | 0 | 0 |
| Wild Oats | 100 | 100 | 100 | 70 | 90 | 80 | 0 | 40 | 0 | 0 |
| Cocklebur | 100 | 100 | 100 | 50 | 80 | 80 | 50 | 40 | 20 | 0 |
| Morningglory | 100 | 100 | 100 | 40 | 80 | 100 | 30 | 40 | 0 | 0 |
| Teaweed | 100 | 100 | 100 | 80 | 70 | 90 | 70 | 30 | 0 | 0 |
| Sicklepod | 100 | 90 | 100 | 90 | 50 | 90 | 80 | 30 | 20 | 0 |
| Jimsonweed | 100 | 100 | 100 | 100 | 80 | 100 | 80 | 30 | 50 | 0 |
| Velvetleaf | 100 | 100 | 100 | 100 | 80 | 100 | 90 | 30 | 60 | 0 |

Table B (continued)

| | Compound 4 | | | |
|---|---|---|---|---|
| POSTEMERGENCE Rate g/ha | 62 | 16 | 4 | 1 |
| Corn | 100 | 100 | 80 | 40 |
| Wheat | 100 | 100 | 40 | 0 |
| Barley | - | - | - | - |
| Rice | 100 | 100 | 60 | 30 |
| Soybean | 100 | 100 | 100 | 60 |
| Cotton | 100 | 90 | 70 | 30 |
| Sugar beet | 100 | 100 | 100 | 100 |
| Rape | 100 | 100 | 100 | 100 |
| Crabgrass | 100 | 80 | 50 | 30 |
| Johnsongrass | 100 | 100 | 70 | 30 |
| Blackgrass | 100 | 100 | 60 | 30 |
| Barnyardgrass | 100 | 100 | 60 | 30 |
| Nutsedge | 100 | 90 | 50 | 30 |
| Giant Foxtail | 100 | 90 | 60 | 30 |
| Wild Oats | 100 | 90 | 30 | 0 |
| Cocklebur | 100 | 90 | 50 | 30 |
| Morningglory | 100 | 100 | 80 | 50 |
| Teaweed | 100 | 80 | 60 | 40 |
| Sicklepod | 100 | 80 | 60 | 30 |
| Jimsonweed | 100 | 100 | 70 | 40 |
| Velvetleaf | 100 | 100 | 100 | 60 |
| PREEMERGENCE Rate g/ha | 250 | 62 | 16 | 4 |
| Corn | 100 | 100 | 90 | 60 |
| Wheat | 100 | 100 | 90 | 70 |
| Barley | - | - | - | - |
| Rice | 100 | 100 | 100 | 100 |
| Soybean | 100 | 100 | 90 | 60 |
| Cotton | 100 | 90 | 60 | 30 |
| Sugar beet | 100 | 100 | 100 | 90 |
| Rape | 100 | 100 | 100 | 50 |
| Crabgrass | 100 | 90 | 50 | 30 |
| Johnsongrass | 100 | 100 | 100 | 70 |
| Blackgrass | 100 | 100 | 100 | 90 |
| Barnyardgrass | 100 | 100 | 100 | 60 |
| Nutsedge | 100 | 90 | 80 | 60 |
| Giant Foxtail | 100 | 100 | 100 | 60 |
| Wild Oats | 100 | 90 | 70 | 70 |
| Cocklebur | 100 | 100 | 80 | 50 |
| Morningglory | 100 | 90 | 50 | 30 |
| Teaweed | 100 | 90 | 70 | 30 |
| Sicklepod | 100 | 90 | 60 | 30 |
| Jimsonweed | 100 | 90 | 80 | 50 |
| Velvetleaf | 100 | 100 | 80 | 50 |

Table B (continued)

| | Compound 5 | | | |
|---|---|---|---|---|
| POSTEMERGENCE | | | | |
| Rate g/ha | 62 | 16 | 4 | 1 |
| Corn | 100 | 90 | 60 | 40 |
| Wheat | 70 | 40 | 0 | 0 |
| Barley | - | - | - | - |
| Rice | 100 | 90 | 30 | 0 |
| Soybean | 90 | 90 | 60 | 40 |
| Cotton | 100 | 90 | 60 | 30 |
| Sugar beet | 100 | 100 | 100 | 90 |
| Rape | 100 | 100 | 100 | 100 |
| Crabgrass | 70 | 50 | 40 | 30 |
| Johnsongrass | 100 | 100 | 80 | 40 |
| Blackgrass | 100 | 100 | 60 | 30 |
| Barnyardgrass | 100 | 100 | 80 | 40 |
| Nutsedge | 100 | 100 | 80 | 70 |
| Giant Foxtail | 100 | 80 | 30 | 0 |
| Wild Oats | 70 | 40 | 0 | 0 |
| Cocklebur | 100 | 100 | 60 | 30 |
| Morningglory | 100 | 90 | 80 | 60 |
| Teaweed | 90 | 70 | 50 | 30 |
| Sicklepod | 70 | 60 | 40 | 30 |
| Jimsonweed | 100 | 90 | 60 | 30 |
| Velvetleaf | 100 | 100 | 60 | 30 |
| PREEMERGENCE | | | | |
| Rate g/ha | 250 | 62 | 16 | 4 |
| Corn | 100 | 100 | 90 | 30 |
| Wheat | 100 | 100 | 50 | 0 |
| Barley | - | - | - | - |
| Rice | 100 | 100 | 100 | 100 |
| Soybean | 100 | 90 | 80 | 60 |
| Cotton | 100 | 80 | 50 | 30 |
| Sugar beet | 100 | 100 | 100 | 80 |
| Rape | 100 | 100 | 80 | 60 |
| Crabgrass | 90 | 70 | 50 | 30 |
| Johnsongrass | 100 | 100 | 100 | 100 |
| Blackgrass | 100 | 100 | 100 | 90 |
| Barnyardgrass | 100 | 100 | 90 | 70 |
| Nutsedge | 100 | 100 | 100 | 90 |
| Giant Foxtail | 100 | 100 | 90 | 60 |
| Wild Oats | 90 | 80 | 50 | 30 |
| Cocklebur | 100 | 90 | 70 | 50 |
| Morningglory | 100 | 80 | 70 | 30 |
| Teaweed | 90 | 70 | 50 | 30 |
| Sicklepod | 90 | 70 | 50 | 50 |
| Jimsonweed | 100 | 90 | 60 | 30 |
| Velvetleaf | 100 | 90 | 60 | 30 |

Table B (continued)

| | Compound 8 | | | |
|---|---|---|---|---|
| POSTEMERGENCE | | | | |
| Rate g/ha | 62 | 16 | 4 | 1 |
| Corn | 100 | 80 | 50 | 30 |
| Wheat | 30 | 0 | 0 | 0 |
| Barley | - | - | - | - |
| Rice | 90 | 70 | 0 | 0 |
| Soybean | 100 | 80 | 70 | 0 |
| Cotton | 100 | 90 | 60 | 40 |
| Sugar beet | 100 | 100 | 100 | 90 |
| Rape | 100 | 100 | 100 | 90 |
| Crabgrass | 90 | 50 | 30 | 0 |
| Johnsongrass | 90 | 50 | 0 | 0 |
| Blackgrass | 100 | 90 | 30 | 0 |
| Barnyardgrass | 90 | 50 | 0 | 0 |
| Nutsedge | 100 | 70 | 70 | 40 |
| Giant Foxtail | 90 | 50 | 0 | 0 |
| Wild Oats | 30 | 0 | 0 | 0 |
| Cocklebur | 100 | 90 | 50 | 30 |
| Morningglory | 100 | 90 | 60 | 30 |
| Teaweed | 100 | 70 | 30 | 30 |
| Sicklepod | 90 | 50 | 30 | 0 |
| Jimsonweed | 100 | 100 | 60 | 30 |
| Velvetleaf | 100 | 100 | 70 | 50 |
| PREEMERGENCE | | | | |
| Rate g/ha | 250 | 62 | 16 | 4 |
| Corn | 100 | 90 | 30 | 0 |
| Wheat | 80 | 30 | 0 | 0 |
| Barley | - | - | - | - |
| Rice | 100 | 100 | 100 | 20 |
| Soybean | 100 | 80 | 40 | 0 |
| Cotton | 100 | 60 | 20 | 0 |
| Sugar beet | 100 | 100 | 100 | 50 |
| Rape | 100 | 100 | 90 | 40 |
| Crabgrass | 80 | 70 | 40 | 20 |
| Johnsongrass | 100 | 90 | 60 | 20 |
| Blackgrass | 100 | 90 | 70 | 30 |
| Barnyardgrass | 100 | 90 | 20 | 0 |
| Nutsedge | 100 | 100 | 80 | 60 |
| Giant Foxtail | 100 | 90 | - | 0 |
| Wild Oats | 100 | 90 | 80 | 0 |
| Cocklebur | 100 | 80 | 20 | 0 |
| Morningglory | 100 | 60 | 20 | 0 |
| Teaweed | 100 | 90 | 90 | 20 |
| Sicklepod | 100 | 90 | 30 | 0 |
| Jimsonweed | 100 | 100 | 80 | 30 |
| Velvetleaf | 100 | 100 | 60 | 20 |

Table B (continued)

| | Compound 13 | | | |
|---|---|---|---|---|
| POSTEMERGENCE | | | | |
| Rate g/ha | 62 | 16 | 4 | 1 |
| Corn | 100 | 100 | 70 | 40 |
| Wheat | 30 | 0 | 0 | 0 |
| Barley | - | - | - | - |
| Rice | 90 | 30 | 0 | 0 |
| Soybean | 100 | 100 | 100 | 70 |
| Cotton | 100 | 100 | 90 | 50 |
| Sugar beet | 100 | 100 | 100 | 100 |
| Rape | 100 | 100 | 100 | 100 |
| Crabgrass | 90 | 60 | 50 | 30 |
| Johnsongrass | 100 | 90 | 60 | 30 |
| Blackgrass | 100 | 70 | 40 | 0 |
| Barnyardgrass | 100 | 90 | 60 | 30 |
| Nutsedge | 100 | 100 | 90 | 80 |
| Giant Foxtail | 100 | 80 | 30 | 0 |
| Wild Oats | 60 | 30 | 0 | 0 |
| Cocklebur | 100 | 70 | 50 | 30 |
| Morningglory | 100 | 100 | 100 | 50 |
| Teaweed | 100 | 90 | 60 | 40 |
| Sicklepod | 100 | 100 | 80 | 40 |
| Jimsonweed | 100 | 100 | 60 | 30 |
| Velvetleaf | 100 | 100 | 100 | 70 |
| PREEMERGENCE | | | | |
| Rate g/ha | 250 | 62 | 16 | 4 |
| Corn | 100 | 90 | 90 | 20 |
| Wheat | 30 | 0 | 0 | 0 |
| Barley | - | - | - | - |
| Rice | 100 | 100 | 100 | 70 |
| Soybean | 100 | 100 | 90 | 70 |
| Cotton | 100 | 80 | 80 | 20 |
| Sugar beet | 100 | 100 | 100 | 90 |
| Rape | 100 | 100 | 90 | 90 |
| Crabgrass | 90 | 70 | 50 | 0 |
| Johnsongrass | 100 | 100 | 100 | 60 |
| Blackgrass | 100 | 90 | 80 | 50 |
| Barnyardgrass | 100 | 100 | 90 | 60 |
| Nutsedge | 100 | 100 | 100 | 90 |
| Giant Foxtail | 100 | 90 | 70 | 50 |
| Wild Oats | - | - | - | - |
| Cocklebur | 100 | 70 | 80 | 20 |
| Morningglory | 100 | 90 | 80 | 30 |
| Teaweed | 100 | 90 | 90 | 20 |
| Sicklepod | 100 | 100 | 80 | 20 |
| Jimsonweed | 100 | 100 | 90 | 50 |
| Velvetleaf | 100 | 100 | 90 | 80 |

Table B (continued)

| | Compound 14 | | | |
|---|---|---|---|---|
| POSTEMERGENCE | | | | |
| Rate g/ha | 16 | 4 | 1 | .25 |
| Corn | 100 | 80 | 60 | 40 |
| Wheat | 30 | 0 | 0 | 0 |
| Barley | - | - | - | - |
| Rice | 60 | 30 | 0 | 0 |
| Soybean | 100 | 100 | 70 | 60 |
| Cotton | 100 | 70 | 50 | 30 |
| Sugar beet | 100 | 100 | 90 | 70 |
| Rape | 100 | 100 | 90 | 60 |
| Crabgrass | 30 | 0 | 0 | 0 |
| Johnsongrass | 100 | 80 | 60 | 30 |
| Blackgrass | 70 | 30 | 0 | 0 |
| Barnyardgrass | 100 | 80 | 50 | 30 |
| Nutsedge | 90 | 70 | 50 | 30 |
| Giant Foxtail | 70 | 30 | 0 | 0 |
| Wild Oats | 40 | 0 | 0 | 0 |
| Cocklebur | 100 | 90 | 60 | 30 |
| Morningglory | 90 | 70 | 50 | 30 |
| Teaweed | 90 | 50 | 30 | 0 |
| Sicklepod | 80 | 50 | 0 | 0 |
| Jimsonweed | 90 | 90 | 50 | 30 |
| Velvetleaf | 100 | 90 | 50 | 30 |
| PREEMERGENCE | | | | |
| Rate g/ha | 62 | 16 | 4 | 1 |
| Corn | 90 | 70 | 50 | 30 |
| Wheat | 80 | 40 | 0 | 0 |
| Barley | - | - | - | - |
| Rice | 100 | 100 | 70 | 40 |
| Soybean | 100 | 90 | 70 | 60 |
| Cotton | 80 | 70 | 50 | 30 |
| Sugar beet | 100 | 100 | 100 | 80 |
| Rape | 100 | 100 | 60 | 60 |
| Crabgrass | 90 | 70 | 50 | 30 |
| Johnsongrass | 100 | 80 | 50 | 30 |
| Blackgrass | 100 | 90 | 60 | 30 |
| Barnyardgrass | 100 | 70 | 50 | 0 |
| Nutsedge | 100 | 100 | 90 | 80 |
| Giant Foxtail | 100 | 60 | 40 | 0 |
| Wild Oats | 80 | 40 | 0 | 0 |
| Cocklebur | 100 | 90 | 70 | 50 |
| Morningglory | 90 | 70 | 50 | 30 |
| Teaweed | 90 | 80 | 50 | 30 |
| Sicklepod | 90 | 70 | 50 | 30 |
| Jimsonweed | 90 | 70 | 50 | 30 |
| Velvetleaf | 90 | 70 | 50 | 30 |

Table B (continu d)

| | Compound 16 | | | |
|---|---|---|---|---|
| POSTEMERGENCE Rate g/ha | 62 | 16 | 4 | 1 |
| Corn | 0 | 0 | 0 | 0 |
| Wheat | 0 | 0 | 0 | 0 |
| Barley | - | - | - | - |
| Rice | 60 | 0 | 0 | 0 |
| Soybean | 100 | 70 | 40 | 0 |
| Cotton | 90 | 70 | 50 | 30 |
| Sugar beet | 100 | 100 | 90 | 70 |
| Rape | 100 | 100 | 90 | 60 |
| Crabgrass | 30 | 0 | 0 | 0 |
| Johnsongrass | 90 | 70 | 30 | 0 |
| Blackgrass | 70 | 30 | 0 | 0 |
| Barnyardgrass | 90 | 50 | 0 | 0 |
| Nutsedge | 90 | 70 | 60 | 40 |
| Giant Foxtail | 0 | 0 | 0 | 0 |
| Wild Oats | 0 | 0 | 0 | 0 |
| Cocklebur | 90 | 80 | 50 | 30 |
| Morningglory | 90 | 80 | 60 | 30 |
| Teaweed | 70 | 30 | 30 | 0 |
| Sicklepod | 50 | 30 | 0 | 0 |
| Jimsonweed | 100 | 80 | 50 | 30 |
| Velvetleaf | 100 | 80 | 30 | 0 |
| PREEMERGENCE Rate g/ha | 250 | 62 | 16 | 4 |
| Corn | 80 | 50 | 30 | 0 |
| Wheat | 90 | 40 | 0 | 0 |
| Barley | - | - | - | - |
| Rice | 100 | 100 | 90 | 60 |
| Soybean | 100 | 80 | 60 | 30 |
| Cotton | 90 | 70 | 50 | 30 |
| Sugar beet | 100 | 100 | 100 | 100 |
| Rape | 100 | 90 | 60 | 0 |
| Crabgrass | 90 | 70 | 50 | 40 |
| Johnsongrass | 100 | 80 | 50 | 30 |
| Blackgrass | 100 | 90 | 60 | 30 |
| Barnyardgrass | 100 | 80 | 40 | 0 |
| Nutsedge | 100 | 100 | 100 | 90 |
| Giant Foxtail | 80 | 60 | 30 | 0 |
| Wild Oats | 90 | 40 | 0 | 0 |
| Cocklebur | 100 | 80 | 70 | 30 |
| Morningglory | 100 | 80 | 50 | 30 |
| Teaweed | 90 | 80 | 50 | 30 |
| Sicklepod | 90 | 70 | 50 | 30 |
| Jimsonweed | 90 | 70 | 50 | 30 |
| Velvetleaf | 90 | 70 | 50 | 30 |

Table B (continued)

| | Compound 20 | | |
|---|---|---|---|
| POSTEMERGENCE | | | |
| Rate g/ha | 62 | 16 | 4 |
| Corn | 100 | 90 | 40 |
| Wheat | 90 | 60 | 20 |
| Barley | - | - | - |
| Rice | 95 | 90 | 85 |
| Soybean | 95 | 90 | 80 |
| Cotton | 100 | 100 | 90 |
| Sugar beet | 95 | 90 | 90 |
| Rape | 100 | 100 | 100 |
| Crabgrass | 60 | 30 | 20 |
| Johnsongrass | 100 | 100 | 85 |
| Blackgrass | 100 | 95 | 85 |
| Barnyardgrass | 100 | 95 | 80 |
| Nutsedge | 100 | 80 | 60 |
| Giant Foxtail | 90 | 85 | 60 |
| Wild Oats | 85 | 70 | 30 |
| Cocklebur | 100 | 95 | 85 |
| Morningglory | 100 | 100 | 90 |
| Teaweed | 90 | 95 | 90 |
| Sicklepod | 70 | 25 | 30 |
| Jimsonweed | 95 | 90 | 30 |
| Velvetleaf | 100 | 100 | 90 |
| PREEMERGENCE | | | |
| Rate g/ha | 250 | 62 | 16 |
| Corn | 100 | 95 | 90 |
| Wheat | 95 | 95 | 80 |
| Barley | - | - | - |
| Rice | 100 | 100 | 100 |
| Soybean | 95 | 95 | 85 |
| Cotton | 95 | 90 | 80 |
| Sugar beet | 100 | 95 | 90 |
| Rape | 100 | 95 | 80 |
| Crabgrass | 90 | 30 | 10 |
| Johnsongrass | 95 | 95 | 90 |
| Blackgrass | 100 | 95 | 80 |
| Barnyardgrass | 100 | 95 | 90 |
| Nutsedge | 100 | 100 | 100 |
| Giant Foxtail | 100 | 95 | 90 |
| Wild Oats | 90 | 85 | 65 |
| Cocklebur | 95 | 90 | 90 |
| Morningglory | 95 | 90 | 80 |
| Teaweed | 90 | 80 | 50 |
| Sicklepod | 90 | 50 | 10 |
| Jimsonweed | 100 | 90 | 60 |
| Velvetleaf | 100 | 95 | 70 |

Table B (continued)

Compound 21

| | POSTEMERGENCE | | | PREEMERGENCE | | |
|---|---|---|---|---|---|---|
| Rate g/ha | 62 | 16 | 4 | 250 | 62 | 16 |
| Corn | 100 | 90 | 70 | 90 | 60 | 20 |
| Wheat | 0 | 0 | 0 | 40 | 0 | 0 |
| Barley | 0 | 0 | 0 | 0 | 0 | 0 |
| Rice | 80 | 20 | 0 | - | - | - |
| Soybean | 100 | 90 | 50 | 80 | 80 | 30 |
| Cotton | 60 | 0 | 0 | 90 | 20 | 0 |
| Sugar beet | 90 | 80 | 0 | 90 | 70 | 50 |
| Rape | 100 | 90 | 30 | 80 | 60 | 0 |
| Crabgrass | 40 | 0 | 0 | 90 | 0 | 0 |
| Johnsongrass | 100 | 70 | 20 | 100 | 80 | 0 |
| Blackgrass | 80 | 60 | 0 | 100 | 50 | 0 |
| Barnyardgrass | 100 | 70 | 30 | 100 | 50 | 0 |
| Nutsedge | 0 | 0 | 0 | 0 | 0 | 0 |
| Giant Foxtail | 100 | 60 | 20 | 90 | 70 | 0 |
| Green Foxtail | 70 | 20 | 0 | 90 | 90 | 30 |
| Cheatgrass | 0 | 0 | 0 | 50 | 0 | 0 |
| Wild Oats | 0 | 0 | 0 | 60 | 20 | 0 |
| Wild Buckwheat | 100 | 80 | 0 | 100 | 50 | 50 |
| Viola | 40 | 20 | 0 | 100 | - | 20 |
| Lambsquarter | 90 | 50 | 0 | 90 | 70 | 0 |
| Cocklebur | 60 | 0 | 0 | 30 | 20 | 0 |
| Morningglory | 50 | 0 | 50 | 70 | 20 | 0 |
| Teaweed | 40 | 20 | 0 | 80 | 30 | 0 |
| Sicklepod | 50 | 0 | 0 | 50 | 0 | 0 |
| Jimsonweed | 80 | 70 | 20 | 60 | 0 | 0 |
| Velvetleaf | 30 | 0 | 0 | 30 | 0 | 0 |

Table B (continued)

Compound 22

| | POSTEMERGENCE | | | PREEMERGENCE | | |
|---|---|---|---|---|---|---|
| Rate g/ha | 62 | 16 | 4 | 250 | 62 | 16 |
| Corn | 100 | 100 | 70 | 90 | 80 | 0 |
| Wheat | 70 | 20 | 0 | 50 | 0 | 0 |
| Barley | 0 | 0 | 0 | 60 | 20 | 0 |
| Rice | 90 | 50 | 20 | - | - | - |
| Soybean | 100 | 100 | 80 | 90 | 80 | 30 |
| Cotton | 80 | 70 | 20 | 90 | 80 | 20 |
| Sugar beet | 100 | 90 | 70 | 80 | 70 | 80 |
| Rape | 100 | 100 | 70 | 90 | 80 | 70 |
| Crabgrass | 60 | 30 | 0 | 70 | 70 | 20 |
| Johnsongrass | 100 | 100 | 70 | 100 | 90 | 80 |
| Blackgrass | 100 | 70 | 50 | 90 | 70 | 70 |
| Barnyardgrass | 100 | 100 | 50 | 100 | 90 | 50 |
| Nutsedge | 0 | 0 | 0 | 0 | 0 | 0 |
| Giant Foxtail | 100 | 100 | 50 | 100 | 90 | 70 |
| Green Foxtail | 100 | 80 | 50 | 100 | 90 | 50 |
| Cheatgrass | 0 | 0 | 0 | 60 | 0 | 0 |
| Wild Oats | 60 | 20 | 0 | 50 | 50 | 20 |
| Wild Buckwheat | 80 | 80 | 50 | 90 | 60 | 20 |
| Viola | 80 | - | 20 | 90 | 50 | 30 |
| Lambsquarter | 80 | 60 | 50 | 90 | 60 | 0 |
| Cocklebur | 100 | 60 | 20 | - | 30 | 0 |
| Morningglory | 70 | 60 | 20 | 80 | 60 | 20 |
| Teaweed | 70 | 50 | 20 | 70 | 40 | 0 |
| Sicklepod | 80 | 50 | 20 | 70 | 30 | 0 |
| Jimsonweed | 90 | 70 | 50 | 70 | 30 | 0 |
| Velvetleaf | 70 | 30 | 20 | 60 | 30 | 0 |

Table B (continued)

| | Compound 23 | | | |
|---|---|---|---|---|
| POSTEMERGENCE | | | | |
| Rate g/ha | 62 | 16 | 4 | 1 |
| Corn | 50 | 30 | 0 | 0 |
| Wheat | 40 | 0 | 0 | 0 |
| Barley | - | - | - | - |
| Rice | 70 | 40 | 0 | 0 |
| Soybean | 100 | 100 | 80 | 60 |
| Cotton | 100 | 100 | 70 | 40 |
| Sugar beet | 100 | 100 | 100 | 100 |
| Rape | 100 | 100 | 100 | 100 |
| Crabgrass | 60 | 40 | 30 | 0 |
| Johnsongrass | 100 | 100 | 80 | 60 |
| Blackgrass | 100 | 70 | 30 | 0 |
| Barnyardgrass | 100 | 100 | 80 | 60 |
| Nutsedge | 100 | 100 | 70 | 50 |
| Giant Foxtail | 70 | 50 | 30 | 0 |
| Wild Oats | 40 | 0 | 0 | 0 |
| Cocklebur | 100 | 100 | 100 | 100 |
| Morningglory | 100 | 80 | 60 | 30 |
| Teaweed | 90 | 70 | 50 | 30 |
| Sicklepod | 100 | 70 | 50 | 30 |
| Jimsonweed | 100 | 100 | 70 | 50 |
| Velvetleaf | 100 | 100 | 100 | 90 |
| PREEMERGENCE | | | | |
| Rate g/ha | 250 | 62 | 16 | 4 |
| Corn | 70 | 50 | 30 | 0 |
| Wheat | 80 | 60 | 40 | 0 |
| Barley | - | - | - | - |
| Rice | 100 | 100 | 80 | 50 |
| Soybean | 100 | 80 | 60 | 40 |
| Cotton | 90 | 70 | 40 | 0 |
| Sugar beet | 100 | 100 | 100 | 80 |
| Rape | 100 | 90 | 70 | 50 |
| Crabgrass | 90 | 70 | 50 | 30 |
| Johnsongrass | 100 | 90 | 80 | 60 |
| Blackgrass | 100 | 100 | 80 | 50 |
| Barnyardgrass | 100 | 80 | 60 | 40 |
| Nutsedge | 100 | 100 | 80 | 60 |
| Giant Foxtail | 100 | 100 | 90 | 70 |
| Wild Oats | 70 | 50 | 30 | 0 |
| Cocklebur | 90 | 80 | 70 | 50 |
| Morningglory | 70 | 50 | 30 | 0 |
| Teaweed | 90 | 70 | 50 | - |
| Sicklepod | 90 | 70 | 40 | 0 |
| Jimsonweed | 100 | 80 | 60 | 40 |
| Velvetleaf | 100 | 80 | 60 | 40 |

Table B (continued)

| | Compound 24 | | | |
|---|---|---|---|---|
| POSTEMERGENCE | | | | |
| te g/ha | 62 | 16 | 4 | 1 |
| Corn | 0 | 0 | 0 | 0 |
| Wheat | 30 | 0 | 0 | 0 |
| Barley | - | - | - | - |
| Rice | 30 | 0 | 0 | 0 |
| Soybean | 100 | 100 | 80 | 60 |
| Cotton | 100 | 90 | 70 | 40 |
| Sugar beet | 100 | 100 | 100 | 90 |
| Rape | 100 | 100 | 100 | 100 |
| Crabgrass | 30 | 0 | 0 | 0 |
| Johnsongrass | 100 | 70 | 50 | 30 |
| Blackgrass | 100 | 100 | 70 | 40 |
| Barnyardgrass | 80 | 60 | 30 | 0 |
| Nutsedge | 100 | 90 | 70 | 50 |
| Giant Foxtail | 70 | 30 | 0 | 0 |
| Wild Oats | 30 | 0 | 0 | 0 |
| Cocklebur | 100 | 100 | 100 | 90 |
| Morningglory | 100 | 90 | 70 | 50 |
| Teaweed | 70 | 50 | 30 | 0 |
| Sicklepod | 70 | 50 | 30 | 0 |
| Jimsonweed | 100 | 70 | 50 | 30 |
| Velvetleaf | 100 | 100 | 70 | 50 |
| PREEMERGENCE | | | | |
| Rate g/ha | 250 | 62 | 16 | 4 |
| Corn | 30 | 0 | 0 | 0 |
| Wheat | 70 | 30 | 0 | 0 |
| Barley | - | - | - | - |
| Rice | 90 | 70 | 50 | 30 |
| Soybean | 90 | 70 | 50 | 30 |
| Cotton | 70 | 50 | 30 | 0 |
| Sugar beet | 100 | 100 | 90 | 80 |
| Rape | 100 | 90 | 70 | 50 |
| Crabgrass | 90 | 60 | 30 | 0 |
| Johnsongrass | 90 | 80 | 70 | 50 |
| Blackgrass | 90 | 80 | 70 | 40 |
| Barnyardgrass | 90 | 80 | 60 | 30 |
| Nutsedge | 100 | 80 | 60 | 40 |
| Giant Foxtail | 100 | 90 | 70 | 40 |
| Wild Oats | 70 | 40 | 30 | 0 |
| Cocklebur | 90 | 70 | 50 | 30 |
| Morningglory | 80 | 60 | 40 | 20 |
| Teaweed | 90 | 70 | 50 | 30 |
| Sicklepod | 90 | 70 | 40 | 0 |
| Jimsonweed | 90 | 70 | 50 | 30 |
| Velvetleaf | 90 | 70 | 50 | 30 |

Table B (contin d)

| | Compound 27 | | | |
|---|---|---|---|---|
| POSTEMERGENCE Rate g/ha | 250 | 62 | 16 | 4 |
| Corn | 0 | 0 | 0 | 0 |
| Wheat | 40 | 20 | 0 | 0 |
| Barley | - | - | - | - |
| Rice | 90 | 70 | 50 | 30 |
| Soybean | 90 | 80 | 70 | 50 |
| Cotton | 100 | 90 | 60 | 30 |
| Sugar beet | 100 | 100 | 100 | 100 |
| Rape | 100 | 100 | 100 | 100 |
| Crabgrass | 70 | 50 | 30 | 0 |
| Johnsongrass | 100 | 90 | 70 | 50 |
| Blackgrass | 90 | 60 | 30 | 0 |
| Barnyardgrass | 100 | 90 | 70 | 50 |
| Nutsedge | 100 | 100 | 70 | 50 |
| Giant Foxtail | 70 | 50 | 30 | 0 |
| Wild Oats | 60 | 40 | 20 | 0 |
| Cocklebur | 100 | 90 | 70 | 50 |
| Morningglory | 90 | 80 | 70 | 50 |
| Teaweed | 80 | 60 | 40 | 20 |
| Sicklepod | 80 | 60 | 30 | 0 |
| Jimsonweed | 100 | 70 | 50 | 30 |
| Velvetleaf | 100 | 100 | 80 | 50 |
| PREEMERGENCE Rate g/ha | 250 | 62 | 16 | 4 |
| Corn | 0 | 0 | 0 | 0 |
| Wheat | 70 | 50 | 30 | 0 |
| Barley | - | - | - | - |
| Rice | 100 | 90 | 70 | 50 |
| Soybean | 70 | 50 | 30 | 0 |
| Cotton | 50 | 30 | 0 | 0 |
| Sugar beet | 100 | 90 | 70 | 50 |
| Rape | 90 | 70 | 50 | 30 |
| Crabgrass | 80 | 70 | 60 | 30 |
| Johnsongrass | 90 | 80 | 70 | 40 |
| Blackgrass | 90 | 70 | 50 | 30 |
| Barnyardgrass | 90 | 60 | 30 | 0 |
| Nutsedge | 90 | 70 | 50 | 30 |
| Giant Foxtail | 90 | 60 | 40 | 0 |
| Wild Oats | 80 | 60 | 30 | 0 |
| Cocklebur | 80 | 60 | 40 | 0 |
| Morningglory | 30 | 0 | 0 | 0 |
| Teaweed | 90 | 70 | 50 | 30 |
| Sicklepod | 90 | 70 | 40 | 0 |
| Jimsonweed | 90 | 70 | 50 | 30 |
| Velvetleaf | 90 | 70 | 50 | 30 |

Table B (continued)

Compound 28

| POSTEMERGENCE | | | |
|---|---|---|---|
| Rate g/ha | 250 | 62 | 16 |
| Corn | 0 | 0 | 0 |
| Wheat | 30 | 0 | 0 |
| Rice | 50 | 30 | 0 |
| Soybean | 80 | 60 | 40 |
| Cotton | 100 | 70 | 30 |
| Sugar beet | 100 | 100 | 100 |
| Rape | 100 | 100 | 100 |
| Crabgrass | 50 | 30 | 0 |
| Johnsongrass | 90 | 70 | 50 |
| Blackgrass | 100 | 90 | 70 |
| Barnyardgrass | 90 | 60 | 30 |
| Nutsedge | 70 | 50 | 30 |
| Giant Foxtail | 80 | 60 | 30 |
| Wild Oats | 30 | 0 | 0 |
| Cocklebur | 90 | 70 | 50 |
| Morningglory | 100 | 100 | 70 |
| Teaweed | 80 | 60 | 40 |
| Sicklepod | 50 | 30 | 0 |
| Jimsonweed | 100 | 80 | 60 |
| Velvetleaf | 100 | 80 | 50 |

| PREEMERGENCE | | | | |
|---|---|---|---|---|
| Rate g/ha | 250 | 62 | 16 | 4 |
| Corn | 0 | 0 | 0 | 0 |
| Wheat | 70 | 30 | 0 | 0 |
| Barley | 70 | 30 | 0 | 0 |
| Rice | 100 | 90 | 60 | 30 |
| Soybean | 90 | 60 | 30 | 0 |
| Cotton | 70 | 30 | 0 | 0 |
| Sugar beet | 90 | 70 | 50 | 30 |
| Rape | 90 | 70 | 50 | 30 |
| Crabgrass | 70 | 50 | 30 | 0 |
| Johnsongrass | 100 | 90 | 70 | 50 |
| Blackgrass | 90 | 70 | 50 | 30 |
| Barnyardgrass | 90 | 70 | 50 | 30 |
| Nutsedge | 100 | 70 | 50 | 30 |
| Giant Foxtail | 90 | 70 | 50 | 30 |
| Green Foxtail | 90 | 70 | 50 | 30 |
| Cheatgrass | 60 | 30 | 0 | 0 |
| Wild Oats | 30 | 0 | 0 | 0 |
| Wild Buckwheat | 90 | 70 | 50 | 30 |
| Viola | 90 | 70 | 50 | 30 |
| Lambsquarter | 100 | 70 | 50 | 30 |
| Cocklebur | 90 | 70 | 50 | 30 |
| Morningglory | 90 | 60 | 30 | 0 |
| Sicklepod | 40 | 0 | 0 | 0 |
| Jimsonweed | 90 | 70 | 50 | 30 |
| Velvetleaf | 90 | 70 | 50 | 30 |

Table B (continued)

| | Compound 31 | | | |
|---|---|---|---|---|
| POSTEMERGENCE | | | | |
| Rate g/ha | 62 | 16 | 4 | 1 |
| Corn | 100 | 70 | 50 | 30 |
| Wheat | 0 | 0 | 0 | 0 |
| Barley | - | - | - | - |
| Rice | 70 | 30 | 0 | 0 |
| Soybean | 100 | 90 | 70 | 40 |
| Cotton | 80 | 60 | 30 | 0 |
| Sugar beet | 100 | 100 | 60 | 30 |
| Rape | 100 | 100 | 100 | 100 |
| Crabgrass | 90 | 70 | 50 | 30 |
| Johnsongrass | 100 | 100 | 60 | 30 |
| Blackgrass | 100 | 100 | 60 | 30 |
| Barnyardgrass | 100 | 100 | 70 | 30 |
| Nutsedge | 30 | 0 | 0 | 0 |
| Giant Foxtail | 100 | 100 | 70 | 40 |
| Wild Oats | 70 | 40 | 20 | 0 |
| Cocklebur | 90 | 70 | 50 | 30 |
| Morningglory | 90 | 70 | 50 | 30 |
| Teaweed | 80 | 60 | 30 | 0 |
| Sicklepod | 80 | 60 | 30 | 0 |
| Jimsonweed | 100 | 80 | 60 | 40 |
| Velvetleaf | 100 | 80 | 60 | 40 |
| PREEMERGENCE | | | | |
| Rate g/ha | 250 | 62 | 16 | 4 |
| Corn | 90 | 60 | 0 | 0 |
| Wheat | 60 | 30 | 0 | 0 |
| Barley | - | - | - | - |
| Rice | 100 | 100 | 90 | 70 |
| Soybean | 90 | 70 | 50 | 30 |
| Cotton | 80 | 40 | 0 | 0 |
| Sugar beet | 100 | 90 | 70 | 50 |
| Rape | 90 | 70 | 50 | 20 |
| Crabgrass | 90 | 60 | 30 | 0 |
| Johnsongrass | 100 | 80 | 60 | 40 |
| Blackgrass | 100 | 90 | 70 | 50 |
| Barnyardgrass | 90 | 80 | 60 | 30 |
| Nutsedge | 80 | 60 | 40 | 20 |
| Giant Foxtail | 100 | 80 | 60 | 30 |
| Wild Oats | 60 | 30 | 0 | 0 |
| Cocklebur | 90 | 70 | 50 | 30 |
| Morningglory | 80 | 60 | 40 | 20 |
| Teaweed | 70 | 50 | 30 | 0 |
| Sicklepod | 80 | 60 | 40 | 0 |
| Jimsonweed | 90 | 70 | 50 | 30 |
| Velvetleaf | 90 | 70 | 50 | 30 |

Table B (continued)

| | Compound 32 | | | |
|---|---|---|---|---|
| POSTEMERGENCE Rate g/ha | 62 | 16 | 4 | 1 |
| Corn | 80 | 40 | 0 | 0 |
| Wheat | 80 | 60 | 40 | 20 |
| Barley | - | - | - | - |
| Rice | 100 | 70 | 50 | 30 |
| Soybean | 100 | 100 | 90 | 70 |
| Cotton | 100 | 100 | 100 | 80 |
| Sugar beet | 100 | 100 | 70 | 50 |
| Rape | 100 | 100 | 100 | 100 |
| Crabgrass | 90 | 70 | 50 | 30 |
| Johnsongrass | 100 | 100 | 100 | 80 |
| Blackgrass | 100 | 100 | 90 | 70 |
| Barnyardgrass | 100 | 100 | 100 | 90 |
| Nutsedge | 100 | 100 | 100 | 90 |
| Giant Foxtail | 100 | 90 | 70 | 50 |
| Wild Oats | 80 | 60 | 40 | 20 |
| Cocklebur | 100 | 100 | 100 | 70 |
| Morningglory | 100 | 100 | 90 | 70 |
| Teaweed | 100 | 80 | 60 | 30 |
| Sicklepod | 100 | 100 | 90 | 80 |
| Jimsonweed | 100 | 90 | 80 | 70 |
| Velvetleaf | 100 | 100 | 100 | 90 |
| PREEMERGENCE Rate g/ha | 250 | 62 | 16 | 4 |
| Corn | 90 | 80 | 70 | 40 |
| Wheat | 80 | 60 | 40 | 20 |
| Barley | - | - | - | - |
| Rice | 100 | 100 | 100 | 100 |
| Soybean | 100 | 80 | 60 | 40 |
| Cotton | 90 | 70 | 50 | 30 |
| Sugar beet | 100 | 100 | 90 | 70 |
| Rape | 100 | 90 | 70 | 50 |
| Crabgrass | 90 | 80 | 70 | 60 |
| Johnsongrass | 100 | 100 | 90 | 70 |
| Blackgrass | 90 | 80 | 70 | 50 |
| Barnyardgrass | 100 | 90 | 70 | 40 |
| Nutsedge | 100 | 90 | 80 | 70 |
| Giant Foxtail | 100 | 100 | 80 | 60 |
| Wild Oats | 90 | 70 | 50 | 30 |
| Cocklebur | 90 | 70 | 50 | 30 |
| Morningglory | 100 | 90 | 60 | 30 |
| Teaweed | 100 | 80 | 60 | 40 |
| Sicklepod | 100 | 80 | 40 | 0 |
| Jimsonweed | 90 | 80 | 70 | 50 |
| Velvetleaf | 90 | 80 | 70 | 50 |

Table B (contin' d)

| | Compound 33 | | | |
|---|---|---|---|---|
| POSTEMERGENCE | | | | |
| Rate g/ha | 62 | 16 | 4 | |
| Corn | 80 | 40 | 30 | |
| Wheat | 90 | 70 | 30 | |
| Barley | - | - | - | |
| Rice | 80 | 70 | 30 | |
| Soybean | 90 | 90 | 70 | |
| Cotton | 90 | 90 | 50 | |
| Sugar beet | 100 | 100 | 80 | |
| Rape | 100 | 100 | 100 | |
| Crabgrass | 60 | 30 | 0 | |
| Johnsongrass | 100 | 100 | 60 | |
| Blackgrass | 90 | 70 | 30 | |
| Barnyardgrass | 100 | 70 | 50 | |
| Nutsedge | 100 | 60 | 50 | |
| Giant Foxtail | 90 | 70 | 50 | |
| Wild Oats | 80 | 40 | 30 | |
| Cocklebur | 100 | 80 | 60 | |
| Morningglory | 100 | 100 | 50 | |
| Teaweed | 80 | 50 | 30 | |
| Sicklepod | 90 | 50 | 30 | |
| Jimsonweed | 90 | 80 | 60 | |
| Velvetleaf | 100 | 100 | 90 | |
| PREEMERGENCE | | | | |
| Rate g/ha | 250 | 62 | 16 | 4 |
| Corn | 40 | 0 | 0 | 0 |
| Wheat | 90 | 40 | 0 | 0 |
| Barley | - | - | - | - |
| Rice | 100 | 100 | 30 | 0 |
| Soybean | 90 | 60 | 30 | 0 |
| Cotton | 80 | 60 | 30 | 0 |
| Sugar beet | 100 | 80 | 40 | 0 |
| Rape | 90 | 70 | 50 | 30 |
| Crabgrass | 70 | 50 | 30 | 0 |
| Johnsongrass | 100 | 90 | 70 | 50 |
| Blackgrass | 90 | 70 | 30 | 0 |
| Barnyardgrass | 100 | 70 | 30 | 0 |
| Nutsedge | 90 | 70 | 50 | 30 |
| Giant Foxtail | 100 | 80 | 50 | 30 |
| Wild Oats | 80 | 30 | 0 | 0 |
| Cocklebur | 90 | 60 | 30 | 0 |
| Morningglory | 90 | 60 | 30 | 0 |
| Teaweed | 60 | 30 | 0 | 0 |
| Sicklepod | 90 | 60 | 30 | 0 |
| Jimsonweed | 90 | 70 | 50 | 30 |
| Velvetleaf | 80 | 60 | 40 | 20 |

Table B (continued)

Compound 34

| POSTEMERGENCE g/ha | 62 | 16 | 4 | 1 |
|---|---|---|---|---|
| Corn | 70 | 30 | 0 | 0 |
| Wheat | 60 | 30 | 0 | 0 |
| Barley | - | - | - | - |
| Rice | 90 | 70 | 50 | 30 |
| Soybean | 90 | 90 | 80 | 60 |
| Cotton | 90 | 80 | 50 | 30 |
| Sugar beet | 100 | 100 | 70 | 50 |
| Rape | 100 | 100 | 100 | 100 |
| Crabgrass | 80 | 50 | 30 | 0 |
| Johnsongrass | 100 | 90 | 70 | 30 |
| Blackgrass | 100 | 70 | 30 | 0 |
| Barnyardgrass | 100 | 70 | 50 | 0 |
| Nutsedge | 90 | 70 | 50 | 30 |
| Giant Foxtail | 90 | 70 | 60 | 0 |
| Wild Oats | 70 | 50 | 30 | 0 |
| Cocklebur | 90 | 70 | 30 | 0 |
| Morningglory | 90 | 80 | 60 | 40 |
| Teaweed | 80 | 50 | 30 | 0 |
| Sicklepod | 90 | 50 | 30 | 0 |
| Jimsonweed | 100 | 80 | 70 | 50 |
| Velvetleaf | 100 | 100 | 80 | 60 |

| PREEMERGENCE Rate g/ha | 250 | 62 | 16 | 4 |
|---|---|---|---|---|
| Corn | 60 | 0 | 0 | 0 |
| Wheat | 70 | 30 | 0 | 0 |
| Barley | - | - | - | - |
| Rice | 100 | 100 | 40 | 0 |
| Soybean | 90 | 50 | 0 | 0 |
| Cotton | 60 | 30 | 0 | 0 |
| Sugar beet | 100 | 90 | 70 | 50 |
| Rape | 90 | 70 | 40 | 0 |
| Crabgrass | 70 | 50 | 40 | 30 |
| Johnsongrass | 90 | 70 | 40 | 0 |
| Blackgrass | 100 | 80 | 40 | 0 |
| Barnyardgrass | 90 | 40 | 0 | 0 |
| Nutsedge | 90 | 70 | 50 | 30 |
| Giant Foxtail | 90 | 50 | 0 | 0 |
| Wild Oats | 70 | 30 | 0 | 0 |
| Cocklebur | 90 | 50 | 30 | 0 |
| Morningglory | 80 | 50 | 30 | 0 |
| Teaweed | 80 | 50 | 30 | 0 |
| Sicklepod | 70 | 30 | 0 | 0 |
| Jimsonweed | 90 | 70 | 50 | 30 |
| Velvetleaf | 80 | 70 | 50 | 30 |

Table B (conti ued)

| | Compound 35 | | | |
|---|---|---|---|---|
| POSTEMERGENCE | | | | |
| Rate g/ha | 62 | 16 | 4 | 1 |
| Corn | 30 | 0 | 0 | 0 |
| Wheat | 40 | 0 | 0 | 0 |
| Barley | - | - | - | - |
| Rice | 100 | 70 | 30 | 0 |
| Soybean | 90 | 80 | 60 | 50 |
| Cotton | 100 | 80 | 40 | 0 |
| Sugar beet | 100 | 100 | 100 | 70 |
| Rape | 100 | 100 | 100 | 100 |
| Crabgrass | 60 | 30 | 0 | 0 |
| Johnsongrass | 100 | 100 | 70 | 40 |
| Blackgrass | 100 | 80 | 30 | 0 |
| Barnyardgrass | 100 | 100 | 70 | 30 |
| Nutsedge | 90 | 70 | 50 | 30 |
| Giant Foxtail | 90 | 80 | 50 | 30 |
| Wild Oats | 50 | 0 | 0 | 0 |
| Cocklebur | 100 | 90 | 50 | 30 |
| Morningglory | 100 | 90 | 50 | 30 |
| Teaweed | 90 | 60 | 30 | 0 |
| Sicklepod | 90 | 40 | 0 | 0 |
| Jimsonweed | 100 | 80 | 50 | 30 |
| Velvetleaf | 100 | 100 | 70 | 30 |
| PREEMERGENCE | | | | |
| Rate g/ha | 250 | 62 | 16 | 4 |
| Corn | 30 | 0 | 0 | 0 |
| Wheat | 80 | 60 | 30 | 0 |
| Barley | - | - | - | - |
| Rice | 100 | 100 | 100 | 70 |
| Soybean | 90 | 80 | 70 | 30 |
| Cotton | 90 | 70 | 50 | 30 |
| Sugar beet | 100 | 100 | 90 | 70 |
| Rape | 100 | 90 | 60 | 30 |
| Crabgrass | 80 | 60 | 50 | 30 |
| Johnsongrass | 100 | 100 | 90 | 70 |
| Blackgrass | 100 | 100 | 90 | 50 |
| Barnyardgrass | 100 | 90 | 80 | 50 |
| Nutsedge | 100 | 100 | 70 | 50 |
| Giant Foxtail | 100 | 90 | 80 | 50 |
| Wild Oats | 80 | 60 | 30 | 0 |
| Cocklebur | 90 | 80 | 50 | 40 |
| Morningglory | 90 | 80 | 60 | 30 |
| Teaweed | 90 | 70 | 40 | 0 |
| Sicklepod | 50 | 30 | 0 | 0 |
| Jimsonweed | 100 | 80 | 50 | 30 |
| Velvetleaf | 90 | 80 | 50 | 30 |

Table B (continued)

| | Compound 36 | | | |
|---|---|---|---|---|
| POSTEMERGENCE | | | | |
| Rate g/ha | 62 | 16 | 4 | 1 |
| Corn | 90 | 70 | 30 | 0 |
| Wheat | 80 | 50 | 30 | 0 |
| Barley | - | - | - | - |
| Rice | 90 | 70 | 50 | 30 |
| Soybean | 100 | 80 | 60 | 40 |
| Cotton | 90 | 70 | 40 | 0 |
| Sugar beet | 100 | 100 | 100 | 70 |
| Rape | 0 | 0 | 0 | 0 |
| Crabgrass | 90 | 60 | 30 | 0 |
| Johnsongrass | 100 | 90 | 60 | 30 |
| Blackgrass | 100 | 100 | 70 | 30 |
| Barnyardgrass | 100 | 90 | 70 | 30 |
| Nutsedge | 0 | 0 | 0 | 0 |
| Giant Foxtail | 90 | 70 | 50 | 30 |
| Wild Oats | 90 | 60 | 30 | 0 |
| Cocklebur | 30 | 0 | 0 | 0 |
| Morningglory | 100 | 90 | 70 | 50 |
| Teaweed | 70 | 50 | 30 | 0 |
| Sicklepod | 90 | 60 | 30 | 0 |
| Jimsonweed | 80 | 60 | 40 | 0 |
| Velvetleaf | 80 | 60 | 40 | 0 |
| PREEMERGENCE | | | | |
| Rate g/ha | 250 | 62 | 16 | 4 |
| Corn | 70 | 30 | 0 | 0 |
| Wheat | 60 | 30 | 0 | 0 |
| Barley | - | - | - | - |
| Rice | 100 | 100 | 100 | 90 |
| Soybean | 80 | 40 | 0 | 0 |
| Cotton | 40 | 0 | 0 | 0 |
| Sugar beet | 100 | 90 | 70 | 50 |
| Rape | 80 | 60 | 40 | 0 |
| Crabgrass | 100 | 70 | 50 | 30 |
| Johnsongrass | 100 | 90 | 70 | 40 |
| Blackgrass | 100 | 100 | 80 | 60 |
| Barnyardgrass | 100 | 80 | 40 | 0 |
| Nutsedge | 70 | 50 | 30 | 0 |
| Giant Foxtail | 100 | 90 | 70 | 40 |
| Wild Oats | 70 | 40 | 0 | 0 |
| Cocklebur | - | 0 | 0 | 0 |
| Morningglory | 80 | 60 | 30 | 0 |
| Teaweed | 90 | 70 | 50 | 20 |
| Sicklepod | 90 | 70 | 50 | 30 |
| Jimsonweed | 90 | 70 | 40 | 20 |
| Velvetleaf | 90 | 70 | 50 | 20 |

Table B (continued)

Compound 39

| Rate g/ha | POSTEMERGENCE 62 | 16 | 4 | 1 | PREEMERGENCE 250 | 62 | 16 | 4 |
|---|---|---|---|---|---|---|---|---|
| Corn | 100 | 100 | 70 | 30 | 100 | 80 | 70 | 20 |
| Wheat | 100 | 100 | 80 | 50 | 90 | 70 | 60 | 0 |
| Barley | 100 | 80 | 40 | 0 | 90 | 80 | 20 | 0 |
| Rice | 100 | 100 | 80 | 30 | 100 | 100 | 90 | 60 |
| Soybean | 100 | 100 | 90 | 70 | 100 | 100 | 60 | 60 |
| Cotton | 70 | 30 | 30 | 0 | 80 | 70 | 20 | 0 |
| Sugar beet | 100 | 90 | 40 | 20 | 100 | 100 | 60 | 20 |
| Rape | 100 | 100 | 100 | 80 | 100 | 100 | 90 | 20 |
| Crabgrass | 90 | 50 | 20 | 0 | 100 | 90 | 70 | 20 |
| Johnsongrass | 100 | 100 | 70 | 50 | 100 | 100 | 90 | 80 |
| Blackgrass | 90 | 100 | 70 | 60 | 100 | 90 | 90 | 50 |
| Barnyardgrass | 100 | 100 | 90 | 60 | 100 | 90 | 70 | 30 |
| Nutsedge | 70 | 80 | 30 | 0 | 100 | - | - | - |
| Giant Foxtail | 90 | 40 | 20 | 0 | 100 | 90 | 70 | 30 |
| Green Foxtail | 100 | 70 | 50 | 0 | 100 | 90 | 80 | 70 |
| Cheatgrass | 100 | 100 | 60 | 50 | 100 | 100 | 90 | 70 |
| Wild Oats | 100 | 100 | 90 | 40 | 90 | 90 | 60 | 20 |
| Wild Buckwheat | 100 | 90 | 70 | 0 | 100 | 90 | 80 | 70 |
| Viola | 70 | 80 | 80 | 30 | 100 | 100 | 90 | 20 |
| Lambsquarter | 100 | 90 | 50 | 20 | 100 | 100 | 90 | 60 |
| Cocklebur | 100 | 100 | 100 | 30 | 100 | 80 | 70 | 0 |
| Morningglory | 100 | 100 | 90 | 30 | 100 | 90 | 40 | 0 |
| Teaweed | 90 | 50 | 20 | 0 | 100 | 90 | 60 | 30 |
| Sicklepod | 100 | 100 | 50 | 0 | 100 | 80 | 30 | 0 |
| Jimsonweed | 100 | 100 | 70 | 50 | 100 | 90 | 70 | 50 |
| Velvetleaf | 100 | 90 | 60 | 20 | 100 | 90 | 50 | 20 |

Table B (continued)

Compound 40

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 80 | 20 | 0 | 0 | 80 | 20 | 0 | 0 |
| Wheat | 90 | 80 | 20 | 0 | 80 | 60 | 0 | 0 |
| Barley | 20 | 0 | 0 | 0 | 0 | 0 | 0 | 0 |
| Rice | 90 | 60 | 30 | 0 | 100 | 80 | 50 | 0 |
| Soybean | 100 | 80 | 80 | 50 | 100 | 50 | 30 | 0 |
| Cotton | 70 | 60 | 30 | 0 | 30 | 0 | 0 | 0 |
| Sugar beet | 70 | 50 | 0 | 0 | 90 | 80 | 60 | 0 |
| Rape | 100 | 100 | 70 | 50 | 100 | 90 | 40 | 0 |
| Crabgrass | 40 | 0 | 0 | 0 | 100 | 80 | 20 | 0 |
| Johnsongrass | 80 | 50 | 20 | 0 | 100 | 100 | 90 | 80 |
| Blackgrass | 80 | 50 | 20 | 0 | 100 | 90 | 70 | 50 |
| Barnyardgrass | 90 | 60 | 20 | 0 | 100 | 90 | 80 | 0 |
| Nutsedge | 50 | 0 | 0 | 0 | - | - | 0 | - |
| Giant Foxtail | 60 | 30 | 0 | 0 | 100 | 90 | 70 | 0 |
| Green Foxtail | 60 | 30 | 20 | 0 | 100 | 100 | 40 | 0 |
| Cheatgrass | 80 | 70 | 50 | 0 | 100 | 90 | 70 | 60 |
| Wild Oats | 100 | 40 | 0 | 0 | 90 | 30 | 0 | 0 |
| Wild Buckwheat | 70 | 0 | 0 | 0 | 100 | 70 | 60 | 20 |
| Viola | 80 | - | - | - | 90 | 80 | 20 | 0 |
| Lambsquarter | 90 | 70 | 20 | 0 | 100 | 100 | 80 | 70 |
| Cocklebur | 100 | 90 | 80 | 0 | 90 | - | - | - |
| Morningglory | 100 | 100 | 70 | 30 | 90 | 70 | 20 | 0 |
| Teaweed | 60 | 50 | 30 | 0 | 90 | 90 | 50 | 0 |
| Sicklepod | 100 | 30 | 30 | 20 | 80 | 0 | 0 | 0 |
| Jimsonweed | 90 | 70 | 50 | 0 | 100 | 40 | 20 | 0 |
| Velvetleaf | 100 | 80 | 60 | 0 | 90 | 30 | 0 | 0 |

Table B (continu 1)

Compound 46

| Rate g/ha | POSTEMERGENCE 62 | 16 | 4 | 1 | PREEMERGENCE 250 | 62 | 16 | 4 |
|---|---|---|---|---|---|---|---|---|
| Corn | 100 | 80 | 50 | 0 | 90 | 80 | 0 | 0 |
| Wheat | 80 | 70 | 20 | 0 | 90 | 70 | 40 | 20 |
| Barley | 90 | 80 | 50 | 0 | 90 | 90 | 50 | 0 |
| Rice | 100 | 100 | 70 | 20 | 100 | 100 | 90 | 80 |
| Soybean | 100 | 90 | 80 | 60 | 90 | 80 | 40 | 0 |
| Cotton | 100 | 60 | 50 | 0 | 90 | 70 | 40 | 0 |
| Sugar beet | 100 | 100 | 60 | 20 | 100 | 90 | 70 | 50 |
| Rape | 100 | 100 | 90 | 50 | 100 | 100 | 80 | 50 |
| Crabgrass | 60 | 30 | 0 | 0 | 90 | 50 | 30 | 0 |
| Johnsongrass | 90 | 100 | 80 | 20 | 100 | 70 | 50 | 30 |
| Blackgrass | 100 | 90 | 60 | 0 | 90 | 90 | 50 | 30 |
| Barnyardgrass | 100 | 100 | 80 | 20 | 90 | 70 | 40 | 0 |
| Nutsedge | 30 | - | - | 0 | 90 | 70 | 50 | 30 |
| Giant Foxtail | 100 | 80 | 40 | 0 | 90 | 80 | 50 | 30 |
| Green Foxtail | 100 | 80 | 60 | 0 | 90 | 80 | 50 | 30 |
| Cheatgrass | 40 | 20 | 0 | 0 | 90 | 80 | 30 | 20 |
| Wild Oats | 90 | 90 | 30 | 0 | 70 | 60 | 30 | 0 |
| Wild Buckwheat | 100 | 100 | 80 | 0 | 100 | 80 | 60 | 30 |
| Viola | 90 | 70 | 30 | 30 | 100 | 100 | 100 | 90 |
| Lambsquarter | 100 | 100 | 80 | 50 | 100 | 100 | 70 | 50 |
| Cocklebur | 100 | 70 | 20 | 0 | 40 | 0 | 0 | 0 |
| Morningglory | 100 | 100 | 80 | 60 | 90 | 80 | 70 | 50 |
| Teaweed | 80 | 60 | 40 | 0 | 90 | 70 | 50 | 30 |
| Sicklepod | 100 | 90 | 70 | 0 | 50 | 30 | 0 | 0 |
| Jimsonweed | 100 | 90 | 80 | 20 | 90 | 70 | 50 | 30 |
| Velvetleaf | 100 | 90 | 60 | 20 | 90 | 70 | 50 | 30 |

Table B (continued)

Compound 53

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 0 | 0 | 0 | 0 | 0 | 0 | 0 | 0 |
| Wheat | 60 | 30 | 0 | 0 | 70 | 40 | 20 | 0 |
| Barley | 60 | 30 | 0 | 0 | 60 | 40 | 20 | 0 |
| Rice | 90 | 60 | 30 | 0 | 90 | 80 | 70 | 30 |
| Soybean | 100 | 90 | 80 | 70 | 90 | 80 | 60 | 30 |
| Cotton | 90 | 60 | 20 | 0 | 60 | 50 | 30 | 0 |
| Sugar beet | 100 | 100 | 100 | 90 | 100 | 90 | 80 | 70 |
| Rape | 100 | 100 | 100 | 100 | 100 | 90 | 70 | 50 |
| Crabgrass | 70 | 50 | 30 | 0 | 90 | 70 | 50 | 30 |
| Johnsongrass | 100 | 90 | 70 | 50 | 90 | 80 | 70 | 30 |
| Blackgrass | 100 | 90 | 60 | 30 | 100 | 70 | 50 | 30 |
| Barnyardgrass | 100 | 100 | 90 | 60 | 90 | 80 | 70 | 30 |
| Nutsedge | - | - | - | - | 90 | 80 | 70 | 50 |
| Giant Foxtail | 100 | 90 | 70 | 50 | 90 | 80 | 70 | 30 |
| Green Foxtail | 90 | 70 | 50 | 30 | 90 | 70 | 50 | 30 |
| Cheatgrass | 100 | 90 | 70 | 50 | 90 | 80 | 70 | 30 |
| Wild Oats | 30 | 0 | 0 | 0 | 40 | 20 | 0 | 0 |
| Wild Buckwheat | 100 | 90 | 70 | 50 | 90 | 80 | 70 | 50 |
| Viola | 100 | 90 | 70 | 50 | 100 | 90 | 70 | 50 |
| Lambsquarter | 100 | 90 | 70 | 50 | 90 | 70 | 50 | 30 |
| Cocklebur | 100 | 90 | 70 | 50 | 90 | 70 | 50 | 30 |
| Morningglory | 100 | 100 | 90 | 60 | 90 | 80 | 70 | 60 |
| Teaweed | 90 | 70 | 50 | 30 | 90 | 70 | 50 | 30 |
| Sicklepod | 100 | 100 | 70 | 50 | 90 | 80 | 70 | 60 |
| Jimsonweed | 100 | 90 | 70 | 50 | 90 | 80 | 70 | 50 |
| Velvetleaf | 100 | 100 | 80 | 60 | 90 | 70 | 50 | 30 |

Table B (continued)

Compound 54

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 0 | 0 | 0 | 0 | 0 | 0 | 0 | 0 |
| Wheat | 80 | 60 | 40 | 20 | 80 | 50 | 30 | 0 |
| Barley | 60 | 30 | 0 | 0 | 50 | 30 | 0 | 0 |
| Rice | 90 | 60 | 30 | 0 | 90 | 70 | 50 | 30 |
| Soybean | 100 | 90 | 70 | 50 | 80 | 60 | 30 | 0 |
| Cotton | 90 | 70 | 20 | 0 | 80 | 40 | 0 | 0 |
| Sugar beet | 100 | 100 | 90 | 70 | 100 | 90 | 80 | 70 |
| Rape | 100 | 100 | 100 | 90 | 90 | 80 | 70 | 50 |
| Crabgrass | 60 | 30 | 0 | 0 | 70 | 50 | 30 | 0 |
| Johnsongrass | 100 | 90 | 70 | 50 | 90 | 70 | 50 | 30 |
| Blackgrass | 100 | 90 | 70 | 50 | 90 | 80 | 70 | 50 |
| Barnyardgrass | 100 | 90 | 70 | 50 | 90 | 80 | 70 | 40 |
| Nutsedge | - | - | - | - | 100 | 90 | 70 | 50 |
| Giant Foxtail | 90 | 70 | 50 | 30 | 90 | 80 | 70 | 40 |
| Green Foxtail | 90 | 70 | 50 | 30 | 90 | 80 | 70 | 50 |
| Cheatgrass | 100 | 70 | 50 | 30 | 90 | 80 | 70 | 50 |
| Wild Oats | 30 | 0 | 0 | 0 | 40 | 0 | 0 | 0 |
| Wild Buckwheat | 90 | 70 | 50 | 30 | 90 | 80 | 70 | 50 |
| Viola | 90 | 70 | 50 | 30 | 100 | 100 | 90 | 70 |
| Lambsquarter | 100 | 90 | 70 | 50 | 90 | 80 | 70 | 50 |
| Cocklebur | 100 | 90 | 70 | 50 | 90 | 70 | 50 | 30 |
| Morningglory | 100 | 70 | 50 | 30 | 90 | 80 | 70 | 50 |
| Teaweed | 90 | 70 | 50 | 30 | 90 | 70 | 50 | 30 |
| Sicklepod | 100 | 70 | 50 | 30 | 90 | 70 | 50 | 30 |
| Jimsonweed | 100 | 90 | 70 | 50 | 100 | 70 | 50 | 30 |
| Velvetleaf | 100 | 90 | 70 | 50 | 90 | 70 | 50 | 30 |

Table B (continued)

Compound 55

| Rate g/ha | POSTEMERGENCE 62 | 16 | 4 | 1 | PREEMERGENCE 250 | 62 | 16 | 4 |
|---|---|---|---|---|---|---|---|---|
| Corn | 0 | 0 | 0 | 0 | 0 | 0 | 0 | 0 |
| Wheat | 90 | 60 | 30 | 0 | 80 | 40 | 0 | 0 |
| Barley | 70 | 40 | 0 | 0 | 80 | 30 | 0 | 0 |
| Rice | 90 | 60 | 30 | 0 | 100 | 70 | 50 | 30 |
| Soybean | 90 | 80 | 70 | 50 | 90 | 70 | 30 | 0 |
| Cotton | 90 | 60 | 0 | 0 | 90 | 60 | 30 | 0 |
| Sugar beet | 100 | 100 | 90 | 70 | 100 | 90 | 80 | 70 |
| Rape | 100 | 100 | 100 | 80 | 90 | 80 | 70 | 40 |
| Crabgrass | 80 | 60 | 40 | 20 | 70 | 50 | 30 | 0 |
| Johnsongrass | 100 | 90 | 70 | 50 | 90 | 80 | 70 | 50 |
| Blackgrass | 100 | 90 | 70 | 50 | 100 | 100 | 90 | 80 |
| Barnyardgrass | 100 | 100 | 70 | 50 | 90 | 80 | 70 | 40 |
| Nutsedge | - | - | - | - | 90 | 80 | 70 | 50 |
| Giant Foxtail | 90 | 80 | 70 | 30 | 90 | 80 | 60 | 30 |
| Green Foxtail | 90 | 70 | 50 | 30 | 90 | 80 | 70 | 50 |
| Cheatgrass | 100 | 90 | 70 | 50 | 90 | 80 | 70 | 40 |
| Wild Oats | 70 | 30 | 0 | 0 | 50 | 30 | 0 | 0 |
| Wild Buckwheat | 100 | 90 | 80 | 60 | 90 | 80 | 70 | 50 |
| Viola | 100 | 90 | 70 | 50 | 100 | 100 | 90 | 70 |
| Lambsquarter | 100 | 90 | 70 | 50 | 90 | 70 | 50 | 30 |
| Cocklebur | 100 | 90 | 70 | 50 | 100 | 70 | 50 | 30 |
| Morningglory | 100 | 80 | 60 | 30 | 100 | 90 | 60 | 30 |
| Teaweed | 90 | 70 | 50 | 30 | 90 | 70 | 50 | 30 |
| Sicklepod | 100 | 70 | 50 | 30 | 90 | 70 | 50 | 30 |
| Jimsonweed | 90 | 80 | 60 | 30 | 90 | 70 | 50 | 30 |
| Velvetleaf | 100 | 90 | 70 | 40 | 90 | 70 | 50 | 30 |

Table B (continued)

Compound 56

| | POSTEMERGENCE | | | | PREEMERGENCE | | | |
|---|---|---|---|---|---|---|---|---|
| Rate g/ha | 62 | 16 | 4 | 1 | 250 | 62 | 16 | 4 |
| Corn | 0 | 0 | 0 | 0 | 0 | 0 | 0 | 0 |
| Wheat | 40 | 20 | 0 | 0 | 80 | 40 | 0 | 0 |
| Barley | 20 | 0 | 0 | 0 | 0 | 0 | 0 | 0 |
| Rice | 80 | 60 | 30 | 0 | 90 | 80 | 70 | 40 |
| Soybean | 70 | 30 | 0 | 0 | 80 | 60 | 30 | 0 |
| Cotton | 40 | 0 | 0 | 0 | 70 | 0 | 0 | 0 |
| Sugar beet | 100 | 90 | 70 | 50 | 100 | 90 | 80 | 70 |
| Rape | 100 | 100 | 90 | 80 | 100 | 90 | 80 | 70 |
| Crabgrass | 50 | 30 | 0 | 0 | 70 | 50 | 30 | 0 |
| Johnsongrass | 90 | 70 | 50 | 30 | 100 | 90 | 80 | 60 |
| Blackgrass | 90 | 70 | 50 | 30 | 90 | 80 | 70 | 50 |
| Barnyardgrass | 90 | 70 | 50 | 30 | 100 | 80 | 70 | 50 |
| Nutsedge | - | - | - | - | 90 | 80 | 70 | 50 |
| Giant Foxtail | 90 | 60 | 30 | 0 | 90 | 80 | 70 | 40 |
| Green Foxtail | 90 | 70 | 50 | 30 | 90 | 80 | 70 | 50 |
| Cheatgrass | 90 | 70 | 50 | 30 | 90 | 80 | 70 | 50 |
| Wild Oats | 0 | 0 | 0 | 0 | 30 | 0 | 0 | 0 |
| Wild Buckwheat | 90 | 70 | 50 | 30 | 90 | 70 | 60 | 40 |
| Viola | - | 70 | 50 | 30 | 100 | 100 | 90 | 80 |
| Lambsquarter | 90 | 70 | 50 | 30 | 90 | 70 | 50 | 30 |
| Cocklebur | 90 | 70 | 50 | 30 | 100 | 70 | 50 | 30 |
| Morningglory | 100 | 60 | 30 | 0 | 90 | 70 | 50 | 30 |
| Teaweed | 90 | 70 | 50 | 30 | 90 | 70 | 50 | 30 |
| Sicklepod | 70 | 50 | 30 | 0 | 100 | 70 | 50 | 30 |
| Jimsonweed | 90 | 70 | 50 | 30 | 90 | 70 | 50 | 30 |
| Velvetleaf | 100 | 70 | 50 | 30 | 90 | 70 | 60 | 50 |

## Claims

1. A compound of the formula:

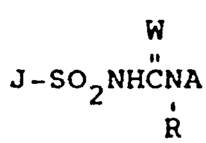

wherein

J is 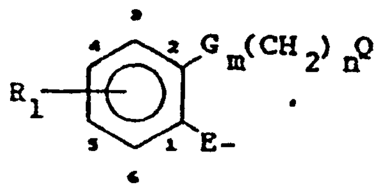 ,

J-1

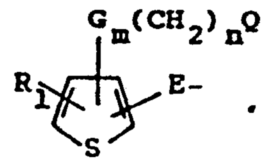 ,

J-2

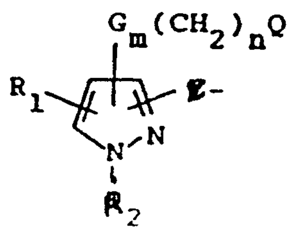 ,

J-3

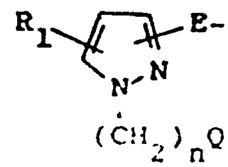 ,

J-4

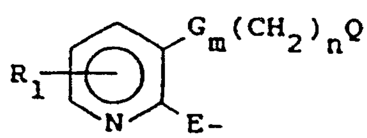 ,

J-5

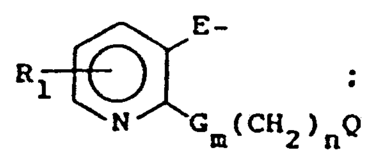 ;

J-6

W is O or S;
G is O, S, SO or $SO_2$;
m is 0 or 1;
n is 0, 1 or 2;
R is H or $CH_3$;
E is a single bond, $CH_2$ or O;

Q is 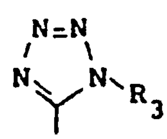 ,

Q-1

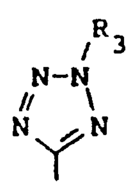 ,

Q-2

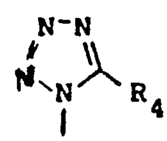 ,

Q-3

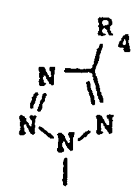 ,

Q-4

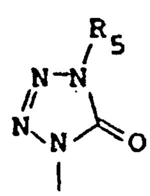 ,

Q-5

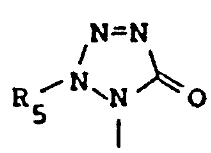 ,

Q-6

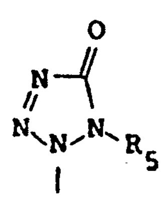 ;

Q-7

$R_1$
is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, CN, $CO_2R_c$, $C_1$-$C_3$ haloalkoxy, $C_1$-$C_3$ haloalkylthio, amino, $C_1$-$C_3$ alkylamino, di($C_1$-$C_3$ alkyl)amino or $C_1$-$C_2$ alkyl substituted with $C_1$-$C_2$ alkoxy, $C_1$-$C_2$ haloalkoxy, $C_1$-$C_2$ alkylthio, $C_1$-$C_2$ haloalkylthio or CN;
$R_2$
is H, $C_1$-$C_3$ alkyl, allyl or phenyl;
$R_3$
is H, $C_1$-$C_6$ alkyl, $C_3$-$C_6$ cycloalkyl, $C_1$-$C_6$ haloalkyl, $CH_2$-($C_2$-$C_5$ alkenyl), $CH_2$($C_2$-$C_5$ haloalkenyl), $CH_2$-($C_2$-$C_5$ alkynyl), $CH_2$($C_2$-$C_5$ haloalkynyl), $C_6H_5$ or $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl;
$R_4$
is H, halogen, $C_1$-$C_6$ alkyl, $C_3$-$C_6$ cycloalkyl, $C_1$-$C_6$ haloalkyl, $C_2$-$C_6$ alkenyl, $C_2$-$C_6$ haloalkenyl, $C_2$-$C_6$ alkynyl, $C_3$-$C_6$ haloalkynyl, $C_6H_5$, $C_1$-$C_4$ alkoxy, $C_1$-$C_4$ alkylthio, $C_1$-$C_4$ alkylsulfinyl, $C_1$-$C_4$ alkylsulfonyl, $OCH_2CH_2O$-($C_1$-$C_2$ alkyl) or di($C_1$-$C_3$ alkyl)amino;
$R_5$
is H, $C_1$-$C_3$ alkyl or allyl;
$R_a$
is H, $C_1$-$C_4$ alkyl, $C_2$-$C_3$ cyanoalkyl, methoxy or ethoxy;
$R_b$
is H, $C_1$-$C_4$ alkyl or $C_3$-$C_4$ alkenyl; or
$R_a$
and $R_b$ may be taken together as $-(CH_2)_3-$, $-(CH_2)_4-$, $-(CH_2)_5-$ or $-CH_2CH_2OCH_2CH_2-$;
$R_c$
is $C_1$-$C_4$ alkyl, $C_3$-$C_4$ alkenyl, $C_3$-$C_4$ alkynyl, $C_2$-$C_4$ haloalkyl, $C_1$-$C_2$ cyanoalkyl, $C_5$-$C_6$ cycloalkyl, $C_4$-$C_7$ cycloalkylalkyl or $C_2$-$C_4$ alkoxyalkyl;

A is 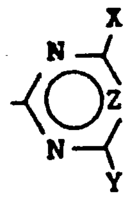, 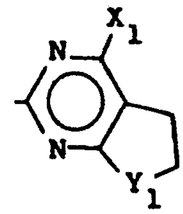, 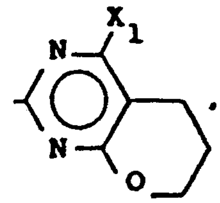,

A-1 A-2 A-3

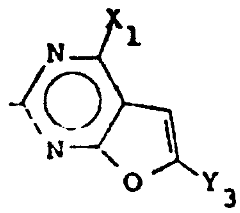, 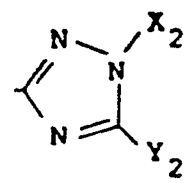, 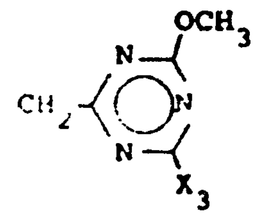

A-4 A-5 A-6

or 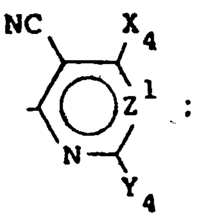 ;

A-7

X
is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y
is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_2$-$C_4$ alkynyl, azido, cyano,

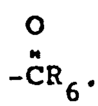, 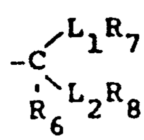, 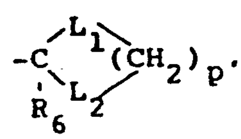,

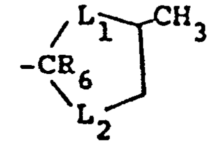

or $-N(OCH_3)CH_3$;

p is 2 or 3;
$L_1$ and $L_2$ are independently O or S;
$R_6$ is H or $CH_3$;
$R_7$ and $R_8$ are independently $C_1$-$C_3$ alkyl;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl;
$Y_4$ is $CH_3$, $OCH_3$, $OC_2H_5$ or Cl; and
$Z^1$ is CH or N;
and their agriculturally suitable salts;
provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;
b) when X or Y is $C_1$ haloalkoxy, then Z is CH;
c) when J is J-2, J-3 or J-4, the substituent $G_m(CH_2)_nQ$ or $(CH_2)_nQ$ and the sulfonylurea bridge are on adjacent ring positions;
d) when J is J-4 and n is O, then Q is Q-1 or Q-2;
e) when E is O, then J is J-1;
f) when W is S, then A is A-1, R is H, and Y is $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$ or

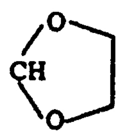 ;

g) when the total number of carbon atoms in X and Y is greater than 4, then the carbon content of $R_1$, $R_3$, $R_4$ and $R_5$ must each be less than or equal to 2;
h) $X_4$ and $Y_4$ cannot simultaneously be Cl;
i) when J is J-1 and m is 0, then n is 0; and
j) when n is 0 and m is 1, then Q is Q-1 or Q-2.

2. A compound of Claim 1 where E is a single bond.

3. A compound of Claim 1 where E is $CH_2$.

4. A compound of Claim 1 where E is O.

5. A compound of Claim 2 where m is 0; W is O; and R is H.

6. A compound of Claim 5 where X is $C_1$-$C_2$ alkyl, $C_1$-$C_2$ alkoxy, Cl, F, Br, I, $OCF_2H$, $CH_2F$, $CF_3$, cyclopropyl, $OCH_2CH_2F$, $OCH_2CHF_2$, $OCH_2CF_3$, $CH_2Cl$ or $CH_2Br$; and Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C{\equiv}CH$, $OCH_2CH_2OCH_3$, $CH_2SCH_3$,

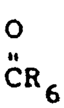 , 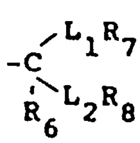 , 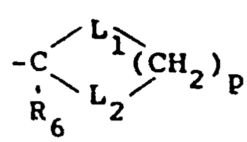 , 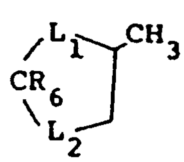 , $OCF_2H$, $SCF_2H$,

cyclopropyl, $C{\equiv}CH$ or $C=CCH_3$.

**7.** A compound of Claim 6 where $R_1$ is H, $CH_3$, F, Cl, Br, $OCH_3$, $SCH_3$, $CH_2CN$, $CH_2OCH_3$, $CF_3$ or $OCF_2H$; and n is O.

**8.** A compound of Claim 7 where $R_3$ is H, $C_1$-$C_3$ alkyl, $CH_2CH_2Cl$, $CH_2CH_2F$, $CH_2CHF_2$, $CH_2CF_3$, allyl or propargyl; and $R_4$ is H, $C_1$-$C_3$ alkyl, $C_1$-$C_2$ alkoxy, $C_1$-$C_2$ alkylthio, $N(CH_3)_2$, allyl, propargyl or $C_1$-$C_2$ alkyl substituted with 1-3 atoms of F, Cl or Br.

**9.** A compound of Claim 8 where A is A-1; Z is CH or N; X is $CH_3$, $OCH_3$, $OCH_2CH_3$, cyclopropyl, Cl or $OCF_2H$; Y is $CH_3$, $OCH_3$, $C_2H_5$, $CH_2OCH_3$, $NHCH_3$ or $CH(OCH_3)_2$.

**10.** A compound of Claim 9 where J is J-1.

**11.** A compound of Claim 10 where $R_3$ and $R_4$ are independently H, $CH_3$ or $C_2H_5$.

**12.** A compound of Claim 9 where J is J-2 to J-6; and $R_1$ is H.

**13.** A compound of Claim 10 where Q is Q-1 to Q-7 and $R_1$ is in the 5- or 6-position.

**14.** A compound of Claim 3 where m is 0; n is 0; J is J-1; W is O; R is H; $R_1$ is H; $R_3$ is H or $C_1$-$C_3$ alkyl; $R_4$ is H or $C_1$-$C_3$ alkyl; A is A-1; Z is CH or N; X is $CH_3$, $OCH_3$, $OCH_2CH_3$, cyclopropyl, Cl or $OCF_2H$; and Y is $CH_3$, $OCH_3$, $C_2H_5$, $CH_2OCH_3$, $NHCH_3$ or $CH(OCH_3)_2$.

**15.** A compound of Claim 4 where m is 0; R is H; $R_1$ is H; $R_3$ is H or $C_1$-$C_3$ alkyl; $R_4$ is H or $C_1$-$C_3$ alkyl; A is A-1; Z is CH or N; X is $CH_3$, $OCH_3$, $OCH_2CH_3$, cyclopropyl, Cl or $OCF_2H$; and Y is $CH_3$, $OCH_3$, $C_2H_5$, $CH_2OCH_3$, $NHCH_3$ or $CH(OCH_3)_2$.

**16.** A compound of Claim 1 which is N-[(4-chloro-6-methoxypyrimidin-2-yl)aminocarbonyl]-2-(1-ethyl-1H-tetrazol-5-yl)benzenesulfonamide.

**17.** A compound of Claim 1 which is N-[(4,6-dimethoxypyrimidin-2-yl)aminocarbonyl]-2-(1-ethyl-1H-tetrazol-5-yl)benzenesulfonamide.

**18.** A compound of claim 1 which is N-[(4,6-dimethoxypyrimidin-2-yl)aminocarbonyl]-2-methyl-6-(1-methyl-1H-tetrazol-5-yl)benzenesulfonamide.

**19.** A compound of claim 1 which is N-[(4-methoxy-6-methylpyrimidin-2-yl)aminocarbonyl]-2-methyl-6-(1-methyl-1H-tetrazol-5-yl)benzenesulfonamide.

**20.** A compound of Claim 1 which is N-[(4,6-dimethylpyrimidin-2-yl)aminocarbonyl]-2-methyl-6-(1-methyl-1H-tetrazol-5-yl)benzenesulfonamide.

**21.** A compound of Claim 1 which is N-[(4,6-dimethoxypyrimidin-2-yl)aminocarbonyl]-2-(5-methyl-1H-tetrazol-1-yl)benzenesulfonamide.

**22.** A compound of Claim 1 which is N-[(4,6-dimethoxypyrimidin-2-yl)aminocarbonyl]-2-(1-methyl-1H-tetrazol-5-yl)benzenesulfonamide.

**23.** A compound of Claim 1 which is N-[(4,6-dimethoxypyrimidin-2-yl)aminocarbonyl]-2-(1-methyl-1H-tetrazol-5-yl)benzenesulfonamide.

**24.** A compound of Claim 1 which is N-[(4,6-dimethylpyrimidin-2-yl)aminocarbonyl]-2-(1-methyl-1H-tetrazol-5-yl)benzenesulfonamide.

**25.** A compound of Claim 1 which is N-[(4-methoxy-6-methylpyrimidin-2-yl)aminocarbonyl]-2-(1-methyl-1H-tetrazol-5-yl)benzenesulfonamide.

**26.** A compound of Claim 1 which is N-[(4-chloro-6-methoxypyrimidin-2-yl)aminocarbonyl]-2-(1-methyl-1H-tetrazol-5-yl)benzenesulfonamide.

**27.** A composition suitable for controlling the growth of undesired vegetation which comprises an effective amount of a compound of Claims 1 to 15 and at least one of the following: surfactant, solid or liquid diluent.

**28.** A composition suitable for controlling the growth of undesired vegetation which comprises an effective amount of a compound any of Claims 16 to 26 and at least one of the following: surfactant, solid or liquid diluent.

**29.** A method for controlling the growth of undesired vegetation which comprises applying to the locus to be protected an effective amount of a compound of Claims 1 to 15.

**30.** A method for controlling the growth of undesired vegetation which comprises applying to the locus to be protected an effective amount of a compound of any of Claims 16 to 26.

**31.** A method for regulating the growth of plants which comprises applying to the locus of such plants an effective but substantially non-phytotoxic amount of a plant growth regulant selected from compounds of any of claims 1 to 26.

**32.** A process for the preparation of a compound of claim 1, which comprises:
(a) reacting a sulfonyl isocyanate or isothiocyanate of formula

$J\ SO_2NCW$ (II)

with an appropriate heterocyclic amine of formula

A-NHR (III) ; or

(b) reacting a sulfonamide of formula

$J\ SO_2NH_2$ (IV)

with a phenyl ester of the appropriate carbamic acid or thiocarbamic acid of formula

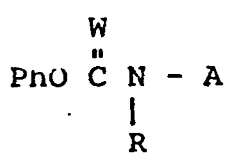 (V)

wherein J, R, W and A as defined in claim 1.

**33.** Compounds of formulae (II) and (IV) as defined in claim 32.

**Claims for the following Contracting State: AT**

**1.** A process for the preparation of a compound of the formula:

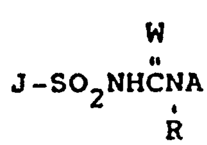

wherein

J is 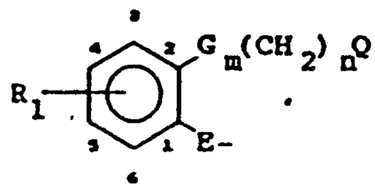 .

J-1

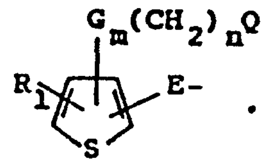 .

J-2

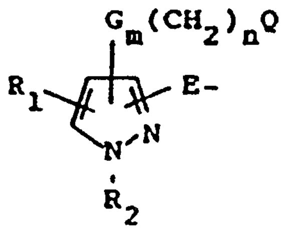 .

J-3

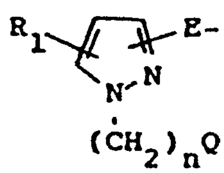 .

J-4

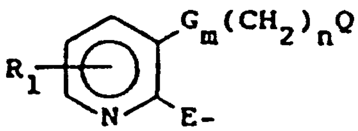 .

J-5

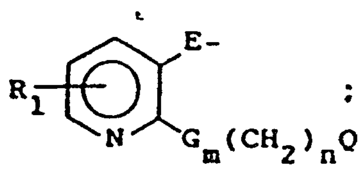 ;

J-6

W is O or S;
G is O, S, SO or $SO_2$;
m is 0 or 1;
n is 0, 1 or 2;
R is H or $CH_3$;
E is a single bond, $CH_2$ or O;

Q is 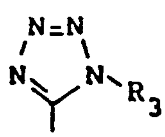 .

Q-1

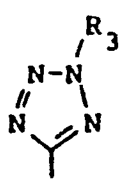 .

Q-2

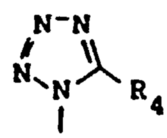 .

Q-3

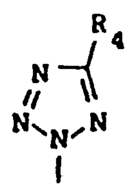 .

Q-4

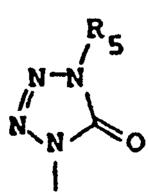 .

Q-5

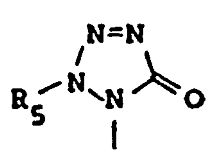 .

Q-6

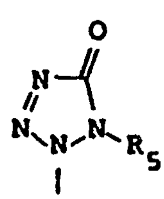 ;

Q-7

$R_1$
is H, $C_1$-$C_3$ alkyl, $C_1$-$C_3$ haloalkyl, halogen, nitro, $C_1$-$C_3$ alkoxy, $SO_2NR_aR_b$, $C_1$-$C_3$ alkylthio, $C_1$-$C_3$ alkylsulfinyl, $C_1$-$C_3$ alkylsulfonyl, CN, $CO_2R_c$, $C_3$-$C_3$ haloalkoxy, $C_1$-$C_3$ haloalkylthio, amino, $C_1$-$C_3$ alkylamino, di($C_1$-$C_3$ alkyl)amino or $C_1$-$C_2$ alkyl substituted with $C_1$-$C_2$ alkoxy, $C_1$-$C_2$ haloalkoxy, $C_1$-$C_2$ alkylthio, $C_1$-$C_2$ haloalkylthio or CN;
$R_2$
is H, $C_1$-$C_3$ alkyl, allyl or phenyl;
$R_3$
is H, $C_1$-$C_6$ alkyl, $C_3$-$C_6$ cycloalkyl, $C_1$-$C_6$ haloalkyl, $CH_2$-($C_2$-$C_5$ alkenyl), $CH_2$($C_2$-$C_5$ haloalkenyl), $CH_2$-($C_2$-$C_5$ alkynyl), $CH_2$($C_2$-$C_5$ haloalkynyl), $C_6H_5$ or $C_1$-$C_4$ alkyl substituted with $C_1$-$C_2$ alkoxy, $C_1$-$C_2$ alkylthio, $C_1$-$C_2$ alkylsulfinyl or $C_1$-$C_2$ alkylsulfonyl;
$R_4$
is H, halogen, $C_1$-$C_6$ alkyl, $C_3$-$C_6$ cycloalkyl, $C_1$-$C_6$ haloalkyl, $C_2$-$C_6$ alkenyl, $C_2$-$C_6$ haloalkenyl, $C_2$-$C_6$ alkynyl, $C_3$-$C_6$ haloalkynyl, $C_6H_5$, $C_1$-$C_4$ alkoxy, $C_1$-$C_4$ alkylthio, $C_1$-$C_4$ alkylsulfinyl, $C_1$-$C_4$ alkylsulfonyl, $OCH_2CH_2O$-($C_1$-$C_2$ alkyl) or di($C_1$-$C_3$ alkyl)amino;
$R_5$
is H, $C_1$-$C_3$ alkyl or allyl;
$R_a$
is H, $C_1$-$C_4$ alkyl, $C_2$-$C_3$ cyanoalkyl, methoxy or ethoxy;
$R_b$
is H, $C_1$-$C_4$ alkyl or $C_3$-$C_4$ alkenyl; or
$R_a$ and $R_b$
may be taken together as $-(CH_2)_3-$, $-(CH_2)_4-$, $-(CH_2)_5-$ or $-CH_2CH_2OCH_2CH_2-$;
$R_c$
is $C_1$-$C_4$ alkyl, $C_3$-$C_4$ alkenyl, $C_3$-$C_4$ alkynyl, $C_2$-$C_4$ haloalkyl, $C_1$-$C_2$ cyanoalkyl, $C_5$-$C_6$ cycloalkyl, $C_4$-$C_7$ cycloalkylalkyl or $C_2$-$C_4$ alkoxyalkyl;

A is 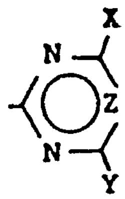, 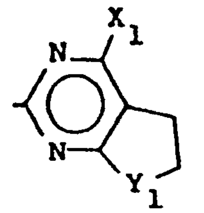, 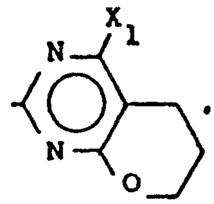,

A-1 A-2 A-3

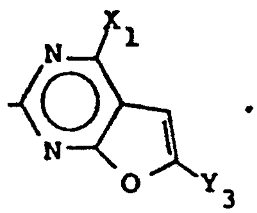, 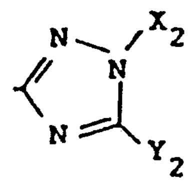, 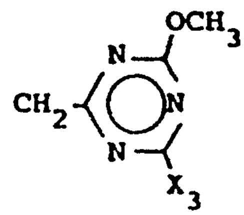

A-4 A-5 A-6

or 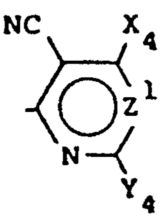 ;

A-7

X
is H, $C_1$-$C_4$ alkyl, $C_1$-$C_4$ alkoxy, $C_1$-$C_4$ haloalkoxy, $C_1$-$C_4$ haloalkyl, $C_1$-$C_4$ haloalkylthio, $C_1$-$C_4$ alkylthio, halogen, $C_2$-$C_5$ alkoxyalkyl, $C_2$-$C_5$ alkoxyalkoxy, amino, $C_1$-$C_3$ alkylamino, di($C_1$-$C_3$ alkyl)amino or $C_3$-$C_5$ cycloalkyl;

Y
is H, $C_1$-$C_4$ alkyl, $C_1$-$C_4$ alkoxy, $C_1$-$C_4$ haloalkoxy, $C_1$-$C_4$ haloalkylthio, $C_1$-$C_4$ alkylthio, $C_2$-$C_5$ alkoxyalkyl, $C_2$-$C_5$ alkoxyalkoxy, amino, $C_1$-$C_3$ alkylamino, di($C_1$-$C_3$ alkyl)amino, $C_3$-$C_4$ alkenyloxy, $C_3$-$C_4$ alkynyloxy, $C_2$-$C_5$ alkylthioalkyl, $C_2$-$C_5$ alkylsulfinylalkyl, $C_2$-$C_5$ alkylsulfonylalkyl, $C_1$-$C_4$ haloalkyl, $C_2$-$C_4$ alkynyl, azido, cyano,

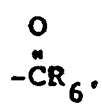, 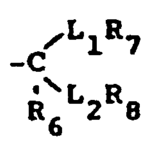, 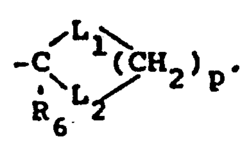,

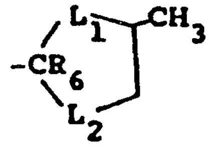

or -N($OCH_3$)$CH_3$;

p is 2 or 3;
$L_1$ and $L_2$ are independently O or S;
$R_6$ is H or $CH_3$;
$R_7$ and $R_8$ are independently $C_1$-$C_3$ alkyl;
Z is CH, N, $CCH_3$, $CC_2H_5$, CCl or CBr;
$Y_1$ is O or $CH_2$;
$X_1$ is $CH_3$, $OCH_3$, $OC_2H_5$ or $OCF_2H$;
$X_2$ is $CH_3$, $C_2H_5$ or $CH_2CF_3$;
$Y_2$ is $OCH_3$, $OC_2H_5$, $SCH_3$, $SC_2H_5$, $CH_3$ or $CH_2CH_3$;
$X_3$ is $CH_3$ or $OCH_3$;
$Y_3$ is H or $CH_3$;
$X_4$ is $CH_3$, $OCH_3$, $OC_2H_5$, $CH_2OCH_3$ or Cl;
$Y_4$ is $CH_3$, $OCH_3$, $OC_2H_5$ or Cl; and
$Z^1$ is CH or N;
and their agriculturally suitable salts;
provided that

a) when X is Cl, F, Br or I, then Z is CH and Y is $OCH_3$, $OC_2H_5$, $N(OCH_3)CH_3$, $NHCH_3$, $N(CH_3)_2$ or $OCF_2H$;
b) when X or Y is $C_1$ haloalkoxy, then Z is CH;
c) when J is J-2, J-3 or J-4, the substituent $G_m(CH_2)_nQ$ or $(CH_2)_nQ$ and the sulfonylurea bridge are on adjacent ring positions;
d) when J is J-4 and n is O, then Q is Q-1 or Q-2;
e) when E is O, then J is J-1;
f) when W is S, then A is A-1, R is H, and Y is $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$, $OCH_2CH_2OCH_3$, $CH(OCH_3)_2$ or

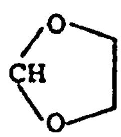 ;

g) when the total number of carbon atoms in X and Y is greater than 4, then the carbon content of $R_1$, $R_3$, $R_4$ and $R_5$ must each be less than or equal to 2;
h) $X_4$ and $Y_4$ cannot simultaneously be Cl;
i) when J is J-1 and m is 0, then n is 0; and
j) when n is 0 and m is 1, then Q is Q-1 or Q-2; which comprises:
(a) reacting a sulfonyl isocyanate or isothiocyanate of formula

J $SO_2NCW$ (II)

with an appropriate heterocyclic amine of formula

A-NHR (III) ; or

(b) reacting a sulfonamide of formula

J $SO_2NH_2$ (IV)

with a phenyl ester of the appropriate carbamic acid or thiocarbamic acid of formula

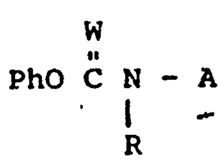 (V)

wherein J, R, W and A as defined above.

2. A process of Claim 1 where E is a single bond.

3. A process of Claim 1 where E is $CH_2$.

4. A process of Claim 1 where E is O.

5. A process of Claim 2 where m is 0; W is O; and R is H.

6. A process of Claim 5 where X is $C_1$-$C_2$ alkyl, $C_1$-$C_2$ alkoxy, Cl, F, Br, I, $OCF_2H$, $CH_2F$, $CF_3$, cyclopropyl, $OCH_2CH_2F$, $OCH_2CHF_2$, $OCH_2CF_3$, $CH_2Cl$ or $CH_2Br$; and Y is H, $C_1$-$C_2$ alkyl, $C_1$-$C_2$ alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C{\equiv}CH$, $OCH_2CH_2OCH_3$, $CH_2SCH_3$,

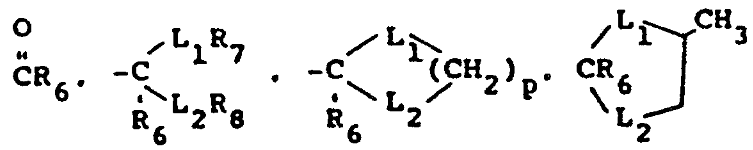

$OCF_2H$, $SCF_2H$, cyclopropyl, $C{\equiv}CH$ or $C{\equiv}CCH_3$.

7. A process of Claim 6 where $R_1$ is H, $CH_3$, F, Cl, Br, $OCH_3$, $SCH_3$, $CH_2CN$, $CH_2OCH_3$, $CF_3$ or $OCF_2H$; and n is O.

8. A process of Claim 7 where $R_3$ is H, $C_1$-$C_3$ alkyl, $CH_2CH_2Cl$, $CH_2CH_2F$, $CH_2CHF_2$, $CH_2CF_3$, allyl or propargyl; and $R_4$ is H, $C_1$-$C_3$ alkyl, $C_1$-$C_2$ alkoxy, $C_1$-$C_2$ alkylthio, $N(CH_3)_2$, allyl, propargyl or $C_1$-$C_2$ alkyl substituted with 1-3 atoms of F, Cl or Br.

9. A process of Claim 8 where A is A-1; Z is CH or N; X is $CH_3$, $OCH_3$, $OCH_2CH_3$, cyclopropyl, Cl or $OCF_2H$; Y is $CH_3$, $OCH_3$, $C_2H_5$, $CH_2OCH_3$, $NHCH_3$ or $CH(OCH_3)_2$.

10. A process of Claim 9 where J is J-1.

11. A process of Claim 10 where $R_3$ and $R_4$ are independently H, $CH_3$ or $C_2H_5$.

12. A process of Claim 9 where J is J-2 to J-6; and $R_1$ is H.

13. A process of Claim 10 where Q is Q-1 to Q-7 and $R_1$ is in the 5- or 6-position.

14. A process of Claim 3 where m is 0; n is 0; J is J-1; W is O; R is H; $R_1$ is H; $R_3$ is H or $C_1$-$C_3$ alkyl; $R_4$ is H or $C_1$-$C_3$ alkyl; A is A-1; Z is CH or N; X is $CH_3$, $OCH_3$, $OCH_2CH_3$, cyclopropyl, Cl or $OCF_2H$; and Y is $CH_3$, $OCH_3$, $C_2H_5$, $CH_2OCH_3$, $NHCH_3$ or $CH(OCH_3)_2$.

15. A process of Claim 4 where m is 0; R is H; $R_1$ is H; $R_3$ is H or $C_1$-$C_3$ alkyl; $R_4$ is H or $C_1$-$C_3$ alkyl; A is A-1; Z is CH or N; X is $CH_3$, $OCH_3$, $OCH_2CH_3$, cyclopropyl, Cl or $OCF_2H$; and Y is $CH_3$, $OCH_3$, $C_2H_5$, $CH_2OCH_3$, $NHCH_3$ or $CH(OCH_3)_2$.

16. A process of Claim 1 wherein the product is N-[(4-chloro-6-methoxypyrimidin-2-yl)aminocarbonyl]-2-(1-ethyl-1H-tetrazol-5-yl)benzenesulfonamide, or an agriculturally suitable salt thereof.

17. A process of Claim 1 wherein the product is N-[(4,6-dimethoxypyrimidin-2-yl)aminocarbonyl]-2-(1-ethyl-1H-tetrazol-5-yl)benzenesulfonamide, or an agriculturally suitable salt thereof.

18. A process of Claim 1 wherein the product is N-[(4,6-dimethoxypyrimidin-2-yl)aminocarbonyl]-2-methyl-6-(1-methyl-1H-tetrazol-5-yl)benzenesulfonamide or an agriculturally suitable salt thereof.

**19.** A process of Claim 1 wherein the product is N-[(4-methoxy-6-methylpyrimidin-2-yl)aminocarbonyl]-2-methyl-6-(1-methyl-1H-tetrazol-5-yl)benzenesulfonamide or an agriculturally suitable salt thereof.

**20.** A process of Claim 1 wherein the product is N-[(4,6-dimethylpyrimidin-2-yl)aminocarbonyl]-2-methyl-6-(1-methyl-1H-tetrazol-5-yl)benzenesulfonamide or an agriculturally suitable salt thereof.

**21.** A process of Claim 1 wherein the product is N-[(4,6-dimethoxypyrimidin 2-yl)aminocarbonyl]-2-(5-methyl-1H-tetrazol-1-yl benzenesulfonamide or an agriculturally suitable salt thereof.

**22.** A process of Claim 1 wherein the product is N-[(4,6-dimethoxypyrimidin-2-yl)aminocarbonyl]-2-(1-methyl-1H-tetrazol-5-yl benzenesulfonamide or an agriculturally suitable salt thereof.

**23.** A process of Claim 1 wherein the product is N-[(4,6-dimethoxypyrimidin-2-yl)aminocarbonyl]-2-(1-methyl-1H-tetrazol-5-yl)benzenesulfonamide or an agriculturally suitable salt thereof.

**24.** A process of Claim 1 wherein the product is N-[(4,6-dimethylpyrimidin-2-yl)aminocarbonyl]-2-(1-methyl-1H-tetrazol-5-yl)benzenesulfonamide or an agriculturally suitable salt thereof.

**25.** A process of Claim 1 wherein the product is N-[(4-methoxy-6-methylpyrimidin-2-yl)aminocarbonyl]-2-(1-methyl-1H-tetrazol-5-yl)benzenesulfonamide or an agriculturally suitable salt thereof.

**26.** A process of Claim 1 wherein the product is N-[(4-chloro-6-methoxypyrimidin-2-yl)aminocarbonyl]-2-(1-methyl-1H-tetrazol-5-yl)benzenesulfonamide or an agriculturally suitable salt thereof.

**27.** A composition suitable for controlling the growth of undesired vegetation which comprises an effective amount of a compound of formula (I) as defined in any of Claims 1 to 15 and at least one of the following: surfactant, solid or liquid diluent.

**28.** A composition suitable for controlling the growth of undesired vegetation which comprises an effective amount of a compound of formula (I) as defined in any of Claims 16 to 26 and at least one of the following: surfactant, solid or liquid diluent.

**29.** A method for controlling the growth of undesired vegetation which comprises applying to the locus to be protected an effective amount of a compound of formula (I) as defined in any of Claims 1 to 15.

**30.** A method for controlling the growth of undesired vegetation which comprises applying to the locus to be protected an effective amount of a compound of formula (I) as defined in any of Claims 16 to 26.

**31.** A method for regulating the growth of plants which comprises applying to the locus of such plants an effective but substantially non-phytotoxic amount of a plant growth regulant selected from compounds of formula (I) as defined in any of Claims 1 to 26.

**Revendications**

**1.** Un composé de la formule :

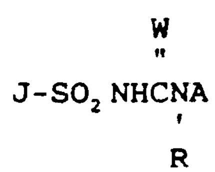

dans laquelle

J est 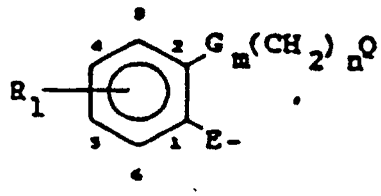 , 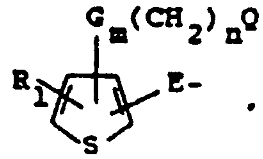 ,

J-1 J-2

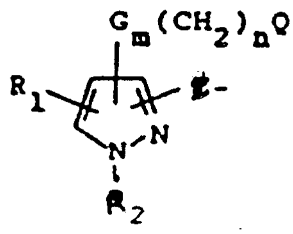 , 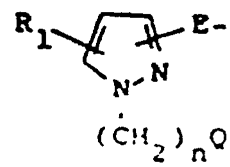 ,

J-3 J-4

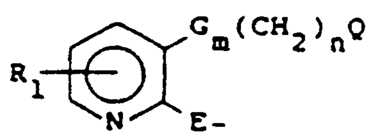 , 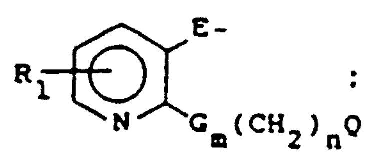 ;

J-5 J-6

W est O ou S ;
G est O, S, SO ou $SO_2$ ;
m est 0 ou 1 ;
n est 0, 1 ou 2 ;
R est H ou $CH_3$ ;
E est une liaison simple, $CH_2$ ou O ;

Q est 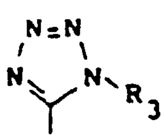 , 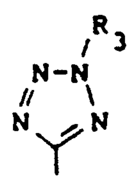 , 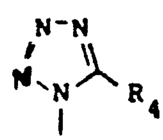 , 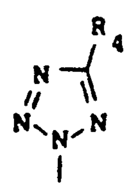 ,

Q-1 Q-2 Q-3 Q-4

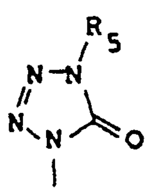 , 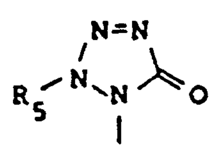 , 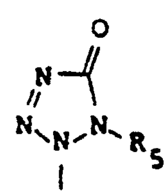 ;

Q-5 Q-6 Q-7

$R_1$

est H, un groupe alkyle en $C_1$-$C_3$, halogénalkyle en $C_1$-$C_3$, halogéno, nitro, alcoxy en $C_1$-$C_3$, $SO_2NR_aR_b$, alkylthio en $C_1$-$C_3$, alkylsulfinyle en $C_1$-$C_3$, alkylsulfonyle en $C_1$-$C_3$, CN, $CO_2R_c$, halogénalcoxy en $C_1$-$C_3$, halogénalkylthio en $C_1$-$C_3$, amino, alkylamino en $C_1$-$C_3$, di(alkyle en $C_1$-$C_3$)amino ou alkyle en $C_1$-$C_2$ substitué par alcoxy en $C_1$-$C_2$, halogénalcoxy en $C_1$-$C_2$, alkylthio en $C_1$-$C_2$, halogénalkylthio en $C_1$-$C_2$ ou CN ;

$R_2$

est H, un groupe alkyle en $C_1$-$C_3$, allyle ou phényle ;

$R_3$

est H, un groupe alkyle en $C_1$-$C_6$, cycloalkyle en $C_3$-$C_6$, halogénalkyle en $C_1$-$C_6$, $CH_2$ (alcényle en $C_2$-$C_5$), $CH_2$(halogénalcényle en $C_2$-$C_5$), $CH_2$(alcynyle en $C_2$-$C_5$), $CH_2$(halogénalcynyle en $C_2$-$C_5$), $C_6H_5$ ou alkyle en $C_1$-$C_4$ substitué par alcoxy en $C_1$-$C_2$, alkylthio en $C_1$-$C_2$, alkylsulfinyle en $C_1$-$C_2$ ou alkylsulfonyle en $C_1$-$C_2$ ;

$R_4$

est H, un halogène, un groupe alkyle en $C_1$-$C_6$, cycloalkyle en $C_3$-$C_6$, halogénalkyle en $C_1$-$C_6$, alcényle en $C_2$-$C_6$, halogénalcényle en $C_2$-$C_6$, alcynyle en $C_2$-$C_6$, halogénalcynyle en $C_3$-$C_6$, $C_6H_5$, alcoxy en $C_1$-$C_4$, alkylthio en $C_1$-$C_4$, alkylsulfinyle en $C_1$-$C_4$, alkylsulfonyle en $C_1$-$C_4$, $OCH_2CH_2O$(alkyle en $C_1$-$C_2$) ou di(alkyle en $C_1$-$C_3$)amino ;

$R_5$

est H, un groupe alkyle en $C_1$-$C_3$ ou allyle ;

$R_a$

est H, un groupe alkyle en $C_1$-$C_4$, cyanoalkyle en $C_2$-$C_3$, méthoxy ou éthoxy ;

$R_b$

est H, un groupe alkyle en $C_1$-$C_4$ ou alcényle en $C_3$-$C_4$ ; ou bien

$R_a$ et $R_b$

peuvent être pris ensemble sous la forme $-(CH_2)_3-$, $-(CH_2)_4-$, $-(CH_2)_5-$ ou $-CH_2CH_2OCH_2CH_2-$ ;

$R_c$

est un groupe alkyle en $C_1$-$C_4$, alcényle en $C_3$-$C_4$, alcynyle en $C_3$-$C_4$, halogénalkyle en $C_2$-$C_4$, cyanoalkyle en $C_1$-$C_2$, cycloalkyle en $C_5$-$C_6$, cycloalkylalkyle en $C_4$-$C_7$ ou alcoxyalkyle en $C_2$-$C_4$ ;

A est 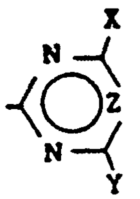 , 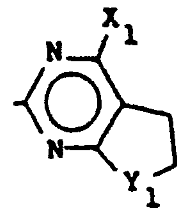 , 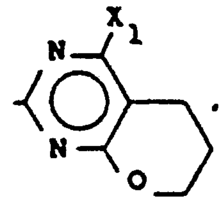

A-1 A-2 A-3

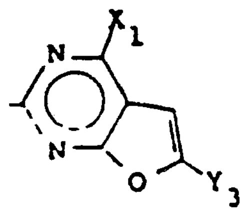 , 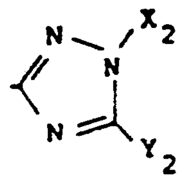 , 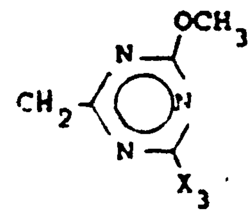

A-4 A-5 A-6

ou 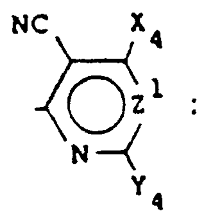 ;

A-7

X

est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$)amino ou cycloalkyle en $C_3$-$C_5$ ;

Y

est H, un groupe alkyle en $C_1$-$C_4$, alcoxy en $C_1$-$C_4$, halogénalcoxy en $C_1$-$C_4$, halogénalkylthio en $C_1$-$C_4$, alkylthio en $C_1$-$C_4$, alcoxyalkyle en $C_2$-$C_5$, alcoxyalcoxy en $C_2$-$C_5$, amino, alkylamino en $C_1$-$C_3$, di(alkyle en $C_1$-$C_3$)amino, alcényloxy en $C_3$-$C_4$, alcynyloxy en $C_3$-$C_4$, alkylthioalkyle en $C_2$-$C_5$,alkylsulfinylalkyle en $C_2$-$C_5$, alkylsulfonylalkyle en $C_2$-$C_5$, halogénalkyle en $C_1$-$C_4$, alcynyle en $C_2$-$C_4$, azido, cyano,

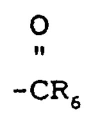 ,

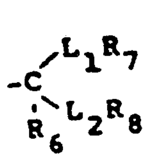 , 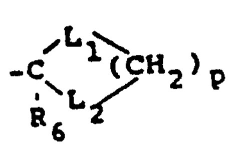 , 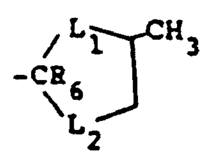

ou $-N(OCH_3)CH_3$ ;

p est 2 ou 3 ;
$L_1$ et $L_2$ sont indépendamment O ou S ;
$R_6$ est H ou $CH_3$ ;
$R_7$ et $R_8$ sont indépendamment un groupe alkyle en $C_1$-$C_3$ ;
Z est CH, N, $CCH_3$, $CC_2H_5$, CCl ou CBr ;
$Y_1$ est O ou $CH_2$ ;
$X_1$ est $CH_3$, $OCH_3$, $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $CH_3$ ou $CH_2CH_3$ ;
$X_3$ est $CH_3$ ou $OCH_3$ ;
$Y_3$ est H ou $CH_3$ ;
$X_4$ est $CH_3$, $OCH_3$, $OC_2H_5$, $CH_2OCH_3$ ou Cl ;
$Y_4$ est $CH_3$, $OCH_3$, $OC_2H_5$ ou Cl ; et
$Z^1$ est CH ou N ;
et ses sels utilisables en agriculture ;
avec les conditions suivantes

a) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$ ;
b) si x ou Y est un groupe halogénalcoxy en $C_1$, alors Z est CH ;
c) si J est J-2, J-3 ou J-4, le substituant $G_m(CH_2)_nQ$ ou $(CH_2)_nQ$ et le pont de sulfonylurée se trouvent sur des positions cycliques adjacentes ;
d) si J est J-4 et n est O, alors Q est Q-1 ou Q-2 ;
e) si E est O, alors J est J-1 ;
f) si W est S, alors A est A-1, R est H, et Y est $CH_3$, $OCH_3$, $OC_2H_5$, $CH_2OCH_3$, $C_2H_5$, $CF_3$, $SCH_3$, $OCH_2CH=CH_2$, $OCH_2C \equiv CH$ $OCH_2CH_2OCH_3$, $CH(OCH_3)_2$,
ou

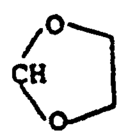 ;

g) si le nombre total d'atomes de carbone dans X et Y est supérieur à 4, alors le nombre d'atomes de carbone de $R_1$, $R_3$, $R_4$ et $R_5$ doit être pour chacun inférieur ou égal à 2 ;
h) $X_4$ et $Y_4$ ne peuvent pas être simultanément Cl ;
i) si J est J-1 et m est 0, alors n est 0 ; et
j) si n est 0 et m est 1, alors Q est Q-1 ou Q-2.

**2.** Un composé de la revendication 1, où E est une liaison simple.

**3.** Un composé de la revendication 1, où E est $CH_2$.

**4.** Un composé de la revendication 1, où E est O.

**5.** Un composé de la revendication 2, où m est 0 ; W est O ; et R est H.

**6.** Un composé de la revendication 5, où X est un groupe alkyle en $C_1$-$C_2$, alcoxy en $C_1$-$C_2$, Cl, F, Br, I, $OCF_2H$, $CH_2F$, $CF_3$, cyclopropyle, $OCH_2CH_2F$, $OCH_2CHF_2$, $OCH_2CF_3$, $CH_2Cl$ ou $CH_2Br$ ; et Y est H, un groupe alkyle en $C_1$-$C_2$, alcoxy en $C_1$-$C_2$, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C \equiv CH$,
$OCH_2CH_2OCH_3$, $CH_2SCH_3$

, 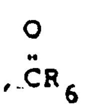 , 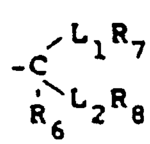 , 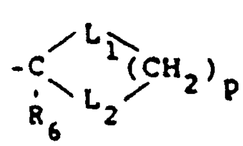 , 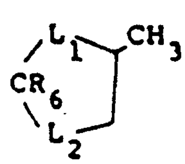 .

$OCF_2H$, $SCF_2H$, cyclopropyle, C≡CH ou $C≡CCH_3$.

7. Un composé de la revendication 6, où $R_1$ est H, $CH_3$, F, Cl, Br, $OCH_3$, $SCH_3$, $CH_2CN$, $CH_2OCH_3$, $CF_3$ ou $OCF_2H$ ; et n est 0.

8. Un composé de la revendication 7, où $R_3$ est H, un groupe alkyle en $C_1$-$C_3$, $CH_2CH_2Cl$, $CH_2CH_2F$, $CH_2CHF_2$, $CH_2CF_3$, allyle ou propargyle ; et $R_4$ est H, un groupe alkyle en $C_1$-$C_3$, alcoxy en $C_1$-$C_2$, alkylthio en $C_1$-$C_2$, $N(CH_3)_2$, allyle, propargyle ou alkyle en $C_1$-$C_2$ substitué par 1 à 3 atomes de F, Cl ou Br.

9. Un composé de la revendication 8, où A est A-1 ; Z est CH ou N ; X est $CH_3$, $OCH_3$, $OCH_2CH_3$, un groupe cyclopropyle, Cl ou $OCF_2H$ ; Y est $CH_3$, $OCH_3$, $C_2H_5$, $CH_2OCH_3$, $NHCH_3$ ou $CH(OCH_3)_2$.

10. Un composé de la revendication 9, où J est J-1.

11. Un composé de la revendication 10, où $R_3$ et $R_4$ sont indépendamment H, $CH_3$ ou $C_2H_5$.

12. Un composé de la revendication 9, où J est J-2 à J-6 ; et $R_1$ est H.

13. Un composé de la revendication 10, où Q est Q-1 à Q-7 et $R_1$ est à la position 5 ou 6.

14. Un composé de la revendication 3, où m est 0 ; n est 0 ; J est J-1 ; W est O ; R est H ; $R_1$ est H ; $R_3$ est H ou un groupe alkyle en $C_1$ -$C_3$ ; $R_4$ est H ou un groupe alkyle en $C_1$-$C_3$ ; A est A-1 ; Z est CH ou N ; X est $CH_3$, $OCH_3$, $OCH_2CH_3$, un groupe cyclopropyle, Cl ou $OCF_2H$ ; et Y est $CH_3$, $OCH_3$, $C_2H_5$, $CH_2OCH_3$, $NHCH_3$ ou $CH(OCH_3)_2$.

15. Un composé de la revendication 4, où m est 0 ; R est H ; $R_1$ est H ; $R_3$ est H ou un groupe alkyle en $C_1$-$C_3$ ; $R_4$ est H ou un groupe alkyle en $C_1$-$C_3$ ; A est A-1 ; Z est CH ou N ; X est $CH_3$, $OCH_3$, $OCH_2CH_3$, un groupe cyclopropyle, Cl ou $OCF_2H$ ; et Y est $CH_3$, $OCH_3$, $C_2H_5$, $CH_2OCH_3$, $NHCH_3$ ou $CH(OCH_3)_2$.

16. Un composé de la revendication 1, qui est le N-[(4-chloro-6-méthoxypyrimidine-2-yl)aminocarbonyl]-2-(1-éthyl-1H-tétrazole-5-yl)benzène-sulfonamide.

17. Un composé de la revendication 1, qui est le N-[(4,6-diméthoxypyrimidine-2-yl)aminocarbonyl]-2-(1-éthyl-1H-tétrazole-5-yl)benzène-sulfonamide.

18. Un composé de la revendication 1, qui est le N-[(4,6-diméthoxypyrimidine-2-yl)aminocarbonyl]-2-méthyl-6-(1-méthyl-1H-tétrazole-5-yl)benzène-sulfonamide.

19. Un composé de la revendication 1, qui est le N-[(4-méthoxy-6-méthylpyrimidine-2-yl)aminocarbonyl]-2-méthyl-6-(1-méthyl-1H-tétrazole-5-yl)benzène-sulfonamide.

20. Un composé de la revendication 1, qui est le N-[(4,6-diméthylpyrimidine-2-yl)aminocarbonyl]-2-mèthyl-6-(1-méthyl-1H-tétrazole-5-yl)benzène-sulfonamide.

21. Un composé de la revendication 1, qui est le N-[(4,6-diméthoxypyrimidine-2-yl )aminocarbonyl]-2-(5-méthyl-1H-tétrazole-1-yl)benzène-sulfonamide.

22. Un composé de la revendication 1, qui est le N-[(4,6-diméthoxypyrimidine-2-yl)aminocarbonyl]-2-(1-méthyl-1H-tétrazole-5-yl)benzène-sulfonamide.

23. Un composé de la revendication 1, qui est le N-[(4,6-diméthoxypyrimidine-2-yl)aminocarbonyl]-2-(1-méthyl-1H-tétrazole-5-yl)benzène-sulfonamide.

24. Un composé de la revendication 1, qui est le N-[(4,6-diméthylpyrimidine-2-yl)aminocarbonyl]-2-(1-méthyl-1H-tétrazole-5-yl)benzène-sulfonamide.

**25.** Un composé de la revendication 1, qui est le N-[(4-méthoxy-6-méthylpyrimidine-2-yl)aminocarbonyl]-2-(1-méthyl-1H-tétrazole-5-yl)benzène-sulfonamide.

**26.** Un composé de la revendication 1, qui est le N-[(4-chloro-6-méthoxypyrimidine-2-yl)aminocarbonyl]-2-(1-méthyl-1H-tétrazole-5-yl)benzène-sulfonamide.

**27.** Une composition convenant pour combattre la croissance de végétation indésirable, qui comprend une quantité efficace d'un composé des revendications 1 à 15 et au moins l'un des ingrédients suivants : agent tensio-actif, diluant solide ou liquide.

**28.** Une composition convenant pour combattre la croissance de végétation indésirable, qui comprend une quantité efficace d'un composé de l'une quelconque des revendications 16 à 26 et au moins l'un des ingrédients suivants : agent tensio-actif, diluant solide ou liquide.

**29.** Un procédé pour combattre la croissance de végétation indésirable, qui consiste à appliquer au site à protéger une quantité efficace d'un composé des revendications 1 à 15.

**30.** Un procédé pour combattre la croissance de végétation indésirable, qui consiste à appliquer au site à protéger une quantité efficace d'un composé de l'une quelconque des revendications 16 à 26.

**31.** Un procédé pour réguler la croissance de plantes, qui consiste à appliquer au site où se trouvent ces plantes une quantité efficace mais sensiblement non phytotoxique d'un régulateur de la croissance de plantes choisi parmi les composés de l'une quelconque des revendications 1 à 26.

**32.** Un procédé pour la préparation d'un composé de la revendication 1, qui consiste à :

(a) faire réagir un isocyanate ou isothiocyanate de sulfonyle de formule

$JSO_2NCW$ (II)

avec une amine hétérocyclique appropriée de formule

A-NHR (III) ; ou

(b) faire réagir un sulfonamide de formule

$JSO_2NH_2$ (IV)

avec un ester de phényle de l'acide carbamique ou acide thiocarbamique approprié de formule

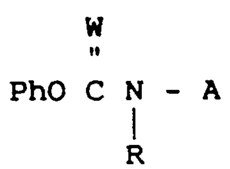

(V)

où J, R, W et A sont tels que définis dans la revendication 1.

**33.** Composés des formules (II) et (IV) telles que définies dans la revendication 32.

**Revendications pour l'Etat contractant suivant: AT**

**1.** Un procédé de préparation d'un composé de la formule :

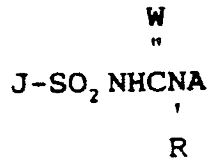

dans laquelle

J est 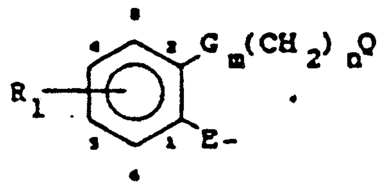 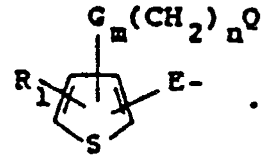

J-1 J-2

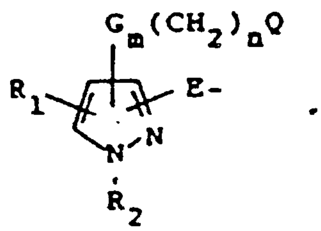 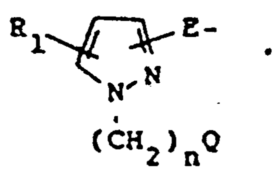

J-3 J-4

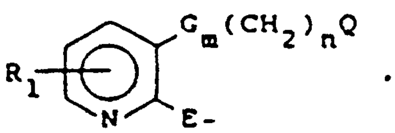 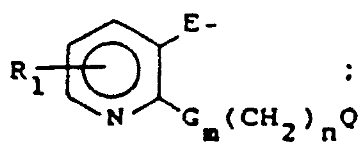

J-5 J-6

W est O ou S ;
G est O, S, SO ou $SO_2$ ;
m est 0 ou 1 ;
n est 0, 1 ou 2 ;
R est H ou $CH_3$ ;
E est une liaison simple, $CH_2$ ou O ;

Q est 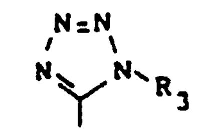 , 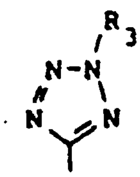 , 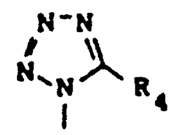 , 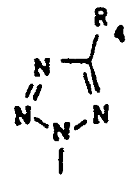 ,

Q-1 Q-2 Q-3 Q-4

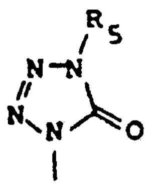 , 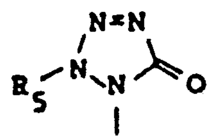 , 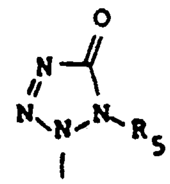 ;

Q-5 Q-6 Q-7

$R_1$
est H, un groupe alkyle en $C_1$-$C_3$, halogénalkyle en $C_1$-$C_3$, halogéno, nitro, alcoxy en $C_1$-$C_3$, $SO_2NR_aR_b$, alkylthio en $C_1$-$C_3$, alkylsulfinyle en $C_1$-$C_3$, alkylsulfonyle en $C_1$-$C_3$, CN, $CO_2R_c$, halogénalcoxy en $C_1$-$C_3$, halogénalkylthio en $C_1$-$C_3$, amino, alkylamino en $C_1$-$C_3$, di(alkyle en $C_1$-$C_3$)amino ou alkyle en $C_1$-$C_2$ substitué par alcoxy en $C_1$-$C_2$, halogénalcoxy en $C_1$-$C_2$, alkylthio en $C_1$-$C_2$, halogénalkylthio en $C_1$-$C_2$ ou CN ;

$R_2$
est H, un groupe alkyle en $C_1$-$C_3$, allyle ou phényle ;

$R_3$
est H, un groupe alkyle en $C_1$-$C_6$, cycloalkyle en $C_3$-$C_6$, halogénalkyle en $C_1$-$C_6$, $CH_2$(alcényle en $C_2$-$C_5$), $CH_2$(halogénalcényle en $C_2$-$C_5$), $CH_2$(alcynyle en $C_2$-$C_5$), $CH_2$ (halogénalcynyle en $C_2$-$C_5$), $C_6H_5$ ou alkyle en $C_1$-$C_4$ substitué par alcoxy en $C_1$-$C_2$, alkylthio en $C_1$-$C_2$, alkylsulfinyle en $C_1$-$C_2$ ou alkylsulfonyle en $C_1$-$C_2$ ;

$R_4$
est H, un halogène, un groupe alkyle en $C_1$-$C_6$, cycloalkyle en $C_3$-$C_6$, halogénalkyle en $C_1$-$C_6$, alcényle en $C_2$-$C_6$, halogénalcényle en $C_2$-$C_6$, alcynyle en $C_2$-$C_6$, halogénalcynyle en $C_3$-$C_6$, $C_6H_5$, alcoxy en $C_1$-$C_4$, alkylthio en $C_1$-$C_4$, alkylsulfinyle en $C_1$-$C_4$, alkylsulfonyle en $C_1$-$C_4$, $OCH_2CH_2O$(alkyle en $C_1$-$C_2$) ou di(alkyle en $C_1$-$C_3$)amino ;

$R_5$
est H, un groupe alkyle en $C_1$-$C_3$ ou allyle ;

$R_a$
est H, un groupe alkyle en $C_1$-$C_4$, cyanoalkyle en $C_2$-$C_3$, méthoxy ou éthoxy ;

$R_b$
est H, un groupe alkyle en $C_1$-$C_4$ ou alcényle en $C_3$-$C_4$ ; ou bien

$R_a$ et $R_b$
peuvent être pris ensemble sous la forme $-(CH_2)_3-$, $-(CH_2)_4-$, $-(CH_2)_5-$ ou $-CH_2CH_2OCH_2CH_2-$ ;

$R_c$
est un groupe alkyle en $C_1$-$C_4$, alcényle en $C_3$-$C_4$, alcynyle en $C_3$-$C_4$, halogénalkyle en $C_2$-$C_4$, cyanoalkyle en $C_1$-$C_2$, cycloalkyle en $C_5$-$C_6$, cycloalkylalkyle en $C_4$-$C_7$ ou alcoxyalkyle en $C_2$-$C_4$ ;

A est 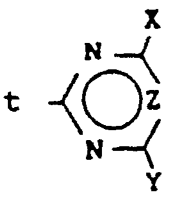 , 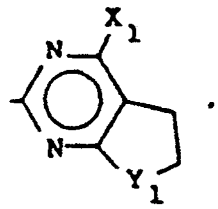 , 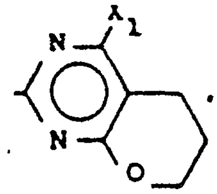 ,

A-1 A-2 A-3

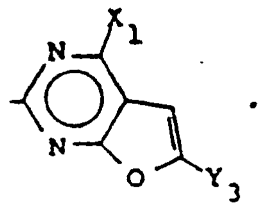 , 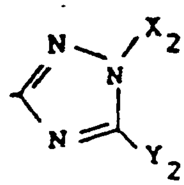 , 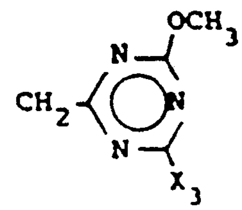

A-4 A-5 A-6

ou 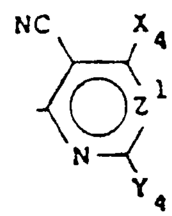 ;

A-7

X
est H, un groupe alkyle en $C_1$-$C_4$, alcoxy en $C_1$-$C_4$, halogénalcoxy en $C_1$-$C_4$, halogénalkyle en $C_1$-$C_4$, halogénalkylthio en $C_1$-$C_4$, alkylthio en $C_1$-$C_4$, halogéno, alcoxyalkyle en $C_2$-$C_5$, alcoxyalcoxy en $C_2$-$C_5$, amino, alkylamino en $C_1$-$C_3$, di(alkyle en $C_1$-$C_3$) amino ou cycloalkyle en $C_3$-$C_5$ ;

Y
est H, un groupe alkyle en $C_1$-$C_4$, alcoxy en $C_1$-$C_4$, halogénalcoxy en $C_1$-$C_4$,halogénalkylthio en $C_1$-$C_4$, alkylthio en $C_1$-$C_4$, alcoxyalkyle en $C_2$-$C_5$, alcoxyalcoxy en $C_2$-$C_5$, amino, alkylamino en $C_1$-$C_3$, di-(alkyle en $C_1$-$C_3$)amino, alcényloxy en $C_3$-$C_4$, alcynyloxy en $C_3$-$C_4$, alkylthioalkyle en $C_2$-$C_5$,alkylsulfinylalkyle en $C_2$-$C_5$, alkylsulfonylalkyle en $C_2$-$C_5$, halogénalkyle en $C_1$-$C_4$, alcynyle en $C_2$-$C_4$, azido, cyano,

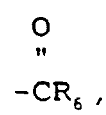 ,

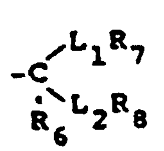 , 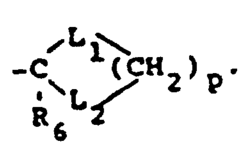 , 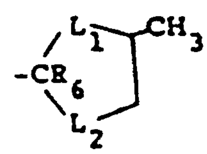

ou -N(OCH₃)CH₃ ;

p est 2 ou 3 ;
$L_1$ et $L_2$ sont indépendamment O ou S ;
$R_6$ est H ou $CH_3$ ;
$R_7$ et $R_8$ sont indépendamment un groupe alkyle en $C_1$-$C_3$ ;
Z est CH, N, $CCH_3$, $CC_2H_5$, CCl ou CBr ;
$Y_1$ est O ou $CH_2$ ;
$X_1$ est $CH_3$, $OCH_3$, $OC_2H_5$ ou $OCF_2H$ ;
$X_2$ est $CH_3$, $C_2H_5$ ou $CH_2CF_3$ ;
$Y_2$ est $OCH_3$, $OC_2H_5$, $SCH_3$, $SC_2H_5$, $CH_3$ ou $CH_2$ $CH_3$ ;
$X_3$ est $CH_3$ ou $OCH_3$ ;
$Y_3$ est H ou $CH_3$ ;
$X_4$ est $CH_3$, $OCH_3$, $OC_2H_5$, $CH_2OCH_3$ ou Cl ;
$Y_4$ est $CH_3$, $OCH_3$, $OC_2H_5$ ou Cl ; et
$Z^1$ est CH ou N ;
et de ses sels utilisables en agriculture ;
avec les conditions suivantes

a) si X est Cl, F, Br ou I, alors Z est CH et Y est $OCH_3$, $OC_2H_5$, $N(OCH_3)CH_3$, $NHCH_3$, $N(CH_3)_2$ ou $OCF_2H$ ;

b) si X ou Y est un groupe halogénalcoxy en $C_1$, alors Z est CH ;

c) si J est J-2, J-3 ou J-4, le substituant $G_m(CH_2)_nQ$ ou $(CH_2)_nQ$ et le pont de sulfonylurée se trouvent sur des positions cycliques adjacentes ;

d) si J est J-4 et n est O, alors Q est Q-1 ou Q-2 ;

e) si E est O, alors J est J-1 ;

f) si W est S, alors A est A-1, R est H, et Y est $CH_3$, $OCH_3$, $OC_2H_5$, $CH_2OCH_3$, $C_2H_5$, $CF_3$, $SCH_3$, $OCH_2CH=CH_2$, $OCH_2C \equiv CH$, $OCH_2CH_2OCH_3$, $CH(OCH_3)_2$,
ou

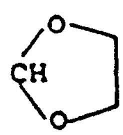 ;

g) si le nombre total d'atomes de carbone dans X et Y est supérieur à 4, alors le nombre d'atomes de carbone de $R_1$, $R_3$, $R_4$ et $R_5$ doit être pour chacun inférieur ou égal à 2 ;

h) $X_4$ et $Y_4$ ne peuvent pas être simultanément Cl ;

i) si J est J-1 et m est 0, alors n est 0 ; et

j) si n est 0 et m est 1, alors Q est Q-1 ou Q-2, qui consiste à :

(a) faire réagir un isocyanate ou isothiocyanate de sulfonyle de formule

$JSO_2NCW$ (II)

avec une amine hétérocyclique appropriée de formule

A-NHR (III) ; ou

(b) faire réagir un sulfonamide de formule

$JSO_2NH_2$ (IV)

avec un ester de phényle de l'acide carbamique ou acide thiocarbamique approprié de formule

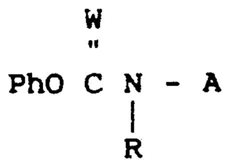 (V)

où J, R, W et A sont tels que définis ci-dessus.

**2.** Un procédé selon la revendication 1, où E est une liaison simple.

**3.** Un procédé selon la revendication 1, où E est $CH_2$.

**4.** Un procédé selon la revendication 1, où E est O.

**5.** Un procédé selon la revendication 2, où m est 0 ; W est O ; et R est H.

**6.** Un procédé selon la revendication 5, où X est un groupe alkyle en $C_1$-$C_2$, alcoxy en $C_1$-$C_2$, Cl, F, Br, I, $OCF_2H$, $CH_2F$, $CF_3$, cyclopropyle, $OCH_2CH_2F$, $OCH_2CHF_2$, $OCH_2CF_3$, $CH_2Cl$ ou $CH_2Br$ ; et Y est H, un groupe alkyle en $C_1$-$C_2$, alcoxy en $C_1$-$C_2$, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$,
$OCH_2CH_2OCH_3$, $CH_2SCH_3$,

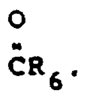, 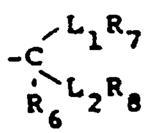, 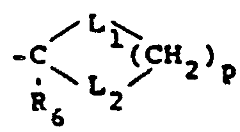, 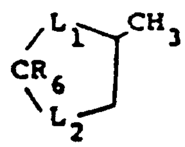

$OCF_2H$, $SCF_2H$, cyclopropyle, $C\equiv CH$ ou $C\equiv CCH_3$.

**7.** Un procédé selon la revendication 6, où $R_1$ est H, $CH_3$, F, Cl, Br, $OCH_3$, $SCH_3$, $CH_2CN$, $CH_2OCH_3$, $CF_3$ ou $OCF_2H$ ; et n est O.

**8.** Un procédé selon la revendication 7, où $R_3$ est H, un groupe alkyle en $C_1$-$C_3$, $CH_2CH_2Cl$, $CH_2CH_2F$, $CH_2CHF_2$, $CH_2CF_3$, allyle ou propargyle ; et $R_4$ est H, un groupe alkyle en $C_1$-$C_3$, alcoxy en $C_1$-$C_2$, alkylthio en $C_1$-$C_2$, $N(CH_3)_2$, allyle, propargyle ou alkyle en $C_1$-$C_2$ substitué par 1 à 3 atomes de F, Cl ou Br.

**9.** Un procédé selon la revendication 8, où A est A-1 ; Z est CH ou N ; X est $CH_3$, $OCH_3$, $OCH_2CH_3$, un groupe cyclopropyle, Cl ou $OCF_2H$ ; Y est $CH_3$, $OCH_3$, $C_2H_5$, $CH_2OCH_3$, $NHCH_3$ ou $CH(OCH_3)_2$.

**10.** Un procédé selon la revendication 9, où J est J-1.

**11.** Un procédé selon la revendication 10, où $R_3$ et $R_4$ sont indépendamment H, $CH_3$ ou $C_2H_5$.

**12.** Un procédé selon la revendication 9, où J est J-2 à J-6 ; et $R_1$ est H.

**13.** Un procédé selon la revendication 10, où Q est Q-1 à Q-7 et $R_1$ est à la position 5 ou 6.

**14.** Un procédé selon la revendication 3, où m est 0 ; n est 0 ; J est J-1 ; W est O ; R est H ; $R_1$ est H ; $R_3$ est H ou un groupe alkyle en $C_1$-$C_3$ ; $R_4$ est H ou un groupe alkyle en $C_1$-$C_3$ ; A est A-1 ; Z est CH ou N ; X est $CH_3$, $OCH_3$, $OCH_2CH_3$, un groupe cyclopropyle, Cl ou $OCF_2H$ ; et Y est $CH_3$, $OCH_3$, $C_2H_5$, $CH_2OCH_3$, $NHCH_3$ ou $CH(OCH_3)_2$.

**15.** Un procédé selon la revendication 4, où m est 0 ; R est H ; $R_1$ est H ; $R_3$ est H ou un groupe alkyle en $C_1$-$C_3$ ; $R_4$ est H ou un groupe alkyle en $C_1$-$C_3$ ; A est A-1 ; Z est CH ou N ; X est $CH_3$, $OCH_3$, $OCH_2CH_3$, un groupe cyclopropyle, Cl ou $OCF_2H$ ; et Y est $CH_3$, $OCH_3$, $C_2H_5$, $CH_2OCH_3$, $NHCH_3$ ou

$CH(OCH_3)_2$.

**16.** Un procédé selon la revendication 1, où le produit est le N-[(4-chloro-6-méthoxypyrimidine-2-yl)-aminocarbonyl]-2-(1-éthyl-1H-tétrazole-5-yl)benzène-sulfonamide.

**17.** Un procédé selon la revendication 1, où le produit est le N-[(4,6-diméthoxypyrimidine-2-yl)-aminocarbonyl]-2-(1-éthyl-1H-tétrazole-5-yl)benzène-sulfonamide.

**18.** Un procédé selon la revendication 1, où le produit est le N-[(4,6-diméthoxypyrimidine-2-yl )aminocarbonyl]-2-méthyl-6-(1-méthyl-1H-tétrazole-5-yl)benzène-sulfonamide.

**19.** Un procédé selon la revendication 1, où le produit est le N-[(4-méthoxy-6-méthylpyrimidine-2-yl)-aminocarbonyl]-2-méthyl-6-(1-méthyl-1H-tétrazole-5-yl)benzène-sulfonamide.

**20.** Un procédé selon la revendication 1, où le produit est le N-[(4,6-diméthylpyrimidine-2-yl)-aminocarbonyl]-2-méthyl-6-(1-méthyl-1H-tétrazole-5-yl)benzène-sulfonamide.

**21.** Un procédé selon la revendication 1, où le produit est le N-[(4,6-diméthoxypyrimidine-2-yl)-aminocarbonyl]-2-(5-méthyl-1H-tétrazole-1-yl)benzène-sulfonamide.

**22.** Un procédé selon la revendication 1, où le produit est le N-[(4,6-diméthoxypyrimidine-2-yl)-aminocarbonyl]-2-(1-méthyl-1H-tétrazole-5-yl)benzène-sulfonamide.

**23.** Un procédé selon la revendication 1, où le produit est le N-[(4,6-diméthoxypyrimidine-2-yl)-aminocarbonyl]-2-(1-méthyl-1H-tétrazole-5-yl)benzène-sulfonamide.

**24.** Un procédé selon la revendication 1, où le produit est le N-[(4,6-diméthylpyrimidine-2-yl)-aminocarbonyl]-2-(1-méthyl-1H-tétrazole-5-yl)benzène-sulfonamide.

**25.** Un procédé selon la revendication 1, où le produit est le N-[(4-méthoxy-6-méthylpyrimidine-2-yl)-aminocarbonyl]-2-(1-méthyl-1H-tétrazole-5-yl)benzène-sulfonamide.

**26.** Un procédé selon la revendication 1, où le produit est le N-[(4-chloro-6-méthoxypyrimidine-2-yl)-aminocarbonyl]-2-(1-mèthyl-1H-tétrazole-5-yl)benzène-sulfonamide.

**27.** Une composition convenant pour combattre la croissance de végétation indésirable, qui comprend une quantité efficace d'un composé de la formule (I) tel que défini à l'une quelconque des revendications 1 à 15 et au moins l'un des ingrédients suivants : agent tensio-actif, diluant solide ou liquide.

**28.** Une composition convenant pour combattre la croissance de végétation indésirable, qui comprend une quantité efficace d'un composé de la formule (I) tel que défini à l'une quelconque des revendications 16 à 26 et au moins l'un des ingrédients suivants : agent tensio-actif, diluant solide ou liquide.

**29.** Un procédé pour combattre la croissance de végétation indésirable, qui consiste à appliquer au site à protéger une quantité efficace d'un composé de formule (I) tel que défini à l'une quelconque des revendications 1 à 15.

**30.** Un procédé pour combattre la croissance de végétation indésirable, qui consiste à appliquer au site à protéger une quantité efficace d'un composé de formule (I) tel que défini à l'une quelconque des revendications 16 à 26.

**31.** Un procédé pour réguler la croissance de plantes, qui consiste à appliquer au site où se trouvent ces plantes une quantité efficace mais sensiblement non phytotoxique d'un régulateur de la croissance de plantes choisi parmi les composés de formule (I) tel que défini à l'une quelconque des revendications 1 à 26.

**Patentansprüche**

1. Verbindung der Formel

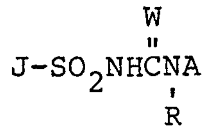

worin J

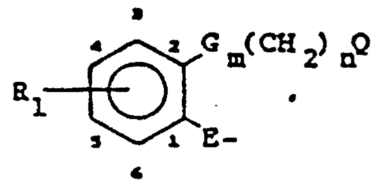

J-1

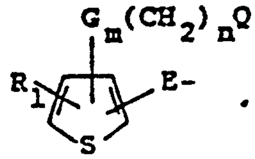

J-2

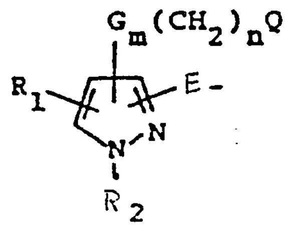

J-3

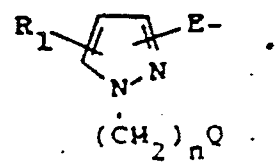

J-4

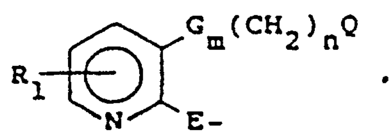

J-5

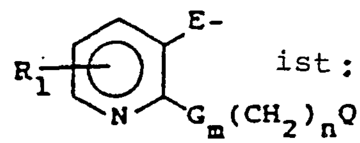

ist:

J-6

W O oder S ist;
G O, S, SO oder $SO_2$ ist;
m 0 oder 1 ist;
n 0, 1 oder 2 ist;
R H oder $CH_3$ ist;
E eine Einfachbindung, $CH_2$ oder O ist;

Q 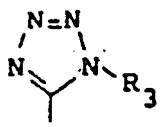 , 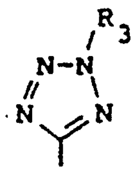 , 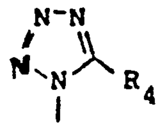 , 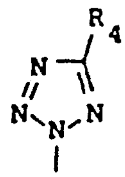 ,

Q-1 Q-2 Q-3 Q-4

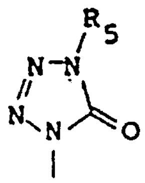 , 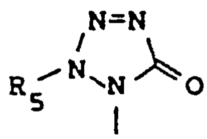 , 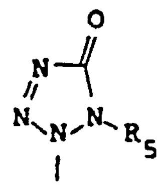 ist;

Q-5 Q-6 Q-7

$R_1$
H, $C_1$-$C_3$-Alkyl, $C_1$-$C_3$-Halogenalkyl, Halogen, Nitro, $C_1$-$C_3$-Alkoxy, $SO_2NR_aR_b$, $C_1$-$C_3$-Alkylthio, $C_1$-$C_3$-Alkylsulfinyl, $C_1$-$C_3$-Alkylsulfonyl, CN, $CO_2R_c$, $C_1$-$C_3$-Halogenalkoxy, $C_1$-$C_3$-Halogenalkylthio, Amino, $C_1$-$C_3$-Alkylamino, Di($C_1$-$C_3$-alkyl)-amino oder mit $C_1$-$C_2$-Alkoxy, $C_1$-$C_2$-Halogenalkoxy, $C_1$-$C_2$-Alkylthio, $C_1$-$C_2$-Halogenalkylthio oder CN substituiertes $C_1$-$C_2$-Alkyl ist;
$R_2$
H, $C_1$-$C_3$-Alkyl, Allyl oder Phenyl ist;
$R_3$
H, $C_1$-$C_6$-Alkyl, $C_3$-$C_6$-Cycloalkyl, $C_1$-$C_6$-Halogenalkyl, $CH_2$-($C_2$-$C_5$-Alkenyl), $CH_2$($C_2$-$C_5$-Halogenalkenyl), $CH_2$($C_2$-$C_5$-Alkinyl), $CH_2$($C_2$-$C_5$-Halogenalkinyl), $C_6H_5$ oder mit $C_1$-$C_2$-Alkoxy, $C_1$-$C_2$-Alkylthio, $C_1$-$C_2$-Alkylsulfinyl oder $C_1$-$C_2$-Alkylsulfonyl substituiertes $C_1$-$C_4$-Alkyl ist;
$R_4$
H, Halogen, $C_1$-$C_6$-Alkyl, $C_3$-$C_6$-Cycloalkyl, $C_1$-$C_6$-Halogenalkyl, $C_2$-$C_6$-Alkenyl, $C_2$-$C_6$-Halogenalkenyl, $C_2$-$C_6$-Alkinyl, $C_3$-$C_6$-Halogenalkinyl, $C_6H_5$, $C_1$-$C_4$-Alkoxy, $C_1$-$C_4$-Alkylthio, $C_1$-$C_4$-Alkylsulfinyl, $C_1$-$C_4$-Alkylsulfonyl, $OCH_2CH_2O$-($C_1$-$C_2$-Alkyl) oder Di-($C_1$-$C_3$-alkyl)amino ist;
$R_5$
H, $C_1$-$C_3$-Alkyl oder Allyl ist;
$R_a$
H, $C_1$-$C_4$-Alkyl, $C_2$-$C_3$-Cyanoalkyl, Methoxy oder Ethoxy ist;
$R_b$
H, $C_1$-$C_4$-Alkyl oder $C_3$-$C_4$-Alkenyl ist; oder
$R_a$
und $R_b$ als $-(CH_2)_3-$, $-(CH_2)_4-$, $-(CH_2)_5-$ oder $-CH_2CH_2OCH_2CH_2-$ zusammengenommen werden können;
$R_c$
$C_1$-$C_4$-Alkyl, $C_3$-$C_4$-Alkenyl, $C_3$-$C_4$-Alkinyl, $C_2$-$C_4$-Halogenalkyl, $C_1$-$C_2$-Cyanoalkyl, $C_5$-$C_6$-Cycloalkyl, $C_4$-$C_7$-Cycloalkylalkyl oder $C_2$-$C_4$-Alkoxyalkyl ist;

A 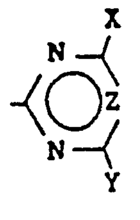 , 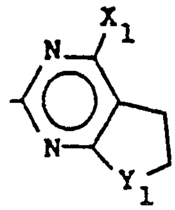 , 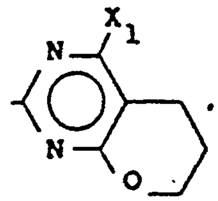 ,

A-1 A-2 A-3

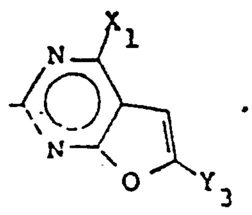 , 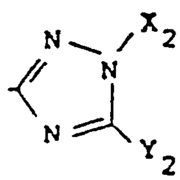 , 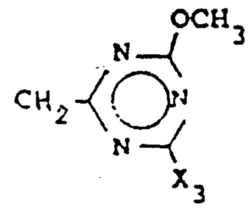 oder

A-4 A-5 A-6

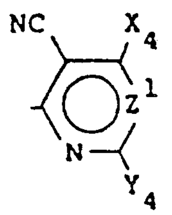 ; ist;

A-7

X

H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)-amino oder $C_3$-$C_5$-Cycloalkyl ist;

Y

H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$C_4$-Alkinyloxy, $C_2$-$C_5$-Alkylthioalkyl, $C_2$-$C_5$-Alkylsulfinylalkyl, $C_2$-$C_5$-Alkylsulfonylalkyl, $C_1$-$C_4$-Halogenalkyl, $C_2$-$C_4$-Alkinyl, Azido, Cyano,

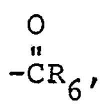 , 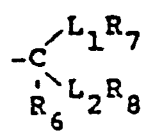 , 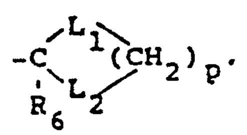 , 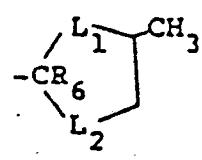

oder $-N(OCH_3)CH_3$ ist;

p 2 oder 3 ist;

$L_1$ und $L_2$ unabhängig O oder S sind;

$R_6$ H oder $CH_3$ ist;

$R_7$ und $R_8$ unabhängig $C_1$-$C_3$-Alkyl sind;

Z CH, N, $CCH_3$, $CC_2H_5$, CCl oder CBr ist;

$Y_1$ O oder $CH_2$ ist;

$X_1$ $CH_3$, $OCH_3$, $OC_2H_5$ oder $OCF_2H$ ist;

$X_2$ $CH_3$, $C_2H_5$ oder $CH_2CF_3$ ist;

$Y_2$ $OCH_3$, $OC_2H_5$, $SCH_3$, $SC_2H_5$, $CH_3$ oder $CH_2CH_3$ ist;
$X_3$ $CH_3$ oder $OCH_3$ ist;
$Y_3$ H oder $CH_3$ ist;
$X_4$ $CH_3$, $OCH_3$, $OC_2H_5$, $CH_2OCH_3$ oder Cl ist;
$Y_4$ $CH_3$, $OCH_3$, $OC_2H_5$ oder Cl ist; und
$Z^1$ CH oder N ist;
und ihre landwirtschaftlich geeigneten Salze, mit der Maßgabe, daß

a) wenn X Cl, F, Br oder I ist, dann Z CH ist und Y $OCH_3$, $OC_2H_5$, $N(OCH_3)CH_3$, $NHCH_3$, $N(CH_3)_2$ oder $OCF_2H$ ist;
b) wenn X oder Y $C_1$-Halogenalkoxy ist, dann Z CH ist;
c) wenn J J-2, J-3 oder J-4 ist, der Substituent $G_m(CH_2)_nQ$ oder $(CH_2)_nQ$ und die Sulfonylharnstoffbrücke an aneinandergrenzenden Ringpositionen sind;
d) wenn J J-4 ist und n O ist, dann Q Q-1 oder Q-2 ist;
e) wenn E O ist, dann J J-1 ist;
f) wenn W S ist, dann A A-1 ist, R H ist und Y $CH_3$, $OCH_3$, $OC_2H_5$, $CH_2OCH_3$, $C_2H_5$, $CF_3$, $SCH_3$, $OCH_2CH=CH_2$, $OCH_2C\equiv CH$,
$OCH_2CH_2OCH_3$, $CH(OCH_3)_2$ oder

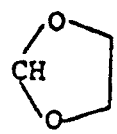

ist;
g) wenn die Gesamtzahl der Kohlenstoffatome in X und Y größer als 4 ist, dann der Kohlenstoffgehalt von $R_1$, $R_3$, $R_4$ und $R_5$ jeweils kleiner oder gleich 2 ist;
h) $X_4$ und $Y_4$ nicht gleichzeitig Cl sein können;
i) wenn J J-1 ist und m 0 ist, dann n 0 ist; und
j) wenn n 0 ist und m 1 ist, dann Q Q-1 oder Q-2 ist.

2. Verbindung nach Anspruch 1, worin E eine Einfachbindung ist.

3. Verbindung nach Anspruch 1, worin E $CH_2$ ist.

4. Verbindung nach Anspruch 1, worin E O ist,

5. Verbindung nach Anspruch 2, worin m 0 ist; W O ist; und R H ist.

6. Verbindung nach Anspruch 5, worin X $C_1$-$C_2$-Alkyl, $C_1$-$C_2$-Alkoxy, Cl, F, Br, I, $OCF_2H$, $CH_2F$, $CF_3$, Cyclopropyl, $OCH_2CH_2F$, $OCH_2CHF_2$, $OCH_2CF_3$, $CH_2Cl$ oder $CH_2Br$ ist und Y H, $C_1$-$C_2$-Alkyl, $C_1$-$C_2$-Alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C\equiv CH$, $OCH_2CH_2OCH_3$, $CH_2SCH_3$,

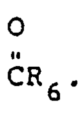, 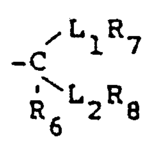, 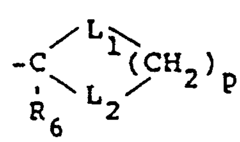, 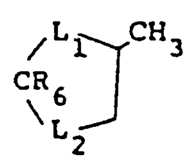,

$OCF_2H$, $SCF_2H$,
Cyclopropyl, $C\equiv CH$ oder $C\equiv CCH_3$ ist.

7. Verbindung nach Anspruch 6, worin $R_1$ H, $CH_3$, F, Cl, Br, $OCH_3$, $SCH_3$, $CH_2CN$, $CH_2OCH_3$, $CF_3$ oder $OCF_2H$ ist und n 0 ist.

8. Verbindung nach Anspruch 7, worin $R_3$ H, $C_1$-$C_3$-Alkyl, $CH_2CH_2Cl$, $CH_2CH_2F$, $CH_2CHF_2$, $CH_2CF_3$, Allyl oder Propargyl ist und $R_4$ H, $C_1$-$C_3$-Alkyl, $C_1$-$C_2$-Alkoxy, $C_1$-$C_2$-Alkylthio, $N(CH_3)_2$, Allyl, Propargyl oder

mit 1-3 Atomen aus F, Cl oder Br substituiertes $C_1$-$C_2$-Alkyl ist.

9. Verbindung nach Anspruch 8, worin A A-1 ist, Z CH oder N ist, X $CH_3$, $OCH_3$, $OCH_2CH_3$, Cyclopropyl, Cl oder $OCF_2H$ ist, Y $CH_3$, $OCH_3$, $C_2H_5$, $CH_2OCH_3$, $NHCH_3$ oder $CH(OCH_3)_2$ ist.

10. Verbindung nach Anspruch 9, worin J J-1 ist.

11. Verbindung nach Anspruch 10, worin $R_3$ und $R_4$ unabhängig H, $CH_3$ oder $C_2H_5$ sind.

12. Verbindung nach Anspruch 9, worin J J-2 bis J-6 ist und $R_1$ $R_1$ H ist.

13. Verbindung nach Anspruch 10, worin Q Q-1 bis Q-7 ist und $R_1$ in der 5- oder 6-Stellung ist.

14. Verbindung nach Anspruch 3, worin m 0 ist, n 0 ist, J J-1 ist, W O ist, R H ist, $R_1$ H ist, $R_3$ H oder $C_1$-$C_3$-Alkyl ist, $R_4$ H oder $C_1$-$C_3$-Alkyl ist; A A-1 ist, Z CH oder N ist, X $CH_3$, $OCH_3$, $OCH_2CH_3$, Cyclopropyl, Cl oder $OCF_2H$ ist und Y $CH_3$, $OCH_3$, $C_2H_5$, $CH_2OCH_3$, $NHCH_3$ oder $CH(OCH_3)_2$ ist.

15. Verbindung nach Anspruch 4, worin m 0 ist, R H ist, $R_1$ H ist, $R_3$ H oder $C_1$-$C_3$-Alkyl ist; $R_4$ H oder $C_1$-$C_3$-Alkyl ist, A A-1 ist, Z CH oder N ist; X $CH_3$, $OCH_3$, $OCH_2CH_3$, Cyclopropyl, Cl oder $OCF_2H$ ist und Y $CH_3$, $OCH_3$, $C_2H_5$, $CH_2OCH_3$, $NHCH_3$ oder $CH(OCH_3)_2$ ist.

16. Verbindung nach Anspruch 1, welche N-[(4-Chlor-6-methoxypyrimidin-2-yl)aminocarbonyl]-2-(1-ethyl-1H-tetrazol-5yl)-benzolsulfonamid ist.

17. Verbindung nach Anspruch 1, welche N-[(4,6-Dimethoxypyrimidin-2-yl)aminocarbonyl]-2-(1-ethyl-1H-tetrazol-5-yl)benzolsulfonamid ist.

18. Verbindung nach Anspruch 1, welche N-[(4,6-Dimethoxypyrimidin-2-yl)aminocarbonyl]-2-methyl-6-(1-methyl-1H-tetrazol-5-yl)benzolsulfonamid ist.

19. Verbindung nach Anspruch 1, welche N-[(4-Methoxy-6-methylpyrimidin-2-yl)aminocarbonyl]-2-methyl-6-(1-methyl-1H-tetrazol-5-yl)benzolsulfonamid ist.

20. Verbindung nach Anspruch 1, welche N-[(4,6-Dimethylpyrimidin-2-yl)aminocarbonyl]-2-methyl-6-(1-methyl-1H-tetrazol-5-yl)benzolsulfonamid ist.

21. Verbindung nach Anspruch 1, welche N-[(4,6-Dimethoxypyrimidin-2-yl)aminocarbonyl]-2-(5-methyl-1H-tetrazol-1-yl)benzolsulfonamid ist.

22. Verbindung nach Anspruch 1, welche N-[(4,6-Dimethoxypyrimidin-2-yl)aminocarbonyl]-2-(1-methyl-1H-tetrazol-5-yl)benzolsulfonamid ist.

23. Verbindung nach Anspruch 1, welche N-[(4,6-Dimethoxypyrimidin-2-yl)aminocarbonyl]-2-(1-methyl-1H-tetrazol-5-yl)benzolsulfonamid ist.

24. Verbindung nach Anspruch 1, welche N-[(4,6-Dimethylpyrimidin-2-yl)aminocarbonyl]-2-(1-methyl-1H-tetrazol-5-yl)benzolsulfonamid ist.

25. Verbindung nach Anspruch 1, welche N-[(4-Methoxy-6-methylpyrimidin-2-yl)aminocarbonyl]-2-(1-methyl-1H-tetrazol-5-yl)-benzolsulfonamid ist.

26. Verbindung nach Anspruch 1, welche N-[(4-Chlor-6-methoxypyrimidin-2-yl)aminocarbonyl]-2-(1-methyl-1H-tetrazol-5-yl)benzolsulfonamid ist.

27. Zur Bekämpfung des Wachstums unerwünschter Vegetation geeignete Zusammensetzung, welche eine wirksame Menge einer Verbindung nach Anspruch 1 bis 15 und wenigstens eines der folgenden umfaßt: oberflächenaktives Mittel, festes oder flüssiges Verdünnungsmittel.

**28.** Zur Bekämpfung des Wachstums unerwünschter Vegetation geeignete Zusammensetzung, welche eine wirksame Menge einer Verbindung nach einem der Ansprüche 16 bis 26 und wenigstens eines der folgenden umfaßt: oberflächenaktives Mittel, festes oder flüssiges Verdünnungsmittel.

**29.** Verfahren zur Bekämpfung des Wachstums unerwünschter Vegetation, welches die Anwendung einer wirksamen Menge einer Verbindung nach Anspruch 1 bis 15 auf den zu schützenden Ort umfaßt.

**30.** Verfahren zur Bekämpfung des Wachstums unerwünschter Vegetation, welches die Anwendung einer wirksamen Menge einer Verbindung nach einem der Ansprüche 16 bis 26 auf den zu schützenden Ort umfaßt.

**31.** Verfahren zur Steuerung des Wachstums von Pflanzen, welches die Anwendung einer wirksamen, jedoch im wesentlichen nicht phytotoxischen Menge eines Pflanzenwachstumsregulans, ausgewählt aus Verbindungen nach einem der Ansprüche 1 bis 26, auf den Ort solcher Pflanzen umfaßt.

**32.** Verfahren zur Herstellung einer Verbindung nach Anspruch 1 durch:
(a) Umsetzen eines Sulfonylisocyanats oder -isothiocyanats der Formel

$J\ SO_2NCW$ (II)

mit einem geeigneten heterocyclischen Amin der Formel

A-NHR (III)

oder
(b) Umsetzen eines Sulfonamids der Formel

$J\ SO_2NH_2$ (IV)

mit einem Phenylester der geeigneten Carbaminsäure oder Thiocarbaminsäure der Formel

$$PhO\ \overset{\overset{\displaystyle W}{\|}}{C}\ \underset{\underset{\displaystyle R}{|}}{N} - A$$

worin J, R, W und A wie in Anspruch 1 definiert sind.

**33.** Verbindungen der Formeln (II) und (IV), wie in Anspruch 32 definiert.

**Patentansprüche für folgenden Vertragsataat: AT**

**1.** Verfahren zur Herstellung einer Verbindung der Formel

$$J-SO_2NH\overset{\overset{\displaystyle W}{\|}}{C}\underset{\underset{\displaystyle R}{|}}{N}A$$

worin J

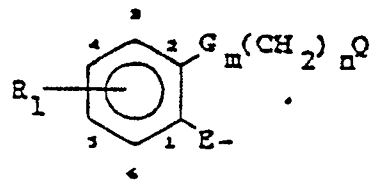,

J-1

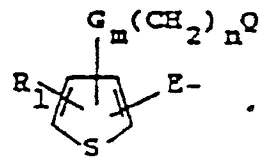,

J-2

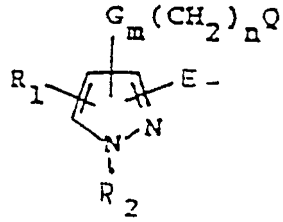,

J-3

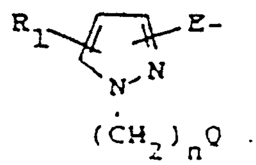,

J-4

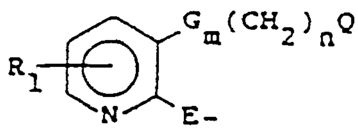,

J-5

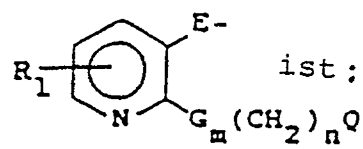

ist;

J-6

W O oder S ist;
G O, S, SO oder $SO_2$ ist;
m 0 oder 1 ist;
n 0, 1 oder 2 ist;
R H oder $CH_3$ ist;
E eine Einfachbindung, $CH_2$ oder O ist;

Q

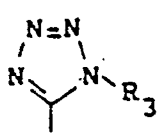,

Q-1

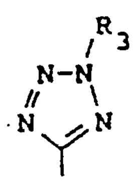,

Q-2

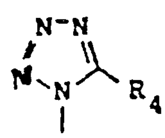,

Q-3

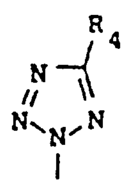,

Q-4

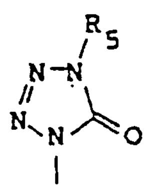,

Q-5

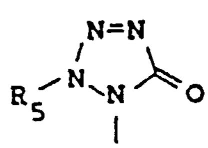,

Q-6

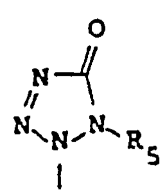

Q-7

ist;

$R_1$
H, $C_1$-$C_3$-Alkyl, $C_1$-$C_3$-Halogenalkyl, Halogen, Nitro, $C_1$-$C_3$-Alkoxy, $SO_2NR_aR_b$, $C_1$-$C_3$-Alkylthio, $C_1$-$C_3$-Alkylsulfinyl, $C_1$-$C_3$-Alkylsulfonyl, CN, $CO_2R_c$, $C_1$-$C_3$-Halogenalkoxy, $C_1$-$C_3$-Halogenalkylthio, Amino, $C_1$-$C_3$-Alkylamino, Di($C_1$-$C_3$-alkyl)-amino oder mit $C_1$-$C_2$-Alkoxy, $C_1$-$C_2$-Halogenalkoxy, $C_1$-$C_2$-Alkylthio, $C_1$-$C_2$-Halogenalkylthio oder CN substituiertes $C_1$-$C_2$-Alkyl ist;
$R_2$
H, $C_1$-$C_3$-Alkyl, Allyl oder Phenyl ist;
$R_3$
H, $C_1$-$C_6$-Alkyl, $C_3$-$C_6$-Cycloalkyl, $C_1$-$C_6$-Halogenalkyl, $CH_2$- ($C_2$-$C_5$-Alkenyl), $CH_2$($C_2$-$C_5$-Halogenalkenyl), $CH_2$($C_2$-$C_5$-Alkinyl), $CH_2$($C_2$-$c_5$-Halogenalkinyl), $C_6H_5$ oder mit $C_1$-$C_2$-Alkoxy, $C_1$-$C_2$-Alkylthio, $C_1$-$C_2$-Alkylsulfinyl oder $C_1$-$C_2$-Alkylsulfonyl substituiertes $C_1$-$C_4$-Alkyl ist;
$R_4$
H, Halogen, $C_1$-$C_6$-Alkyl, $C_3$-$C_6$-Cycloalkyl, $C_1$-$C_6$-Halogenalkyl, $C_2$-$C_6$ -Alkenyl, $C_2$-$C_6$-Halogenalkenyl, $C_2$-$C_6$-Alkinyl, $C_3$-$C_6$-Halogenalkinyl, $C_6H_5$, $C_1$-$C_4$-Alkoxy, $C_1$-$C_4$-Alkylthio, $C_1$-$C_4$-Alkylsulfinyl, $C_1$-$C_4$-Alkylsulfonyl, $OCH_2CH_2O$-($C_1$-$C_2$-Alkyl) oder Di-($C_1$-$C_3$-alkyl)amino ist;
$R_5$
H, $C_1$-$C_3$-Alkyl oder Allyl ist;
$R_a$
H, $C_1$-$C_4$-Alkyl, $C_2$-$C_3$-Cyanoalkyl, Methoxy oder Ethoxy ist;
$R_b$
H, $C_1$-$C_4$-Alkyl oder $C_3$-$C_4$-Alkenyl ist; oder
$R_a$ und $R_b$
als $-(CH_2)_3-$, $-(CH_2)_4-$, $-(CH_2)_5-$ oder $-CH_2CH_2OCH_2CH_2-$ zusammengenommen werden können;
$R_c$
$C_1$-$C_4$-Alkyl, $C_3$-$C_4$-Alkenyl, $C_3$-$C_4$-Alkinyl, $C_2$-$C_4$-Halogenalkyl, $C_1$-$C_2$-Cyanoalkyl, $C_5$-$C_6$-Cycloalkyl, $C_4$-$C_7$-Cycloalkylalkyl oder $C_2$-$C_4$-Alkoxyalkyl ist;

A 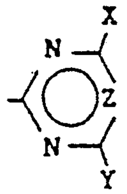 , 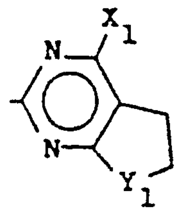 , 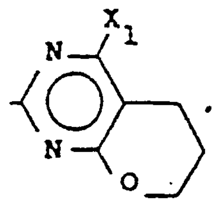 ,

A-1 A-2 A-3

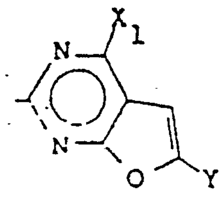 , 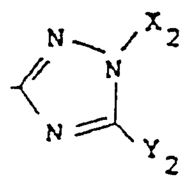 , 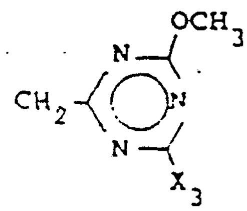 oder

A-4 A-5 A-6

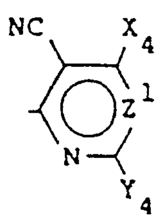 ; ist;

A-7

X
H, $C_1$-$C_4$-Alkyl, $C_1$-$C_4$-Alkoxy, $C_1$-$C_4$-Halogenalkoxy, $C_1$-$C_4$-Halogenalkyl, $C_1$-$C_4$-Halogenalkylthio, $C_1$-$C_4$-Alkylthio, Halogen, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)-amino oder $C_3$-$C_5$-Cycloalkyl ist;
Y
H, $C_1$-$C_4$-Alkyl, $C_1$-$C_4$-Alkoxy, $C_1$-$C_4$-Halogenalkoxy, $C_1$-$C_4$-Halogenalkylthio, $C_1$-$C_4$-Alkylthio, $C_2$-$C_5$-Alkoxyalkyl, $C_2$-$C_5$-Alkoxyalkoxy, Amino, $C_1$-$C_3$-Alkylamino, Di($C_1$-$C_3$-alkyl)amino, $C_3$-$C_4$-Alkenyloxy, $C_3$-$c_4$-Alkinyloxy, $C_2$-$C_5$-Alkylthioalkyl, $C_2$-$C_5$-Alkylsulfinylalkyl, $C_2$-$C_5$-Alkylsulfonylalkyl, $C_1$-$C_4$-Halogenalkyl, $C_2$-$C_4$-Alkinyl, Azido, Cyano,

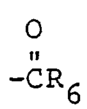 , 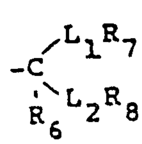 , 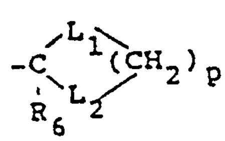 , 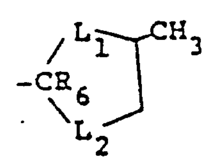

oder $-N(OCH_3)CH_3$ ist;
p 2 oder 3 ist;
$L_1$ und $L_2$ unabhängig O oder S sind;
$R_6$ H oder $CH_3$ ist;
$R_7$ und $R_8$ unabhängig $C_1$-$C_3$-Alkyl sind;
Z CH, N, $CCH_3$, $CC_2H_5$, CCl oder CBr ist;
$Y_1$ O oder $CH_2$ ist;
$X_1$ $CH_3$, $OCH_3$, $OC_2H_5$ oder $OCF_2H$ ist;

$X_2$ $CH_3$, $C_2H_5$ oder $CH_2CF_3$ ist;
$Y_2$ $OCH_3$, $OC_2H_5$, $SCH_3$, $SC_2H_5$, $CH_3$ oder $CH_2CH_3$ ist;
$X_3$ $CH_3$ oder $OCH_3$ ist;
$Y_3$ H oder $CH_3$ ist;
$x_4$ $CH_3$, $OCH_3$, $OC_2H_5$, $CH_2OCH_3$ oder Cl ist;
$Y_4$ $CH_3$, $OCH_3$, $OC_2H_5$ oder Cl ist; und
Z CH oder N ist;
und ihre landwirtschaftlich geeigneten Salze, mit der Maßgabe, daß

a) wenn X Cl, F, Br oder I ist, dann Z CH ist und Y $OCH_3$, $OC_2H_5$, $N(OCH_3)CH_3$, $NHCH_3$, $N(CH_3)_2$ oder $OCF_2H$ ist;
b) wenn X oder Y $C_1$-Halogenalkoxy ist, dann Z CH ist;
c) wenn J J-2, J-3 oder J-4 ist, der Substituent $G_m(CH_2)_nQ$ oder $(CH_2)_nQ$ und die Sulfonylharnstoffbrücke an aneinandergrenzenden Ringpositionen sind;
d) wenn J J-4 ist und n O ist, dann Q Q-1 oder Q-2 ist;
e) wenn E O ist, dann J J-1 ist;
f) wenn W S ist, dann A A-1 ist, R H ist und Y $CH_3$, $OCH_3$, $OC_2H_5$, $CH_2OCH_3$, $C_2H_5$, $CF_3$, $SCH_3$, $OCH_2CH=CH_2$, $OCH_2C{\equiv}CH$,
$OCH_2CH_2OCH_3$, $CH(OCH_3)_2$ oder

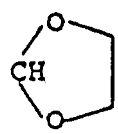

ist;
g) wenn die Gesamtzahl der Kohlenstoffatome in X und Y größer als 4 ist, dann der Kohlenstoffgehalt von $R_1$, $R_3$, $R_4$ und $R_5$ jeweils kleiner oder gleich 2 ist;
h) $X_4$ und $Y_4$ nicht gleichzeitig Cl sein können;
i) wenn J J-1 ist und m 0 ist, dann n 0 ist; und
j) wenn n 0 ist und m 1 ist, dann Q Q-1 oder Q-2 ist, durch
(a) Umsetzen eines Sulfonylisocyanats oder -isothiocyanats der Formel

J $SO_2NCW$ (II)

mit einem geeigneten heterocyclischen Amin der Formel

A-NHR (III)

oder
(b) Umsetzen eines Sulfonamids der Formel

J $SO_2NH_2$ (IV)

mit einem Phenylester der geeigneten Carbaminsäure oder Thiocarbaminsäure der Formel

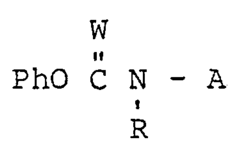

worin J, R, W und A wie oben definiert sind.

2. Verfahren nach Anspruch 1, worin E eine Einfachbindung ist.

3. Verfahren nach Anspruch 1, worin E $CH_2$ ist.

**4.** Verfahren nach Anspruch 1, worin E O ist.

**5.** Verfahren nach Anspruch 2, worin m 0 ist, W O ist und R H ist.

**6.** Verfahren nach Anspruch 5, worin X $C_1$-$C_2$-Alkyl, $C_1$-$C_2$-Alkoxy, Cl, F, Br, I, $OCF_2H$, $CH_2F$, $CF_3$, Cyclopropyl, $OCH_2CH_2F$, $OCH_2CHF_2$, $OCH_2CF_3$, $CH_2Cl$ oder $CH_2Br$ ist und Y H, $C_1$-$C_2$-Alkyl, $C_1$-$C_2$-Alkoxy, $CH_2OCH_3$, $CH_2OCH_2CH_3$, $NHCH_3$, $N(OCH_3)CH_3$, $N(CH_3)_2$, $CF_3$, $SCH_3$, $OCH_2CH=CH_2$, $OCH_2C \equiv CH$, $OCH_2CH_2OCH_3$, $CH_2SCH_3$,

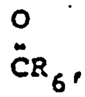, 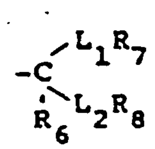, 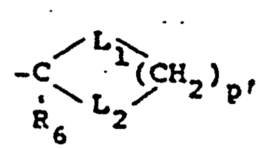, 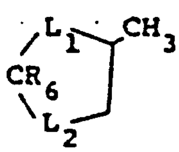, $OCF_2H$, $SCF_2H$,

Cyclopropyl, C CH oder C $CCH_3$ ist.

**7.** Verfahren nach Anspruch 6, worin $R_1$ H, $CH_3$, F, Cl, Br, $OCH_3$, $SVH_3$, $CH_2CN$, $CH_2OCH_3$, $CF_3$ oder $OCF_2H$ ist und n 0 ist.

**8.** Verfahren nach Anspruch 7, worin $R_3$ H, $C_1$-$C_3$-Alkyl, $CH_2CH_2Cl$, $CH_2CH_2F$, $CH_2CHF_2$, $CH_2CF_3$, Allyl oder Propargyl ist und $R_4$ H, $C_1$-$C_3$-Alkyl, $C_1$-$C_2$-Alkoxy, $C_1$-$C_2$-Alkylthio, $N(CH_3)_2$, Allyl, Propargyl oder mit 1-3 Atomen aus F, Cl oder Br substituiertes $C_1$-$C_2$-Alkyl ist.

**9.** Verfahren nach Anspruch 8, worin A A-1 ist, Z CH oder N ist, X $CH_3$, $OCH_3$, $OCH_2CH_3$, Cyclopropyl, Cl oder $OCF_2H$ ist, Y $CH_3$, $OCH_3$, $C_2H_5$, $CH_2OCH_3$, $NHCH_3$ oder $CH(OCH_3)_2$ ist.

**10.** Verfahren nach Anspruch 9, worin J J-1 ist.

**11.** Verfahren nach Anspruch 10, worin $R_3$ und $R_4$ unabhängig H, $CH_3$ oder $C_2H_5$ sind.

**12.** Verfahren nach Anspruch 9, worin J J-2 bis J-6 ist und $R_1$ H ist.

**13.** Verfahren nach Anspruch 10, worin Q Q-1 bis Q-7 ist und $R_1$ in der 5- oder 6-Stellung ist.

**14.** Verfahren nach Anspruch 3, worin m 0 ist, n 0 ist, J J-1 ist, W O ist, R H ist, $R_1$ H ist, $R_3$ H oder $C_1$-$C_3$-Alkyl ist, $R_4$ H oder $C_1$-$C_3$-Alkyl ist, A A-1 ist, Z CH oder N ist, X $CH_3$, $OCH_3$, $OCH_2CH_3$, Cyclopropyl, Cl oder $OCF_2H$ ist und Y $CH_3$, $OCH_3$, $C_2H_5$, $CH_2OCH_3$, $NHCH_3$ oder $CH(OCH_3)_2$ ist.

**15.** Verfahren nach Anspruch 4, worin m 0 ist, R H ist, $R_1$ H ist, $R_3$ H oder $C_1$-$C_3$-Alkyl ist, $R_4$ H oder $C_1$-$C_3$-Alkyl ist, A A-1 ist, Z CH oder N ist, X $CH_3$, $OCH_3$, $OCH_2CH_3$, Cyclopropyl, Cl oder $OCF_2H$ ist und Y $CH_3$, $OCH_3$, $C_2H_5$, $CH_2OCH_3$, $NHCH_3$ oder $CH(OCH_3)_2$ ist.

**16.** Verfahren nach Anspruch 1, worin das Produkt N-[(4-Chlor-6-methoxypyrimidin-2-yl)aminocarbonyl]-2-(1-ethyl-1H-tetrazol-5-yl)benzolsulfonamid oder ein landwirtschaftlich geeignetes Salz davon ist.

**17.** Verfahren nach Anspruch 1, worin das Produkt N-[(4,6-Dimethoxypyrimidin-2-yl)aminocarbonyl]-2-(1-ethyl-1H-tetrazol-5-yl)benzolsulfonamid oder ein landwirtschaftlich geeignetes Salz davon ist.

**18.** Verfahren nach Anspruch 1, worin das Produkt N-[(4,6-Dimethoxypyrimidin-2-yl)aminocarbonyl]-2-methyl-6-(1-methyl-1H-tetrazol-5-yl)benzolsulfonamid oder ein landwirtschaftlich geeignetes Salz davon ist.

**19.** Verfahren nach Anspruch 1, worin das Produkt N-[(4-Methoxy-6-methylpyrimidin-2-yl)aminocarbonyl]-2-methyl-6-(1-methyl-1H-tetrazol-5-yl)benzolsulfonamid oder ein landwirtschaftlich geeignetes Salz davon ist.

**20.** Verfahren nach Anspruch 1, worin das Produkt N-[(4,6-Dimethylpyrimidin-2-yl)aminocarbonyl]-2-methyl-6-(1-methyl-1H-tetrazol-5-yl)benzolsulfonamid oder ein landwirtschaftlich geeignetes Salz davon ist.

**21.** Verfahren nach Anspruch 1, worin das Produkt N-[(4,6-Dimethoxypyrimidin-2-yl)aminocarbonyl]-2-(5-methyl-1H-tetrazol-1-yl)benzolsulfonamid oder ein landwirtschaftlich geeignetes Salz davon ist.

**22.** Verfahren nach Anspruch 1, worin das Produkt N-[(4,6-Dimethoxypyrimidin-2-yl)aminocarbonyl]-2-(1-methyl-1H-tetrazol-5-yl)benzolsulfonamid oder ein landwirtschaftlich geeignetes Salz davon ist.

**23.** Verfahren nach Anspruch 1, worin das Produkt N-[(4,6-Dimethoxypyrimidin-2-yl)aminocarbonyl]-2-(1-methyl-1H-tetrazol-5-yl)benzolsulfonamid oder ein landwirtschaftlich geeignetes Salz davon ist.

**24.** Verfahren nach Anspruch 1, worin das Produkt N-[(4,6-Dimethylpyrimidin-2-yl)aminocarbonyl]-2-(1-methyl-1H-tetrazol-5-yl)benzolsulfonamid oder ein landwirtschaftlich geeignetes Salz davon ist.

**25.** Verfahren nach Anspruch 1, worin das Produkt N-[(4-Methoxy-6-methylpyrimidin-2-yl)aminocarbonyl]-2-(1-methyl-1H-tetrazol-5-yl)benzolsulfonamid oder ein landwirtschaftlich geeignetes Salz davon ist.

**26.** Verfahren nach Anspruch 1, worin das Produkt N-[(4-Chlor-6-methoxypyrimidin-2-yl)aminocarbonyl]-2-(1-methyl-1H-tetrazol-5-yl)benzolsulfonamid oder ein landwirtschaftlich geeignetes Salz davon ist.

**27.** Zur Bekämpfung des Wachstums unerwünschter Vegetation geeignete Zusammensetzung, welche eine wirksame Menge einer Verbindung der Formel (I), wie in einem der Ansprüche 1 bis 15 definiert, und wenigstens eines der folgenden umfaßt: oberflächenaktives Mittel, festes oder flüssiges Verdünnungsmittel.

**28.** Zur Bekämpfung des Wachstums unerwünschter Vegetation geeignete Zusammensetzung, welche eine wirksame Menge einer Verbindung der Formel (I), wie in einem der Ansprüche 16 bis 26 definiert, und wenigstens eines der folgenden umfaßt: oberflächenaktives Mittel, festes oder flüssiges Verdünnungsmittel.

**29.** Verfahren zur Bekämpfung des Wachstums unerwünschter Vegetation, welches die Anwendung einer wirksamen Menge einer Verbindung der Formel (I), wie in einem der Ansprüche 1 bis 15 definiert, auf den zu schützenden Ort umfaßt.

**30.** Verfahren zur Bekämpfung des Wachstums unerwünschter Vegetation, welches die Anwendung einer wirksamen Menge einer Verbindung der Formel (I), wie in einem der Ansprüche 16 bis 26 definiert, auf den Ort solcher Pflanzen umfaßt.

**31.** Verfahren zur Steuerung des Wachstums von Pflanzen, welches die Anwendung einer wirksamen, jedoch im wesentlichen nicht phytotoxischen Menge eines Pflanzenwachstumsregulans, ausgewählt aus Verbindungen der Formel (I), wie in einem der Ansprüche 1 bis 26 definiert, auf den Ort solcher Pflanzen umfaßt.